# EUROPEAN PATENT APPLICATION

(11) **EP 2 514 839 A1**
(43) Date of publication of application: **24.10.2012**
(21) Application number: 12172160.9
(22) Date of filing: 18.07.2008
(51) Int. Cl.: C12Q 1/68

(54) **Methods for quantitating small RNA molecules**

(30) Priority: 18.07.2007 US 779759
(62) Divisional of application: 08782037.9
(71) Applicant: Merck Sharp & Dohme Corporation, Rahway, NJ 07065 (US)
(72) Inventor: Raymond, Christopher K., Seattle WA98115 (US)
(74) Representative: Brady, Paul Andrew

(57) **Abstract**

In one aspect, the present invention provides methods, compositions and kits for amplifying a microRNA molecule to produce DNA molecules. The methods each include the steps of: (a) using primer extension to make a DNA molecule that is complementary to a target microRNA molecule; and (b) using a universal forward primer and a reverse primer to amplify the DNA molecule to produce amplified DNA molecules. In some embodiments of the method, at least one of the forward primer and the reverse primer comprise at least one locked nucleic acid molecule.

SEQUENCE LISTING

<110>  Merck Sharp & Dohme Corp
       Raymond, Christopher K.

<120>  METHODS FOR QUANTITATING SMALL RNA MOLECULES

<130>  14813 EP1 / PAB

<150>  US 11/779,759
<151>  2007-07-18

<150>  US 60/647,178
<151>  2005-01-25

<150>  PCT/US2006/002591
<151>  2006-01-25

<150>  US 10/579,029
<151>  2006-05-11

<160>  1043

<170>  PatentIn version 3.2

<210>  1
<211>  20
<212>  DNA
<213>  Artificial Sequence

<220>
<223>  Primer

<400>  1
catgatcagc tgggccaaga                                                  20


<210>  2
<211>  33
<212>  DNA
<213>  Artificial Sequence

<220>
<223>  Primer

<400>  2
catgatcagc tgggccaaga aactatacaa cct                                   33


<210>  3
<211>  31
<212>  DNA
<213>  Artificial Sequence

<220>
<223>  Primer

<400>  3
catgatcagc tgggccaaga tacatacttc t                                     31


<210>  4
<211>  34
<212>  DNA
<213>  Artificial Sequence

<220>
<223>  Primer

<400>  4
catgatcagc tgggccaaga cacaaaccat tatg                                    34


<210>  5
<211>  35
<212>  DNA
<213>  Artificial Sequence

<220>
<223>  Primer

<400>  5
catgatcagc tgggccaaga cgccaatatt tacgt                                   35


<210>  6
<211>  31
<212>  DNA
<213>  Artificial Sequence

<220>
<223>  Primer

<400>  6
catgatcagc tgggccaaga tcaacatcag t                                       31


<210>  7
<211>  32
<212>  DNA
<213>  Artificial Sequence

<220>
<223>  Primer

<400>  7
catgatcagc tgggccaaga ctgttcctgc tg                                      32


<210>  8
<211>  36
<212>  DNA
<213>  Artificial Sequence

<220>
<223>  Primer

<400>  8
catgatcagc tgggccaaga acaaacacca ttgtca                                  36


<210>  9
<211>  36
<212>  DNA
<213>  Artificial Sequence

<220>
<223>  Primer

<400>  9

catgatcagc tgggccaaga tggcattcac cgcgtg                                36


<210>  10
<211>  32
<212>  DNA
<213>  Artificial Sequence

<220>
<223>  Primer

<400>  10
catgatcagc tgggccaaga tgagctacag tg                                   32


<210>  11
<211>  36
<212>  DNA
<213>  Artificial Sequence

<220>
<223>  Primer

<400>  11
catgatcagc tgggccaaga aagggattcc tgggaa                                36


<210>  12
<211>  33
<212>  DNA
<213>  Artificial Sequence

<220>
<223>  Primer

<400>  12
catgatcagc tgggccaaga cccctatcac gat                                  33


<210>  13
<211>  20
<212>  DNA
<213>  Artificial Sequence

<220>
<223>  Primer

<400>  13
catgatcagc tgggccaaga                                                 20


<210>  14
<211>  15
<212>  DNA
<213>  Artificial Sequence

<220>
<223>  Primer


<220>
<221>  misc_feature
<222>  (3)..(7)
<223>  Nucleotide at position 3 and/or 7 may be modified by
       2'-O,4'-C-methylene-beta-D-ribofuranosyl moiety

<400>  14
tgaggtagta ggttg                                                        15


<210>  15
<211>  17
<212>  DNA
<213>  Artificial Sequence

<220>
<223>  Primer



<220>
<221>  misc_feature
<222>  (3)..(8)
<223>  Nucleotide at position 3 and/or 6 and/or 8 may be modified by
       2'-O,4'-C-methylene-beta-D-ribofuranosyl moiety

<400>  15
tggaatgtaa agaagta                                                      17


<210>  16
<211>  15
<212>  DNA
<213>  Artificial Sequence

<220>
<223>  Primer



<220>
<221>  misc_feature
<222>  (4)..(7)
<223>  Nucleotide at position 4 and/or 7 may be modified by
       2'-O,4'-C-methylene-beta-D-ribofuranosyl moiety

<400>  16
tagcagcaca taatg                                                        15


<210>  17
<211>  14
<212>  DNA
<213>  Artificial Sequence

<220>
<223>  Primer



<220>
<221>  misc_feature
<222>  (2)..(5)
<223>  Nucleotide at position 2 and/or 5 may be modified by
       2'-O,4'-C-methylene-beta-D-ribofuranosyl moiety

<400>  17
tagcagcacg taaa                                                         14


<210>  18
<211>  17
<212>  DNA

<213>  Artificial Sequence

<220>
<223>  Primer



<220>
<221>  misc_feature
<222>  (2)..(6)
<223>  Nucleotide at position 2 and/or 4 and/or 6 may be modified by
       2'-O,4'-C-methylene-beta-D-ribofuranosyl moiety

<400>  18
tagcttatca gactgat                                                    17


<210>  19
<211>  14
<212>  DNA
<213>  Artificial Sequence

<220>
<223>  Primer



<220>
<221>  misc_feature
<222>  (4)..(4)
<223>  Nucleotide at position 4 may be modified by
       2'-O,4'-C-methylene-beta-D-ribofuranosyl moiety

<400>  19
tggctcagtt cagc                                                       14


<210>  20
<211>  13
<212>  DNA
<213>  Artificial Sequence

<220>
<223>  Primer



<220>
<221>  misc_feature
<222>  (2)..(6)
<223>  Nucleotide at position 2 and/or 3 and/or 6 may be modified by
       2'-O,4'-C-methylene-beta-D-ribofuranosyl moiety

<400>  20
tggagtgtga caa                                                        13


<210>  21
<211>  12
<212>  DNA
<213>  Artificial Sequence

<220>
<223>  Primer



<220>
<221>  misc_feature

<222>  (2)..(5)
<223>  Nucleotide at position 2 and/or 5 may be modified by
       2'-O,4'-C-methylene-beta-D-ribofuranosyl moiety

<400>  21
ttaaggcacg cg                                                          12


<210>  22
<211>  15
<212>  DNA
<213>  Artificial Sequence

<220>
<223>  Primer


<220>
<221>  misc_feature
<222>  (3)..(6)
<223>  Nucleotide at position 3 and/or 6 may be modified by
       2'-O,4'-C-methylene-beta-D-ribofuranosyl moiety

<400>  22
tgagatgaag cactg                                                       15


<210>  23
<211>  14
<212>  DNA
<213>  Artificial Sequence

<220>
<223>  Primer


<220>
<221>  misc_feature
<222>  (3)..(3)
<223>  Nucleotide at position 3 may be modified by
       2'-O,4'-C-methylene-beta-D-ribofuranosyl moiety

<400>  23
gtccagtttt ccca                                                        14


<210>  24
<211>  16
<212>  DNA
<213>  Artificial Sequence

<220>
<223>  Primer


<220>
<221>  misc_feature
<222>  (2)..(7)
<223>  Nucleotide at position 2 and/or 5 and/or 7 may be modified by
       2'-O,4'-C-methylene-beta-D-ribofuranosyl moiety

<400>  24
ttaatgctaa tcgtga                                                      16

<210>  25
<211>  32
<212>  DNA
<213>  Artificial Sequence

<220>
<223>  Primer

<400>  25
catgatcagc tgggccaaga gccaatattt ct                                    32


<210>  26
<211>  31
<212>  DNA
<213>  Artificial Sequence

<220>
<223>  Primer

<400>  26
catgatcagc tgggccaaga gccaatattt c                                     31


<210>  27
<211>  30
<212>  DNA
<213>  Artificial Sequence

<220>
<223>  Primer

<400>  27
catgatcagc tgggccaaga gccaatattt                                       30


<210>  28
<211>  29
<212>  DNA
<213>  Artificial Sequence

<220>
<223>  Primer

<400>  28
catgatcagc tgggccaaga gccaatatt                                        29


<210>  29
<211>  28
<212>  DNA
<213>  Artificial Sequence

<220>
<223>  Primer

<400>  29
catgatcagc tgggccaaga gccaatat                                         28


<210>  30
<211>  27
<212>  DNA
<213>  Artificial Sequence

<220>
<223>  Primer

<400>  30
catgatcagc tgggccaaga gccaata                                          27


<210>  31
<211>  26
<212>  DNA
<213>  Artificial Sequence

<220>
<223>  Primer

<400>  31
catgatcagc tgggccaaga gccaat                                           26


<210>  32
<211>  25
<212>  DNA
<213>  Artificial Sequence

<220>
<223>  Primer

<400>  32
catgatcagc tgggccaaga gccaa                                            25


<210>  33
<211>  24
<212>  DNA
<213>  Artificial Sequence

<220>
<223>  Primer

<400>  33
catgatcagc tgggccaaga gcca                                             24


<210>  34
<211>  23
<212>  DNA
<213>  Artificial Sequence

<220>
<223>  Primer

<400>  34
catgatcagc tgggccaaga gcc                                              23


<210>  35
<211>  32
<212>  DNA
<213>  Artificial Sequence

<220>
<223>  Primer

<400>  35
catgatcagc tgggccaaga gtctgtcaat tc                                    32

<210>  36
<211>  31
<212>  DNA
<213>  Artificial Sequence

<220>
<223>  Primer

<400>  36
catgatcagc tgggccaaga gtctgtcaat t                                    31


<210>  37
<211>  30
<212>  DNA
<213>  Artificial Sequence

<220>
<223>  Primer

<400>  37
catgatcagc tgggccaaga gtctgtcaat                                      30


<210>  38
<211>  29
<212>  DNA
<213>  Artificial Sequence

<220>
<223>  Primer

<400>  38
catgatcagc tgggccaaga gtctgtcaa                                       29


<210>  39
<211>  28
<212>  DNA
<213>  Artificial Sequence

<220>
<223>  Primer

<400>  39
catgatcagc tgggccaaga gtctgtca                                        28


<210>  40
<211>  27
<212>  DNA
<213>  Artificial Sequence

<220>
<223>  Primer

<400>  40
catgatcagc tgggccaaga gtctgtc                                         27


<210>  41
<211>  26
<212>  DNA

<213>  Artificial Sequence

<220>
<223>  Primer

<400>  41
catgatcagc tgggccaaga gtctgt                                          26


<210>  42
<211>  25
<212>  DNA
<213>  Artificial Sequence

<220>
<223>  Primer

<400>  42
catgatcagc tgggccaaga gtctg                                           25


<210>  43
<211>  24
<212>  DNA
<213>  Artificial Sequence

<220>
<223>  Primer

<400>  43
catgatcagc tgggccaaga gtct                                            24


<210>  44
<211>  23
<212>  DNA
<213>  Artificial Sequence

<220>
<223>  Primer

<400>  44
catgatcagc tgggccaaga gtc                                             23


<210>  45
<211>  15
<212>  DNA
<213>  Artificial Sequence

<220>
<223>  Primer


<220>
<221>  misc_feature
<222>  (2)..(5)
<223>  Nucleotide at position 2 and/or 5 may be modified by
       2'-O,4'-C-methylene-beta-D-ribofuranosyl moiety

<400>  45
tagcagcaca gaaat                                                      15


<210>  46

<211>  15
<212>  DNA
<213>  Artificial Sequence

<220>
<223>  Primer


<220>
<221>  misc_feature
<222>  (2)..(5)
<223>  Nucleotide at position 2 and/or 3 and/or 5 may be modified by
       2'-O,4'-C-methylene-beta-D-ribofuranosyl moiety

<400>  46
atgacctatg aattg                                                    15


<210>  47
<211>  30
<212>  DNA
<213>  Artificial Sequence

<220>
<223>  Primer

<400>  47
catgatcagc tgggccaaga tacatacttc                                    30


<210>  48
<211>  16
<212>  DNA
<213>  Artificial Sequence

<220>
<223>  Primer


<220>
<221>  misc_feature
<222>  (2)..(8)
<223>  Nucleotide at position 2 and/or 3 and/or 6 and/or 8 may be
       modified by 2'-O,4'-C-methylene-beta-D-ribofuranosyl moiety

<400>  48
tggaatgtaa agaagt                                                   16


<210>  49
<211>  30
<212>  DNA
<213>  Artificial Sequence

<220>
<223>  Primer

<400>  49
catgatcagc tgggccaaga caacaaaatc                                    30


<210>  50
<211>  18
<212>  DNA
<213>  Artificial Sequence

<220>
<223>  Primer


<220>
<221>  misc_feature
<222>  (2)..(6)
<223>  Nucleotide at position 2 and/or 6 may be modified by
       2'-O,4'-C-methylene-beta-D-ribofuranosyl moiety

<400>  50
tggaagacta gtgatttt                                                    18


<210>  51
<211>  30
<212>  DNA
<213>  Artificial Sequence

<220>
<223>  Primer

<400>  51
catgatcagc tgggccaaga actttcggtt                                       30


<210>  52
<211>  16
<212>  DNA
<213>  Artificial Sequence

<220>
<223>  Primer


<220>
<221>  misc_feature
<222>  (5)..(8)
<223>  Nucleotide at position 5 and/or 8 may be modified by
       2'-O,4'-C-methylene-beta-D-ribofuranosyl moiety

<400>  52
taaagctaga taaccg                                                      16


<210>  53
<211>  30
<212>  DNA
<213>  Artificial Sequence

<220>
<223>  Primer

<400>  53
catgatcagc tgggccaaga cacaaattcg                                       30


<210>  54
<211>  15
<212>  DNA
<213>  Artificial Sequence

<220>
<223>  Primer

<220>
<221>  misc_feature
<222>  (2)..(4)
<223>  Nucleotide at position 2 and/or 4 may be modified by
      2'-O,4'-C-methylene-beta-D-ribofuranosyl moiety

<400>  54
taccctgtag atccg                                                    15


<210>  55
<211>  31
<212>  DNA
<213>  Artificial Sequence

<220>
<223>  Primer

<400>  55
catgatcagc tgggccaaga acaaattcgg t                                  31


<210>  56
<211>  16
<212>  DNA
<213>  Artificial Sequence

<220>
<223>  Primer


<220>
<221>  misc_feature
<222>  (3)..(9)
<223>  Nucleotide at position 3 and/or 6 and/or 9 may be modified by
      2'-O,4'-C-methylene-beta-D-ribofuranosyl moiety

<400>  56
taccctgtag aaccga                                                   16


<210>  57
<211>  30
<212>  DNA
<213>  Artificial Sequence

<220>
<223>  Primer

<400>  57
catgatcagc tgggccaaga cacaaaccat                                    30


<210>  58
<211>  15
<212>  DNA
<213>  Artificial Sequence

<220>
<223>  Primer


<220>

<221>  misc_feature
<222>  (2)..(4)
<223>  Nucleotide at position 2 and/or 4 may be modified by
       2'-O,4'-C-methylene-beta-D-ribofuranosyl moiety

<400>  58
tagcagcaca taatg                                                    15


<210>  59
<211>  29
<212>  DNA
<213>  Artificial Sequence

<220>
<223>  Primer

<400>  59
catgatcagc tgggccaaga tgtaaacca                                     29


<210>  60
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  60
tagcagcaca tcat                                                     14


<210>  61
<211>  29
<212>  DNA
<213>  Artificial Sequence

<220>
<223>  Primer

<400>  61
catgatcagc tgggccaaga cgccaatat                                     29


<210>  62
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by

2'-O,4'-C-methylene-beta-D-ribofuranosyl moiety

<400>  62
tagcagcacg taaa                                                          14


<210>  63
<211>  30
<212>  DNA
<213>  Artificial Sequence

<220>
<223>  Primer

<400>  63
catgatcagc tgggccaaga acaagtgcct                                         30


<210>  64
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  64
actgcagtga aggc                                                          14


<210>  65
<211>  29
<212>  DNA
<213>  Artificial Sequence

<220>
<223>  Primer

<400>  65
catgatcagc tgggccaaga actacctgc                                          29


<210>  66
<211>  16
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  66

caaagtgctt acagtg                                                    16

<210>  67
<211>  29
<212>  DNA
<213>  Artificial Sequence

<220>
<223>  Primer

<400>  67
catgatcagc tgggccaaga tcagttttg                                       29

<210>  68
<211>  15
<212>  DNA
<213>  Artificial Sequence

<220>
<223>  Primer

<220>
<221>  misc_feature
<222>  (3)..(5)
<223>  Nucleotide at position 3 and/or 5 may be modified by
       2'-O,4'-C-methylene-beta-D-ribofuranosyl moiety

<400>  68
tgtgcaaatc tatgc                                                      15

<210>  69
<211>  30
<212>  DNA
<213>  Artificial Sequence

<220>
<223>  Primer

<400>  69
catgatcagc tgggccaaga tcagttttgc                                      30

<210>  70
<211>  14
<212>  DNA
<213>  Artificial Sequence

<220>
<223>  Primer

<220>
<221>  misc_feature
<222>  (3)..(5)
<223>  Nucleotide at position 3 and/or 5 may be modified by
       2'-O,4'-C-methylene-beta-D-ribofuranosyl moiety

<400>  70
tgtgcaaatc catg                                                       14

<210>  71
<211>  28
<212>  DNA
<213>  Artificial Sequence

<220>
<223>  Primer

<400>  71
catgatcagc tgggccaaga ctacctgc                                          28


<210>  72
<211>  18
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  72
taaagtgctt atagtgca                                                     18


<210>  73
<211>  29
<212>  DNA
<213>  Artificial Sequence

<220>
<223>  Primer

<400>  73
catgatcagc tgggccaaga tcaacatca                                         29


<210>  74
<211>  18
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  74
tagcttatca gactgatg                                                     18


<210>  75
<211>  30
<212>  DNA

<213>  Artificial Sequence

<220>
<223>  Primer

<400>  75
catgatcagc tgggccaaga ggaaatccct                                        30


<210>  76
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  76
atcacattgc cagg                                                         14


<210>  77
<211>  30
<212>  DNA
<213>  Artificial Sequence

<220>
<223>  Primer

<400>  77
catgatcagc tgggccaaga ggtaatccct                                        30


<210>  78
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  78
atcacattgc cagg                                                         14


<210>  79
<211>  30
<212>  DNA
<213>  Artificial Sequence

<220>

<223>  Primer

<400>  79
catgatcagc tgggccaaga tcagaccgag                                        30


<210>  80
<211>  15
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  80
cattgcactt gtctc                                                        15


<210>  81
<211>  29
<212>  DNA
<213>  Artificial Sequence

<220>
<223>  Primer

<400>  81
catgatcagc tgggccaaga gcctatcct                                         29


<210>  82
<211>  17
<212>  DNA
<213>  Artificial Sequence

<220>
<223>  Primer


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  82
ttcaagtaat ccaggat                                                      17


<210>  83
<211>  29
<212>  DNA
<213>  Artificial Sequence

<220>
<223>  Primer

<400>  83

catgatcagc tgggccaaga aacctatcc                                          29

<210>  84
<211>  17
<212>  DNA
<213>  Artificial Sequence

<220>
<223>  Primer

<220>
<221>  misc_feature
<222>  (3)..(8)
<223>  Nucleotide at position 3 and/or 5 and/or 8 may be modified by
       2'-O,4'-C-methylene-beta-D-ribofuranosyl moiety

<400>  84
ttcaagtaat tcaggat                                                    17

<210>  85
<211>  30
<212>  DNA
<213>  Artificial Sequence

<220>
<223>  Primer

<400>  85
catgatcagc tgggccaaga gcggaactta                                      30

<210>  86
<211>  14
<212>  DNA
<213>  Artificial Sequence

<220>
<223>  Primer

<220>
<221>  misc_feature
<222>  (3)..(5)
<223>  Nucleotide at position 3 and/or 5 may be modified by
       2'-O,4'-C-methylene-beta-D-ribofuranosyl moiety

<400>  86
ttcacagtgg ctaa                                                       14

<210>  87
<211>  30
<212>  DNA
<213>  Artificial Sequence

<220>
<223>  Primer

<400>  87
catgatcagc tgggccaaga gcagaactta                                      30

<210>  88
<211>  14
<212>  DNA
<213>  Artificial Sequence

<220>
<223>  Primer

<220>
<221>  misc_feature
<222>  (3)..(5)
<223>  Nucleotide at position 3 and/or 5 may be modified by
       2'-O,4'-C-methylene-beta-D-ribofuranosyl moiety

<400>  88
ttcacagtgg ctaa                                                    14

<210>  89
<211>  30
<212>  DNA
<213>  Artificial Sequence

<220>
<223>  Primer

<400>  89
catgatcagc tgggccaaga ctcaatagac                                   30

<210>  90
<211>  14
<212>  DNA
<213>  Artificial Sequence

<220>
<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  90
aaggagctca cagt                                                    14

<210>  91
<211>  28
<212>  DNA
<213>  Artificial Sequence

<220>
<223>  Primer

<400>  91
catgatcagc tgggccaaga aaccgatt                                     28

<210>  92
<211>  16
<212>  DNA

<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  92
tagcaccatc tgaaat                                                    16


<210>  93
<211>  29
<212>  DNA
<213>  Artificial Sequence

<220>
<223>  Primer

<400>  93
catgatcagc tgggccaaga aacactgat                                      29


<210>  94
<211>  19
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  94
tagcaccatt tgaaatcag                                                 19


<210>  95
<211>  30
<212>  DNA
<213>  Artificial Sequence

<220>
<223>  Primer

<400>  95
catgatcagc tgggccaaga cttccagtcg                                     30


<210>  96
<211>  16
<212>  DNA
<213>  Artificial Sequence

<220>

<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  96
tgtaaacatc ctcgac                                                    16

<210>  97
<211>  30
<212>  DNA
<213>  Artificial Sequence

<220>
<223>  Primer

<400>  97
catgatcagc tgggccaaga agctgagtgt                                     30

<210>  98
<211>  17
<212>  DNA
<213>  Artificial Sequence

<220>
<223>  Primer

<220>
<221>  misc_feature
<222>  (4)..(7)
<223>  Nucleotide at position 4 and/or 7 may be modified by
       2'-O,4'-C-methylene-beta-D-ribofuranosyl moiety

<400>  98
tgtaaacatc ctacact                                                   17

<210>  99
<211>  30
<212>  DNA
<213>  Artificial Sequence

<220>
<223>  Primer

<400>  99
catgatcagc tgggccaaga gctgagagtg                                     30

<210>  100
<211>  17
<212>  DNA
<213>  Artificial Sequence

<220>
<223>  Primer

<220>
<221>  misc_feature
<222>  (4)..(7)
<223>  Nucleotide at position 4 and/or 7 may be modified by
      2'-O,4'-C-methylene-beta-D-ribofuranosyl moiety

<400>  100
tgtaaacatc ctacact                                                    17


<210>  101
<211>  30
<212>  DNA
<213>  Artificial Sequence

<220>
<223>  Primer

<400>  101
catgatcagc tgggccaaga cttccagtcg                                      30


<210>  102
<211>  14
<212>  DNA
<213>  Artificial Sequence

<220>
<223>  Primer


<220>
<221>  misc_feature
<222>  (2)..(7)
<223>  Nucleotide at position 2 and/or 7 may be modified by
      2'-O,4'-C-methylene-beta-D-ribofuranosyl moiety

<400>  102
tgtaaacatc cccg                                                       14


<210>  103
<211>  29
<212>  DNA
<213>  Artificial Sequence

<220>
<223>  Primer

<400>  103
catgatcagc tgggccaaga gctgtaaac                                       29


<210>  104
<211>  17
<212>  DNA
<213>  Artificial Sequence

<220>
<223>  Primer


<220>
<221>  misc_feature
<222>  (5)..(9)

<223>  Nucleotide at position 5 and/or 9 may be modified by
       2'-O,4'-C-methylene-beta-D-ribofuranosyl moiety

<400>  104
ctttcagtcg gatgttt                                                    17


<210>  105
<211>  30
<212>  DNA
<213>  Artificial Sequence

<220>
<223>  Primer

<400>  105
catgatcagc tgggccaaga tccagtcaag                                      30


<210>  106
<211>  16
<212>  DNA
<213>  Artificial Sequence

<220>
<223>  Primer


<220>
<221>  misc_feature
<222>  (3)..(7)
<223>  Nucleotide at position 3 and/or 7 may be modified by
       2'-O,4'-C-methylene-beta-D-ribofuranosyl moiety

<400>  106
tgtaaacatc cttgac                                                     16


<210>  107
<211>  30
<212>  DNA
<213>  Artificial Sequence

<220>
<223>  Primer

<400>  107
catgatcagc tgggccaaga cagctatgcc                                      30


<210>  108
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  108
ggcaagatgc tggc                                                          14


<210>  109
<211>  30
<212>  DNA
<213>  Artificial Sequence

<220>
<223>  Primer

<400>  109
catgatcagc tgggccaaga gcaacttagt                                         30


<210>  110
<211>  17
<212>  DNA
<213>  Artificial Sequence

<220>
<223>  Primer


<220>
<221>  misc_feature
<222>  (6)..(8)
<223>  Nucleotide at position 6 and/or 8 may be modified by
       2'-O,4'-C-methylene-beta-D-ribofuranosyl moiety

<400>  110
tattgcacat tactaag                                                       17


<210>  111
<211>  29
<212>  DNA
<213>  Artificial Sequence

<220>
<223>  Primer

<400>  111
catgatcagc tgggccaaga caatgcaac                                          29


<210>  112
<211>  15
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  112
gtgcattgta gttgc                                                         15

<210>  113
<211>  30
<212>  DNA
<213>  Artificial Sequence

<220>
<223>  Primer

<400>  113
catgatcagc tgggccaaga aacaaccagc                                        30


<210>  114
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  114
tggcagtgtc ttag                                                         14


<210>  115
<211>  30
<212>  DNA
<213>  Artificial Sequence

<220>
<223>  Primer

<400>  115
catgatcagc tgggccaaga caatcagcta                                        30


<210>  116
<211>  14
<212>  DNA
<213>  Artificial Sequence

<220>
<223>  Primer



<220>
<221>  misc_feature
<222>  (3)..(5)
<223>  Nucleotide at position 3 and/or 5 may be modified by
       2'-O,4'-C-methylene-beta-D-ribofuranosyl moiety

<400>  116
taggcagtgt catt                                                         14


<210>  117
<211>  30

<212>  DNA
<213>  Artificial Sequence

<220>
<223>  Primer

<400>  117
catgatcagc tgggccaaga gcaatcagct                                        30


<210>  118
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  118
aggcagtgta gtta                                                         14


<210>  119
<211>  30
<212>  DNA
<213>  Artificial Sequence

<220>
<223>  Primer

<400>  119
catgatcagc tgggccaaga caggccggga                                        30


<210>  120
<211>  15
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  120
tattgcactt gtccc                                                        15


<210>  121
<211>  30
<212>  DNA
<213>  Artificial Sequence

<220>
<223>  Primer

<400>  121
catgatcagc tgggccaaga ctacctgcac                                        30


<210>  122
<211>  14
<212>  DNA
<213>  Artificial Sequence

<220>
<223>  Primer


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  122
aaagtgctgt tcgt                                                         14


<210>  123
<211>  30
<212>  DNA
<213>  Artificial Sequence

<220>
<223>  Primer

<400>  123
catgatcagc tgggccaaga tgctcaataa                                        30


<210>  124
<211>  19
<212>  DNA
<213>  Artificial Sequence

<220>
<223>  Primer


<220>
<221>  misc_feature
<222>  (3)..(7)
<223>  Nucleotide at position 3 and/or 7 may be modified by
       2'-O,4'-C-methylene-beta-D-ribofuranosyl moiety

<400>  124
ttcaacgggt atttattga                                                    19


<210>  125
<211>  30
<212>  DNA
<213>  Artificial Sequence

<220>
<223>  Primer

<400>  125
catgatcagc tgggccaaga gcaaaaatgt                                        30


<210>  126
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  126
tttggcacta gcac                                                         14


<210>  127
<211>  30
<212>  DNA
<213>  Artificial Sequence

<220>
<223>  Primer

<400>  127
catgatcagc tgggccaaga aacaatacaa                                        30


<210>  128
<211>  15
<212>  DNA
<213>  Artificial Sequence

<220>
<223>  Primer


<220>
<221>  misc_feature
<222>  (4)..(7)
<223>  Nucleotide at position 4 and/or 7 may be modified by
       2'-O,4'-C-methylene-beta-D-ribofuranosyl moiety

<400>  128
tgaggtagta agttg                                                        15


<210>  129
<211>  30
<212>  DNA
<213>  Artificial Sequence

<220>
<223>  Primer

<400>  129
catgatcagc tgggccaaga cacaagatcg                                        30

<210>  130
<211>  14
<212>  DNA
<213>  Artificial Sequence

<220>
<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  130
aacccgtaga tccg                                                          14

<210>  131
<211>  30
<212>  DNA
<213>  Artificial Sequence

<220>
<223>  Primer

<400>  131
catgatcagc tgggccaaga cgcaaggtcg                                         30

<210>  132
<211>  14
<212>  DNA
<213>  Artificial Sequence

<220>
<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  132
cacccgtaga accg                                                          14

<210>  133
<211>  30
<212>  DNA
<213>  Artificial Sequence

<220>
<223>  Primer

<400>  133
catgatcagc tgggccaaga cacaagttcg                                         30

<210>  134
<211>  14

<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  134
aacccgtaga tccg                                                         14


<210>  135
<211>  30
<212>  DNA
<213>  Artificial Sequence

<220>
<223>  Primer

<400>  135
catgatcagc tgggccaaga cttcagttat                                        30


<210>  136
<211>  18
<212>  DNA
<213>  Artificial Sequence

<220>
<223>  Primer


<220>
<221>  misc_feature
<222>  (3)..(6)
<223>  Nucleotide at position 3 and/or 6 may be modified by
       2'-O,4'-C-methylene-beta-D-ribofuranosyl moiety

<400>  136
tacagtactg tgataact                                                     18


<210>  137
<211>  30
<212>  DNA
<213>  Artificial Sequence

<220>
<223>  Primer

<400>  137
catgatcagc tgggccaaga tcatagccct                                        30


<210>  138
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  138
agcagcattg taca                                                          14


<210>  139
<211>  30
<212>  DNA
<213>  Artificial Sequence

<220>
<223>  Primer

<400>  139
catgatcagc tgggccaaga acaggagtct                                         30


<210>  140
<211>  15
<212>  DNA
<213>  Artificial Sequence

<220>
<223>  Primer


<220>
<221>  misc_feature
<222>  (2)..(6)
<223>  Nucleotide at position 2 and/or 6 may be modified by
       2'-O,4'-C-methylene-beta-D-ribofuranosyl moiety

<400>  140
tcaaatgctc agact                                                         15


<210>  141
<211>  30
<212>  DNA
<213>  Artificial Sequence

<220>
<223>  Primer

<400>  141
catgatcagc tgggccaaga gctacctgca                                         30


<210>  142
<211>  16
<212>  DNA
<213>  Artificial Sequence

<220>
<223>  Primer

<220>
<221>  misc_feature
<222>  (4)..(6)
<223>  Nucleotide at position 4 and/or 6 may be modified by
       2'-O,4'-C-methylene-beta-D-ribofuranosyl moiety

<400>  142
aaaagtgctt acagtg                                                    16


<210>  143
<211>  30
<212>  DNA
<213>  Artificial Sequence

<220>
<223>  Primer

<400>  143
catgatcagc tgggccaaga atctgcactg                                     30


<210>  144
<211>  15
<212>  DNA
<213>  Artificial Sequence

<220>
<223>  Primer


<220>
<221>  misc_feature
<222>  (2)..(6)
<223>  Nucleotide at position 2 and/or 6 may be modified by
       2'-O,4'-C-methylene-beta-D-ribofuranosyl moiety

<400>  144
taaagtgctg acagt                                                     15


<210>  145
<211>  28
<212>  DNA
<213>  Artificial Sequence

<220>
<223>  Primer

<400>  145
catgatcagc tgggccaaga tgatagcc                                       28


<210>  146
<211>  15
<212>  DNA
<213>  Artificial Sequence

<220>
<223>  Primer


<220>
<221>  misc_feature

<222>  (2)..(4)
<223>  Nucleotide at position 2 and/or 4 may be modified by
       2'-O,4'-C-methylene-beta-D-ribofuranosyl moiety

<400>  146
agcagcattg tacag                                                        15


<210>  147
<211>  30
<212>  DNA
<213>  Artificial Sequence

<220>
<223>  Primer

<400>  147
catgatcagc tgggccaaga acaaacacca                                        30


<210>  148
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  148
tggagtgtga caat                                                         14


<210>  149
<211>  30
<212>  DNA
<213>  Artificial Sequence

<220>
<223>  Primer

<400>  149
catgatcagc tgggccaaga tggcattcac                                        30


<210>  150
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  150
ttaaggcacg cggt                                                         14


<210>  151
<211>  30
<212>  DNA
<213>  Artificial Sequence

<220>
<223>  Primer

<400>  151
catgatcagc tgggccaaga cacaggttaa                                        30


<210>  152
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  152
tccctgagac cctt                                                         14


<210>  153
<211>  30
<212>  DNA
<213>  Artificial Sequence

<220>
<223>  Primer

<400>  153
catgatcagc tgggccaaga tcacaagtta                                        30


<210>  154
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  154
tccctgagac ccta                                                         14

<210>  155
<211>  30
<212>  DNA
<213>  Artificial Sequence

<220>
<223>  Primer

<400>  155
catgatcagc tgggccaaga gcattattac                                        30


<210>  156
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  156
tcgtaccgtg agta                                                          14


<210>  157
<211>  28
<212>  DNA
<213>  Artificial Sequence

<220>
<223>  Primer

<400>  157
catgatcagc tgggccaaga cgcgtacc                                          28


<210>  158
<211>  19
<212>  DNA
<213>  Artificial Sequence

<220>
<223>  Primer


<220>
<221>  misc_feature
<222>  (2)..(9)
<223>  Nucleotide at position 2 and/or 5 and/or 9 may be modified by
       2'-O,4'-C-methylene-beta-D-ribofuranosyl moiety

<400>  158
cattattact tttggtacg                                                     19


<210>  159

<211>  30
<212>  DNA
<213>  Artificial Sequence

<220>
<223>  Primer

<400>  159
catgatcagc tgggccaaga agccaagctc                                        30


<210>  160
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  160
tcggatccgt ctga                                                         14


<210>  161
<211>  30
<212>  DNA
<213>  Artificial Sequence

<220>
<223>  Primer

<400>  161
catgatcagc tgggccaaga aaaagagacc                                        30


<210>  162
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  162
tcacagtgaa ccgg                                                         14


<210>  163
<211>  30
<212>  DNA
<213>  Artificial Sequence

<220>
<223>  Primer

<400>  163
catgatcagc tgggccaaga gaaagagacc                                        30


<210>  164
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  164
tcacagtgaa ccgg                                                         14


<210>  165
<211>  30
<212>  DNA
<213>  Artificial Sequence

<220>
<223>  Primer

<400>  165
catgatcagc tgggccaaga gcaagcccag                                        30


<210>  166
<211>  14
<212>  DNA
<213>  Artificial Sequence

<220>
<223>  Primer


<220>
<221>  misc_feature
<222>  (6)..(8)
<223>  Nucleotide at position 6 and/or 8 may be modified by
       2'-O,4'-C-methylene-beta-D-ribofuranosyl moiety

<400>  166
ctttttgcgg tctg                                                         14


<210>  167
<211>  30
<212>  DNA
<213>  Artificial Sequence

<220>
<223>  Primer

<400>  167
catgatcagc tgggccaaga atgccctttt                                        30


<210>  168
<211>  17
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  168
cagtgcaatg ttaaaag                                                      17


<210>  169
<211>  30
<212>  DNA
<213>  Artificial Sequence

<220>
<223>  Primer

<400>  169
catgatcagc tgggccaaga atgccctttc                                        30


<210>  170
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  170
cagtgcaatg atga                                                         14


<210>  171
<211>  30
<212>  DNA
<213>  Artificial Sequence

<220>
<223>  Primer

<400>  171
catgatcagc tgggccaaga cgaccatggc                                        30

<210>  172
<211>  15
<212>  DNA
<213>  Artificial Sequence

<220>
<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  172
taacagtcta cagcc                                                    15

<210>  173
<211>  30
<212>  DNA
<213>  Artificial Sequence

<220>
<223>  Primer

<400>  173
catgatcagc tgggccaaga acagctggtt                                    30

<210>  174
<211>  14
<212>  DNA
<213>  Artificial Sequence

<220>
<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  174
ttggtcccct tcaa                                                     14

<210>  175
<211>  30
<212>  DNA
<213>  Artificial Sequence

<220>
<223>  Primer

<400>  175
catgatcagc tgggccaaga tagctggttg                                    30

<210>  176

<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  176
ttggtcccct tcaa                                                         14


<210>  177
<211>  30
<212>  DNA
<213>  Artificial Sequence

<220>
<223>  Primer

<400>  177
catgatcagc tgggccaaga ccctctggtc                                        30


<210>  178
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  178
tgtgactggt tgac                                                         14


<210>  179
<211>  30
<212>  DNA
<213>  Artificial Sequence

<220>
<223>  Primer

<400>  179
catgatcagc tgggccaaga tcacatagga                                        30


<210>  180
<211>  17
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  180
tatggctttt tattcct                                                    17


<210>  181
<211>  30
<212>  DNA
<213>  Artificial Sequence

<220>
<223>  Primer

<400>  181
catgatcagc tgggccaaga cacataggaa                                      30


<210>  182
<211>  16
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  182
tatggctttt cattcc                                                     16


<210>  183
<211>  30
<212>  DNA
<213>  Artificial Sequence

<220>
<223>  Primer

<400>  183
catgatcagc tgggccaaga tccatcatca                                      30


<210>  184
<211>  18
<212>  DNA
<213>  Artificial Sequence

<220>
<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  184
actccatttg ttttgatg                                                    18


<210>  185
<211>  30
<212>  DNA
<213>  Artificial Sequence

<220>
<223>  Primer

<400>  185
catgatcagc tgggccaaga ctacgcgtat                                       30


<210>  186
<211>  18
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  186
tattgcttaa gaatacgc                                                    18


<210>  187
<211>  29
<212>  DNA
<213>  Artificial Sequence

<220>
<223>  Primer

<400>  187
catgatcagc tgggccaaga cggcctgat                                        29


<210>  188
<211>  14
<212>  DNA
<213>  Artificial Sequence

<220>
<223>  Primer


<220>

<221>  misc_feature
<222>  (2)..(4)
<223>  Nucleotide at position 2 and/or 4 may be modified by
       2'-O,4'-C-methylene-beta-D-ribofuranosyl moiety

<400>  188
agctggtgtt gtga                                                         14


<210>  189
<211>  30
<212>  DNA
<213>  Artificial Sequence

<220>
<223>  Primer

<400>  189
catgatcagc tgggccaaga agacacgtgc                                        30


<210>  190
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  190
tctacagtgc acgt                                                         14


<210>  191
<211>  30
<212>  DNA
<213>  Artificial Sequence

<220>
<223>  Primer

<400>  191
catgatcagc tgggccaaga ctaccatagg                                        30


<210>  192
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by

2'-O,4'-C-methylene-beta-D-ribofuranosyl moiety

<400> 192
agtggtttta ccct 14

<210> 193
<211> 29
<212> DNA
<213> Artificial Sequence

<220>
<223> Primer

<400> 193
catgatcagc tgggccaaga ccatcttta 29

<210> 194
<211> 16
<212> DNA
<213> Artificial Sequence

<220>
<223> Primer

<220>
<221> misc_feature
<222> (4)..(7)
<223> Nucleotide at position 4 and/or 7 may be modified by
      2'-O,4'-C-methylene-beta-D-ribofuranosyl moiety

<400> 194
taacactgtc tggtaa 16

<210> 195
<211> 28
<212> DNA
<213> Artificial Sequence

<220>
<223> Primer

<400> 195
catgatcagc tgggccaaga tccataaa 28

<210> 196
<211> 16
<212> DNA
<213> Artificial Sequence

<220>
<223> Primer

<220>
<221> misc_feature
<222> (4)..(6)
<223> Nucleotide at position 4 and/or 6 may be modified by
      2'-O,4'-C-methylene-beta-D-ribofuranosyl moiety

<400> 196

tgtagtgttt cctact                                                        16

<210>  197
<211>  28
<212>  DNA
<213>  Artificial Sequence

<220>
<223>  Primer

<400>  197
catgatcagc tgggccaaga tgagctac                                          28

<210>  198
<211>  15
<212>  DNA
<213>  Artificial Sequence

<220>
<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  198
tgagatgaag cactg                                                        15

<210>  199
<211>  29
<212>  DNA
<213>  Artificial Sequence

<220>
<223>  Primer

<400>  199
catgatcagc tgggccaaga ctagtacat                                         29

<210>  200
<211>  16
<212>  DNA
<213>  Artificial Sequence

<220>
<223>  Primer

<220>
<221>  misc_feature
<222>  (3)..(9)
<223>  Nucleotide at position 3 and/or 5 and/or 9 may be modified by
       2'-O,4'-C-methylene-beta-D-ribofuranosyl moiety

<400>  200
tacagtatag atgatg                                                       16

<210> 201
<211> 29
<212> DNA
<213> Artificial Sequence

<220>
<223> Primer

<400> 201
catgatcagc tgggccaaga aagggattc 29


<210> 202
<211> 14
<212> DNA
<213> Artificial Sequence

<220>
<223> Primer


<220>
<221> misc_feature
<222> (2)..(4)
<223> Nucleotide at position 2 and/or 4 may be modified by
2'-O,4'-C-methylene-beta-D-ribofuranosyl moiety

<400> 202
gtccagtttt ccca 14


<210> 203
<211> 28
<212> DNA
<213> Artificial Sequence

<220>
<223> Primer

<400> 203
catgatcagc tgggccaaga aacccatg 28


<210> 204
<211> 16
<212> DNA
<213> Artificial Sequence

<220>
<223> Primer


<220>
<221> misc_feature
<222> (2)..(4)
<223> Nucleotide at position 2 and/or 4 may be modified by
2'-O,4'-C-methylene-beta-D-ribofuranosyl moiety

<400> 204
tgagaactga attcca 16


<210> 205
<211> 30
<212> DNA

<213>  Artificial Sequence

<220>
<223>  Primer

<400>  205
catgatcagc tgggccaaga gcagaagcat                                        30


<210>  206
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  206
gtgtgtggaa atgc                                                         14


<210>  207
<211>  29
<212>  DNA
<213>  Artificial Sequence

<220>
<223>  Primer

<400>  207
catgatcagc tgggccaaga acaaagttc                                         29


<210>  208
<211>  18
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  208
tcagtgcact acagaact                                                     18


<210>  209
<211>  29
<212>  DNA
<213>  Artificial Sequence

<220>

<223>  Primer

<400>  209
catgatcagc tgggccaaga acaaagttc                                        29


<210>  210
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  210
tcagtgcatc acag                                                        14


<210>  211
<211>  29
<212>  DNA
<213>  Artificial Sequence

<220>
<223>  Primer

<400>  211
catgatcagc tgggccaaga ggagtgaag                                        29


<210>  212
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  212
tctggctccg tgtc                                                        14


<210>  213
<211>  28
<212>  DNA
<213>  Artificial Sequence

<220>
<223>  Primer

<400>  213

catgatcagc tgggccaaga cactggta                                          28

<210>  214
<211>  14
<212>  DNA
<213>  Artificial Sequence

<220>
<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  214
tctcccaacc cttg                                                      14

<210>  215
<211>  29
<212>  DNA
<213>  Artificial Sequence

<220>
<223>  Primer

<400>  215
catgatcagc tgggccaaga cctcaagga                                     29

<210>  216
<211>  15
<212>  DNA
<213>  Artificial Sequence

<220>
<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  216
actagactga agctc                                                     15

<210>  217
<211>  29
<212>  DNA
<213>  Artificial Sequence

<220>
<223>  Primer

<400>  217
catgatcagc tgggccaaga cccaagttc                                     29

<210>  218
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  218
tcagtgcatg acag                                                         14


<210>  219
<211>  29
<212>  DNA
<213>  Artificial Sequence

<220>
<223>  Primer

<400>  219
catgatcagc tgggccaaga tcacttttg                                         29


<210>  220
<211>  16
<212>  DNA
<213>  Artificial Sequence

<220>
<223>  Primer



<220>
<221>  misc_feature
<222>  (4)..(7)
<223>  Nucleotide at position 4 and/or 7 may be modified by
       2'-O,4'-C-methylene-beta-D-ribofuranosyl moiety

<400>  220
ttgcatagtc acaaaa                                                       16


<210>  221
<211>  29
<212>  DNA
<213>  Artificial Sequence

<220>
<223>  Primer

<400>  221
catgatcagc tgggccaaga aataggtca                                         29


<210>  222
<211>  17
<212>  DNA

<213>  Artificial Sequence

<220>
<223>  Primer



<220>
<221>  misc_feature
<222>  (6)..(8)
<223>  Nucleotide at position 6 and/or 8 may be modified by
       2'-O,4'-C-methylene-beta-D-ribofuranosyl moiety

<400>  222
aatcatacac ggttgac                                                    17


<210>  223
<211>  29
<212>  DNA
<213>  Artificial Sequence

<220>
<223>  Primer

<400>  223
catgatcagc tgggccaaga cgaaggcaa                                       29


<210>  224
<211>  15
<212>  DNA
<213>  Artificial Sequence

<220>
<223>  Primer



<220>
<221>  misc_feature
<222>  (3)..(8)
<223>  Nucleotide at position 3 and/or 8 may be modified by
       2'-O,4'-C-methylene-beta-D-ribofuranosyl moiety

<400>  224
taggttatcc gtgtt                                                      15


<210>  225
<211>  28
<212>  DNA
<213>  Artificial Sequence

<220>
<223>  Primer

<400>  225
catgatcagc tgggccaaga cccctatc                                        28


<210>  226
<211>  20
<212>  DNA
<213>  Artificial Sequence

<220>

<223>  Primer

<220>
<221>  misc_feature
<222>  (3)..(5)
<223>  Nucleotide at position 2 and/or 5 may be modified by
       2'-O,4'-C-methylene-beta-D-ribofuranosyl moiety

<400>  226
ttaatgctaa tcgtgatagg                                                  20

<210>  227
<211>  29
<212>  DNA
<213>  Artificial Sequence

<220>
<223>  Primer

<400>  227
catgatcagc tgggccaaga actcaccga                                        29

<210>  228
<211>  16
<212>  DNA
<213>  Artificial Sequence

<220>
<223>  Primer

<220>
<221>  misc_feature
<222>  (3)..(7)
<223>  Nucleotide at position 3 and/or 7 may be modified by
       2'-O,4'-C-methylene-beta-D-ribofuranosyl moiety

<400>  228
aacattcaac gctgtc                                                      16

<210>  229
<211>  29
<212>  DNA
<213>  Artificial Sequence

<220>
<223>  Primer

<400>  229
catgatcagc tgggccaaga actcaccga                                        29

<210>  230
<211>  16
<212>  DNA
<213>  Artificial Sequence

<220>
<223>  Primer

<220>
<221>  misc_feature
<222>  (3)..(7)
<223>  Nucleotide at position 3 and/or 7 may be modified by
      2'-O,4'-C-methylene-beta-D-ribofuranosyl moiety

<400>  230
aacattcaac ctgtcg                                                    16


<210>  231
<211>  30
<212>  DNA
<213>  Artificial Sequence

<220>
<223>  Primer

<400>  231
catgatcagc tgggccaaga tagttggcaa                                     30


<210>  232
<211>  15
<212>  DNA
<213>  Artificial Sequence

<220>
<223>  Primer


<220>
<221>  misc_feature
<222>  (2)..(4)
<223>  Nucleotide at position 2 and/or 4 may be modified by
      2'-O,4'-C-methylene-beta-D-ribofuranosyl moiety

<400>  232
tggttctaga cttgc                                                     15


<210>  233
<211>  29
<212>  DNA
<213>  Artificial Sequence

<220>
<223>  Primer

<400>  233
catgatcagc tgggccaaga tgtgagttc                                      29


<210>  234
<211>  14
<212>  DNA
<213>  Artificial Sequence

<220>
<223>  Primer


<220>
<221>  misc_feature
<222>  (4)..(6)

<223>  Nucleotide at position 4 and/or 6 may be modified by
      2'-O,4'-C-methylene-beta-D-ribofuranosyl moiety

<400>  234
tttggcaatg gtag                                                    14


<210>  235
<211>  29
<212>  DNA
<213>  Artificial Sequence

<220>
<223>  Primer

<400>  235
catgatcagc tgggccaaga cagtgaatt                                    29


<210>  236
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
      2'-O,4'-C-methylene-beta-D-ribofuranosyl moiety

<400>  236
tatggcactg gtag                                                    14


<210>  237
<211>  29
<212>  DNA
<213>  Artificial Sequence

<220>
<223>  Primer

<400>  237
catgatcagc tgggccaaga acccttatc                                    29


<210>  238
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
      2'-O,4'-C-methylene-beta-D-ribofuranosyl moiety

<400>  238
tggacggaga actg                                                          14


<210>  239
<211>  29
<212>  DNA
<213>  Artificial Sequence

<220>
<223>  Primer

<400>  239
catgatcagc tgggccaaga aagcccaaa                                          29


<210>  240
<211>  19
<212>  DNA
<213>  Artificial Sequence

<220>
<223>  Primer


<220>
<221>  misc_feature
<222>  (3)..(10)
<223>  Nucleotide at position 3 and/or 5 and/or 10 may be modified by
       2'-O,4'-C-methylene-beta-D-ribofuranosyl moiety

<400>  240
caaagaattc tccttttgg                                                     19


<210>  241
<211>  30
<212>  DNA
<213>  Artificial Sequence

<220>
<223>  Primer

<400>  241
catgatcagc tgggccaaga cggctgcaac                                         30


<210>  242
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  242
tcgtgtcttg tgtt                                                          14

<210>  243
<211>  30
<212>  DNA
<213>  Artificial Sequence

<220>
<223>  Primer

<400>  243
catgatcagc tgggccaaga accctccacc                                        30


<210>  244
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  244
catcccttgc atgg                                                         14


<210>  245
<211>  29
<212>  DNA
<213>  Artificial Sequence

<220>
<223>  Primer

<400>  245
catgatcagc tgggccaaga actgatatc                                         29


<210>  246
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  246
gtgcctactg agct                                                         14


<210>  247
<211>  29

<212>  DNA
<213>  Artificial Sequence

<220>
<223>  Primer

<400>  247
catgatcagc tgggccaaga acctaatat                                          29


<210>  248
<211>  20
<212>  DNA
<213>  Artificial Sequence

<220>
<223>  Primer


<220>
<221>  misc_feature
<222>  (2)..(6)
<223>  Nucleotide at position 2 and/or 4 and/or 6 may be modified by
       2'-O,4'-C-methylene-beta-D-ribofuranosyl moiety

<400>  248
tgatatgttt gatatattag                                                    20


<210>  249
<211>  29
<212>  DNA
<213>  Artificial Sequence

<220>
<223>  Primer

<400>  249
catgatcagc tgggccaaga agctgcttt                                          29


<210>  250
<211>  17
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  250
caacggaatc ccaaaag                                                       17


<210>  251
<211>  29
<212>  DNA
<213>  Artificial Sequence

<220>
<223>  Primer

<400>  251
catgatcagc tgggccaaga ggctgtcaa                                         29


<210>  252
<211>  17
<212>  DNA
<213>  Artificial Sequence

<220>
<223>  Primer


<220>
<221>  misc_feature
<222>  (2)..(5)
<223>  Nucleotide at position 2 and/or 5 may be modified by
       2'-O,4'-C-methylene-beta-D-ribofuranosyl moiety

<400>  252
ctgacctatg aattgac                                                      17


<210>  253
<211>  29
<212>  DNA
<213>  Artificial Sequence

<220>
<223>  Primer

<400>  253
catgatcagc tgggccaaga ctgggactt                                         29


<210>  254
<211>  15
<212>  DNA
<213>  Artificial Sequence

<220>
<223>  Primer


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  254
aactggccta caaag                                                        15


<210>  255
<211>  28
<212>  DNA
<213>  Artificial Sequence

<220>
<223>  Primer

<400>  255
catgatcagc tgggccaaga tccacatg                                          28


<210>  256
<211>  16
<212>  DNA
<213>  Artificial Sequence

<220>
<223>  Primer


<220>
<221>  misc_feature
<222>  (3)..(6)
<223>  Nucleotide at position 3 and/or 6 may be modified by
       2'-O,4'-C-methylene-beta-D-ribofuranosyl moiety

<400>  256
tgtaacagca actcca                                                       16


<210>  257
<211>  29
<212>  DNA
<213>  Artificial Sequence

<220>
<223>  Primer

<400>  257
catgatcagc tgggccaaga gccaatatt                                         29


<210>  258
<211>  16
<212>  DNA
<213>  Artificial Sequence

<220>
<223>  Primer


<220>
<221>  misc_feature
<222>  (2)..(7)
<223>  Nucleotide at position 2 and/or 4 and/or 7 may be modified by
       2'-O,4'-C-methylene-beta-D-ribofuranosyl moiety

<400>  258
tagcagcaca gaaata                                                       16


<210>  259
<211>  30
<212>  DNA
<213>  Artificial Sequence

<220>
<223>  Primer

<400>  259
catgatcagc tgggccaaga ccaacaacag                                        30

<210>  260
<211>  15
<212>  DNA
<213>  Artificial Sequence

<220>
<223>  Primer



<220>
<221>  misc_feature
<222>  (3)..(6)
<223>  Nucleotide at position 3 and/or 6 may be modified by
       2'-O,4'-C-methylene-beta-D-ribofuranosyl moiety

<400>  260
taggtagttt cctgt                                                    15


<210>  261
<211>  30
<212>  DNA
<213>  Artificial Sequence

<220>
<223>  Primer

<400>  261
catgatcagc tgggccaaga ccaacaacat                                    30


<210>  262
<211>  17
<212>  DNA
<213>  Artificial Sequence

<220>
<223>  Primer



<220>
<221>  misc_feature
<222>  (3)..(5)
<223>  Nucleotide at position 3 and/or 5 may be modified by
       2'-O,4'-C-methylene-beta-D-ribofuranosyl moiety

<400>  262
taggtagttt catgttg                                                  17


<210>  263
<211>  29
<212>  DNA
<213>  Artificial Sequence

<220>
<223>  Primer

<400>  263
catgatcagc tgggccaaga gctgggtgg                                     29


<210>  264
<211>  14

<212>  DNA
<213>  Artificial Sequence

<220>
<223>  Primer


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  264
ttcaccacct tctc                                                          14


<210>  265
<211>  28
<212>  DNA
<213>  Artificial Sequence

<220>
<223>  Primer

<400>  265
catgatcagc tgggccaaga cctatctc                                           28


<210>  266
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  266
ggtccagagg ggag                                                          14


<210>  267
<211>  29
<212>  DNA
<213>  Artificial Sequence

<220>
<223>  Primer

<400>  267
catgatcagc tgggccaaga aaccaatgt                                          29


<210>  268
<211>  15
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  268
tacagtagtc tgcac                                                        15


<210>  269
<211>  29
<212>  DNA
<213>  Artificial Sequence

<220>
<223>  Primer

<400>  269
catgatcagc tgggccaaga gaacaggta                                         29


<210>  270
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  270
cccagtgttc agac                                                         14


<210>  271
<211>  30
<212>  DNA
<213>  Artificial Sequence

<220>
<223>  Primer

<400>  271
catgatcagc tgggccaaga gaacagatag                                        30


<210>  272
<211>  14
<212>  DNA
<213>  Artificial Sequence

<220>
<223>  Primer

<220>
<221>  misc_feature
<222>  (2)..(4)
<223>  Nucleotide at position 2 and/or 4 may be modified by
      2'-O,4'-C-methylene-beta-D-ribofuranosyl moiety

<400>  272
cccagtgttt agac                                                         14


<210>  273
<211>  29
<212>  DNA
<213>  Artificial Sequence

<220>
<223>  Primer

<400>  273
catgatcagc tgggccaaga acatcgtta                                         29


<210>  274
<211>  14
<212>  DNA
<213>  Artificial Sequence

<220>
<223>  Primer


<220>
<221>  misc_feature
<222>  (4)..(7)
<223>  Nucleotide at position 4 and/or 7 may be modified by
      2'-O,4'-C-methylene-beta-D-ribofuranosyl moiety

<400>  274
taacactgtc tggt                                                         14


<210>  275
<211>  29
<212>  DNA
<213>  Artificial Sequence

<220>
<223>  Primer

<400>  275
catgatcagc tgggccaaga gtcatcatt                                         29


<210>  276
<211>  17
<212>  DNA
<213>  Artificial Sequence

<220>
<223>  Primer


<220>
<221>  misc_feature

<222>  (6)..(9)
<223>  Nucleotide at position 6 and/or 9 may be modified by
       2'-O,4'-C-methylene-beta-D-ribofuranosyl moiety

<400>  276
taatactgcc tggtaat                                                    17


<210>  277
<211>  30
<212>  DNA
<213>  Artificial Sequence

<220>
<223>  Primer

<400>  277
catgatcagc tgggccaaga ttttcccatg                                      30


<210>  278
<211>  15
<212>  DNA
<213>  Artificial Sequence

<220>
<223>  Primer


<220>
<221>  misc_feature
<222>  (2)..(10)
<223>  Nucleotide at position 2 and/or 4 and/or 10 may be modified by
       2'-O,4'-C-methylene-beta-D-ribofuranosyl moiety

<400>  278
agaggtatag ggcat                                                      15


<210>  279
<211>  29
<212>  DNA
<213>  Artificial Sequence

<220>
<223>  Primer

<400>  279
catgatcagc tgggccaaga ctagtggtc                                       29


<210>  280
<211>  17
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  280
gtgaaatgtt taggacc                                                      17


<210>  281
<211>  29
<212>  DNA
<213>  Artificial Sequence

<220>
<223>  Primer

<400>  281
catgatcagc tgggccaaga aggcatagg                                         29


<210>  282
<211>  15
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  282
ttccctttgt catcc                                                        15


<210>  283
<211>  30
<212>  DNA
<213>  Artificial Sequence

<220>
<223>  Primer

<400>  283
catgatcagc tgggccaaga cagactccgg                                        30


<210>  284
<211>  14
<212>  DNA
<213>  Artificial Sequence

<220>
<223>  Primer



<220>
<221>  misc_feature
<222>  (2)..(6)
<223>  Nucleotide at position 2 and/or 6 may be modified by
       2'-O,4'-C-methylene-beta-D-ribofuranosyl moiety

<400>  284
tccttcattc cacc                                                         14

<210>  285
<211>  27
<212>  DNA
<213>  Artificial Sequence

<220>
<223>  Primer

<400>  285
catgatcagc tgggccaaga ccacaca                                          27


<210>  286
<211>  18
<212>  DNA
<213>  Artificial Sequence

<220>
<223>  Primer


<220>
<221>  misc_feature
<222>  (2)..(6)
<223>  Nucleotide at position 2 and/or 3 and/or 6 may be modified by
       2'-O,4'-C-methylene-beta-D-ribofuranosyl moiety

<400>  286
tggaatgtaa ggaagtgt                                                    18


<210>  287
<211>  33
<212>  DNA
<213>  Artificial Sequence

<220>
<223>  Primer

<400>  287
catgatcagc tgggccaaga acaagctttt tgc                                  33


<210>  288
<211>  17
<212>  DNA
<213>  Artificial Sequence

<220>
<223>  Primer


<220>
<221>  misc_feature
<222>  (5)..(9)
<223>  Nucleotide at position 5 and/or 7 and/or 9 may be modified by
       2'-O,4'-C-methylene-beta-D-ribofuranosyl moiety

<400>  288
ataagacgag caaaaag                                                     17


<210>  289

<211>  30
<212>  DNA
<213>  Artificial Sequence

<220>
<223>  Primer

<400>  289
catgatcagc tgggccaaga tcagccgctg                                        30


<210>  290
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  290
ctgtgcgtgt gaca                                                         14


<210>  291
<211>  29
<212>  DNA
<213>  Artificial Sequence

<220>
<223>  Primer

<400>  291
catgatcagc tgggccaaga aggcgaagg                                         29


<210>  292
<211>  15
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  292
ttccctttgt catcc                                                        15


<210>  293
<211>  29
<212>  DNA
<213>  Artificial Sequence

<220>
<223>  Primer

<400>  293
catgatcagc tgggccaaga ggccgtgac                                        29


<210>  294
<211>  16
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  294
taacagtctc cagtca                                                      16


<210>  295
<211>  30
<212>  DNA
<213>  Artificial Sequence

<220>
<223>  Primer

<400>  295
catgatcagc tgggccaaga ggtacaatca                                       30


<210>  296
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  296
accatcgacc gttg                                                        14


<210>  297
<211>  30
<212>  DNA
<213>  Artificial Sequence

<220>
<223>  Primer

<400>  297
catgatcagc tgggccaaga ctgcctgtct                                        30


<210>  298
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  298
acagcaggca caga                                                         14


<210>  299
<211>  29
<212>  DNA
<213>  Artificial Sequence

<220>
<223>  Primer

<400>  299
catgatcagc tgggccaaga gtctgtcaa                                         29


<210>  300
<211>  17
<212>  DNA
<213>  Artificial Sequence

<220>
<223>  Primer


<220>
<221>  misc_feature
<222>  (2)..(5)
<223>  Nucleotide at position 2 and/or 5 may be modified by
       2'-O,4'-C-methylene-beta-D-ribofuranosyl moiety

<400>  300
atgacctatg aattgac                                                      17


<210>  301
<211>  29
<212>  DNA
<213>  Artificial Sequence

<220>
<223>  Primer

<400>  301
catgatcagc tgggccaaga cacagttgc                                         29

<210>  302
<211>  15
<212>  DNA
<213>  Artificial Sequence

<220>
<223>  Primer



<220>
<221>  misc_feature
<222>  (4)..(7)
<223>  Nucleotide at position 4 and/or 7 may be modified by
       2'-O,4'-C-methylene-beta-D-ribofuranosyl moiety

<400>  302
taatctcagc tggca                                                        15


<210>  303
<211>  29
<212>  DNA
<213>  Artificial Sequence

<220>
<223>  Primer

<400>  303
catgatcagc tgggccaaga atccaatca                                         29


<210>  304
<211>  18
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  304
tactgcatca ggaactga                                                     18


<210>  305
<211>  29
<212>  DNA
<213>  Artificial Sequence

<220>
<223>  Primer

<400>  305
catgatcagc tgggccaaga acatggtta                                         29


<210>  306

<211>  16
<212>  DNA
<213>  Artificial Sequence

<220>
<223>  Primer


<220>
<221>  misc_feature
<222>  (4)..(9)
<223>  Nucleotide at position 4 and/or 9 may be modified by
       2'-O,4'-C-methylene-beta-D-ribofuranosyl moiety

<400>  306
ttgtgcttga tctaac                                                       16


<210>  307
<211>  30
<212>  DNA
<213>  Artificial Sequence

<220>
<223>  Primer

<400>  307
catgatcagc tgggccaaga aaagtgtcag                                        30


<210>  308
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  308
ccacaccgta tctg                                                         14


<210>  309
<211>  29
<212>  DNA
<213>  Artificial Sequence

<220>
<223>  Primer

<400>  309
catgatcagc tgggccaaga gaaacccag                                         29


<210>  310
<211>  15
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  310
agctacattg tctgc                                                        15


<210>  311
<211>  28
<212>  DNA
<213>  Artificial Sequence

<220>
<223>  Primer

<400>  311
catgatcagc tgggccaaga gagaccca                                          28


<210>  312
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  312
agctacatct ggct                                                         14


<210>  313
<211>  30
<212>  DNA
<213>  Artificial Sequence

<220>
<223>  Primer

<400>  313
catgatcagc tgggccaaga ggggtatttg                                        30


<210>  314
<211>  15
<212>  DNA
<213>  Artificial Sequence

<220>
<223>  Primer

<220>
<221>  misc_feature
<222>  (3)..(5)
<223>  Nucleotide at position 3 and/or 5 may be modified by
      2'-O,4'-C-methylene-beta-D-ribofuranosyl moiety

<400>  314
tgtcagtttg tcaaa                                                        15


<210>  315
<211>  28
<212>  DNA
<213>  Artificial Sequence

<220>
<223>  Primer

<400>  315
catgatcagc tgggccaaga taaacgga                                          28


<210>  316
<211>  16
<212>  DNA
<213>  Artificial Sequence

<220>
<223>  Primer


<220>
<221>  misc_feature
<222>  (2)..(5)
<223>  Nucleotide at position 2 and/or 5 may be modified by
      2'-O,4'-C-methylene-beta-D-ribofuranosyl moiety

<400>  316
caagtcacta gtggtt                                                       16


<210>  317
<211>  29
<212>  DNA
<213>  Artificial Sequence

<220>
<223>  Primer

<400>  317
catgatcagc tgggccaaga acaggattg                                         29


<210>  318
<211>  15
<212>  DNA
<213>  Artificial Sequence

<220>
<223>  Primer


<220>

<221>  misc_feature
<222>  (2)..(4)
<223>  Nucleotide at position 2 and/or 4 may be modified by
      2'-O,4'-C-methylene-beta-D-ribofuranosyl moiety

<400>  318
agggcccccc ctcaa                                                        15


<210>  319
<211>  29
<212>  DNA
<213>  Artificial Sequence

<220>
<223>  Primer

<400>  319
catgatcagc tgggccaaga atgtatgtg                                         29


<210>  320
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
      2'-O,4'-C-methylene-beta-D-ribofuranosyl moiety

<400>  320
tggtttaccg tccc                                                         14


<210>  321
<211>  30
<212>  DNA
<213>  Artificial Sequence

<220>
<223>  Primer

<400>  321
catgatcagc tgggccaaga gctttgacaa                                        30


<210>  322
<211>  17
<212>  DNA
<213>  Artificial Sequence

<220>
<223>  Primer


<220>
<221>  misc_feature
<222>  (2)..(4)
<223>  Nucleotide at position 2 and/or 4 may be modified by

2′-O,4′-C-methylene-beta-D-ribofuranosyl moiety

<400>  322
cagtgcaata gtattgt                                                    17


<210>  323
<211>  30
<212>  DNA
<213>  Artificial Sequence

<220>
<223>  Primer

<400>  323
catgatcagc tgggccaaga aaagcaagta                                      30


<210>  324
<211>  15
<212>  DNA
<213>  Artificial Sequence

<220>
<223>  Primer


<220>
<221>  misc_feature
<222>  (5)..(7)
<223>  Nucleotide at position 5 and/or 7 may be modified by
       2′-O,4′-C-methylene-beta-D-ribofuranosyl moiety

<400>  324
taaacgtgga tgtac                                                      15


<210>  325
<211>  30
<212>  DNA
<213>  Artificial Sequence

<220>
<223>  Primer

<400>  325
catgatcagc tgggccaaga tcaccaaaac                                      30


<210>  326
<211>  15
<212>  DNA
<213>  Artificial Sequence

<220>
<223>  Primer


<220>
<221>  misc_feature
<222>  (2)..(5)
<223>  Nucleotide at position 2 and/or 5 may be modified by
       2′-O,4′-C-methylene-beta-D-ribofuranosyl moiety

<400>  326

taagtgcttc catgt                                                          15

<210>  327
<211>  30
<212>  DNA
<213>  Artificial Sequence

<220>
<223>  Primer

<400>  327
catgatcagc tgggccaaga agaaagcact                                          30

<210>  328
<211>  17
<212>  DNA
<213>  Artificial Sequence

<220>
<223>  Primer

<220>
<221>  misc_feature
<222>  (2)..(8)
<223>  Nucleotide at position 2 and/or 8 may be modified by
       2'-O,4'-C-methylene-beta-D-ribofuranosyl moiety

<400>  328
actttaacat ggaagtg                                                        17

<210>  329
<211>  30
<212>  DNA
<213>  Artificial Sequence

<220>
<223>  Primer

<400>  329
catgatcagc tgggccaaga ctactaaaac                                          30

<210>  330
<211>  15
<212>  DNA
<213>  Artificial Sequence

<220>
<223>  Primer

<220>
<221>  misc_feature
<222>  (2)..(5)
<223>  Nucleotide at position 2 and/or 5 may be modified by
       2'-O,4'-C-methylene-beta-D-ribofuranosyl moiety

<400>  330
taagtgcttc catgt                                                          15

<210>  331
<211>  30
<212>  DNA
<213>  Artificial Sequence

<220>
<223>  Primer

<400>  331
catgatcagc tgggccaaga acactcaaac                                        30


<210>  332
<211>  15
<212>  DNA
<213>  Artificial Sequence

<220>
<223>  Primer


<220>
<221>  misc_feature
<222>  (2)..(5)
<223>  Nucleotide at position 2 and/or 5 may be modified by
       2'-O,4'-C-methylene-beta-D-ribofuranosyl moiety

<400>  332
taagtgcttc catgt                                                        15


<210>  333
<211>  29
<212>  DNA
<213>  Artificial Sequence

<220>
<223>  Primer

<400>  333
catgatcagc tgggccaaga cagcaggta                                         29


<210>  334
<211>  17
<212>  DNA
<213>  Artificial Sequence

<220>
<223>  Primer


<220>
<221>  misc_feature
<222>  (3)..(9)
<223>  Nucleotide at position 3 and/or 6 and/or 9 may be modified by
       2'-O,4'-C-methylene-beta-D-ribofuranosyl moiety

<400>  334
tttaacatgg gggtacc                                                      17


<210>  335
<211>  29
<212>  DNA

<213>  Artificial Sequence

<220>
<223>  Primer

<400>  335
catgatcagc tgggccaaga ccactgaaa                                          29


<210>  336
<211>  19
<212>  DNA
<213>  Artificial Sequence

<220>
<223>  Primer


<220>
<221>  misc_feature
<222>  (2)..(5)
<223>  Nucleotide at position 2 and/or 5 may be modified by
       2'-O,4'-C-methylene-beta-D-ribofuranosyl moiety

<400>  336
taagtgcttc catgtttca                                                     19


<210>  337
<211>  28
<212>  DNA
<213>  Artificial Sequence

<220>
<223>  Primer

<400>  337
catgatcagc tgggccaaga ttcgccct                                           28


<210>  338
<211>  18
<212>  DNA
<213>  Artificial Sequence

<220>
<223>  Primer


<220>
<221>  misc_feature
<222>  (5)..(8)
<223>  Nucleotide at position 5 and/or 8 may be modified by
       2'-O,4'-C-methylene-beta-D-ribofuranosyl moiety

<400>  338
aaaagctggg ttgagagg                                                      18


<210>  339
<211>  30
<212>  DNA
<213>  Artificial Sequence

<220>

<223>  Primer

<400>  339
catgatcagc tgggccaaga agaggtcgac                                        30


<210>  340
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  340
gcacattaca cggt                                                         14


<210>  341
<211>  30
<212>  DNA
<213>  Artificial Sequence

<220>
<223>  Primer

<400>  341
catgatcagc tgggccaaga ccagcagcac                                        30


<210>  342
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  342
ccactgcccc aggt                                                         14


<210>  343
<211>  30
<212>  DNA
<213>  Artificial Sequence

<220>
<223>  Primer

<400>  343

catgatcagc tgggccaaga acaccaatgc                                    30

<210>  344
<211>  14
<212>  DNA
<213>  Artificial Sequence

<220>
<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  344
cgcatcccct aggg                                                        14

<210>  345
<211>  30
<212>  DNA
<213>  Artificial Sequence

<220>
<223>  Primer

<400>  345
catgatcagc tgggccaaga acacttactg                                    30

<210>  346
<211>  14
<212>  DNA
<213>  Artificial Sequence

<220>
<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  346
cctagtaggt gtcc                                                        14

<210>  347
<211>  30
<212>  DNA
<213>  Artificial Sequence

<220>
<223>  Primer

<400>  347
catgatcagc tgggccaaga ctggaggaag                                    30

<210>  348
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  348
cctctgggcc cttc                                                         14


<210>  349
<211>  30
<212>  DNA
<213>  Artificial Sequence

<220>
<223>  Primer

<400>  349
catgatcagc tgggccaaga acggaagggc                                        30


<210>  350
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  350
ctggccctct ctgc                                                         14


<210>  351
<211>  30
<212>  DNA
<213>  Artificial Sequence

<220>
<223>  Primer

<400>  351
catgatcagc tgggccaaga tctctgcagg                                        30


<210>  352
<211>  14
<212>  DNA

<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  352
gcaaagcaca cggc                                                    14


<210>  353
<211>  30
<212>  DNA
<213>  Artificial Sequence

<220>
<223>  Primer

<400>  353
catgatcagc tgggccaaga ttctaggata                                   30


<210>  354
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  354
gcccctgggc ctat                                                    14


<210>  355
<211>  30
<212>  DNA
<213>  Artificial Sequence

<220>
<223>  Primer

<400>  355
catgatcagc tgggccaaga aaaggcatca                                   30


<210>  356
<211>  15
<212>  DNA
<213>  Artificial Sequence

<220>

<223>  Primer


<220>
<221>  misc_feature
<222>  (2)..(4)
<223>  Nucleotide at position 2 and/or 4 may be modified by
      2'-O,4'-C-methylene-beta-D-ribofuranosyl moiety

<400>  356
tccagctcct atatg                                                    15


<210>  357
<211>  30
<212>  DNA
<213>  Artificial Sequence

<220>
<223>  Primer

<400>  357
catgatcagc tgggccaaga tcaacaaaat                                    30


<210>  358
<211>  17
<212>  DNA
<213>  Artificial Sequence

<220>
<223>  Primer


<220>
<221>  misc_feature
<222>  (2)..(4)
<223>  Nucleotide at position 2 and/or 4 may be modified by
      2'-O,4'-C-methylene-beta-D-ribofuranosyl moiety

<400>  358
tccagcatca gtgattt                                                  17


<210>  359
<211>  29
<212>  DNA
<213>  Artificial Sequence

<220>
<223>  Primer

<400>  359
catgatcagc tgggccaaga tgagctcct                                     29


<210>  360
<211>  15
<212>  DNA
<213>  Artificial Sequence

<220>
<223>  Primer

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  360
tccctgtcct ccagg                                                        15


<210>  361
<211>  30
<212>  DNA
<213>  Artificial Sequence

<220>
<223>  Primer

<400>  361
catgatcagc tgggccaaga ggctataaag                                        30


<210>  362
<211>  14
<212>  DNA
<213>  Artificial Sequence

<220>
<223>  Primer


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  362
tccgtctcag ttac                                                         14


<210>  363
<211>  28
<212>  DNA
<213>  Artificial Sequence

<220>
<223>  Primer

<400>  363
catgatcagc tgggccaaga gacgggtg                                          28


<210>  364
<211>  16
<212>  DNA
<213>  Artificial Sequence

<220>
<223>  Primer


<220>
<221>  misc_feature
<222>  (2)..(4)

<223>  Nucleotide at position 2 and/or 4 may be modified by
       2'-O,4'-C-methylene-beta-D-ribofuranosyl moiety

<400>  364
tctcacacag aaatcg                                                    16


<210>  365
<211>  30
<212>  DNA
<213>  Artificial Sequence

<220>
<223>  Primer

<400>  365
catgatcagc tgggccaaga gccctggact                                     30


<210>  366
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  366
tgctgactcc tagt                                                      14


<210>  367
<211>  30
<212>  DNA
<213>  Artificial Sequence

<220>
<223>  Primer

<400>  367
catgatcagc tgggccaaga agaggcaggc                                     30


<210>  368
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  368
tgtctgcccg catg                                                          14


<210>  369
<211>  30
<212>  DNA
<213>  Artificial Sequence

<220>
<223>  Primer

<400>  369
catgatcagc tgggccaaga tacagatgga                                         30


<210>  370
<211>  15
<212>  DNA
<213>  Artificial Sequence

<220>
<223>  Primer


<220>
<221>  misc_feature
<222>  (4)..(9)
<223>  Nucleotide at position 4 and/or 6 and/or 9 may be modified by
       2'-O,4'-C-methylene-beta-D-ribofuranosyl moiety

<400>  370
aattgcacgg tatcc                                                         15


<210>  371
<211>  30
<212>  DNA
<213>  Artificial Sequence

<220>
<223>  Primer

<400>  371
catgatcagc tgggccaaga tcaccattgc                                         30


<210>  372
<211>  17
<212>  DNA
<213>  Artificial Sequence

<220>
<223>  Primer


<220>
<221>  misc_feature
<222>  (4)..(6)
<223>  Nucleotide at position 4 and/or 6 may be modified by
       2'-O,4'-C-methylene-beta-D-ribofuranosyl moiety

<400>  372
aattgcactt tagcaat                                                       17

<210>  373
<211>  30
<212>  DNA
<213>  Artificial Sequence

<220>
<223>  Primer

<400>  373
catgatcagc tgggccaaga aaacgtggaa                                        30


<210>  374
<211>  18
<212>  DNA
<213>  Artificial Sequence

<220>
<223>  Primer


<220>
<221>  misc_feature
<222>  (2)..(8)
<223>  Nucleotide at position 2 and/or 8 may be modified by
       2'-O,4'-C-methylene-beta-D-ribofuranosyl moiety

<400>  374
acatagagga aattccac                                                     18


<210>  375
<211>  30
<212>  DNA
<213>  Artificial Sequence

<220>
<223>  Primer

<400>  375
catgatcagc tgggccaaga ccaggttcca                                        30


<210>  376
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  376
gcctgctggg gtgg                                                         14


<210>  377
<211>  30

<212>  DNA
<213>  Artificial Sequence

<220>
<223>  Primer

<400>  377
catgatcagc tgggccaaga acactcaaaa                                        30


<210>  378
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  378
gtgccgccat cttt                                                         14


<210>  379
<211>  30
<212>  DNA
<213>  Artificial Sequence

<220>
<223>  Primer

<400>  379
catgatcagc tgggccaaga acgctcaaat                                        30


<210>  380
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  380
aaagtgctgc gaca                                                         14


<210>  381
<211>  30
<212>  DNA
<213>  Artificial Sequence

<220>
<223>  Primer

<400>  381
catgatcagc tgggccaaga ggaaagcgcc                                        30


<210>  382
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  382
actcaaaatg gggg                                                         14


<210>  383
<211>  30
<212>  DNA
<213>  Artificial Sequence

<220>
<223>  Primer

<400>  383
catgatcagc tgggccaaga acaccccaaa                                        30


<210>  384
<211>  18
<212>  DNA
<213>  Artificial Sequence

<220>
<223>  Primer


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  384
gaagtgcttc gattttgg                                                     18


<210>  385
<211>  29
<212>  DNA
<213>  Artificial Sequence

<220>
<223>  Primer

<400>  385
catgatcagc tgggccaaga cacttatca                                          29


<210>  386
<211>  20
<212>  DNA
<213>  Artificial Sequence

<220>
<223>  Primer


<220>
<221>  misc_feature
<222>  (3)..(9)
<223>  Nucleotide at position 3 and/or 5 and/or 9 may be modified by
       2'-O,4'-C-methylene-beta-D-ribofuranosyl moiety

<400>  386
ttataataca acctgataag                                                    20


<210>  387
<211>  30
<212>  DNA
<213>  Artificial Sequence

<220>
<223>  Primer

<400>  387
catgatcagc tgggccaaga tcacgcgagc                                         30


<210>  388
<211>  14
<212>  DNA
<213>  Artificial Sequence

<220>
<223>  Primer


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  388
tttgttcgtt cggc                                                          14


<210>  389
<211>  28
<212>  DNA
<213>  Artificial Sequence

<220>
<223>  Primer

<400>  389
catgatcagc tgggccaaga aacatgga                                           28

<210>  390
<211>  18
<212>  DNA
<213>  Artificial Sequence

<220>
<223>  Primer



<220>
<221>  misc_feature
<222>  (3)..(9)
<223>  Nucleotide at position 3 and/or 6 and/or 9 may be modified by
       2'-O,4'-C-methylene-beta-D-ribofuranosyl moiety

<400>  390
atcatagagg aaaatcca                                                    18


<210>  391
<211>  30
<212>  DNA
<213>  Artificial Sequence

<220>
<223>  Primer

<400>  391
catgatcagc tgggccaaga acacaggacc                                       30


<210>  392
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  392
ctcctgactc cagg                                                        14


<210>  393
<211>  27
<212>  DNA
<213>  Artificial Sequence

<220>
<223>  Primer

<400>  393
catgatcagc tgggccaaga tacgttc                                          27


<210>  394
<211>  17

<212>  DNA
<213>  Artificial Sequence

<220>
<223>  Primer



<220>
<221>  misc_feature
<222>  (2)..(5)
<223>  Nucleotide at position 2 and/or 5 may be modified by
       2'-O,4'-C-methylene-beta-D-ribofuranosyl moiety

<400>  394
tggtagacta tggaacg                                                    17


<210>  395
<211>  30
<212>  DNA
<213>  Artificial Sequence

<220>
<223>  Primer

<400>  395
catgatcagc tgggccaaga gcgcatgttc                                      30


<210>  396
<211>  14
<212>  DNA
<213>  Artificial Sequence

<220>
<223>  Primer



<220>
<221>  misc_feature
<222>  (2)..(5)
<223>  Nucleotide at position 2 and/or 5 may be modified by
       2'-O,4'-C-methylene-beta-D-ribofuranosyl moiety

<400>  396
tggttgacca taga                                                       14


<210>  397
<211>  30
<212>  DNA
<213>  Artificial Sequence

<220>
<223>  Primer

<400>  397
catgatcagc tgggccaaga aagatgtgga                                      30


<210>  398
<211>  18
<212>  DNA
<213>  Artificial Sequence

<220>
<223>  Primer


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  398
tatgtaatat ggtccaca                                                    18


<210>  399
<211>  29
<212>  DNA
<213>  Artificial Sequence

<220>
<223>  Primer

<400>  399
catgatcagc tgggccaaga acagagagc                                        29


<210>  400
<211>  16
<212>  DNA
<213>  Artificial Sequence

<220>
<223>  Primer


<220>
<221>  misc_feature
<222>  (5)..(8)
<223>  Nucleotide at position 5 and/or 8 may be modified by
       2'-O,4'-C-methylene-beta-D-ribofuranosyl moiety

<400>  400
tatacaaggg caagct                                                      16


<210>  401
<211>  30
<212>  DNA
<213>  Artificial Sequence

<220>
<223>  Primer

<400>  401
catgatcagc tgggccaaga cgaatccacc                                       30


<210>  402
<211>  15
<212>  DNA
<213>  Artificial Sequence

<220>
<223>  Primer

<220>
<221>  misc_feature
<222>  (2)..(4)
<223>  Nucleotide at position 2 and/or 4 may be modified by
      2'-O,4'-C-methylene-beta-D-ribofuranosyl moiety

<400>  402
gaagttgttc gtggt                                                        15


<210>  403
<211>  30
<212>  DNA
<213>  Artificial Sequence

<220>
<223>  Primer

<400>  403
catgatcagc tgggccaaga agccacaatc                                        30


<210>  404
<211>  18
<212>  DNA
<213>  Artificial Sequence

<220>
<223>  Primer


<220>
<221>  misc_feature
<222>  (2)..(6)
<223>  Nucleotide at position 2 and/or 6 may be modified by
      2'-O,4'-C-methylene-beta-D-ribofuranosyl moiety

<400>  404
agatcagaag gtgattgt                                                     18


<210>  405
<211>  29
<212>  DNA
<213>  Artificial Sequence

<220>
<223>  Primer

<400>  405
catgatcagc tgggccaaga acaggccat                                         29


<210>  406
<211>  17
<212>  DNA
<213>  Artificial Sequence

<220>
<223>  Primer


<220>
<221>  misc_feature

<222>  (3)..(10)
<223>  Nucleotide at position 3 and/or 5 and/or 8 and/or 10 may be
       modified by 2'-O,4'-C-methylene-beta-D-ribofuranosyl moiety

<400>  406
aatataacac agatggc                                                    17


<210>  407
<211>  30
<212>  DNA
<213>  Artificial Sequence

<220>
<223>  Primer

<400>  407
catgatcagc tgggccaaga acggctagtg                                      30


<210>  408
<211>  18
<212>  DNA
<213>  Artificial Sequence

<220>
<223>  Primer


<220>
<221>  misc_feature
<222>  (2)..(5)
<223>  Nucleotide at position 2 and/or 5 may be modified by
       2'-O,4'-C-methylene-beta-D-ribofuranosyl moiety

<400>  408
acttcacctg gtccacta                                                   18


<210>  409
<211>  30
<212>  DNA
<213>  Artificial Sequence

<220>
<223>  Primer

<400>  409
catgatcagc tgggccaaga ggccttctga                                      30


<210>  410
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  410
ctggacttag ggtc                                                         14


<210>  411
<211>  30
<212>  DNA
<213>  Artificial Sequence

<220>
<223>  Primer

<400>  411
catgatcagc tgggccaaga ggccttctga                                        30


<210>  412
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  412
ctggacttgg agtc                                                         14


<210>  413
<211>  30
<212>  DNA
<213>  Artificial Sequence

<220>
<223>  Primer

<400>  413
catgatcagc tgggccaaga ctgaggggcc                                        30


<210>  414
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  414
agctcggtct gagg                                                         14

<210>  415
<211>  30
<212>  DNA
<213>  Artificial Sequence

<220>
<223>  Primer

<400>  415
catgatcagc tgggccaaga ttcaaaacat                                        30


<210>  416
<211>  19
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  416
cagcagcaat tcatgtttt                                                    19


<210>  417
<211>  30
<212>  DNA
<213>  Artificial Sequence

<220>
<223>  Primer

<400>  417
catgatcagc tgggccaaga ggcggacacg                                        30


<210>  418
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  418
atcgggaatg tcgt                                                         14


<210>  419

<211>  31
<212>  DNA
<213>  Artificial Sequence

<220>
<223>  Primer

<400>  419
catgatcagc tgggccaaga acggttttac c                                    31


<210>  420
<211>  18
<212>  DNA
<213>  Artificial Sequence

<220>
<223>  Primer


<220>
<221>  misc_feature
<222>  (2)..(9)
<223>  Nucleotide at position 2 and/or 7 and/or 9 may be modified by
       2'-O,4'-C-methylene-beta-D-ribofuranosyl moiety

<400>  420
taatactgtc tggtaaaa                                                   18


<210>  421
<211>  30
<212>  DNA
<213>  Artificial Sequence

<220>
<223>  Primer

<400>  421
catgatcagc tgggccaaga tgcatgacgg                                      30


<210>  422
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  422
tgtcttgcag gccg                                                       14


<210>  423
<211>  30
<212>  DNA
<213>  Artificial Sequence

<220>
<223>  Primer

<400>  423
catgatcagc tgggccaaga atgggacatc                                        30


<210>  424
<211>  16
<212>  DNA
<213>  Artificial Sequence

<220>
<223>  Primer


<220>
<221>  misc_feature
<222>  (4)..(8)
<223>  Nucleotide at position 4 and/or 8 may be modified by
       2'-O,4'-C-methylene-beta-D-ribofuranosyl moiety

<400>  424
ttgcatatgt aggatg                                                       16


<210>  425
<211>  30
<212>  DNA
<213>  Artificial Sequence

<220>
<223>  Primer

<400>  425
catgatcagc tgggccaaga accagctaac                                        30


<210>  426
<211>  18
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  426
tggcagtgta ttgttagc                                                     18


<210>  427
<211>  30
<212>  DNA
<213>  Artificial Sequence

<220>
<223>  Primer

<400>  427
catgatcagc tgggccaaga tattaggaac                                        30


<210>  428
<211>  15
<212>  DNA
<213>  Artificial Sequence

<220>
<223>  Primer


<220>
<221>  misc_feature
<222>  (5)..(7)
<223>  Nucleotide at position 5 and/or 7 may be modified by
       2'-O,4'-C-methylene-beta-D-ribofuranosyl moiety

<400>  428
tttttgcgat gtgtt                                                        15


<210>  429
<211>  30
<212>  DNA
<213>  Artificial Sequence

<220>
<223>  Primer

<400>  429
catgatcagc tgggccaaga aaactcagta                                        30


<210>  430
<211>  19
<212>  DNA
<213>  Artificial Sequence

<220>
<223>  Primer


<220>
<221>  misc_feature
<222>  (4)..(10)
<223>  Nucleotide at position 4 and/or 7 and/or 10 may be modified by
       2'-O,4'-C-methylene-beta-D-ribofuranosyl moiety

<400>  430
aaaccgttac cattactga                                                    19


<210>  431
<211>  28
<212>  DNA
<213>  Artificial Sequence

<220>
<223>  Primer

<400>  431
catgatcagc tgggccaaga aactatac                                          28

<210>  432
<211>  15
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  432
tgaggtagta ggttg                                                        15


<210>  433
<211>  28
<212>  DNA
<213>  Artificial Sequence

<220>
<223>  Primer

<400>  433
catgatcagc tgggccaaga aaccacac                                          28


<210>  434
<211>  28
<212>  DNA
<213>  Artificial Sequence

<220>
<223>  Primer

<400>  434
catgatcagc tgggccaaga aaccatac                                          28


<210>  435
<211>  28
<212>  DNA
<213>  Artificial Sequence

<220>
<223>  Primer

<400>  435
catgatcagc tgggccaaga actatgca                                          28


<210>  436
<211>  15
<212>  DNA
<213>  Artificial Sequence

<220>
<223>  Primer

<220>
<221>  misc_feature
<222>  (2)..(4)
<223>  Nucleotide at position 2 and/or 4 may be modified by
      2'-O,4'-C-methylene-beta-D-ribofuranosyl moiety

<400>  436
agaggtagta ggttg                                                        15


<210>  437
<211>  28
<212>  DNA
<213>  Artificial Sequence

<220>
<223>  Primer

<400>  437
catgatcagc tgggccaaga actataca                                          28


<210>  438
<211>  15
<212>  DNA
<213>  Artificial Sequence

<220>
<223>  Primer


<220>
<221>  misc_feature
<222>  (2)..(4)
<223>  Nucleotide at position 2 and/or 4 may be modified by
      2'-O,4'-C-methylene-beta-D-ribofuranosyl moiety

<400>  438
tgaggtagga ggttg                                                        15


<210>  439
<211>  28
<212>  DNA
<213>  Artificial Sequence

<220>
<223>  Primer

<400>  439
catgatcagc tgggccaaga aactatac                                          28


<210>  440
<211>  15
<212>  DNA
<213>  Artificial Sequence

<220>
<223>  Primer


<220>
<221>  misc_feature

<222>  (2)..(4)
<223>  Nucleotide at position 2 and/or 4 may be modified by
       2'-O,4'-C-methylene-beta-D-ribofuranosyl moiety

<400>  440
tgaggtagta gattg                                                        15


<210>  441
<211>  28
<212>  DNA
<213>  Artificial Sequence

<220>
<223>  Primer

<400>  441
catgatcagc tgggccaaga actgtaca                                          28


<210>  442
<211>  15
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  442
tgaggtagta gtttg                                                        15


<210>  443
<211>  28
<212>  DNA
<213>  Artificial Sequence

<220>
<223>  Primer

<400>  443
catgatcagc tgggccaaga acagcaca                                          28


<210>  444
<211>  15
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  444
tgaggtagta gtttg                                                        15


<210>  445
<211>  30
<212>  DNA
<213>  Artificial Sequence

<220>
<223>  Primer

<400>  445
catgatcagc tgggccaaga acaaaagttg                                        30


<210>  446
<211>  16
<212>  DNA
<213>  Artificial Sequence

<220>
<223>  Primer


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  446
atcacacaaa ggcaac                                                       16


<210>  447
<211>  27
<212>  DNA
<213>  Artificial Sequence

<220>
<223>  Primer

<400>  447
catgatcagc tgggccaaga acgtgga                                           27


<210>  448
<211>  17
<212>  DNA
<213>  Artificial Sequence

<220>
<223>  Primer


<220>
<221>  misc_feature
<222>  (3)..(9)
<223>  Nucleotide at position 3, 6 and/or 9 may be modified by
       2'-O,4'-C-methylene-beta-D-ribofuranosyl moiety

<220>
<221>  misc_feature

<222>  (3)..(9)
<223>  Nucleotide at position 3 and/or 6 and/or 9 may be modified by
       2'-O,4'-C-methylene-beta-D-ribofuranosyl moiety

<400>  448
atcatagagg aaaatcc                                                    17


<210>  449
<211>  30
<212>  DNA
<213>  Artificial Sequence

<220>
<223>  Primer

<400>  449
catgatcagc tgggccaaga acagttcttc                                      30


<210>  450
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  450
aagctgccag ttga                                                       14


<210>  451
<211>  29
<212>  DNA
<213>  Artificial Sequence

<220>
<223>  Primer

<400>  451
catgatcagc tgggccaaga ccatcatta                                       29


<210>  452
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  452
taatactgcc gggt                                                         14


<210>  453
<211>  30
<212>  DNA
<213>  Artificial Sequence

<220>
<223>  Primer

<400>  453
catgatcagc tgggccaaga ctgttcctgc                                        30


<210>  454
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  454
tggctcagtt cagc                                                         14


<210>  455
<211>  30
<212>  DNA
<213>  Artificial Sequence

<220>
<223>  Primer

<400>  455
catgatcagc tgggccaaga accgatttca                                        30


<210>  456
<211>  16
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  456
tagcaccatt tgaaat                                                       16

<210>  457
<211>  30
<212>  DNA
<213>  Artificial Sequence

<220>
<223>  Primer

<400>  457
catgatcagc tgggccaaga tatctgcact                                        30


<210>  458
<211>  15
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  458
taaggtgcat ctagt                                                        15


<210>  459
<211>  30
<212>  DNA
<213>  Artificial Sequence

<220>
<223>  Primer

<400>  459
catgatcagc tgggccaaga gaactgcctt                                        30


<210>  460
<211>  14
<212>  DNA
<213>  Artificial Sequence

<220>
<223>  Primer



<220>
<221>  misc_feature
<222>  (2)..(4)
<223>  Nucleotide at position 2 and/or 4 may be modified by
       2'-O,4'-C-methylene-beta-D-ribofuranosyl moiety

<400>  460
tggagagaaa ggca                                                         14


<210>  461

<211>  28
<212>  DNA
<213>  Artificial Sequence

<220>
<223>  Primer

<400>  461
catgatcagc tgggccaaga cccaccga                                          28


<210>  462
<211>  16
<212>  DNA
<213>  Artificial Sequence

<220>
<223>  Primer


<220>
<221>  misc_feature
<222>  (3)..(7)
<223>  Nucleotide at position 3 and/or 7 may be modified by
       2'-O,4'-C-methylene-beta-D-ribofuranosyl moiety

<400>  462
aacattcatt gctgtc                                                       16


<210>  463
<211>  30
<212>  DNA
<213>  Artificial Sequence

<220>
<223>  Primer

<400>  463
catgatcagc tgggccaaga acaagtgccc                                        30


<210>  464
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  464
actgcagtga gggc                                                         14


<210>  465
<211>  30
<212>  DNA
<213>  Artificial Sequence

<220>
<223>  Primer

<400>  465
catgatcagc tgggccaaga agccacagtc                                        30


<210>  466
<211>  18
<212>  DNA
<213>  Artificial Sequence

<220>
<223>  Primer


<220>
<221>  misc_feature
<222>  (2)..(6)
<223>  Nucleotide at position 2 and/or 6 may be modified by
       2'-O,4'-C-methylene-beta-D-ribofuranosyl moiety

<400>  466
agatcagaag gtgactgt                                                     18


<210>  467
<211>  28
<212>  DNA
<213>  Artificial Sequence

<220>
<223>  Primer

<400>  467
catgatcagc tgggccaaga acgtggat                                          28


<210>  468
<211>  19
<212>  DNA
<213>  Artificial Sequence

<220>
<223>  Primer


<220>
<221>  misc_feature
<222>  (2)..(9)
<223>  Nucleotide at position 2 and/or 9 may be modified by
       2'-O,4'-C-methylene-beta-D-ribofuranosyl moiety

<400>  468
atcgtagagg aaaatccac                                                    19


<210>  469
<211>  17
<212>  DNA
<213>  Artificial Sequence

<220>
<223>  Primer

<220>
<221>  misc_feature
<222>  (2)..(5)
<223>  Nucleotide at position 2 and/or 5 may be modified by
      2'-O,4'-C-methylene-beta-D-ribofuranosyl moiety

<400>  469
atgacctatg atttgac                                                    17


<210>  470
<211>  29
<212>  DNA
<213>  Artificial Sequence

<220>
<223>  Primer

<400>  470
catgatcagc tgggccaaga tgtgaacaa                                       29


<210>  471
<211>  17
<212>  DNA
<213>  Artificial Sequence

<220>
<223>  Primer


<220>
<221>  misc_feature
<222>  (4)..(9)
<223>  Nucleotide at position 4 and/or 7 and/or 9 may be modified by
      2'-O,4'-C-methylene-beta-D-ribofuranosyl moiety

<400>  471
attcctagaa attgttc                                                    17


<210>  472
<211>  29
<212>  DNA
<213>  Artificial Sequence

<220>
<223>  Primer

<400>  472
catgatcagc tgggccaaga tacctgcac                                       29


<210>  473
<211>  16
<212>  DNA
<213>  Artificial Sequence

<220>
<223>  Primer


<220>

<221>  misc_feature
<222>  (4)..(6)
<223>  Nucleotide at position 4 and/or 6 may be modified by
       2'-O,4'-C-methylene-beta-D-ribofuranosyl moiety

<400>  473
caaagtgcta acagtg                                                       16


<210>  474
<211>  30
<212>  DNA
<213>  Artificial Sequence

<220>
<223>  Primer

<400>  474
catgatcagc tgggccaaga tccaaaacat                                        30


<210>  475
<211>  30
<212>  DNA
<213>  Artificial Sequence

<220>
<223>  Primer

<400>  475
catgatcagc tgggccaaga gaacaggtag                                        30


<210>  476
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  476
cctagtaggt gctc                                                         14


<210>  477
<211>  15
<212>  DNA
<213>  Artificial Sequence

<220>
<223>  Primer


<220>
<221>  misc_feature
<222>  (2)..(4)
<223>  Nucleotide at position 2 and/or 4 may be modified by

2'-O,4'-C-methylene-beta-D-ribofuranosyl moiety

<400> 477
actagactga ggctc 15

<210> 478
<211> 14
<212> DNA
<213> Artificial Sequence

<220>
<223> Primer

<220>
<221> misc_feature
<222> (2)..(4)
<223> Nucleotide at position 2 and/or 4 may be modified by
2'-O,4'-C-methylene-beta-D-ribofuranosyl moiety

<400> 478
gcaaagcaca gggc 14

<210> 479
<211> 31
<212> DNA
<213> Artificial Sequence

<220>
<223> Primer

<400> 479
catgatcagc tgggccaaga acggcattac c 31

<210> 480
<211> 18
<212> DNA
<213> Artificial Sequence

<220>
<223> Primer

<220>
<221> misc_feature
<222> (2)..(9)
<223> Nucleotide at position 2 and/or 7 and/or 9 may be modified by
2'-O,4'-C-methylene-beta-D-ribofuranosyl moiety

<400> 480
taatactgtc tggtaatg 18

<210> 481
<211> 29
<212> DNA
<213> Artificial Sequence

<220>
<223> Primer

<400> 481

catgatcagc tgggccaaga atccagtca                                    29

<210>  482
<211>  14
<212>  DNA
<213>  Artificial Sequence

<220>
<223>  Primer

<220>
<221>  misc_feature
<222>  (3)..(5)
<223>  Nucleotide at position 3 and/or 5 may be modified by
       2'-O,4'-C-methylene-beta-D-ribofuranosyl moiety

<400>  482
taggcagtgt aatt                                                   14

<210>  483
<211>  18
<212>  DNA
<213>  Artificial Sequence

<220>
<223>  Primer

<220>
<221>  misc_feature
<222>  (3)..(5)
<223>  Nucleotide at position 3 and/or 5 may be modified by
       2'-O,4'-C-methylene-beta-D-ribofuranosyl moiety

<400>  483
tatgtagtat ggtccaca                                               18

<210>  484
<211>  30
<212>  DNA
<213>  Artificial Sequence

<220>
<223>  Primer

<400>  484
catgatcagc tgggccaaga gcactggact                                  30

<210>  485
<211>  14
<212>  DNA
<213>  Artificial Sequence

<220>
<223>  Primer

<220>
<221>  misc_feature
<222>  (2)..(4)

<223>  Nucleotide at position 2 and/or 4 may be modified by
      2'-O,4'-C-methylene-beta-D-ribofuranosyl moiety

<400>  485
tgctgacccc tagt                                                    14


<210>  486
<211>  29
<212>  DNA
<213>  Artificial Sequence

<220>
<223>  Primer

<400>  486
catgatcagc tgggccaaga aacaaaatc                                    29


<210>  487
<211>  18
<212>  DNA
<213>  Artificial Sequence

<220>
<223>  Primer


<220>
<221>  misc_feature
<222>  (2)..(6)
<223>  Nucleotide at position 2 and/or 6 may be modified by
      2'-O,4'-C-methylene-beta-D-ribofuranosyl moiety

<400>  487
tggaagactt gtgatttt                                                18


<210>  488
<211>  14
<212>  DNA
<213>  Artificial Sequence

<220>
<223>  Primer


<220>
<221>  misc_feature
<222>  (2)..(4)
<223>  Nucleotide at position 2 and/or 4 may be modified by
      2'-O,4'-C-methylene-beta-D-ribofuranosyl moiety

<400>  488
tgtctgcccg agtg                                                    14


<210>  489
<211>  20
<212>  DNA
<213>  Artificial Sequence

<220>
<223>  Primer

<220>
<221>  misc_feature
<222>  (3)..(5)
<223>  Nucleotide at position 3 and/or 5 may be modified by
       2'-O,4'-C-methylene-beta-D-ribofuranosyl moiety

<400>  489
ttaatgctaa ttgtgatagg                                                   20


<210>  490
<211>  15
<212>  DNA
<213>  Artificial Sequence

<220>
<223>  Primer


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  490
ttcagctcct atatg                                                        15


<210>  491
<211>  29
<212>  DNA
<213>  Artificial Sequence

<220>
<223>  Primer

<400>  491
catgatcagc tgggccaaga aggcaaagg                                         29


<210>  492
<211>  31
<212>  DNA
<213>  Artificial Sequence

<220>
<223>  Primer

<400>  492
catgatcagc tgggccaaga acacaaattc g                                      31


<210>  493
<211>  16
<212>  DNA
<213>  Artificial Sequence

<220>
<223>  Primer


<220>
<221>  misc_feature

<222>  (2)..(7)
<223>  Nucleotide at position 2 and/or 4 and/or 7 may be modified by
       2'-O,4'-C-methylene-beta-D-ribofuranosyl moiety

<400>  493
ccctgtagaa ccgaat                                                    16


<210>  494
<211>  15
<212>  DNA
<213>  Artificial Sequence

<220>
<223>  Primer

<400>  494
tcacagtgaa ccggt                                                     15


<210>  495
<211>  15
<212>  DNA
<213>  Artificial Sequence

<220>
<223>  Primer

<400>  495
agctggtgtt gtgaa                                                     15


<210>  496
<211>  16
<212>  DNA
<213>  Artificial Sequence

<220>
<223>  Primer

<400>  496
tgagatgaag cactgt                                                    16


<210>  497
<211>  16
<212>  DNA
<213>  Artificial Sequence

<220>
<223>  Primer

<400>  497
tctcccaacc cttgta                                                    16


<210>  498
<211>  15
<212>  DNA
<213>  Artificial Sequence

<220>
<223>  Primer

<400>  498

aacattcaac gctgt 15

<210> 499
<211> 16
<212> DNA
<213> Artificial Sequence

<220>
<223> Primer

<400> 499
tgtaacagca actcca 16

<210> 500
<211> 30
<212> DNA
<213> Artificial Sequence

<220>
<223> Synthetic

<400> 500
catgatcagc tgggccaaga aagaagtgca 30

<210> 501
<211> 29
<212> DNA
<213> Artificial Sequence

<220>
<223> Synthetic

<400> 501
catgatcagc tgggccaaga aagaagtgc 29

<210> 502
<211> 28
<212> DNA
<213> Artificial Sequence

<220>
<223> Synthetic

<400> 502
catgatcagc tgggccaaga aagaagtg 28

<210> 503
<211> 27
<212> DNA
<213> Artificial Sequence

<220>
<223> Synthetic

<400> 503
catgatcagc tgggccaaga aagaagt 27

<210> 504
<211> 17

<212>  DNA
<213>  Artificial Sequence

<220>
<223>  Synthetic


<220>
<221>  misc_feature
<222>  (4)..(10)
<223>  Nucleotide at position 4 and/or 8 and/or 10 may be modified by
       2'-O,4'-C-methylene-beta-D-ribofuranosyl moiety

<400>  504
aaacaaacat ggtgcac                                                    17


<210>  505
<211>  15
<212>  DNA
<213>  Artificial Sequence

<220>
<223>  Synthetic


<220>
<221>  misc_feature
<222>  (4)..(8)
<223>  Nucleotide at position 4 and/or 5 and/or 8 may be modified by
       2'-O,4'-C-methylene-beta-D-ribofuranosyl moiety

<400>  505
aaacaaacat ggtgc                                                      15


<210>  506
<211>  30
<212>  DNA
<213>  Artificial Sequence

<220>
<223>  Synthetic

<400>  506
catgatcagc tgggccaaga ggcacacaaa                                      30


<210>  507
<211>  29
<212>  DNA
<213>  Artificial Sequence

<220>
<223>  Synthetic

<400>  507
catgatcagc tgggccaaga ggcacacaa                                       29


<210>  508
<211>  28
<212>  DNA
<213>  Artificial Sequence

<220>
<223>  Synthetic

<400>  508
catgatcagc tgggccaaga ggcacaca                                          28


<210>  509
<211>  27
<212>  DNA
<213>  Artificial Sequence

<220>
<223>  Synthetic

<400>  509
catgatcagc tgggccaaga ggcacac                                           27


<210>  510
<211>  18
<212>  DNA
<213>  Artificial Sequence

<220>
<223>  Synthetic


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  510
aaagtgcttc cactttgt                                                     18


<210>  511
<211>  16
<212>  DNA
<213>  Artificial Sequence

<220>
<223>  Synthetic


<220>
<221>  misc_feature
<222>  (3)..(7)
<223>  Nucleotide at position 3 and/or 5 and/or 7 may be modified by
       2'-O,4'-C-methylene-beta-D-ribofuranosyl moiety

<400>  511
aaagtgcttc cacttt                                                       16


<210>  512
<211>  30
<212>  DNA
<213>  Artificial Sequence

<220>
<223>  Synthetic

<400>  512
catgatcagc tgggccaaga gacaaacaaa                                        30


<210>  513
<211>  29
<212>  DNA
<213>  Artificial Sequence

<220>
<223>  Synthetic

<400>  513
catgatcagc tgggccaaga gacaaacaa                                         29


<210>  514
<211>  28
<212>  DNA
<213>  Artificial Sequence

<220>
<223>  Synthetic

<400>  514
catgatcagc tgggccaaga gacaaaca                                          28


<210>  515
<211>  27
<212>  DNA
<213>  Artificial Sequence

<220>
<223>  Synthetic

<400>  515
catgatcagc tgggccaaga gacaaac                                           27


<210>  516
<211>  18
<212>  DNA
<213>  Artificial Sequence

<220>
<223>  Synthetic


<220>
<221>  misc_feature
<222>  (3)..(10)
<223>  Nucleotide at position 3 and/or 5 and/or 7 and/or 10 may be
       modified by 2'-O,4'-C-methylene-beta-D-ribofuranosyl moiety

<400>  516
aaagtgcatc cattttgt                                                     18


<210>  517
<211>  16
<212>  DNA
<213>  Artificial Sequence

<220>

<223>  Synthetic

<220>
<221>  misc_feature
<222>  (3)..(7)
<223>  Nucleotide at position 3 and/or 5 and/or 7 may be modified by
       2'-O,4'-C-methylene-beta-D-ribofuranosyl moiety

<400>  517
aaagtgcatc catttt                                                    16

<210>  518
<211>  30
<212>  DNA
<213>  Artificial Sequence

<220>
<223>  Synthetic

<400>  518
catgatcagc tgggccaaga gtaacactct                                     30

<210>  519
<211>  29
<212>  DNA
<213>  Artificial Sequence

<220>
<223>  Synthetic

<400>  519
catgatcagc tgggccaaga gtaacactc                                      29

<210>  520
<211>  28
<212>  DNA
<213>  Artificial Sequence

<220>
<223>  Synthetic

<400>  520
catgatcagc tgggccaaga gtaacact                                       28

<210>  521
<211>  27
<212>  DNA
<213>  Artificial Sequence

<220>
<223>  Synthetic

<400>  521
catgatcagc tgggccaaga gtaacac                                        27

<210>  522
<211>  20
<212>  DNA
<213>  Artificial Sequence

<220>
<223>  Synthetic


<220>
<221>  misc_feature
<222>  (3)..(7)
<223>  Nucleotide at position 3 and/or 5 and/or 7 may be modified by
      2'-O,4'-C-methylene-beta-D-ribofuranosyl moiety

<400>  522
aaagtgcatc cttttagagt                                                   20


<210>  523
<211>  18
<212>  DNA
<213>  Artificial Sequence

<220>
<223>  Synthetic


<220>
<221>  misc_feature
<222>  (3)..(7)
<223>  Nucleotide at position 3 and/or 5 and/or 7 may be modified by
      2'-O,4'-C-methylene-beta-D-ribofuranosyl moiety

<400>  523
aaagtgcatc cttttaga                                                     18


<210>  524
<211>  30
<212>  DNA
<213>  Artificial Sequence

<220>
<223>  Synthetic

<400>  524
catgatcagc tgggccaaga aaacctctaa                                        30


<210>  525
<211>  29
<212>  DNA
<213>  Artificial Sequence

<220>
<223>  Synthetic

<400>  525
catgatcagc tgggccaaga aaacctcta                                         29


<210>  526
<211>  28
<212>  DNA
<213>  Artificial Sequence

<220>
<223>  Synthetic

<400>  526
catgatcagc tgggccaaga aaacctct                                          28


<210>  527
<211>  27
<212>  DNA
<213>  Artificial Sequence

<220>
<223>  Synthetic

<400>  527
catgatcagc tgggccaaga aaacctc                                           27


<210>  528
<211>  17
<212>  DNA
<213>  Artificial Sequence

<220>
<223>  Synthetic


<220>
<221>  misc_feature
<222>  (3)..(7)
<223>  Nucleotide at position 3 and/or 5 and/or 7 may be modified by
       2'-O,4'-C-methylene-beta-D-ribofuranosyl moiety

<400>  528
aaagtgcatc cttttag                                                      17


<210>  529
<211>  15
<212>  DNA
<213>  Artificial Sequence

<220>
<223>  Synthetic


<220>
<221>  misc_feature
<222>  (3)..(7)
<223>  Nucleotide at position 3 and/or 5 and/or 7 may be modified by
       2'-O,4'-C-methylene-beta-D-ribofuranosyl moiety

<400>  529
aaagtgcatc ctttt                                                        15


<210>  530
<211>  30
<212>  DNA
<213>  Artificial Sequence

<220>
<223>  Synthetic

<400>  530
catgatcagc tgggccaaga atcctctaaa                                        30

<210>  531
<211>  29
<212>  DNA
<213>  Artificial Sequence

<220>
<223>  Synthetic

<400>  531
catgatcagc tgggccaaga atcctctaa                                         29


<210>  532
<211>  28
<212>  DNA
<213>  Artificial Sequence

<220>
<223>  Synthetic

<400>  532
catgatcagc tgggccaaga atcctcta                                          28


<210>  533
<211>  27
<212>  DNA
<213>  Artificial Sequence

<220>
<223>  Synthetic

<400>  533
catgatcagc tgggccaaga atcctct                                           27


<210>  534
<211>  18
<212>  DNA
<213>  Artificial Sequence

<220>
<223>  Synthetic


<220>
<221>  misc_feature
<222>  (3)..(7)
<223>  Nucleotide at position 3 and/or 5 and/or 7 may be modified by
       2'-O,4'-C-methylene-beta-D-ribofuranosyl moiety

<400>  534
aaagtgcatc tttttaga                                                     18


<210>  535
<211>  16
<212>  DNA
<213>  Artificial Sequence

<220>
<223>  Synthetic

<220>
<221>  misc_feature
<222>  (3)..(7)
<223>  Nucleotide at position 3 and/or 5 and/or 7 may be modified by
      2'-O,4'-C-methylene-beta-D-ribofuranosyl moiety

<400>  535
aaagtgcatc ttttta                                                       16


<210>  536
<211>  30
<212>  DNA
<213>  Artificial Sequence

<220>
<223>  Synthetic

<400>  536
catgatcagc tgggccaaga acactctaaa                                        30


<210>  537
<211>  29
<212>  DNA
<213>  Artificial Sequence

<220>
<223>  Synthetic

<400>  537
catgatcagc tgggccaaga acactctaa                                         29


<210>  538
<211>  28
<212>  DNA
<213>  Artificial Sequence

<220>
<223>  Synthetic

<400>  538
catgatcagc tgggccaaga acactcta                                          28


<210>  539
<211>  27
<212>  DNA
<213>  Artificial Sequence

<220>
<223>  Synthetic

<400>  539
catgatcagc tgggccaaga acactct                                           27


<210>  540
<211>  18
<212>  DNA
<213>  Artificial Sequence

<220>

<223>  Synthetic

<220>
<221>  misc_feature
<222>  (2)..(6)
<223>  Nucleotide at position 2 and/or 6 may be modified by
       2'-O,4'-C-methylene-beta-D-ribofuranosyl moiety

<400>  540
caaagtgcct ccctttag                                                    18

<210>  541
<211>  16
<212>  DNA
<213>  Artificial Sequence

<220>
<223>  Synthetic

<220>
<221>  misc_feature
<222>  (2)..(6)
<223>  Nucleotide at position 2 and/or 4 and/or 6 may be modified by
       2'-O,4'-C-methylene-beta-D-ribofuranosyl moiety

<400>  541
caaagtgcct cccttt                                                      16

<210>  542
<211>  30
<212>  DNA
<213>  Artificial Sequence

<220>
<223>  Synthetic

<400>  542
catgatcagc tgggccaaga acagtccaaa                                       30

<210>  543
<211>  29
<212>  DNA
<213>  Artificial Sequence

<220>
<223>  Synthetic

<400>  543
catgatcagc tgggccaaga acagtccaa                                        29

<210>  544
<211>  28
<212>  DNA
<213>  Artificial Sequence

<220>
<223>  Synthetic

<400>  544

catgatcagc tgggccaaga acagtcca                                          28

<210>  545
<211>  27
<212>  DNA
<213>  Artificial Sequence

<220>
<223>  Synthetic

<400>  545
catgatcagc tgggccaaga acagtcc                                           27

<210>  546
<211>  17
<212>  DNA
<213>  Artificial Sequence

<220>
<223>  Synthetic

<220>
<221>  misc_feature
<222>  (3)..(5)
<223>  Nucleotide at position 3 and/or 5 may be modified by
       2'-O,4'-C-methylene-beta-D-ribofuranosyl moiety

<400>  546
aaagtgcttc cctttgg                                                      17

<210>  547
<211>  15
<212>  DNA
<213>  Artificial Sequence

<220>
<223>  Synthetic

<220>
<221>  misc_feature
<222>  (3)..(6)
<223>  Nucleotide at position 3 and/or 5 and/or 6 may be modified by
       2'-O,4'-C-methylene-beta-D-ribofuranosyl moiety

<400>  547
aaagtgcttc ccttt                                                        15

<210>  548
<211>  30
<212>  DNA
<213>  Artificial Sequence

<220>
<223>  Synthetic

<400>  548
catgatcagc tgggccaaga ccctctaaaa                                        30

<210>  549
<211>  29
<212>  DNA
<213>  Artificial Sequence

<220>
<223>  Synthetic

<400>  549
catgatcagc tgggccaaga ccctctaaa                                          29


<210>  550
<211>  28
<212>  DNA
<213>  Artificial Sequence

<220>
<223>  Synthetic

<400>  550
catgatcagc tgggccaaga ccctctaa                                           28


<210>  551
<211>  27
<212>  DNA
<213>  Artificial Sequence

<220>
<223>  Synthetic

<400>  551
catgatcagc tgggccaaga ccctcta                                            27


<210>  552
<211>  17
<212>  DNA
<213>  Artificial Sequence

<220>
<223>  Synthetic


<220>
<221>  misc_feature
<222>  (3)..(6)
<223>  Nucleotide at position 3 and/or 5 and/or 6 may be modified by
       2'-O,4'-C-methylene-beta-D-ribofuranosyl moiety

<400>  552
aaagtgcttc cttttag                                                       17


<210>  553
<211>  16
<212>  DNA
<213>  Artificial Sequence

<220>
<223>  Synthetic


<220>

<221>  misc_feature
<222>  (3)..(7)
<223>  Nucleotide at position 3 and/or 5 and/or 7 may be modified by
       2'-O,4'-C-methylene-beta-D-ribofuranosyl moiety

<400>  553
aaagtgcttc ctttta                                                       16


<210>  554
<211>  30
<212>  DNA
<213>  Artificial Sequence

<220>
<223>  Synthetic

<400>  554
catgatcagc tgggccaaga aacccaccaa                                        30


<210>  555
<211>  29
<212>  DNA
<213>  Artificial Sequence

<220>
<223>  Synthetic

<400>  555
catgatcagc tgggccaaga aacccacca                                         29


<210>  556
<211>  28
<212>  DNA
<213>  Artificial Sequence

<220>
<223>  Synthetic

<400>  556
catgatcagc tgggccaaga aacccacc                                          28


<210>  557
<211>  27
<212>  DNA
<213>  Artificial Sequence

<220>
<223>  Synthetic

<400>  557
catgatcagc tgggccaaga aacccac                                           27


<210>  558
<211>  18
<212>  DNA
<213>  Artificial Sequence

<220>
<223>  Synthetic

<220>
<221>  misc_feature
<222>  (3)..(5)
<223>  Nucleotide at position 3 and/or 5 may be modified by
       2'-O,4'-C-methylene-beta-D-ribofuranosyl moiety

<400>  558
aaagtgcttc tctttggt                                                    18


<210>  559
<211>  16
<212>  DNA
<213>  Artificial Sequence

<220>
<223>  Synthetic


<220>
<221>  misc_feature
<222>  (3)..(7)
<223>  Nucleotide at position 3 and/or 5 and/or 7 may be modified by
       2'-O,4'-C-methylene-beta-D-ribofuranosyl moiety

<400>  559
aaagtgcttc tctttg                                                      16


<210>  560
<211>  30
<212>  DNA
<213>  Artificial Sequence

<220>
<223>  Synthetic

<400>  560
catgatcagc tgggccaaga ccctcaaaaa                                       30


<210>  561
<211>  29
<212>  DNA
<213>  Artificial Sequence

<220>
<223>  Synthetic

<400>  561
catgatcagc tgggccaaga ccctcaaaa                                        29


<210>  562
<211>  28
<212>  DNA
<213>  Artificial Sequence

<220>
<223>  Synthetic

<400>  562
catgatcagc tgggccaaga ccctcaaa                                         28

<210>  563
<211>  27
<212>  DNA
<213>  Artificial Sequence

<220>
<223>  Synthetic

<400>  563
catgatcagc tgggccaaga ccctcaa                                          27


<210>  564
<211>  17
<212>  DNA
<213>  Artificial Sequence

<220>
<223>  Synthetic


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  564
   aaagtgcttc ctttttg                                                      17


<210>  565
<211>  16
<212>  DNA
<213>  Artificial Sequence

<220>
<223>  Synthetic


<220>
<221>  misc_feature
<222>  (3)..(6)
<223>  Nucleotide at position 3 and/or 5 and/or 6 may be modified by
       2'-O,4'-C-methylene-beta-D-ribofuranosyl moiety

<400>  565
aaagtgcttc cttttt                                                      16


<210>  566
<211>  30
<212>  DNA
<213>  Artificial Sequence

<220>
<223>  Synthetic

<400>  566
catgatcagc tgggccaaga aaccctctaa                                       30


<210>  567
<211>  29

<212>  DNA
<213>  Artificial Sequence

<220>
<223>  Synthetic

<400>  567
catgatcagc tgggccaaga aaccctcta                                         29


<210>  568
<211>  28
<212>  DNA
<213>  Artificial Sequence

<220>
<223>  Synthetic

<400>  568
catgatcagc tgggccaaga aaccctct                                          28


<210>  569
<211>  27
<212>  DNA
<213>  Artificial Sequence

<220>
<223>  Synthetic

<400>  569
catgatcagc tgggccaaga aaccctc                                           27


<210>  570
<211>  17
<212>  DNA
<213>  Artificial Sequence

<220>
<223>  Synthetic


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  570
aagtgcttcc ttttaga                                                      17


<210>  571
<211>  15
<212>  DNA
<213>  Artificial Sequence

<220>
<223>  Synthetic


<220>
<221>  misc_feature
<222>  (2)..(5)

<223>  Nucleotide at position 2 and/or 4 and/or 5 may be modified by
      2'-O,4'-C-methylene-beta-D-ribofuranosyl moiety

<400>  571
aagtgcttcc tttta                                                    15


<210>  572
<211>  30
<212>  DNA
<213>  Artificial Sequence

<220>
<223>  Synthetic

<400>  572
catgatcagc tgggccaaga aaaaaggtta                                    30


<210>  573
<211>  29
<212>  DNA
<213>  Artificial Sequence

<220>
<223>  Synthetic

<400>  573
catgatcagc tgggccaaga aaaaaggtt                                     29


<210>  574
<211>  28
<212>  DNA
<213>  Artificial Sequence

<220>
<223>  Synthetic

<400>  574
catgatcagc tgggccaaga aaaaaggt                                      28


<210>  575
<211>  27
<212>  DNA
<213>  Artificial Sequence

<220>
<223>  Synthetic

<400>  575
catgatcagc tgggccaaga aaaaagg                                       27


<210>  576
<211>  17
<212>  DNA
<213>  Artificial Sequence

<220>
<223>  Synthetic


<220>

<221>  misc_feature
<222>  (3)..(5)
<223>  Nucleotide at position 3 and/or 5 may be modified by
       2'-O,4'-C-methylene-beta-D-ribofuranosyl moiety

<400>  576
aacacaccca gctaacc                                                    17


<210>  577
<211>  15
<212>  DNA
<213>  Artificial Sequence

<220>
<223>  Synthetic


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  577
aacacaccca gctaa                                                      15


<210>  578
<211>  30
<212>  DNA
<213>  Artificial Sequence

<220>
<223>  Synthetic

<400>  578
catgatcagc tgggccaaga aacccaccga                                      30


<210>  579
<211>  29
<212>  DNA
<213>  Artificial Sequence

<220>
<223>  Synthetic

<400>  579
catgatcagc tgggccaaga aacccaccg                                       29


<210>  580
<211>  28
<212>  DNA
<213>  Artificial Sequence

<220>
<223>  Synthetic

<400>  580
catgatcagc tgggccaaga aacccacc                                        28


<210>  581

<211>  27
<212>  DNA
<213>  Artificial Sequence

<220>
<223>  Synthetic

<400>  581
catgatcagc tgggccaaga aacccac                                          27


<210>  582
<211>  19
<212>  DNA
<213>  Artificial Sequence

<220>
<223>  Synthetic


<220>
<221>  misc_feature
<222>  (3)..(7)
<223>  Nucleotide at position 3 and/or 7 may be modified by
       2'-O,4'-C-methylene-beta-D-ribofuranosyl moiety

<400>  582
aacattcatt gttgtcggt                                                   19


<210>  583
<211>  17
<212>  DNA
<213>  Artificial Sequence

<220>
<223>  Synthetic


<220>
<221>  misc_feature
<222>  (3)..(7)
<223>  Nucleotide at position 3 and/or 5 and/or 7 may be modified by
       2'-O,4'-C-methylene-beta-D-ribofuranosyl moiety

<400>  583
aacattcatt gttgtcg                                                     17


<210>  584
<211>  30
<212>  DNA
<213>  Artificial Sequence

<220>
<223>  Synthetic

<400>  584
catgatcagc tgggccaaga aaagcgggac                                       30


<210>  585
<211>  29
<212>  DNA
<213>  Artificial Sequence

<220>
<223>  Synthetic

<400>  585
catgatcagc tgggccaaga aaagcggga                                         29


<210>  586
<211>  28
<212>  DNA
<213>  Artificial Sequence

<220>
<223>  Synthetic

<400>  586
catgatcagc tgggccaaga aaagcggg                                          28


<210>  587
<211>  27
<212>  DNA
<213>  Artificial Sequence

<220>
<223>  Synthetic

<400>  587
catgatcagc tgggccaaga aaagcgg                                           27


<210>  588
<211>  19
<212>  DNA
<213>  Artificial Sequence

<220>
<223>  Synthetic


<220>
<221>  misc_feature
<222>  (3)..(5)
<223>  Nucleotide at position 3  and/or 5 may be modified by
       2'-O,4'-C-methylene-beta-D-ribofuranosyl moiety

<400>  588
aactggccct caaagtccc                                                    19


<210>  589
<211>  17
<212>  DNA
<213>  Artificial Sequence

<220>
<223>  Synthetic


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  589
aactggccct caaagtc                                                    17


<210>  590
<211>  30
<212>  DNA
<213>  Artificial Sequence

<220>
<223>  Synthetic

<400>  590
catgatcagc tgggccaaga actcacacct                                      30


<210>  591
<211>  29
<212>  DNA
<213>  Artificial Sequence

<220>
<223>  Synthetic

<400>  591
catgatcagc tgggccaaga actcacacc                                       29


<210>  592
<211>  28
<212>  DNA
<213>  Artificial Sequence

<220>
<223>  Synthetic

<400>  592
catgatcagc tgggccaaga actcacac                                        28


<210>  593
<211>  27
<212>  DNA
<213>  Artificial Sequence

<220>
<223>  Synthetic

<400>  593
catgatcagc tgggccaaga actcaca                                         27


<210>  594
<211>  19
<212>  DNA
<213>  Artificial Sequence

<220>
<223>  Synthetic


<220>
<221>  misc_feature
<222>  (4)..(8)

<223>  Nucleotide at position 4 and/or 8 may be modified by
       2'-O,4'-C-methylene-beta-D-ribofuranosyl moiety

<400>  594
aatccttgga acctaggtg                                                    19


<210>  595
<211>  17
<212>  DNA
<213>  Artificial Sequence

<220>
<223>  Synthetic


<220>
<221>  misc_feature
<222>  (3)..(8)
<223>  Nucleotide at position 3 and/or 5 and/or 8 may be modified by
       2'-O,4'-C-methylene-beta-D-ribofuranosyl moiety

<400>  595
aatccttgga acctagg                                                      17


<210>  596
<211>  30
<212>  DNA
<213>  Artificial Sequence

<220>
<223>  Synthetic

<400>  596
catgatcagc tgggccaaga ttcacaccta                                        30


<210>  597
<211>  29
<212>  DNA
<213>  Artificial Sequence

<220>
<223>  Synthetic

<400>  597
catgatcagc tgggccaaga ttcacacct                                         29


<210>  598
<211>  28
<212>  DNA
<213>  Artificial Sequence

<220>
<223>  Synthetic

<400>  598
catgatcagc tgggccaaga ttcacacc                                          28


<210>  599
<211>  27
<212>  DNA

<213>  Artificial Sequence

<220>
<223>  Synthetic

<400>  599
catgatcagc tgggccaaga ttcacac                                          27


<210>  600
<211>  18
<212>  DNA
<213>  Artificial Sequence

<220>
<223>  Synthetic


<220>
<221>  misc_feature
<222>  (3)..(8)
<223>  Nucleotide at position 3 and/or 8 may be modified by
      2'-O,4'-C-methylene-beta-D-ribofuranosyl moiety

<400>  600
aatccttgga acctaggt                                                    18


<210>  601
<211>  16
<212>  DNA
<213>  Artificial Sequence

<220>
<223>  Synthetic


<220>
<221>  misc_feature
<222>  (3)..(8)
<223>  Nucleotide at position 3 and/or 5 and/or 8 may be modified by
      2'-O,4'-C-methylene-beta-D-ribofuranosyl moiety

<400>  601
aatccttgga acctag                                                      16


<210>  602
<211>  30
<212>  DNA
<213>  Artificial Sequence

<220>
<223>  Synthetic

<400>  602
catgatcagc tgggccaaga cagaatcctt                                       30


<210>  603
<211>  29
<212>  DNA
<213>  Artificial Sequence

<220>

<223>  Synthetic

<400>  603
catgatcagc tgggccaaga cagaatcct                                         29


<210>  604
<211>  28
<212>  DNA
<213>  Artificial Sequence

<220>
<223>  Synthetic

<400>  604
catgatcagc tgggccaaga cagaatcc                                          28


<210>  605
<211>  27
<212>  DNA
<213>  Artificial Sequence

<220>
<223>  Synthetic

<400>  605
catgatcagc tgggccaaga cagaatc                                           27


<210>  606
<211>  17
<212>  DNA
<213>  Artificial Sequence

<220>
<223>  Synthetic


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  606
atgcacctgg gcaagga                                                      17


<210>  607
<211>  15
<212>  DNA
<213>  Artificial Sequence

<220>
<223>  Synthetic


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  607

atgcacctgg gcaag                                                        15


<210>  608
<211>  30
<212>  DNA
<213>  Artificial Sequence

<220>
<223>  Synthetic

<400>  608
catgatcagc tgggccaaga ctgaaccctt                                       30


<210>  609
<211>  29
<212>  DNA
<213>  Artificial Sequence

<220>
<223>  Synthetic

<400>  609
catgatcagc tgggccaaga ctgaaccct                                        29


<210>  610
<211>  28
<212>  DNA
<213>  Artificial Sequence

<220>
<223>  Synthetic

<400>  610
catgatcagc tgggccaaga ctgaaccc                                         28


<210>  611
<211>  27
<212>  DNA
<213>  Artificial Sequence

<220>
<223>  Synthetic

<400>  611
catgatcagc tgggccaaga ctgaacc                                          27


<210>  612
<211>  17
<212>  DNA
<213>  Artificial Sequence

<220>
<223>  Synthetic


<220>
<221>  misc_feature
<222>  (2)..(6)
<223>  Nucleotide at position 2 and/or 6 may be modified by
       2'-O,4'-C-methylene-beta-D-ribofuranosyl moiety

<400>  612
atgcacctgg gcaaggg                                                    17


<210>  613
<211>  15
<212>  DNA
<213>  Artificial Sequence

<220>
<223>  Synthetic


<220>
<221>  misc_feature
<222>  (2)..(6)
<223>  Nucleotide at position 2 and/or 6 may be modified by
       2'-O,4'-C-methylene-beta-D-ribofuranosyl moiety

<400>  613
atgcacctgg gcaag                                                      15


<210>  614
<211>  30
<212>  DNA
<213>  Artificial Sequence

<220>
<223>  Synthetic

<400>  614
catgatcagc tgggccaaga tctcacccag                                      30


<210>  615
<211>  29
<212>  DNA
<213>  Artificial Sequence

<220>
<223>  Synthetic

<400>  615
catgatcagc tgggccaaga tctcaccca                                       29


<210>  616
<211>  28
<212>  DNA
<213>  Artificial Sequence

<220>
<223>  Synthetic

<400>  616
catgatcagc tgggccaaga tctcaccc                                        28


<210>  617
<211>  27
<212>  DNA
<213>  Artificial Sequence

<220>
<223>  Synthetic

<400>  617
catgatcagc tgggccaaga tctcacc                                          27


<210>  618
<211>  17
<212>  DNA
<213>  Artificial Sequence

<220>
<223>  Synthetic


<220>
<221>  misc_feature
<222>  (3)..(8)
<223>  Nucleotide at position 3 and/or 4 and/or 8 may be modified by
       2'-O,4'-C-methylene-beta-D-ribofuranosyl moiety

<400>  618
aatcctttgt ccctggg                                                     17


<210>  619
<211>  16
<212>  DNA
<213>  Artificial Sequence

<220>
<223>  Synthetic


<220>
<221>  misc_feature
<222>  (4)..(8)
<223>  Nucleotide at position 4 and/or 8 may be modified by
       2'-O,4'-C-methylene-beta-D-ribofuranosyl moiety

<400>  619
aatcctttgt ccctgg                                                      16


<210>  620
<211>  30
<212>  DNA
<213>  Artificial Sequence

<220>
<223>  Synthetic

<400>  620
catgatcagc tgggccaaga tttcacccag                                       30


<210>  621
<211>  29
<212>  DNA
<213>  Artificial Sequence

<220>
<223>  Synthetic

<400>  621
catgatcagc tgggccaaga tttcaccca                                          29


<210>  622
<211>  28
<212>  DNA
<213>  Artificial Sequence

<220>
<223>  Synthetic

<400>  622
catgatcagc tgggccaaga tttcaccc                                           28


<210>  623
<211>  27
<212>  DNA
<213>  Artificial Sequence

<220>
<223>  Synthetic

<400>  623
catgatcagc tgggccaaga tttcacc                                            27


<210>  624
<211>  17
<212>  DNA
<213>  Artificial Sequence

<220>
<223>  Synthetic


<220>
<221>  misc_feature
<222>  (3)..(6)
<223>  Nucleotide at position 3 and/or 4 and/or 6 may be modified by
       2'-O,4'-C-methylene-beta-D-ribofuranosyl moiety

<400>  624
aatcctttgt ccctggg                                                       17


<210>  625
<211>  15
<212>  DNA
<213>  Artificial Sequence

<220>
<223>  Synthetic


<220>
<221>  misc_feature
<222>  (3)..(6)
<223>  Nucleotide at position 3 and/or 4 and/or 6 may be modified by
       2'-O,4'-C-methylene-beta-D-ribofuranosyl moiety

<400>  625
aatcctttgt ccctg                                                         15

<210>  626
<211>  30
<212>  DNA
<213>  Artificial Sequence

<220>
<223>  Synthetic

<400>  626
catgatcagc tgggccaaga agtggatgac                                        30


<210>  627
<211>  29
<212>  DNA
<213>  Artificial Sequence

<220>
<223>  Synthetic

<400>  627
catgatcagc tgggccaaga agtggatga                                         29


<210>  628
<211>  28
<212>  DNA
<213>  Artificial Sequence

<220>
<223>  Synthetic

<400>  628
catgatcagc tgggccaaga agtggatg                                          28


<210>  629
<211>  27
<212>  DNA
<213>  Artificial Sequence

<220>
<223>  Synthetic

<400>  629
catgatcagc tgggccaaga agtggat                                           27


<210>  630
<211>  16
<212>  DNA
<213>  Artificial Sequence

<220>
<223>  Synthetic


<220>
<221>  misc_feature
<222>  (4)..(6)
<223>  Nucleotide at position 4 and/or 6 may be modified by
       2'-O,4'-C-methylene-beta-D-ribofuranosyl moiety

<400>  630

aatcgtacag ggtcat                                                    16

<210>  631
<211>  14
<212>  DNA
<213>  Artificial Sequence

<220>
<223>  Synthetic

<220>
<221>  misc_feature
<222>  (2)..(6)
<223>  Nucleotide at position 2 and/or 4 and/or 6 may be modified by
       2'-O,4'-C-methylene-beta-D-ribofuranosyl moiety

<400>  631
aatcgtacag ggtc                                                      14

<210>  632
<211>  30
<212>  DNA
<213>  Artificial Sequence

<220>
<223>  Synthetic

<400>  632
catgatcagc tgggccaaga gctgccgtat                                     30

<210>  633
<211>  29
<212>  DNA
<213>  Artificial Sequence

<220>
<223>  Synthetic

<400>  633
catgatcagc tgggccaaga gctgccgta                                      29

<210>  634
<211>  28
<212>  DNA
<213>  Artificial Sequence

<220>
<223>  Synthetic

<400>  634
catgatcagc tgggccaaga gctgccgt                                       28

<210>  635
<211>  27
<212>  DNA
<213>  Artificial Sequence

<220>
<223>  Synthetic

<400>  635
catgatcagc tgggccaaga gctgccg                                          27


<210>  636
<211>  18
<212>  DNA
<213>  Artificial Sequence

<220>
<223>  Synthetic


<220>
<221>  misc_feature
<222>  (3)..(6)
<223>  Nucleotide at position 3 and/or 6 may be modified by
       2'-O,4'-C-methylene-beta-D-ribofuranosyl moiety

<400>  636
agtgacatca catatacg                                                    18


<210>  637
<211>  17
<212>  DNA
<213>  Artificial Sequence

<220>
<223>  Synthetic


<220>
<221>  misc_feature
<222>  (2)..(6)
<223>  Nucleotide at position 2 and/or 3 and/or 6 may be modified by
       2'-O,4'-C-methylene-beta-D-ribofuranosyl moiety

<400>  637
agtgacatca catatac                                                     17


<210>  638
<211>  30
<212>  DNA
<213>  Artificial Sequence

<220>
<223>  Synthetic

<400>  638
catgatcagc tgggccaaga gctgccatat                                       30


<210>  639
<211>  29
<212>  DNA
<213>  Artificial Sequence

<220>
<223>  Synthetic

<400>  639
catgatcagc tgggccaaga gctgccata                                        29

<210>  640
<211>  28
<212>  DNA
<213>  Artificial Sequence

<220>
<223>  Synthetic

<400>  640
catgatcagc tgggccaaga gctgccat                                          28


<210>  641
<211>  27
<212>  DNA
<213>  Artificial Sequence

<220>
<223>  Synthetic

<400>  641
catgatcagc tgggccaaga gctgcca                                           27


<210>  642
<211>  18
<212>  DNA
<213>  Artificial Sequence

<220>
<223>  Synthetic


<220>
<221>  misc_feature
<222>  (6)..(8)
<223>  Nucleotide at position 6 and/or 8 may be modified by
       2'-O,4'-C-methylene-beta-D-ribofuranosyl moiety

<400>  642
aatgacacca catatatg                                                     18


<210>  643
<211>  15
<212>  DNA
<213>  Artificial Sequence

<220>
<223>  Synthetic


<220>
<221>  misc_feature
<222>  (3)..(8)
<223>  Nucleotide at position 3 and/or 6 and/or 8 may be modified by
       2'-O,4'-C-methylene-beta-D-ribofuranosyl moiety

<400>  643
aatgacacca catat                                                        15


<210>  644

<211>  30
<212>  DNA
<213>  Artificial Sequence

<220>
<223>  Synthetic

<400>  644
catgatcagc tgggccaaga gctgccatat                                        30


<210>  645
<211>  29
<212>  DNA
<213>  Artificial Sequence

<220>
<223>  Synthetic

<400>  645
catgatcagc tgggccaaga gctgccata                                         29


<210>  646
<211>  28
<212>  DNA
<213>  Artificial Sequence

<220>
<223>  Synthetic

<400>  646
catgatcagc tgggccaaga gctgccat                                          28


<210>  647
<211>  27
<212>  DNA
<213>  Artificial Sequence

<220>
<223>  Synthetic

<400>  647
catgatcagc tgggccaaga gctgcca                                           27


<210>  648
<211>  18
<212>  DNA
<213>  Artificial Sequence

<220>
<223>  Synthetic


<220>
<221>  misc_feature
<222>  (3)..(8)
<223>  Nucleotide at position 3 and/or 6 and/or 8 may be modified by
       2'-O,4'-C-methylene-beta-D-ribofuranosyl moiety

<400>  648
aatgacatca catatatg                                                     18

<210>  649
<211>  17
<212>  DNA
<213>  Artificial Sequence

<220>
<223>  Synthetic


<220>
<221>  misc_feature
<222>  (4)..(8)
<223>  Nucleotide at position 4 and/or 6 and/or 8 may be modified by
       2'-O,4'-C-methylene-beta-D-ribofuranosyl moiety

<400>  649
aatgacatca catatat                                                    17


<210>  650
<211>  30
<212>  DNA
<213>  Artificial Sequence

<220>
<223>  Synthetic

<400>  650
catgatcagc tgggccaaga tcaacgggag                                      30


<210>  651
<211>  29
<212>  DNA
<213>  Artificial Sequence

<220>
<223>  Synthetic

<400>  651
catgatcagc tgggccaaga tcaacggga                                       29


<210>  652
<211>  28
<212>  DNA
<213>  Artificial Sequence

<220>
<223>  Synthetic

<400>  652
catgatcagc tgggccaaga tcaacggg                                        28


<210>  653
<211>  27
<212>  DNA
<213>  Artificial Sequence

<220>
<223>  Synthetic

<400>  653

catgatcagc tgggccaaga tcaacgg                                          27

<210>  654
<211>  18
<212>  DNA
<213>  Artificial Sequence

<220>
<223>  Synthetic

<220>
<221>  misc_feature
<222>  (3)..(6)
<223>  Nucleotide at position 3 and/or 6 may be modified by
       2'-O,4'-C-methylene-beta-D-ribofuranosyl moiety

<400>  654
aatgacacga tcactccc                                                 18

<210>  655
<211>  16
<212>  DNA
<213>  Artificial Sequence

<220>
<223>  Synthetic

<220>
<221>  misc_feature
<222>  (3)..(6)
<223>  Nucleotide at position 3 and/or 4 and/or 6 may be modified by
       2'-O,4'-C-methylene-beta-D-ribofuranosyl moiety

<400>  655
aatgacacga tcactc                                                   16

<210>  656
<211>  30
<212>  DNA
<213>  Artificial Sequence

<220>
<223>  Synthetic

<400>  656
catgatcagc tgggccaaga tgcacaaccc                                    30

<210>  657
<211>  29
<212>  DNA
<213>  Artificial Sequence

<220>
<223>  Synthetic

<400>  657
catgatcagc tgggccaaga tgcacaacc                                     29

<210>  658
<211>  28
<212>  DNA
<213>  Artificial Sequence

<220>
<223>  Synthetic

<400>  658
catgatcagc tgggccaaga tgcacaac                                          28


<210>  659
<211>  27
<212>  DNA
<213>  Artificial Sequence

<220>
<223>  Synthetic

<400>  659
catgatcagc tgggccaaga tgcacaa                                           27


<210>  660
<211>  18
<212>  DNA
<213>  Artificial Sequence

<220>
<223>  Synthetic


<220>
<221>  misc_feature
<222>  (4)..(4)
<223>  Nucleotide at position 4 may be modified by
       2'-O,4'-C-methylene-beta-D-ribofuranosyl moiety

<400>  660
aatggcgcca ctagggtt                                                     18


<210>  661
<211>  16
<212>  DNA
<213>  Artificial Sequence

<220>
<223>  Synthetic


<220>
<221>  misc_feature
<222>  (4)..(6)
<223>  Nucleotide at position 4 and/or 6 may be modified by
       2'-O,4'-C-methylene-beta-D-ribofuranosyl moiety

<400>  661
aatggcgcca ctaggg                                                       16


<210>  662
<211>  30
<212>  DNA

<213>  Artificial Sequence

<220>
<223>  Synthetic

<400>  662
catgatcagc tgggccaaga gaattcatca                                        30


<210>  663
<211>  29
<212>  DNA
<213>  Artificial Sequence

<220>
<223>  Synthetic

<400>  663
catgatcagc tgggccaaga gaattcatc                                         29


<210>  664
<211>  28
<212>  DNA
<213>  Artificial Sequence

<220>
<223>  Synthetic

<400>  664
catgatcagc tgggccaaga gaattcat                                          28


<210>  665
<211>  27
<212>  DNA
<213>  Artificial Sequence

<220>
<223>  Synthetic

<400>  665
catgatcagc tgggccaaga gaattca                                           27


<210>  666
<211>  17
<212>  DNA
<213>  Artificial Sequence

<220>
<223>  Synthetic


<220>
<221>  misc_feature
<222>  (4)..(4)
<223>  Nucleotide at position 4 may be modified by
       2'-O,4'-C-methylene-beta-D-ribofuranosyl moiety

<400>  666
agaggctggc cgtgatg                                                      17


<210>  667

<211>  15
<212>  DNA
<213>  Artificial Sequence

<220>
<223>  Synthetic


<220>
<221>  misc_feature
<222>  (4)..(4)
<223>  Nucleotide at position 4 may be modified by
       2'-O,4'-C-methylene-beta-D-ribofuranosyl moiety

<400>  667
agaggctggc cgtga                                                        15


<210>  668
<211>  30
<212>  DNA
<213>  Artificial Sequence

<220>
<223>  Synthetic

<400>  668
catgatcagc tgggccaaga agagaggaga                                        30


<210>  669
<211>  29
<212>  DNA
<213>  Artificial Sequence

<220>
<223>  Synthetic

<400>  669
catgatcagc tgggccaaga agagaggag                                         29


<210>  670
<211>  28
<212>  DNA
<213>  Artificial Sequence

<220>
<223>  Synthetic

<400>  670
catgatcagc tgggccaaga agagagga                                          28


<210>  671
<211>  27
<212>  DNA
<213>  Artificial Sequence

<220>
<223>  Synthetic

<400>  671
catgatcagc tgggccaaga agagagg                                           27

<210>  672
<211>  18
<212>  DNA
<213>  Artificial Sequence

<220>
<223>  Synthetic


<220>
<221>  misc_feature
<222>  (3)..(8)
<223>  Nucleotide at position 3 and/or 8 may be modified by
       2'-O,4'-C-methylene-beta-D-ribofuranosyl moiety

<400>  672
agtcatacac ggctctcc                                                    18


<210>  673
<211>  16
<212>  DNA
<213>  Artificial Sequence

<220>
<223>  Synthetic


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  673
agtcatacac ggctct                                                      16


<210>  674
<211>  30
<212>  DNA
<213>  Artificial Sequence

<220>
<223>  Synthetic

<400>  674
catgatcagc tgggccaaga cgaatataac                                       30


<210>  675
<211>  29
<212>  DNA
<213>  Artificial Sequence

<220>
<223>  Synthetic

<400>  675
catgatcagc tgggccaaga cgaatataa                                        29


<210>  676
<211>  28

<212>  DNA
<213>  Artificial Sequence

<220>
<223>  Synthetic

<400>  676
catgatcagc tgggccaaga cgaatata                                          28


<210>  677
<211>  27
<212>  DNA
<213>  Artificial Sequence

<220>
<223>  Synthetic

<400>  677
catgatcagc tgggccaaga cgaatat                                           27


<210>  678
<211>  16
<212>  DNA
<213>  Artificial Sequence

<220>
<223>  Synthetic


<220>
<221>  misc_feature
<222>  (2)..(6)
<223>  Nucleotide at position 2 and/or 4 and/or 6 may be modified by
       2'-O,4'-C-methylene-beta-D-ribofuranosyl moiety

<400>  678
agatcgaccg tgttat                                                       16


<210>  679
<211>  14
<212>  DNA
<213>  Artificial Sequence

<220>
<223>  Synthetic


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  679
agatcgaccg tgtt                                                         14


<210>  680
<211>  30
<212>  DNA
<213>  Artificial Sequence

<220>
<223>  Synthetic

<400>  680
catgatcagc tgggccaaga cctccagccc                                        30


<210>  681
<211>  29
<212>  DNA
<213>  Artificial Sequence

<220>
<223>  Synthetic

<400>  681
catgatcagc tgggccaaga cctccagcc                                         29


<210>  682
<211>  28
<212>  DNA
<213>  Artificial Sequence

<220>
<223>  Synthetic

<400>  682
catgatcagc tgggccaaga cctccagc                                          28


<210>  683
<211>  27
<212>  DNA
<213>  Artificial Sequence

<220>
<223>  Synthetic

<400>  683
catgatcagc tgggccaaga cctccag                                           27


<210>  684
<211>  19
<212>  DNA
<213>  Artificial Sequence

<220>
<223>  Synthetic


<220>
<221>  misc_feature
<222>  (2)..(2)
<223>  Nucleotide at position 2 may be modified by
       2'-O,4'-C-methylene-beta-D-ribofuranosyl moiety

<400>  684
aggaagccct ggaggggct                                                    19


<210>  685
<211>  17
<212>  DNA

<213>  Artificial Sequence

<220>
<223>  Synthetic


<220>
<221>  misc_feature
<222>  (2)..(2)
<223>  Nucleotide at position 2 may be modified by
       2'-O,4'-C-methylene-beta-D-ribofuranosyl moiety

<400>  685
aggaagccct ggagggg                                                    17


<210>  686
<211>  30
<212>  DNA
<213>  Artificial Sequence

<220>
<223>  Synthetic

<400>  686
catgatcagc tgggccaaga gccagctaac                                      30


<210>  687
<211>  29
<212>  DNA
<213>  Artificial Sequence

<220>
<223>  Synthetic

<400>  687
catgatcagc tgggccaaga gccagctaa                                       29


<210>  688
<211>  28
<212>  DNA
<213>  Artificial Sequence

<220>
<223>  Synthetic

<400>  688
catgatcagc tgggccaaga gccagcta                                        28


<210>  689
<211>  27
<212>  DNA
<213>  Artificial Sequence

<220>
<223>  Synthetic

<400>  689
catgatcagc tgggccaaga gccagct                                         27


<210>  690

<211>  17
<212>  DNA
<213>  Artificial Sequence

<220>
<223>  Synthetic


<220>
<221>  misc_feature
<222>  (2)..(5)
<223>  Nucleotide at position 2 and/or 5 may be modified by
       2'-O,4'-C-methylene-beta-D-ribofuranosyl moiety

<400>  690
aggcagtgta ttgttag                                                    17


<210>  691
<211>  15
<212>  DNA
<213>  Artificial Sequence

<220>
<223>  Synthetic


<220>
<221>  misc_feature
<222>  (3)..(7)
<223>  Nucleotide at position 3 and/or 5 and/or 7 may be modified by
       2'-O,4'-C-methylene-beta-D-ribofuranosyl moiety

<400>  691
aggcagtgta ttgtt                                                      15


<210>  692
<211>  30
<212>  DNA
<213>  Artificial Sequence

<220>
<223>  Synthetic

<400>  692
catgatcagc tgggccaaga ccagctagca                                      30


<210>  693
<211>  29
<212>  DNA
<213>  Artificial Sequence

<220>
<223>  Synthetic

<400>  693
catgatcagc tgggccaaga ccagctagc                                       29


<210>  694
<211>  28
<212>  DNA
<213>  Artificial Sequence

<220>
<223>  Synthetic

<400>  694
catgatcagc tgggccaaga ccagctag                                          28


<210>  695
<211>  27
<212>  DNA
<213>  Artificial Sequence

<220>
<223>  Synthetic

<400>  695
catgatcagc tgggccaaga ccagcta                                           27


<210>  696
<211>  16
<212>  DNA
<213>  Artificial Sequence

<220>
<223>  Synthetic


<220>
<221>  misc_feature
<222>  (2)..(5)
<223>  Nucleotide at position 2 and/or 5 may be modified by
       2'-O,4'-C-methylene-beta-D-ribofuranosyl moiety

<400>  696
aggcagtgca ttgcta                                                       16


<210>  697
<211>  14
<212>  DNA
<213>  Artificial Sequence

<220>
<223>  Synthetic


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  697
aggcagtgca ttgc                                                         14


<210>  698
<211>  30
<212>  DNA
<213>  Artificial Sequence

<220>
<223>  Synthetic

<400>  698
catgatcagc tgggccaaga cgagccaggt                                        30


<210>  699
<211>  29
<212>  DNA
<213>  Artificial Sequence

<220>
<223>  Synthetic

<400>  699
catgatcagc tgggccaaga cgagccagg                                         29


<210>  700
<211>  28
<212>  DNA
<213>  Artificial Sequence

<220>
<223>  Synthetic

<400>  700
catgatcagc tgggccaaga cgagccag                                          28


<210>  701
<211>  27
<212>  DNA
<213>  Artificial Sequence

<220>
<223>  Synthetic

<400>  701
catgatcagc tgggccaaga cgagcca                                           27


<210>  702
<211>  15
<212>  DNA
<213>  Artificial Sequence

<220>
<223>  Synthetic


<220>
<221>  misc_feature
<222>  (2)..(2)
<223>  Nucleotide at position 2 may be modified by
       2'-O,4'-C-methylene-beta-D-ribofuranosyl moiety

<400>  702
aggcagtgcg acctg                                                        15


<210>  703
<211>  13
<212>  DNA
<213>  Artificial Sequence

<220>
<223>  Synthetic

<220>
<221>  misc_feature
<222>  (2)..(4)
<223>  Nucleotide at position 2 and/or 4 may be modified by
       2'-O,4'-C-methylene-beta-D-ribofuranosyl moiety

<400>  703
aggcagtgcg acc                                                          13

<210>  704
<211>  30
<212>  DNA
<213>  Artificial Sequence

<220>
<223>  Synthetic

<400>  704
catgatcagc tgggccaaga caaagttgct                                        30

<210>  705
<211>  29
<212>  DNA
<213>  Artificial Sequence

<220>
<223>  Synthetic

<400>  705
catgatcagc tgggccaaga caaagttgc                                         29

<210>  706
<211>  28
<212>  DNA
<213>  Artificial Sequence

<220>
<223>  Synthetic

<400>  706
catgatcagc tgggccaaga caaagttg                                          28

<210>  707
<211>  27
<212>  DNA
<213>  Artificial Sequence

<220>
<223>  Synthetic

<400>  707
catgatcagc tgggccaaga caaagtt                                           27

<210>  708
<211>  17
<212>  DNA

<213>  Artificial Sequence

<220>
<223>  Synthetic


<220>
<221>  misc_feature
<222>  (2)..(5)
<223>  Nucleotide at position 2 and/or 5 may be modified by
      2'-O,4'-C-methylene-beta-D-ribofuranosyl moiety

<400>  708
aggttacccg agcaact                                                    17


<210>  709
<211>  15
<212>  DNA
<213>  Artificial Sequence

<220>
<223>  Synthetic


<220>
<221>  misc_feature
<222>  (2)..(4)
<223>  Nucleotide at position 2 and/or 4 may be modified by
      2'-O,4'-C-methylene-beta-D-ribofuranosyl moiety

<400>  709
aggttacccg agcaa                                                      15


<210>  710
<211>  30
<212>  DNA
<213>  Artificial Sequence

<220>
<223>  Synthetic

<400>  710
catgatcagc tgggccaaga aaggggttca                                      30


<210>  711
<211>  29
<212>  DNA
<213>  Artificial Sequence

<220>
<223>  Synthetic

<400>  711
catgatcagc tgggccaaga aaggggttc                                       29


<210>  712
<211>  28
<212>  DNA
<213>  Artificial Sequence

<220>

<223>  Synthetic

<400>  712
catgatcagc tgggccaaga aaggggtt                                          28


<210>  713
<211>  27
<212>  DNA
<213>  Artificial Sequence

<220>
<223>  Synthetic

<400>  713
catgatcagc tgggccaaga aaggggt                                           27


<210>  714
<211>  18
<212>  DNA
<213>  Artificial Sequence

<220>
<223>  Synthetic


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  714
gaatgttgct cggtgaac                                                     18


<210>  715
<211>  16
<212>  DNA
<213>  Artificial Sequence

<220>
<223>  Synthetic


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  715
gaatgttgct cggtga                                                       16


<210>  716
<211>  30
<212>  DNA
<213>  Artificial Sequence

<220>
<223>  Synthetic

<400>  716

catgatcagc tgggccaaga cctcatggaa                                        30

<210>  717
<211>  29
<212>  DNA
<213>  Artificial Sequence

<220>
<223>  Synthetic

<400>  717
catgatcagc tgggccaaga cctcatgga                                         29

<210>  718
<211>  28
<212>  DNA
<213>  Artificial Sequence

<220>
<223>  Synthetic

<400>  718
catgatcagc tgggccaaga cctcatgg                                          28

<210>  719
<211>  27
<212>  DNA
<213>  Artificial Sequence

<220>
<223>  Synthetic

<400>  719
catgatcagc tgggccaaga cctcatg                                           27

<210>  720
<211>  17
<212>  DNA
<213>  Artificial Sequence

<220>
<223>  Synthetic

<220>
<221>  misc_feature
<222>  (3)..(6)
<223>  Nucleotide at position 3 and/or 6 may be modified by
       2'-O,4'-C-methylene-beta-D-ribofuranosyl moiety

<400>  720
agtggggaac ccttcca                                                      17

<210>  721
<211>  15
<212>  DNA
<213>  Artificial Sequence

<220>
<223>  Synthetic

<220>
<221>  misc_feature
<222>  (3)..(5)
<223>  Nucleotide at position 3 and/or 5 may be modified by
      2'-O,4'-C-methylene-beta-D-ribofuranosyl moiety

<400>  721
agtggggaac ccttc                                                        15


<210>  722
<211>  30
<212>  DNA
<213>  Artificial Sequence

<220>
<223>  Synthetic

<400>  722
catgatcagc tgggccaaga tatgaacaat                                        30


<210>  723
<211>  29
<212>  DNA
<213>  Artificial Sequence

<220>
<223>  Synthetic

<400>  723
catgatcagc tgggccaaga tatgaacaa                                         29


<210>  724
<211>  28
<212>  DNA
<213>  Artificial Sequence

<220>
<223>  Synthetic

<400>  724
catgatcagc tgggccaaga tatgaaca                                          28


<210>  725
<211>  27
<212>  DNA
<213>  Artificial Sequence

<220>
<223>  Synthetic

<400>  725
catgatcagc tgggccaaga tatgaac                                           27


<210>  726
<211>  17
<212>  DNA
<213>  Artificial Sequence

<220>
<223>  Synthetic


<220>
<221>  misc_feature
<222>  (2)..(7)
<223>  Nucleotide at position 2 and/or 4 and/or 7 may be modified by
      2'-O,4'-C-methylene-beta-D-ribofuranosyl moiety

<400>  726
attcctagaa attgttc                                                    17


<210>  727
<211>  15
<212>  DNA
<213>  Artificial Sequence

<220>
<223>  Synthetic


<220>
<221>  misc_feature
<222>  (2)..(9)
<223>  Nucleotide at position 2 and/or 4 and/or 7 and/or 9 may be
      modified by 2'-O,4'-C-methylene-beta-D-ribofuranosyl moiety

<400>  727
attcctagaa attgt                                                      15


<210>  728
<211>  30
<212>  DNA
<213>  Artificial Sequence

<220>
<223>  Synthetic

<400>  728
catgatcagc tgggccaaga tgtgaacaat                                      30


<210>  729
<211>  29
<212>  DNA
<213>  Artificial Sequence

<220>
<223>  Synthetic

<400>  729
catgatcagc tgggccaaga tgtgaacaa                                       29


<210>  730
<211>  28
<212>  DNA
<213>  Artificial Sequence

<220>
<223>  Synthetic

<400>  730
catgatcagc tgggccaaga tgtgaaca                                          28


<210>  731
<211>  27
<212>  DNA
<213>  Artificial Sequence

<220>
<223>  Synthetic

<400>  731
catgatcagc tgggccaaga tgtgaac                                           27


<210>  732
<211>  16
<212>  DNA
<213>  Artificial Sequence

<220>
<223>  Synthetic


<220>
<221>  misc_feature
<222>  (2)..(7)
<223>  Nucleotide at position 2 and/or 4 and/or 7 may be modified by
       2'-O,4'-C-methylene-beta-D-ribofuranosyl moiety

<400>  732
attcctagaa attgtt                                                       16


<210>  733
<211>  16
<212>  DNA
<213>  Artificial Sequence

<220>
<223>  Synthetic


<220>
<221>  misc_feature
<222>  (2)..(9)
<223>  Nucleotide at position 2 and/or 4 and/or 7 and/or 9 may be
       modified by 2'-O,4'-C-methylene-beta-D-ribofuranosyl moiety

<400>  733
attcctagaa attgtt                                                       16


<210>  734
<211>  30
<212>  DNA
<213>  Artificial Sequence

<220>
<223>  Synthetic

<400>  734
catgatcagc tgggccaaga acctgcacta                                        30

<210>  735
<211>  29
<212>  DNA
<213>  Artificial Sequence

<220>
<223>  Synthetic

<400>  735
catgatcagc tgggccaaga acctgcact                                         29


<210>  736
<211>  28
<212>  DNA
<213>  Artificial Sequence

<220>
<223>  Synthetic

<400>  736
catgatcagc tgggccaaga acctgcac                                          28


<210>  737
<211>  27
<212>  DNA
<213>  Artificial Sequence

<220>
<223>  Synthetic

<400>  737
catgatcagc tgggccaaga acctgca                                           27


<210>  738
<211>  18
<212>  DNA
<213>  Artificial Sequence

<220>
<223>  Synthetic


<220>
<221>  misc_feature
<222>  (2)..(6)
<223>  Nucleotide at position 2 and/or 4 and/or 6 may be modified by
       2'-O,4'-C-methylene-beta-D-ribofuranosyl moiety

<400>  738
caaagtgctc atagtgca                                                     18


<210>  739
<211>  16
<212>  DNA
<213>  Artificial Sequence

<220>
<223>  Synthetic

<220>
<221>  misc_feature
<222>  (4)..(8)
<223>  Nucleotide at position 4 and/or 6 and/or 8 may be modified by
       2'-O,4'-C-methylene-beta-D-ribofuranosyl moiety

<400>  739
caaagtgctc atagtg                                                    16


<210>  740
<211>  30
<212>  DNA
<213>  Artificial Sequence

<220>
<223>  Synthetic

<400>  740
catgatcagc tgggccaaga cagcatggag                                     30


<210>  741
<211>  29
<212>  DNA
<213>  Artificial Sequence

<220>
<223>  Synthetic

<400>  741
catgatcagc tgggccaaga cagcatgga                                      29


<210>  742
<211>  28
<212>  DNA
<213>  Artificial Sequence

<220>
<223>  Synthetic

<400>  742
catgatcagc tgggccaaga cagcatgg                                       28


<210>  743
<211>  27
<212>  DNA
<213>  Artificial Sequence

<220>
<223>  Synthetic

<400>  743
catgatcagc tgggccaaga cagcatg                                        27


<210>  744
<211>  17
<212>  DNA
<213>  Artificial Sequence

<220>
<223>  Synthetic

<220>
<221>  misc_feature
<222>  (2)..(4)
<223>  Nucleotide at position 2 and/or 4 may be modified by
      2'-O,4'-C-methylene-beta-D-ribofuranosyl moiety

<400>  744
caacctggag gactcca                                                    17


<210>  745
<211>  15
<212>  DNA
<213>  Artificial Sequence

<220>
<223>  Synthetic


<220>
<221>  misc_feature
<222>  (4)..(7)
<223>  Nucleotide at position 4 and/or 7 may be modified by
      2'-O,4'-C-methylene-beta-D-ribofuranosyl moiety

<400>  745
caacctggag gactc                                                      15


<210>  746
<211>  30
<212>  DNA
<213>  Artificial Sequence

<220>
<223>  Synthetic

<400>  746
catgatcagc tgggccaaga acaaaccaca                                      30


<210>  747
<211>  29
<212>  DNA
<213>  Artificial Sequence

<220>
<223>  Synthetic

<400>  747
catgatcagc tgggccaaga acaaaccac                                       29


<210>  748
<211>  28
<212>  DNA
<213>  Artificial Sequence

<220>
<223>  Synthetic

<400>  748
catgatcagc tgggccaaga acaaacca                                        28

<210>  749
<211>  27
<212>  DNA
<213>  Artificial Sequence

<220>
<223>  Synthetic

<400>  749
catgatcagc tgggccaaga acaaacc                                          27


<210>  750
<211>  16
<212>  DNA
<213>  Artificial Sequence

<220>
<223>  Synthetic


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  750
cagcagcaca ctgtgg                                                      16


<210>  751
<211>  15
<212>  DNA
<213>  Artificial Sequence

<220>
<223>  Synthetic


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  751
cagcagcaca ctgtg                                                       15


<210>  752
<211>  30
<212>  DNA
<213>  Artificial Sequence

<220>
<223>  Synthetic

<400>  752
catgatcagc tgggccaaga tgctttgaca                                       30


<210>  753

<211>  29
<212>  DNA
<213>  Artificial Sequence

<220>
<223>  Synthetic

<400>  753
catgatcagc tgggccaaga tgctttgac                                         29


<210>  754
<211>  28
<212>  DNA
<213>  Artificial Sequence

<220>
<223>  Synthetic

<400>  754
catgatcagc tgggccaaga tgctttga                                          28


<210>  755
<211>  27
<212>  DNA
<213>  Artificial Sequence

<220>
<223>  Synthetic

<400>  755
catgatcagc tgggccaaga tgctttg                                           27


<210>  756
<211>  19
<212>  DNA
<213>  Artificial Sequence

<220>
<223>  Synthetic


<220>
<221>  misc_feature
<222>  (2)..(6)
<223>  Nucleotide at position 2 and/or 4 and/or 6 may be modified by
       2'-O,4'-C-methylene-beta-D-ribofuranosyl moiety

<400>  756
cagtgcaatg atattgtca                                                    19


<210>  757
<211>  17
<212>  DNA
<213>  Artificial Sequence

<220>
<223>  Synthetic


<220>
<221>  misc_feature

<222>  (2)..(6)
<223>  Nucleotide at position 2 and/or 4 and/or 6 may be modified by
       2'-O,4'-C-methylene-beta-D-ribofuranosyl moiety

<400>  757
cagtgcaatg atattgt                                                    17


<210>  758
<211>  30
<212>  DNA
<213>  Artificial Sequence

<220>
<223>  Synthetic

<400>  758
catgatcagc tgggccaaga tgctttgaca                                      30


<210>  759
<211>  29
<212>  DNA
<213>  Artificial Sequence

<220>
<223>  Synthetic

<400>  759
catgatcagc tgggccaaga tgctttgac                                       29


<210>  760
<211>  28
<212>  DNA
<213>  Artificial Sequence

<220>
<223>  Synthetic

<400>  760
catgatcagc tgggccaaga tgctttga                                        28


<210>  761
<211>  27
<212>  DNA
<213>  Artificial Sequence

<220>
<223>  Synthetic

<400>  761
catgatcagc tgggccaaga tgctttg                                         27


<210>  762
<211>  19
<212>  DNA
<213>  Artificial Sequence

<220>
<223>  Synthetic

<220>
<221>  misc_feature
<222>  (2)..(6)
<223>  Nucleotide at position 2 and/or 4 and/or 6 may be modified by
      2'-O,4'-C-methylene-beta-D-ribofuranosyl moiety

<400>  762
cagtgcaatg gtattgtca                                                    19


<210>  763
<211>  17
<212>  DNA
<213>  Artificial Sequence

<220>
<223>  Synthetic


<220>
<221>  misc_feature
<222>  (2)..(6)
<223>  Nucleotide at position 2 and/or 4 and/or 6 may be modified by
      2'-O,4'-C-methylene-beta-D-ribofuranosyl moiety

<400>  763
cagtgcaatg gtattgt                                                      17


<210>  764
<211>  30
<212>  DNA
<213>  Artificial Sequence

<220>
<223>  Synthetic

<400>  764
catgatcagc tgggccaaga ttcccccttt                                        30


<210>  765
<211>  29
<212>  DNA
<213>  Artificial Sequence

<220>
<223>  Synthetic

<400>  765
   catgatcagc tgggccaaga ttccccctt                                        29


<210>  766
<211>  28
<212>  DNA
<213>  Artificial Sequence

<220>
<223>  Synthetic

<400>  766
catgatcagc tgggccaaga ttccccct                                          28

<210>  767
<211>  27
<212>  DNA
<213>  Artificial Sequence

<220>
<223>  Synthetic

<400>  767
catgatcagc tgggccaaga ttccccc                                          27


<210>  768
<211>  17
<212>  DNA
<213>  Artificial Sequence

<220>
<223>  Synthetic



<220>
<221>  misc_feature
<222>  (2)..(6)
<223>  Nucleotide at position 2 and/or 4 and/or 6 may be modified by
       2'-O,4'-C-methylene-beta-D-ribofuranosyl moiety

<400>  768
cagtgcaatt aaaaggg                                                     17


<210>  769
<211>  15
<212>  DNA
<213>  Artificial Sequence

<220>
<223>  Synthetic



<220>
<221>  misc_feature
<222>  (2)..(6)
<223>  Nucleotide at position 2 and/or 4 and/or 6 may be modified by
       2'-O,4'-C-methylene-beta-D-ribofuranosyl moiety

<400>  769
cagtgcaatt aaaag                                                       15


<210>  770
<211>  30
<212>  DNA
<213>  Artificial Sequence

<220>
<223>  Synthetic

<400>  770
catgatcagc tgggccaaga acggtcctac                                       30


<210>  771
<211>  29
<212>  DNA

<213>  Artificial Sequence

<220>
<223>  Synthetic

<400>  771
catgatcagc tgggccaaga acggtccta                                        29


<210>  772
<211>  28
<212>  DNA
<213>  Artificial Sequence

<220>
<223>  Synthetic

<400>  772
catgatcagc tgggccaaga acggtcct                                         28


<210>  773
<211>  27
<212>  DNA
<213>  Artificial Sequence

<220>
<223>  Synthetic

<400>  773
catgatcagc tgggccaaga acggtcc                                          27


<210>  774
<211>  17
<212>  DNA
<213>  Artificial Sequence

<220>
<223>  Synthetic


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  774
catgccttga gtgtagg                                                     17


<210>  775
<211>  15
<212>  DNA
<213>  Artificial Sequence

<220>
<223>  Synthetic


<220>
<221>  misc_feature
<222>  (3)..(5)
<223>  Nucleotide at position 3 and/or 5 may be modified by

2'-O,4'-C-methylene-beta-D-ribofuranosyl moiety

<400>  775
catgccttga gtgta                                                        15


<210>  776
<211>  30
<212>  DNA
<213>  Artificial Sequence

<220>
<223>  Synthetic

<400>  776
catgatcagc tgggccaaga ttgagagtgc                                        30


<210>  777
<211>  29
<212>  DNA
<213>  Artificial Sequence

<220>
<223>  Synthetic

<400>  777
catgatcagc tgggccaaga ttgagagtg                                         29


<210>  778
<211>  28
<212>  DNA
<213>  Artificial Sequence

<220>
<223>  Synthetic

<400>  778
catgatcagc tgggccaaga ttgagagt                                          28


<210>  779
<211>  27
<212>  DNA
<213>  Artificial Sequence

<220>
<223>  Synthetic

<400>  779
catgatcagc tgggccaaga ttgagag                                           27


<210>  780
<211>  16
<212>  DNA
<213>  Artificial Sequence

<220>
<223>  Synthetic


<220>
<221>  misc_feature

<222>  (2)..(5)
<223>  Nucleotide at position 2 and/or 5 may be modified by
       2'-O,4'-C-methylene-beta-D-ribofuranosyl moiety

<400>  780
cccagataat ggcact                                                    16


<210>  781
<211>  14
<212>  DNA
<213>  Artificial Sequence

<220>
<223>  Synthetic


<220>
<221>  misc_feature
<222>  (2)..(5)
<223>  Nucleotide at position 2 and/or 4 and/or 5 may be modified by
       2'-O,4'-C-methylene-beta-D-ribofuranosyl moiety

<400>  781
cccagataat ggca                                                      14


<210>  782
<211>  30
<212>  DNA
<213>  Artificial Sequence

<220>
<223>  Synthetic

<400>  782
catgatcagc tgggccaaga ttgagagtgc                                     30


<210>  783
<211>  29
<212>  DNA
<213>  Artificial Sequence

<220>
<223>  Synthetic

<400>  783
catgatcagc tgggccaaga ttgagagtg                                      29


<210>  784
<211>  28
<212>  DNA
<213>  Artificial Sequence

<220>
<223>  Synthetic

<400>  784
catgatcagc tgggccaaga ttgagagt                                       28


<210>  785
<211>  27

<212>  DNA
<213>  Artificial Sequence

<220>
<223>  Synthetic

<400>  785
catgatcagc tgggccaaga ttgagag                                          27


<210>  786
<211>  16
<212>  DNA
<213>  Artificial Sequence

<220>
<223>  Synthetic


<220>
<221>  misc_feature
<222>  (2)..(5)
<223>  Nucleotide at position 2 and/or 5 may be modified by
       2'-O,4'-C-methylene-beta-D-ribofuranosyl moiety

<400>  786
cccagataat agcact                                                      16


<210>  787
<211>  14
<212>  DNA
<213>  Artificial Sequence

<220>
<223>  Synthetic


<220>
<221>  misc_feature
<222>  (2)..(5)
<223>  Nucleotide at position 2 and/or 4 and/or 5 may be modified by
       2'-O,4'-C-methylene-beta-D-ribofuranosyl moiety

<400>  787
cccagataat agca                                                        14


<210>  788
<211>  30
<212>  DNA
<213>  Artificial Sequence

<220>
<223>  Synthetic

<400>  788
catgatcagc tgggccaaga acacaccaag                                       30


<210>  789
<211>  29
<212>  DNA
<213>  Artificial Sequence

<220>
<223>  Synthetic

<400>  789
catgatcagc tgggccaaga acacaccaa                                         29


<210>  790
<211>  28
<212>  DNA
<213>  Artificial Sequence

<220>
<223>  Synthetic

<400>  790
catgatcagc tgggccaaga acacacca                                          28


<210>  791
<211>  27
<212>  DNA
<213>  Artificial Sequence

<220>
<223>  Synthetic

<400>  791
catgatcagc tgggccaaga acacacc                                           27


<210>  792
<211>  18
<212>  DNA
<213>  Artificial Sequence

<220>
<223>  Synthetic


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  792
ggagaaatta tccttggt                                                     18


<210>  793
<211>  17
<212>  DNA
<213>  Artificial Sequence

<220>
<223>  Synthetic


<220>
<221>  misc_feature
<222>  (2)..(5)
<223>  Nucleotide at position 2 and/or 4 and/or 5 may be modified by
       2'-O,4'-C-methylene-beta-D-ribofuranosyl moiety

<400>  793
ggagaaatta tccttgg                                                      17


<210>  794
<211>  30
<212>  DNA
<213>  Artificial Sequence

<220>
<223>  Synthetic

<400>  794
catgatcagc tgggccaaga gaggaaacca                                        30


<210>  795
<211>  29
<212>  DNA
<213>  Artificial Sequence

<220>
<223>  Synthetic

<400>  795
catgatcagc tgggccaaga gaggaaacc                                         29


<210>  796
<211>  28
<212>  DNA
<213>  Artificial Sequence

<220>
<223>  Synthetic

<400>  796
catgatcagc tgggccaaga gaggaaac                                          28


<210>  797
<211>  27
<212>  DNA
<213>  Artificial Sequence

<220>
<223>  Synthetic

<400>  797
catgatcagc tgggccaaga gaggaaa                                           27


<210>  798
<211>  17
<212>  DNA
<213>  Artificial Sequence

<220>
<223>  Synthetic


<220>
<221>  misc_feature
<222>  (3)..(6)
<223>  Nucleotide at position 3 and/or 6 may be modified by

2'-O,4'-C-methylene-beta-D-ribofuranosyl moiety

<400>  798
gtcaacactt gctggtt                                                    17


<210>  799
<211>  15
<212>  DNA
<213>  Artificial Sequence

<220>
<223>  Synthetic


<220>
<221>  misc_feature
<222>  (2)..(6)
<223>  Nucleotide at position 2 and/or 3 and/or 6 may be modified by
       2'-O,4'-C-methylene-beta-D-ribofuranosyl moiety

<400>  799
gtcaacactt gctgg                                                      15


<210>  800
<211>  30
<212>  DNA
<213>  Artificial Sequence

<220>
<223>  Synthetic

<400>  800
catgatcagc tgggccaaga ggaaaccagc                                      30


<210>  801
<211>  29
<212>  DNA
<213>  Artificial Sequence

<220>
<223>  Synthetic

<400>  801
catgatcagc tgggccaaga ggaaaccag                                       29


<210>  802
<211>  28
<212>  DNA
<213>  Artificial Sequence

<220>
<223>  Synthetic

<400>  802
catgatcagc tgggccaaga ggaaacca                                        28


<210>  803
<211>  27
<212>  DNA
<213>  Artificial Sequence

<220>
<223>  Synthetic

<400>  803
catgatcagc tgggccaaga ggaaacc                                          27


<210>  804
<211>  17
<212>  DNA
<213>  Artificial Sequence

<220>
<223>  Synthetic


<220>
<221>  misc_feature
<222>  (3)..(9)
<223>  Nucleotide at position 3, 4, 7 and/or 9 may be modified by
       2'-O,4'-C-methylene-beta-D-ribofuranosyl moiety

<220>
<221>  misc_feature
<222>  (3)..(9)
<223>  Nucleotide at position 3 and/or 4 and/or 7 and/or 9 may be
       modified by 2'-O,4'-C-methylene-beta-D-ribofuranosyl moiety

<400>  804
cgtcaacact tgctggt                                                     17


<210>  805
<211>  15
<212>  DNA
<213>  Artificial Sequence

<220>
<223>  Synthetic


<220>
<221>  misc_feature
<222>  (3)..(9)
<223>  Nucleotide at position 3 and/or 4 and/or 7 and/or 9 may be
       modified by 2'-O,4'-C-methylene-beta-D-ribofuranosyl moiety

<400>  805
cgtcaacact tgctg                                                       15


<210>  806
<211>  30
<212>  DNA
<213>  Artificial Sequence

<220>
<223>  Synthetic

<400>  806
catgatcagc tgggccaaga taactgcact                                       30


<210>  807

<211>  29
<212>  DNA
<213>  Artificial Sequence

<220>
<223>  Synthetic

<400>  807
catgatcagc tgggccaaga taactgcac                                         29


<210>  808
<211>  28
<212>  DNA
<213>  Artificial Sequence

<220>
<223>  Synthetic

<400>  808
catgatcagc tgggccaaga taactgca                                          28


<210>  809
<211>  27
<212>  DNA
<213>  Artificial Sequence

<220>
<223>  Synthetic

<400>  809
catgatcagc tgggccaaga taactgc                                           27


<210>  810
<211>  17
<212>  DNA
<213>  Artificial Sequence

<220>
<223>  Synthetic


<220>
<221>  misc_feature
<222>  (4)..(6)
<223>  Nucleotide at position 4 and/or 6 may be modified by
       2'-O,4'-C-methylene-beta-D-ribofuranosyl moiety

<400>  810
taaggtgcat ctagtgc                                                      17


<210>  811
<211>  15
<212>  DNA
<213>  Artificial Sequence

<220>
<223>  Synthetic


<220>
<221>  misc_feature

<222>  (2)..(6)
<223>  Nucleotide at position 2 and/or 4 and/or 6 may be modified by
       2'-O,4'-C-methylene-beta-D-ribofuranosyl moiety

<400>  811
taaggtgcat ctagt                                                        15


<210>  812
<211>  30
<212>  DNA
<213>  Artificial Sequence

<220>
<223>  Synthetic

<400>  812
catgatcagc tgggccaaga taacagcact                                        30


<210>  813
<211>  29
<212>  DNA
<213>  Artificial Sequence

<220>
<223>  Synthetic

<400>  813
catgatcagc tgggccaaga taacagcac                                         29


<210>  814
<211>  28
<212>  DNA
<213>  Artificial Sequence

<220>
<223>  Synthetic

<400>  814
catgatcagc tgggccaaga taacagca                                          28


<210>  815
<211>  27
<212>  DNA
<213>  Artificial Sequence

<220>
<223>  Synthetic

<400>  815
catgatcagc tgggccaaga taacagc                                           27


<210>  816
<211>  17
<212>  DNA
<213>  Artificial Sequence

<220>
<223>  Synthetic

<220>
<221>  misc_feature
<222>  (2)..(6)
<223>  Nucleotide at position 2 and/or 4 and/or 6 may be modified by
      2'-O,4'-C-methylene-beta-D-ribofuranosyl moiety

<400>  816
taaggtgcat ctagtgc                                                    17


<210>  817
<211>  15
<212>  DNA
<213>  Artificial Sequence

<220>
<223>  Synthetic


<220>
<221>  misc_feature
<222>  (4)..(8)
<223>  Nucleotide at position 4 and/or 6 and/or 8 may be modified by
      2'-O,4'-C-methylene-beta-D-ribofuranosyl moiety

<400>  817
taaggtgcat ctagt                                                      15


<210>  818
<211>  30
<212>  DNA
<213>  Artificial Sequence

<220>
<223>  Synthetic

<400>  818
catgatcagc tgggccaaga cagtactgtt                                      30


<210>  819
<211>  29
<212>  DNA
<213>  Artificial Sequence

<220>
<223>  Synthetic

<400>  819
catgatcagc tgggccaaga cagtactgt                                       29


<210>  820
<211>  28
<212>  DNA
<213>  Artificial Sequence

<220>
<223>  Synthetic

<400>  820
catgatcagc tgggccaaga cagtactg                                        28

<210> 821
<211> 27
<212> DNA
<213> Artificial Sequence

<220>
<223> Synthetic

<400> 821
catgatcagc tgggccaaga cagtact 27


<210> 822
<211> 16
<212> DNA
<213> Artificial Sequence

<220>
<223> Synthetic


<220>
<221> misc_feature
<222> (2)..(5)
<223> Nucleotide at position 2 and/or 5 may be modified by
2'-O,4'-C-methylene-beta-D-ribofuranosyl moiety

<400> 822
tagcagcggg aacagt 16


<210> 823
<211> 14
<212> DNA
<213> Artificial Sequence

<220>
<223> Synthetic


<220>
<221> misc_feature
<222> (2)..(5)
<223> Nucleotide at position 2 and/or 5 may be modified by
2'-O,4'-C-methylene-beta-D-ribofuranosyl moiety

<400> 823
tagcagcggg aaca 14


<210> 824
<211> 30
<212> DNA
<213> Artificial Sequence

<220>
<223> Synthetic

<400> 824
catgatcagc tgggccaaga cgatgtagtc 30


<210> 825
<211> 29
<212> DNA

<213>  Artificial Sequence

<220>
<223>  Synthetic

<400>  825
catgatcagc tgggccaaga cgatgtagt                                          29


<210>  826
<211>  28
<212>  DNA
<213>  Artificial Sequence

<220>
<223>  Synthetic

<400>  826
catgatcagc tgggccaaga cgatgtag                                           28


<210>  827
<211>  27
<212>  DNA
<213>  Artificial Sequence

<220>
<223>  Synthetic

<400>  827
catgatcagc tgggccaaga cgatgta                                            27


<210>  828
<211>  17
<212>  DNA
<213>  Artificial Sequence

<220>
<223>  Synthetic


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  828
tatgtgcctt tggacta                                                       17


<210>  829
<211>  15
<212>  DNA
<213>  Artificial Sequence

<220>
<223>  Synthetic


<220>
<221>  misc_feature
<222>  (3)..(5)
<223>  Nucleotide at position 3 and/or 5 may be modified by

2'-O,4'-C-methylene-beta-D-ribofuranosyl moiety

<400> 829
tatgtgcctt tggac 15

<210> 830
<211> 30
<212> DNA
<213> Artificial Sequence

<220>
<223> Synthetic

<400> 830
catgatcagc tgggccaaga gaggccggga 30

<210> 831
<211> 29
<212> DNA
<213> Artificial Sequence

<220>
<223> Synthetic

<400> 831
catgatcagc tgggccaaga gaggccggg 29

<210> 832
<211> 28
<212> DNA
<213> Artificial Sequence

<220>
<223> Synthetic

<400> 832
catgatcagc tgggccaaga gaggccgg 28

<210> 833
<211> 27
<212> DNA
<213> Artificial Sequence

<220>
<223> Synthetic

<400> 833
catgatcagc tgggccaaga gaggccg 27

<210> 834
<211> 16
<212> DNA
<213> Artificial Sequence

<220>
<223> Synthetic

<220>
<221> misc_feature

<222>  (4)..(6)
<223>  Nucleotide at position 4 and/or 6 may be modified by
       2'-O,4'-C-methylene-beta-D-ribofuranosyl moiety

<400>  834
tattgcactc gtcccg                                                    16


<210>  835
<211>  15
<212>  DNA
<213>  Artificial Sequence

<220>
<223>  Synthetic


<220>
<221>  misc_feature
<222>  (4)..(6)
<223>  Nucleotide at position 4 and/or 6 may be modified by
       2'-O,4'-C-methylene-beta-D-ribofuranosyl moiety

<400>  835
tattgcactc gtccc                                                     15


<210>  836
<211>  30
<212>  DNA
<213>  Artificial Sequence

<220>
<223>  Synthetic

<400>  836
catgatcagc tgggccaaga agaagacggg                                     30


<210>  837
<211>  29
<212>  DNA
<213>  Artificial Sequence

<220>
<223>  Synthetic

<400>  837
catgatcagc tgggccaaga agaagacgg                                      29


<210>  838
<211>  28
<212>  DNA
<213>  Artificial Sequence

<220>
<223>  Synthetic

<400>  838
catgatcagc tgggccaaga agaagacg                                       28


<210>  839
<211>  27

<212>  DNA
<213>  Artificial Sequence

<220>
<223>  Synthetic

<400>  839
catgatcagc tgggccaaga agaagac                                          27


<210>  840
<211>  18
<212>  DNA
<213>  Artificial Sequence

<220>
<223>  Synthetic


<220>
<221>  misc_feature
<222>  (2)..(5)
<223>  Nucleotide at position 2, and/or 5 may be modified by
       2'-O,4'-C-methylene-beta-D-ribofuranosyl moiety

<400>  840
tcactcctct cctcccgt                                                    18


<210>  841
<211>  16
<212>  DNA
<213>  Artificial Sequence

<220>
<223>  Synthetic


<220>
<221>  misc_feature
<222>  (2)..(5)
<223>  Nucleotide at position 2 and/or 5 may be modified by
       2'-O,4'-C-methylene-beta-D-ribofuranosyl moiety

<400>  841
tcactcctct cctccc                                                      16


<210>  842
<211>  30
<212>  DNA
<213>  Artificial Sequence

<220>
<223>  Synthetic

<400>  842
catgatcagc tgggccaaga acaagacggg                                       30


<210>  843
<211>  29
<212>  DNA
<213>  Artificial Sequence

<220>
<223>  Synthetic

<400>  843
catgatcagc tgggccaaga acaagacgg                                         29


<210>  844
<211>  28
<212>  DNA
<213>  Artificial Sequence

<220>
<223>  Synthetic

<400>  844
catgatcagc tgggccaaga acaagacg                                          28


<210>  845
<211>  27
<212>  DNA
<213>  Artificial Sequence

<220>
<223>  Synthetic

<400>  845
catgatcagc tgggccaaga acaagac                                           27


<210>  846
<211>  18
<212>  DNA
<213>  Artificial Sequence

<220>
<223>  Synthetic


<220>
<221>  misc_feature
<222>  (3)..(3)
<223>  Nucleotide at position 3 may be modified by
       2'-O,4'-C-methylene-beta-D-ribofuranosyl moiety

<400>  846
tcactcctcc cctcccgt                                                     18


<210>  847
<211>  16
<212>  DNA
<213>  Artificial Sequence

<220>
<223>  Synthetic


<220>
<221>  misc_feature
<222>  (3)..(3)
<223>  Nucleotide at position 3 may be modified by
       2'-O,4'-C-methylene-beta-D-ribofuranosyl moiety

<400>  847
tcactcctcc cctccc                                                       16


<210>  848
<211>  30
<212>  DNA
<213>  Artificial Sequence

<220>
<223>  Synthetic

<400>  848
catgatcagc tgggccaaga atcgggaggg                                        30


<210>  849
<211>  29
<212>  DNA
<213>  Artificial Sequence

<220>
<223>  Synthetic

<400>  849
catgatcagc tgggccaaga atcgggagg                                         29


<210>  850
<211>  28
<212>  DNA
<213>  Artificial Sequence

<220>
<223>  Synthetic

<400>  850
catgatcagc tgggccaaga atcgggag                                          28


<210>  851
<211>  27
<212>  DNA
<213>  Artificial Sequence

<220>
<223>  Synthetic

<400>  851
catgatcagc tgggccaaga atcggga                                           27


<210>  852
<211>  17
<212>  DNA
<213>  Artificial Sequence

<220>
<223>  Synthetic


<220>
<221>  misc_feature
<222>  (4)..(4)
<223>  Nucleotide at position 4 may be modified by

2'-O,4'-C-methylene-beta-D-ribofuranosyl moiety

<400>  852
tcaggctcag tcccctc                                                    17


<210>  853
<211>  15
<212>  DNA
<213>  Artificial Sequence

<220>
<223>  Synthetic


<220>
<221>  misc_feature
<222>  (3)..(3)
<223>  Nucleotide at position 3 may be modified by
       2'-O,4'-C-methylene-beta-D-ribofuranosyl moiety

<400>  853
tcaggctcag tcccc                                                      15


<210>  854
<211>  30
<212>  DNA
<213>  Artificial Sequence

<220>
<223>  Synthetic

<400>  854
catgatcagc tgggccaaga caggctcaaa                                      30


<210>  855
<211>  29
<212>  DNA
<213>  Artificial Sequence

<220>
<223>  Synthetic

<400>  855
catgatcagc tgggccaaga caggctcaa                                       29


<210>  856
<211>  28
<212>  DNA
<213>  Artificial Sequence

<220>
<223>  Synthetic

<400>  856
catgatcagc tgggccaaga caggctca                                        28


<210>  857
<211>  27
<212>  DNA
<213>  Artificial Sequence

<220>
<223>  Synthetic

<400>  857
catgatcagc tgggccaaga caggctc                                          27


<210>  858
<211>  19
<212>  DNA
<213>  Artificial Sequence

<220>
<223>  Synthetic


<220>
<221>  misc_feature
<222>  (3)..(3)
<223>  Nucleotide at position 3 may be modified by
       2'-O,4'-C-methylene-beta-D-ribofuranosyl moiety

<400>  858
tccctgagga gccctttga                                                   19


<210>  859
<211>  17
<212>  DNA
<213>  Artificial Sequence

<220>
<223>  Synthetic


<220>
<221>  misc_feature
<222>  (3)..(3)
<223>  Nucleotide at position 3 may be modified by
       2'-O,4'-C-methylene-beta-D-ribofuranosyl moiety

<400>  859
tccctgagga gcccttt                                                     17


<210>  860
<211>  30
<212>  DNA
<213>  Artificial Sequence

<220>
<223>  Synthetic

<400>  860
catgatcagc tgggccaaga agagggagac                                       30


<210>  861
<211>  29
<212>  DNA
<213>  Artificial Sequence

<220>
<223>  Synthetic

<400>  861
catgatcagc tgggccaaga agagggaga                                        29


<210>  862
<211>  28
<212>  DNA
<213>  Artificial Sequence

<220>
<223>  Synthetic

<400>  862
catgatcagc tgggccaaga agagggag                                         28


<210>  863
<211>  27
<212>  DNA
<213>  Artificial Sequence

<220>
<223>  Synthetic

<400>  863
catgatcagc tgggccaaga agaggga                                          27


<210>  864
<211>  16
<212>  DNA
<213>  Artificial Sequence

<220>
<223>  Synthetic


<220>
<221>  misc_feature
<222>  (3)..(3)
<223>  Nucleotide at position 3 may be modified by
       2'-O,4'-C-methylene-beta-D-ribofuranosyl moiety

<400>  864
tccgagcctg ggtctc                                                      16


<210>  865
<211>  14
<212>  DNA
<213>  Artificial Sequence

<220>
<223>  Synthetic


<220>
<221>  misc_feature
<222>  (3)..(3)
<223>  Nucleotide at position 3 may be modified by
       2'-O,4'-C-methylene-beta-D-ribofuranosyl moiety

<400>  865
tccgagcctg ggtc                                                        14

<210>  866
<211>  30
<212>  DNA
<213>  Artificial Sequence

<220>
<223>  Synthetic

<400>  866
catgatcagc tgggccaaga ctcggggcag                                        30


<210>  867
<211>  29
<212>  DNA
<213>  Artificial Sequence

<220>
<223>  Synthetic

<400>  867
catgatcagc tgggccaaga ctcggggca                                         29


<210>  868
<211>  28
<212>  DNA
<213>  Artificial Sequence

<220>
<223>  Synthetic

<400>  868
catgatcagc tgggccaaga ctcggggc                                          28


<210>  869
<211>  27
<212>  DNA
<213>  Artificial Sequence

<220>
<223>  Synthetic

<400>  869
catgatcagc tgggccaaga ctcgggg                                           27


<210>  870
<211>  17
<212>  DNA
<213>  Artificial Sequence

<220>
<223>  Synthetic


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  870
tcctgtactg agctgcc                                                      17


<210>  871
<211>  15
<212>  DNA
<213>  Artificial Sequence

<220>
<223>  Synthetic


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  871
tcctgtactg agctg                                                        15


<210>  872
<211>  30
<212>  DNA
<213>  Artificial Sequence

<220>
<223>  Synthetic

<400>  872
catgatcagc tgggccaaga aggtttcccg                                        30


<210>  873
<211>  29
<212>  DNA
<213>  Artificial Sequence

<220>
<223>  Synthetic

<400>  873
catgatcagc tgggccaaga aggtttccc                                         29


<210>  874
<211>  28
<212>  DNA
<213>  Artificial Sequence

<220>
<223>  Synthetic

<400>  874
catgatcagc tgggccaaga aggtttcc                                          28


<210>  875
<211>  27
<212>  DNA
<213>  Artificial Sequence

<220>

<223>  Synthetic

<400>  875
catgatcagc tgggccaaga aggtttc                                          27


<210>  876
<211>  16
<212>  DNA
<213>  Artificial Sequence

<220>
<223>  Synthetic


<220>
<221>  misc_feature
<222>  (2)..(6)
<223>  Nucleotide at position 2 and/or 4 and/or 6 may be modified by
       2'-O,4'-C-methylene-beta-D-ribofuranosyl moiety

<400>  876
tgaaacatac acggga                                                      16


<210>  877
<211>  14
<212>  DNA
<213>  Artificial Sequence

<220>
<223>  Synthetic


<220>
<221>  misc_feature
<222>  (2)..(6)
<223>  Nucleotide at position 2 and/or 4 and/or 6 may be modified by
       2'-O,4'-C-methylene-beta-D-ribofuranosyl moiety

<400>  877
tgaaacatac acgg                                                        14


<210>  878
<211>  30
<212>  DNA
<213>  Artificial Sequence

<220>
<223>  Synthetic

<400>  878
catgatcagc tgggccaaga ctggcacaca                                       30


<210>  879
<211>  29
<212>  DNA
<213>  Artificial Sequence

<220>
<223>  Synthetic

<400>  879

catgatcagc tgggccaaga ctggcacac                                          29

<210>  880
<211>  28
<212>  DNA
<213>  Artificial Sequence

<220>
<223>  Synthetic

<400>  880
catgatcagc tgggccaaga ctggcaca                                           28

<210>  881
<211>  27
<212>  DNA
<213>  Artificial Sequence

<220>
<223>  Synthetic

<400>  881
catgatcagc tgggccaaga ctggcac                                            27

<210>  882
<211>  15
<212>  DNA
<213>  Artificial Sequence

<220>
<223>  Synthetic

<220>
<221>  misc_feature
<222>  (2)..(5)
<223>  Nucleotide at position 2 and/or 5 may be modified by
       2'-O,4'-C-methylene-beta-D-ribofuranosyl moiety

<400>  882
tgaaggtcta ctgtg                                                         15

<210>  883
<211>  14
<212>  DNA
<213>  Artificial Sequence

<220>
<223>  Synthetic

<220>
<221>  misc_feature
<222>  (2)..(5)
<223>  Nucleotide at position 2 and/or 5 may be modified by
       2'-O,4'-C-methylene-beta-D-ribofuranosyl moiety

<400>  883
tgaaggtcta ctgt                                                          14

<210>  884
<211>  30
<212>  DNA
<213>  Artificial Sequence

<220>
<223>  Synthetic

<400>  884
catgatcagc tgggccaaga agcctatgga                                        30


<210>  885
<211>  29
<212>  DNA
<213>  Artificial Sequence

<220>
<223>  Synthetic

<400>  885
catgatcagc tgggccaaga agcctatgg                                         29


<210>  886
<211>  28
<212>  DNA
<213>  Artificial Sequence

<220>
<223>  Synthetic

<400>  886
catgatcagc tgggccaaga agcctatg                                          28


<210>  887
<211>  27
<212>  DNA
<213>  Artificial Sequence

<220>
<223>  Synthetic

<400>  887
catgatcagc tgggccaaga agcctat                                           27


<210>  888
<211>  18
<212>  DNA
<213>  Artificial Sequence

<220>
<223>  Synthetic


<220>
<221>  misc_feature
<222>  (2)..(8)
<223>  Nucleotide at position 2 and/or 4 and/or 8 may be modified by
       2'-O,4'-C-methylene-beta-D-ribofuranosyl moiety

<400>  888
tgagaactga attccata                                                     18

<210>  889
<211>  16
<212>  DNA
<213>  Artificial Sequence

<220>
<223>  Synthetic


<220>
<221>  misc_feature
<222>  (2)..(8)
<223>  Nucleotide at position 2 and/or 4 and/or 8 may be modified by
       2'-O,4'-C-methylene-beta-D-ribofuranosyl moiety

<400>  889
tgagaactga attcca                                                    16


<210>  890
<211>  30
<212>  DNA
<213>  Artificial Sequence

<220>
<223>  Synthetic

<400>  890
catgatcagc tgggccaaga tacacacttc                                     30


<210>  891
<211>  29
<212>  DNA
<213>  Artificial Sequence

<220>
<223>  Synthetic

<400>  891
catgatcagc tgggccaaga tacacactt                                      29


<210>  892
<211>  28
<212>  DNA
<213>  Artificial Sequence

<220>
<223>  Synthetic

<400>  892
catgatcagc tgggccaaga tacacact                                       28


<210>  893
<211>  27
<212>  DNA
<213>  Artificial Sequence

<220>
<223>  Synthetic

<400>  893
catgatcagc tgggccaaga tacacac                                          27


<210>  894
<211>  17
<212>  DNA
<213>  Artificial Sequence

<220>
<223>  Synthetic


<220>
<221>  misc_feature
<222>  (2)..(6)
<223>  Nucleotide at position 2 and/or 3 and/or 6 may be modified by
       2'-O,4'-C-methylene-beta-D-ribofuranosyl moiety

<400>  894
tggaatgtaa agaagtg                                                     17


<210>  895
<211>  15
<212>  DNA
<213>  Artificial Sequence

<220>
<223>  Synthetic


<220>
<221>  misc_feature
<222>  (2)..(6)
<223>  Nucleotide at position 2 and/or 3 and/or 6 may be modified by
       2'-O,4'-C-methylene-beta-D-ribofuranosyl moiety

<400>  895
tggaatgtaa agaag                                                       15


<210>  896
<211>  30
<212>  DNA
<213>  Artificial Sequence

<220>
<223>  Synthetic

<400>  896
catgatcagc tgggccaaga cactgtgggc                                       30


<210>  897
<211>  29
<212>  DNA
<213>  Artificial Sequence

<220>
<223>  Synthetic

<400>  897
catgatcagc tgggccaaga cactgtggg                                        29

<210>  898
<211>  28
<212>  DNA
<213>  Artificial Sequence

<220>
<223>  Synthetic

<400>  898
catgatcagc tgggccaaga cactgtgg                                          28


<210>  899
<211>  27
<212>  DNA
<213>  Artificial Sequence

<220>
<223>  Synthetic

<400>  899
catgatcagc tgggccaaga cactgtg                                           27


<210>  900
<211>  18
<212>  DNA
<213>  Artificial Sequence

<220>
<223>  Synthetic


<220>
<221>  misc_feature
<222>  (2)..(2)
<223>  Nucleotide at position 2 may be modified by
       2'-O,4'-C-methylene-beta-D-ribofuranosyl moiety

<400>  900
tggtgcggag agggccca                                                     18


<210>  901
<211>  15
<212>  DNA
<213>  Artificial Sequence

<220>
<223>  Synthetic


<220>
<221>  misc_feature
<222>  (2)..(2)
<223>  Nucleotide at position 2 may be modified by
       2'-O,4'-C-methylene-beta-D-ribofuranosyl moiety

<400>  901
tggtgcggag agggc                                                        15


<210>  902
<211>  30

<212>  DNA
<213>  Artificial Sequence

<220>
<223>  Synthetic

<400>  902
catgatcagc tgggccaaga actgtgggcc                                        30


<210>  903
<211>  29
<212>  DNA
<213>  Artificial Sequence

<220>
<223>  Synthetic

<400>  903
catgatcagc tgggccaaga actgtgggc                                         29


<210>  904
<211>  28
<212>  DNA
<213>  Artificial Sequence

<220>
<223>  Synthetic

<400>  904
catgatcagc tgggccaaga actgtggg                                          28


<210>  905
<211>  27
<212>  DNA
<213>  Artificial Sequence

<220>
<223>  Synthetic

<400>  905
catgatcagc tgggccaaga actgtgg                                           27


<210>  906
<211>  16
<212>  DNA
<213>  Artificial Sequence

<220>
<223>  Synthetic


<220>
<221>  misc_feature
<222>  (2)..(2)
<223>  Nucleotide at position 2 may be modified by
       2'-O,4'-C-methylene-beta-D-ribofuranosyl moiety

<400>  906
tggtgcggaa agggcc                                                       16

<210>  907
<211>  14
<212>  DNA
<213>  Artificial Sequence

<220>
<223>  Synthetic


<220>
<221>  misc_feature
<222>  (2)..(2)
<223>  Nucleotide at position 2 may be modified by
       2'-O,4'-C-methylene-beta-D-ribofuranosyl moiety

<400>  907
tggtgcggaa aggg                                                          14


<210>  908
<211>  30
<212>  DNA
<213>  Artificial Sequence

<220>
<223>  Synthetic

<400>  908
catgatcagc tgggccaaga gtagtgggcc                                        30


<210>  909
<211>  29
<212>  DNA
<213>  Artificial Sequence

<220>
<223>  Synthetic

<400>  909
catgatcagc tgggccaaga gtagtgggc                                          29


<210>  910
<211>  28
<212>  DNA
<213>  Artificial Sequence

<220>
<223>  Synthetic

<400>  910
catgatcagc tgggccaaga gtagtggg                                           28


<210>  911
<211>  27
<212>  DNA
<213>  Artificial Sequence

<220>
<223>  Synthetic

<400>  911
catgatcagc tgggccaaga gtagtgg                                            27

<210>  912
<211>  17
<212>  DNA
<213>  Artificial Sequence

<220>
<223>  Synthetic


<220>
<221>  misc_feature
<222>  (3)..(3)
<223>  Nucleotide at position 3 may be modified by
       2'-O,4'-C-methylene-beta-D-ribofuranosyl moiety

<400>  912
tgtcactcgg ctcggcc                                                      17


<210>  913
<211>  15
<212>  DNA
<213>  Artificial Sequence

<220>
<223>  Synthetic


<220>
<221>  misc_feature
<222>  (3)..(3)
<223>  Nucleotide at position 3 may be modified by
       2'-O,4'-C-methylene-beta-D-ribofuranosyl moiety

<400>  913
tgtcactcgg ctcgg                                                        15


<210>  914
<211>  30
<212>  DNA
<213>  Artificial Sequence

<220>
<223>  Synthetic

<400>  914
catgatcagc tgggccaaga agaggcaggc                                        30


<210>  915
<211>  29
<212>  DNA
<213>  Artificial Sequence

<220>
<223>  Synthetic

<400>  915
catgatcagc tgggccaaga agaggcagg                                         29


<210>  916

<211>  28
<212>  DNA
<213>  Artificial Sequence

<220>
<223>  Synthetic

<400>  916
catgatcagc tgggccaaga agaggcag                                          28


<210>  917
<211>  27
<212>  DNA
<213>  Artificial Sequence

<220>
<223>  Synthetic

<400>  917
catgatcagc tgggccaaga agaggca                                           27


<210>  918
<211>  18
<212>  DNA
<213>  Artificial Sequence

<220>
<223>  Synthetic


<220>
<221>  misc_feature
<222>  (5)..(5)
<223>  Nucleotide at position 5 may be modified by
       2'-O,4'-C-methylene-beta-D-ribofuranosyl moiety

<400>  918
tgtctgcctg agtgcctg                                                     18


<210>  919
<211>  16
<212>  DNA
<213>  Artificial Sequence

<220>
<223>  Synthetic


<220>
<221>  misc_feature
<222>  (5)..(5)
<223>  Nucleotide at position 5 may be modified by
       2'-O,4'-C-methylene-beta-D-ribofuranosyl moiety

<400>  919
tgtctgcctg agtgcc                                                       16


<210>  920
<211>  30
<212>  DNA
<213>  Artificial Sequence

<220>
<223>  Synthetic

<400>  920
catgatcagc tgggccaaga ttcagttatc                                        30


<210>  921
<211>  29
<212>  DNA
<213>  Artificial Sequence

<220>
<223>  Synthetic

<400>  921
catgatcagc tgggccaaga ttcagttat                                         29


<210>  922
<211>  28
<212>  DNA
<213>  Artificial Sequence

<220>
<223>  Synthetic

<400>  922
catgatcagc tgggccaaga ttcagtta                                          28


<210>  923
<211>  27
<212>  DNA
<213>  Artificial Sequence

<220>
<223>  Synthetic

<400>  923
catgatcagc tgggccaaga ttcagtt                                           27


<210>  924
<211>  18
<212>  DNA
<213>  Artificial Sequence

<220>
<223>  Synthetic


<220>
<221>  misc_feature
<222>  (3)..(6)
<223>  Nucleotide at position 3 and/or 6 may be modified by
       2'-O,4'-C-methylene-beta-D-ribofuranosyl moiety

<400>  924
tgtgacagat tgataact                                                     18


<210>  925
<211>  16

<212>  DNA
<213>  Artificial Sequence

<220>
<223>  Synthetic


<220>
<221>  misc_feature
<222>  (3)..(6)
<223>  Nucleotide at position 3 and/or 4 and/or 6 may be modified by
       2'-O,4'-C-methylene-beta-D-ribofuranosyl moiety

<400>  925
tgtgacagat tgataa                                                    16


<210>  926
<211>  32
<212>  DNA
<213>  Artificial Sequence

<220>
<223>  Synthetic

<400>  926
catgatcagc tgggccaaga cgtgacatga tg                                  32


<210>  927
<211>  29
<212>  DNA
<213>  Artificial Sequence

<220>
<223>  Synthetic

<400>  927
catgatcagc tgggccaaga cgtgacatg                                      29


<210>  928
<211>  28
<212>  DNA
<213>  Artificial Sequence

<220>
<223>  Synthetic

<400>  928
catgatcagc tgggccaaga cgtgacat                                       28


<210>  929
<211>  27
<212>  DNA
<213>  Artificial Sequence

<220>
<223>  Synthetic

<400>  929
catgatcagc tgggccaaga cgtgaca                                        27

<210>  930
<211>  16
<212>  DNA
<213>  Artificial Sequence

<220>
<223>  Synthetic


<220>
<221>  misc_feature
<222>  (4)..(6)
<223>  Nucleotide at position 4 and/or 6 may be modified by
       2'-O,4'-C-methylene-beta-D-ribofuranosyl moiety

<400>  930
ctcggggatc atcatg                                                    16


<210>  931
<211>  15
<212>  DNA
<213>  Artificial Sequence

<220>
<223>  Synthetic


<220>
<221>  misc_feature
<222>  (2)..(4)
<223>  Nucleotide at position 2 and/or 4 may be modified by
       2'-O,4'-C-methylene-beta-D-ribofuranosyl moiety

<400>  931
ctcggggatc atcat                                                     15


<210>  932
<211>  30
<212>  DNA
<213>  Artificial Sequence

<220>
<223>  Synthetic

<400>  932
catgatcagc tgggccaaga aaacatcact                                     30


<210>  933
<211>  29
<212>  DNA
<213>  Artificial Sequence

<220>
<223>  Synthetic

<400>  933
catgatcagc tgggccaaga aaacatcac                                      29


<210>  934
<211>  28
<212>  DNA

<213>  Artificial Sequence

<220>
<223>  Synthetic

<400>  934
catgatcagc tgggccaaga aaacatca                                          28


<210>  935
<211>  27
<212>  DNA
<213>  Artificial Sequence

<220>
<223>  Synthetic

<400>  935
catgatcagc tgggccaaga aaacatc                                           27


<210>  936
<211>  17
<212>  DNA
<213>  Artificial Sequence

<220>
<223>  Synthetic


<220>
<221>  misc_feature
<222>  (2)..(7)
<223>  Nucleotide at position 2 and/or 5 and/or 7 may be modified by
       2'-O,4'-C-methylene-beta-D-ribofuranosyl moiety

<400>  936
ttaagacttg cagtgat                                                      17


<210>  937
<211>  15
<212>  DNA
<213>  Artificial Sequence

<220>
<223>  Synthetic


<220>
<221>  misc_feature
<222>  (2)..(7)
<223>  Nucleotide at position 2 and/or 5 and/or 7 may be modified by
       2'-O,4'-C-methylene-beta-D-ribofuranosyl moiety

<400>  937
ttaagacttg cagtg                                                        15


<210>  938
<211>  30
<212>  DNA
<213>  Artificial Sequence

<220>

<223>  Synthetic

<400>  938
catgatcagc tgggccaaga gttagtggac                                        30


<210>  939
<211>  29
<212>  DNA
<213>  Artificial Sequence

<220>
<223>  Synthetic

<400>  939
catgatcagc tgggccaaga gttagtgga                                         29


<210>  940
<211>  28
<212>  DNA
<213>  Artificial Sequence

<220>
<223>  Synthetic

<400>  940
catgatcagc tgggccaaga gttagtgg                                          28


<210>  941
<211>  27
<212>  DNA
<213>  Artificial Sequence

<220>
<223>  Synthetic

<400>  941
catgatcagc tgggccaaga gttagtg                                           27


<210>  942
<211>  17
<212>  DNA
<213>  Artificial Sequence

<220>
<223>  Synthetic


<220>
<221>  misc_feature
<222>  (3)..(5)
<223>  Nucleotide at position 3 and/or 5 may be modified by
       2'-O,4'-C-methylene-beta-D-ribofuranosyl moiety

<400>  942
tttgtgacct ggtccac                                                      17


<210>  943
<211>  15
<212>  DNA
<213>  Artificial Sequence

<220>
<223>  Synthetic


<220>
<221>  misc_feature
<222>  (3)..(6)
<223>  Nucleotide at position 3 and/or 5 and/or 6 may be modified by
      2'-O,4'-C-methylene-beta-D-ribofuranosyl moiety

<400>  943
tttgtgacct ggtcc                                                        15


<210>  944
<211>  30
<212>  DNA
<213>  Artificial Sequence

<220>
<223>  Synthetic

<400>  944
catgatcagc tgggccaaga tccacatgga                                        30


<210>  945
<211>  29
<212>  DNA
<213>  Artificial Sequence

<220>
<223>  Synthetic

<400>  945
catgatcagc tgggccaaga tccacatgg                                         29


<210>  946
<211>  28
<212>  DNA
<213>  Artificial Sequence

<220>
<223>  Synthetic

<400>  946
catgatcagc tgggccaaga tccacatg                                          28


<210>  947
<211>  27
<212>  DNA
<213>  Artificial Sequence

<220>
<223>  Synthetic

<400>  947
catgatcagc tgggccaaga tccacat                                           27


<210>  948
<211>  16

<212>  DNA
<213>  Artificial Sequence

<220>
<223>  Synthetic


<220>
<221>  misc_feature
<222>  (3)..(6)
<223>  Nucleotide at position 3 and/or 6 may be modified by
       2'-O,4'-C-methylene-beta-D-ribofuranosyl moiety

<400>  948
tgtaacagca actcca                                                    16


<210>  949
<211>  17
<212>  DNA
<213>  Artificial Sequence

<220>
<223>  Synthetic


<220>
<221>  misc_feature
<222>  (3)..(8)
<223>  Nucleotide at position 3 and/or 6 and/or 8 may be modified by
       2'-O,4'-C-methylene-beta-D-ribofuranosyl moiety

<400>  949
tgtaacagca actccat                                                   17


<210>  950
<211>  30
<212>  DNA
<213>  Artificial Sequence

<220>
<223>  Synthetic

<400>  950
catgatcagc tgggccaaga ccacacactt                                     30


<210>  951
<211>  29
<212>  DNA
<213>  Artificial Sequence

<220>
<223>  Synthetic

<400>  951
catgatcagc tgggccaaga ccacacact                                      29


<210>  952
<211>  28
<212>  DNA
<213>  Artificial Sequence

<220>
<223>  Synthetic

<400>  952
catgatcagc tgggccaaga ccacacac                                          28


<210>  953
<211>  27
<212>  DNA
<213>  Artificial Sequence

<220>
<223>  Synthetic

<400>  953
catgatcagc tgggccaaga ccacaca                                           27


<210>  954
<211>  16
<212>  DNA
<213>  Artificial Sequence

<220>
<223>  Synthetic


<220>
<221>  misc_feature
<222>  (2)..(6)
<223>  Nucleotide at position 2 and/or 6 may be modified by
       2'-O,4'-C-methylene-beta-D-ribofuranosyl moiety

<400>  954
tggaatgtaa ggaagt                                                       16


<210>  955
<211>  18
<212>  DNA
<213>  Artificial Sequence

<220>
<223>  Synthetic


<220>
<221>  misc_feature
<222>  (2)..(6)
<223>  Nucleotide at position 2 and/or 3 and/or 6 may be modified by
       2'-O,4'-C-methylene-beta-D-ribofuranosyl moiety

<400>  955
tggaatgtaa ggaagtgt                                                     18


<210>  956
<211>  29
<212>  DNA
<213>  Artificial Sequence

<220>
<223>  Synthetic

<400>  956
catgatcagc tgggccaaga tacatactt                                         29


<210>  957
<211>  28
<212>  DNA
<213>  Artificial Sequence

<220>
<223>  Synthetic

<400>  957
catgatcagc tgggccaaga tacatact                                          28


<210>  958
<211>  27
<212>  DNA
<213>  Artificial Sequence

<220>
<223>  Synthetic

<400>  958
catgatcagc tgggccaaga tacatac                                           27


<210>  959
<211>  17
<212>  DNA
<213>  Artificial Sequence

<220>
<223>  Synthetic


<220>
<221>  misc_feature
<222>  (3)..(8)
<223>  Nucleotide at position 3 and/or 6 and/or 8 may be modified by
       2'-O,4'-C-methylene-beta-D-ribofuranosyl moiety

<400>  959
tggaatgtaa agaagta                                                      17


<210>  960
<211>  30
<212>  DNA
<213>  Artificial Sequence

<220>
<223>  Synthetic

<400>  960
catgatcagc tgggccaaga tcatacagct                                        30


<210>  961
<211>  29
<212>  DNA
<213>  Artificial Sequence

<220>

<223>  Synthetic

<400>  961
catgatcagc tgggccaaga tcatacagc                                        29


<210>  962
<211>  28
<212>  DNA
<213>  Artificial Sequence

<220>
<223>  Synthetic

<400>  962
catgatcagc tgggccaaga tcatacag                                         28


<210>  963
<211>  27
<212>  DNA
<213>  Artificial Sequence

<220>
<223>  Synthetic

<400>  963
catgatcagc tgggccaaga tcataca                                          27


<210>  964
<211>  17
<212>  DNA
<213>  Artificial Sequence

<220>
<223>  Synthetic


<220>
<221>  misc_feature
<222>  (2)..(6)
<223>  Nucleotide at position 2 and/or 6 may be modified by
       2'-O,4'-C-methylene-beta-D-ribofuranosyl moiety

<400>  964
tctttggtta tctagct                                                     17


<210>  965
<211>  20
<212>  DNA
<213>  Artificial Sequence

<220>
<223>  Synthetic


<220>
<221>  misc_feature
<222>  (3)..(10)
<223>  Nucleotide at position 3 and/or 6 and/or 10 may be modified by
       2'-O,4'-C-methylene-beta-D-ribofuranosyl moiety

<400>  965

tctttggtta tctagctgta                                                    20

<210>  966
<211>  22
<212>  RNA
<213>  Artificial Sequence

<220>
<223>  Synthetic

<400>  966
aaacaaacau ggugcacuuc uu                                                  22

<210>  967
<211>  23
<212>  RNA
<213>  Artificial Sequence

<220>
<223>  Synthetic

<400>  967
aaagugcuuc cacuuugugu gcc                                                 23

<210>  968
<211>  23
<212>  RNA
<213>  Artificial Sequence

<220>
<223>  Synthetic

<400>  968
aaagugcauc cauuuuguuu guc                                                 23

<210>  969
<211>  25
<212>  RNA
<213>  Artificial Sequence

<220>
<223>  Synthetic

<400>  969
aaagugcauc cuuuuagagu guuac                                               25

<210>  970
<211>  23
<212>  RNA
<213>  Artificial Sequence

<220>
<223>  Synthetic

<400>  970
aaagugcauc cuuuuagagg uuu                                                 23

<210>  971
<211>  22

<212>  RNA
<213>  Artificial Sequence

<220>
<223>  Synthetic

<400>  971
aaagugcauc uuuuuagagg au                                              22


<210>  972
<211>  23
<212>  RNA
<213>  Artificial Sequence

<220>
<223>  Synthetic

<400>  972
caaagugccu cccuuuagag ugu                                             23


<210>  973
<211>  22
<212>  RNA
<213>  Artificial Sequence

<220>
<223>  Synthetic

<400>  973
aaagugcuuc ccuuuggacu gu                                              22


<210>  974
<211>  21
<212>  RNA
<213>  Artificial Sequence

<220>
<223>  Synthetic

<400>  974
aaagugcuuc cuuuuagagg g                                               21


<210>  975
<211>  23
<212>  RNA
<213>  Artificial Sequence

<220>
<223>  Synthetic

<400>  975
aaagugcuuc ucuuuggugg guu                                             23


<210>  976
<211>  21
<212>  RNA
<213>  Artificial Sequence

<220>
<223>  Synthetic

<400>  976
aaagugcuuc cuuuuugagg g                                                21


<210>  977
<211>  22
<212>  RNA
<213>  Artificial Sequence

<220>
<223>  Synthetic

<400>  977
aagugcuucc uuuuagaggg uu                                               22


<210>  978
<211>  22
<212>  RNA
<213>  Artificial Sequence

<220>
<223>  Synthetic

<400>  978
aacacaccca gcuaaccuuu uu                                               22


<210>  979
<211>  24
<212>  RNA
<213>  Artificial Sequence

<220>
<223>  Synthetic

<400>  979
aacauucauu guugucggug gguu                                             24


<210>  980
<211>  24
<212>  RNA
<213>  Artificial Sequence

<220>
<223>  Synthetic

<400>  980
aacuggcccu caaagucccg cuuu                                             24


<210>  981
<211>  24
<212>  RNA
<213>  Artificial Sequence

<220>
<223>  Synthetic

<400>  981
aauccuugga accuaggugu gagu                                             24

<210>  982
<211>  23
<212>  RNA
<213>  Artificial Sequence

<220>
<223>  Synthetic

<400>  982
aauccuugga accuaggugu gaa                                                23


<210>  983
<211>  22
<212>  RNA
<213>  Artificial Sequence

<220>
<223>  Synthetic

<400>  983
augcaccugg gcaaggauuc ug                                                 22


<210>  984
<211>  22
<212>  RNA
<213>  Artificial Sequence

<220>
<223>  Synthetic

<400>  984
augcaccugg gcaaggguuc ag                                                 22


<210>  985
<211>  22
<212>  RNA
<213>  Artificial Sequence

<220>
<223>  Synthetic

<400>  985
aauccuuugu cccuggguga ga                                                 22


<210>  986
<211>  22
<212>  RNA
<213>  Artificial Sequence

<220>
<223>  Synthetic

<400>  986
aauccuuugu cccuggguga aa                                                 22


<210>  987
<211>  21
<212>  RNA
<213>  Artificial Sequence

<220>
<223>  Synthetic

<400>  987
aaucguacag ggucauccac u                                                21


<210>  988
<211>  23
<212>  RNA
<213>  Artificial Sequence

<220>
<223>  Synthetic

<400>  988
agugacauca cauauacggc agc                                              23


<210>  989
<211>  23
<212>  RNA
<213>  Artificial Sequence

<220>
<223>  Synthetic

<400>  989
aaugacacca cauauauggc agc                                              23


<210>  990
<211>  23
<212>  RNA
<213>  Artificial Sequence

<220>
<223>  Synthetic

<400>  990
aaugacauca cauauauggc agc                                              23


<210>  991
<211>  23
<212>  RNA
<213>  Artificial Sequence

<220>
<223>  Synthetic

<400>  991
   aaugacacga ucacucccgu uga                                             23


<210>  992
<211>  23
<212>  RNA
<213>  Artificial Sequence

<220>
<223>  Synthetic

<400>  992
aauggcgcca cuaggguugu gca                                              23

<210>  993
<211>  22
<212>  RNA
<213>  Artificial Sequence

<220>
<223>  Synthetic

<400>  993
agaggcuggc cgugaugaau uc                                                22


<210>  994
<211>  23
<212>  RNA
<213>  Artificial Sequence

<220>
<223>  Synthetic

<400>  994
agucauacac ggcucuccuc ucu                                               23


<210>  995
<211>  21
<212>  RNA
<213>  Artificial Sequence

<220>
<223>  Synthetic

<400>  995
agaucgaccg uguuauauuc g                                                 21


<210>  996
<211>  24
<212>  RNA
<213>  Artificial Sequence

<220>
<223>  Synthetic

<400>  996
aggaagcccu ggaggggcug gagg                                              24


<210>  997
<211>  22
<212>  RNA
<213>  Artificial Sequence

<220>
<223>  Synthetic

<400>  997
aggcagugua uuguuagcug gc                                                22


<210>  998
<211>  21
<212>  RNA

<213>  Artificial Sequence

<220>
<223>  Synthetic

<400>  998
aggcagugca uugcuagcug g                                                21


<210>  999
<211>  20
<212>  RNA
<213>  Artificial Sequence

<220>
<223>  Synthetic

<400>  999
aggcagugcg accuggcucg                                                  20


<210>  1000
<211>  22
<212>  RNA
<213>  Artificial Sequence

<220>
<223>  Synthetic

<400>  1000
agguuacccg agcaacuuug ca                                               22


<210>  1001
<211>  23
<212>  RNA
<213>  Artificial Sequence

<220>
<223>  Synthetic

<400>  1001
gaauguugcu cggugaaccc cuu                                              23


<210>  1002
<211>  22
<212>  RNA
<213>  Artificial Sequence

<220>
<223>  Synthetic

<400>  1002
aguggggaac ccuuccauga gg                                               22


<210>  1003
<211>  20
<212>  RNA
<213>  Artificial Sequence

<220>
<223>  Synthetic

<400>  1003
auuccuagaa auuguucaua                                                20


<210>  1004
<211>  20
<212>  RNA
<213>  Artificial Sequence

<220>
<223>  Synthetic

<400>  1004
auuccuagaa auuguucaca                                                20


<210>  1005
<211>  23
<212>  RNA
<213>  Artificial Sequence

<220>
<223>  Synthetic

<400>  1005
caaagugcuc auagugcagg uag                                            23


<210>  1006
<211>  22
<212>  RNA
<213>  Artificial Sequence

<220>
<223>  Synthetic

<400>  1006
caaccuggag gacuccaugc ug                                             22


<210>  1007
<211>  21
<212>  RNA
<213>  Artificial Sequence

<220>
<223>  Synthetic

<400>  1007
cagcagcaca cugugguuug u                                              21


<210>  1008
<211>  24
<212>  RNA
<213>  Artificial Sequence

<220>
<223>  Synthetic

<400>  1008
cagugcaaug auauugucaa agca                                           24


<210>  1009

<211>  24
<212>  RNA
<213>  Artificial Sequence

<220>
<223>  Synthetic

<400>  1009
cagugcaaug guauugucaa agca                                              24


<210>  1010
<211>  21
<212>  RNA
<213>  Artificial Sequence

<220>
<223>  Synthetic

<400>  1010
cagugcaauu aaaaggggga a                                                 21


<210>  1011
<211>  22
<212>  RNA
<213>  Artificial Sequence

<220>
<223>  Synthetic

<400>  1011
caugccuuga guguaggacc gu                                                22


<210>  1012
<211>  21
<212>  RNA
<213>  Artificial Sequence

<220>
<223>  Synthetic

<400>  1012
cccagauaau ggcacucuca a                                                 21


<210>  1013
<211>  21
<212>  RNA
<213>  Artificial Sequence

<220>
<223>  Synthetic

<400>  1013
cccagauaau agcacucuca a                                                 21


<210>  1014
<211>  22
<212>  RNA
<213>  Artificial Sequence

<220>

<223>  Synthetic

<400>  1014
ggagaaauua uccuuggugu gu                                                22


<210>  1015
<211>  22
<212>  RNA
<213>  Artificial Sequence

<220>
<223>  Synthetic

<400>  1015
gucaacacuu gcugguuucc uc                                                22


<210>  1016
<211>  22
<212>  RNA
<213>  Artificial Sequence

<220>
<223>  Synthetic

<400>  1016
cgucaacacu ugcugguuuu cu                                                22


<210>  1017
<211>  22
<212>  RNA
<213>  Artificial Sequence

<220>
<223>  Synthetic

<400>  1017
uaaggugcau cuagugcagu ua                                                22


<210>  1018
<211>  22
<212>  RNA
<213>  Artificial Sequence

<220>
<223>  Synthetic

<400>  1018
uaaggugcau cuagugcugu ua                                                22


<210>  1019
<211>  21
<212>  RNA
<213>  Artificial Sequence

<220>
<223>  Synthetic

<400>  1019
uagcagcggg aacaguacug c                                                 21

<210>  1020
<211>  22
<212>  RNA
<213>  Artificial Sequence

<220>
<223>  Synthetic

<400>  1020
uaugugccuu uggacuacau cg                                                22


<210>  1021
<211>  21
<212>  RNA
<213>  Artificial Sequence

<220>
<223>  Synthetic

<400>  1021
uauugcacuc gucccggccu c                                                 21


<210>  1022
<211>  23
<212>  RNA
<213>  Artificial Sequence

<220>
<223>  Synthetic

<400>  1022
ucacuccucu ccucccgucu ucu                                               23


<210>  1023
<211>  23
<212>  RNA
<213>  Artificial Sequence

<220>
<223>  Synthetic

<400>  1023
ucacuccucc ccucccgucu ugu                                               23


<210>  1024
<211>  22
<212>  RNA
<213>  Artificial Sequence

<220>
<223>  Synthetic

<400>  1024
ucaggcucag uccccucccg au                                                22


<210>  1025
<211>  24
<212>  RNA
<213>  Artificial Sequence

<220>
<223>  Synthetic

<400>  1025
ucccugagga gcccuuugag ccug                                              24


<210>  1026
<211>  21
<212>  RNA
<213>  Artificial Sequence

<220>
<223>  Synthetic

<400>  1026
uccgagccug ggucucccuc u                                                 21


<210>  1027
<211>  22
<212>  RNA
<213>  Artificial Sequence

<220>
<223>  Synthetic

<400>  1027
uccuguacug agcugccccg ag                                                22


<210>  1028
<211>  21
<212>  RNA
<213>  Artificial Sequence

<220>
<223>  Synthetic

<400>  1028
ugaaacauac acgggaaacc u                                                 21


<210>  1029
<211>  21
<212>  RNA
<213>  Artificial Sequence

<220>
<223>  Synthetic

<400>  1029
ugaaggucua cugugugcca g                                                 21


<210>  1030
<211>  22
<212>  RNA
<213>  Artificial Sequence

<220>
<223>  Synthetic

<400>  1030

ugagaacuga auuccauagg cu                                                22

<210>  1031
<211>  21
<212>  RNA
<213>  Artificial Sequence

<220>
<223>  Synthetic

<400>  1031
uggaauguaa agaagugugu a                                                 21

<210>  1032
<211>  23
<212>  RNA
<213>  Artificial Sequence

<220>
<223>  Synthetic

<400>  1032
uggugcggag agggcccaca gug                                               23

<210>  1033
<211>  22
<212>  RNA
<213>  Artificial Sequence

<220>
<223>  Synthetic

<400>  1033
uggugcggaa agggcccaca gu                                                22

<210>  1034
<211>  23
<212>  RNA
<213>  Artificial Sequence

<220>
<223>  Synthetic

<400>  1034
ugucacucgg cucggcccac uac                                               23

<210>  1035
<211>  23
<212>  RNA
<213>  Artificial Sequence

<220>
<223>  Synthetic

<400>  1035
ugucugccug agugccugcc ucu                                               23

<210>  1036
<211>  22

<212>  RNA
<213>  Artificial Sequence

<220>
<223>  Synthetic

<400>  1036
ugugacagau ugauaacuga aa                                              22


<210>  1037
<211>  21
<212>  RNA
<213>  Artificial Sequence

<220>
<223>  Synthetic

<400>  1037
cucggggauc aucaugucac g                                               21


<210>  1038
<211>  21
<212>  RNA
<213>  Artificial Sequence

<220>
<223>  Synthetic

<400>  1038
uuaagacuug cagugauguu u                                               21


<210>  1039
<211>  22
<212>  RNA
<213>  Artificial Sequence

<220>
<223>  Synthetic

<400>  1039
uuugugaccu gguccacuaa cc                                              22


<210>  1040
<211>  22
<212>  RNA
<213>  Artificial Sequence

<220>
<223>  Synthetic

<400>  1040
uguaacagca acuccaugug ga                                              22


<210>  1041
<211>  22
<212>  RNA
<213>  Artificial Sequence

<220>
<223>  Synthetic

<400>  1041
uggaauguaa ggaagugugu gg                                                22

<210>  1042
<211>  21
<212>  RNA
<213>  Artificial Sequence

<220>
<223>  Synthetic

<400>  1042
uggaauguaa agaaguaugu a                                                 21

<210>  1043
<211>  23
<212>  RNA
<213>  Artificial Sequence

<220>
<223>  Synthetic

<400>  1043
ucuuugguua ucuagcugua uga                                               23

## Description

### FIELD OF THE INVENTION

The present invention relates to methods, compositions and kits for amplifying and quantitating small RNA molecules.

### BACKGROUND OF THE INVENTION

RNA interference (RNAi) is an evolutionarily conserved process that functions to inhibit gene expression (Bernstein et aL (2001), Nature 409:363-6; Dykxhoorn et aL (2003) Nat. Rev. Mol. Cell. Biol. 4:457-67). The phenomenon of RNAi was first described in *Caenorhabditis elegans,* where injection of double-stranded RNA (dsRNA) led to efficient sequence-specific gene silencing of the mRNA that was complementary to the dsRNA (Fire et al. (1998) Nature 391:806-11). RNAi has also been described in plants as a phenomenon called post-transcriptional gene silencing (PTGS), which is likely used as a viral defense mechanism (Jorgensen (1990) Trends Biotechnol. 8:340-4; Brigneti et al. (1998) EMBO J. 17:6739-46; Hamilton & Baulcombe (1999) Science 286:950-2).

An early indication that the molecules that regulate PTGS were short RNAs processed from longer dsRNA was the identification of short 21 to 22 nucleotide dsRNA derived from the longer dsRNA in plants (Hamilton & Baulcombe (1999) Science 286:950-2). This observation was repeated in *Drosophila* embryo extracts where long dsRNA was found processed into 21-25 nucleotide short RNA by the RNase III type enzyme, Dicer (Elbashir et al. (2001) Nature 411:494-8; Elbashir et al. (2001) EMBO J. 20:6877-88; Elbashir et al. (2001) Genes Dev. 15:188-200). These observations led Elbashir et al. to test if synthetic 21-25 nucleotide synthetic dsRNAs function to specifically inhibit gene expression in *Drosophila* embryo lysates and mammalian cell culture (Elbashir et al. (2001) Nature 411:494-8; Elbashir et al. (2001) EMBO J. 20:6877-88; Elbashir et al. (2001) Genes Dev. 15:188-200). They demonstrated that small interfering RNAs (siRNAs) had the ability to specifically inhibit gene expression in mammalian cell culture without induction of the interferon response.

These observations led to the development of techniques for the reduction, or elimination, of expression of specific genes in mammalian cell culture, such as plasmid-based systems that generate hairpin siRNAs (Brummelkamp et al. (2002) Science 296:550-3; Paddison et al. (2002) Genes Dev. 16:948-58; Paddison et al. (2002) Proc. Natl. Acad. Sci. U.S.A. 99:1443-8; Paul et al. 2002) Nat. Biotechnol. 20:404-8). siRNA molecules can also be introduced into cells, *in vivo,* to inhibit the expression of specific proteins (see, e.g., Soutschek, J., et al., Nature 432 (7014):173-178 (2004)).

siRNA molecules have promise both as therapeutic agents for inhibiting the expression of specific proteins, and as targets for drugs that affect the activity of siRNA molecules that function to regulate the expression of proteins involved in a disease state. A first step in developing such therapeutic agents is to measure the amounts of specific siRNA molecules in different cell types within an organism, and thereby construct an "atlas" of siRNA expression within the body. Additionally, it will be useful to measure changes in the amount of specific siRNA molecules in specific cell types in response to a defined stimulus, or in a disease state.

Short RNA molecules are difficult to quantitate. For example, with respect to the use of PCR to amplify and measure the small RNA molecules, most PCR primers are longer than the small RNA molecules, and so it is difficult to design a primer that has significant overlap with a small RNA molecule, and that selectively hybridizes to the small RNA molecule at the temperatures used for primer extension and PCR amplification reactions.

### SUMMARY OF THE INVENTION

In one aspect, the present invention provides a kit for detecting at least one mammalian microRNA, the kit comprising at least one oligonucleotide primer selected from the group consisting of SEQ ID NO:500 to SEQ ID NO:965. In some embodiments, the kit comprises at least two oligonucleotide primers selected from the group consisting of SEQ ID NO:500 to SEQ ID NO:965. In some embodiments, the at least one oligonucleotide primer comprises a 2'-O,4'-C-methylene-β-D-ribofuranosyl moiety.

In another aspect, the present invention provides an oligonucleotide primer for detecting a mammalian microRNA selected from the group consisting of SEQ ID NO:500 to SEQ ID NO:965. The primer molecules of the invention can be used as primers for detecting mammalian microRNA target molecules, using the methods of the invention described herein.

In another aspect, the present invention provides a kit for detecting at least one mammalian target microRNA. The kit comprises at least one primer set specific for the detection of a target microRNA, the primer set comprising: (1) an extension primer for producing a cDNA molecule complementary to a target microRNA, the extension primer comprising a first portion that hybridizes to a target microRNA and a second portion having a hybridization sequence for a universal forward PCR primer; (2) a universal forward PCR primer for amplifying the cDNA molecule, comprising a sequence selected to hybridize to the hybridization sequence on the extension primer; and (3) a reverse PCR primer for amplifying the cDNA molecule, comprising a sequence selected to hybridize to a portion of the cDNA molecule, wherein the at least one target microRNA is selected from the group consisting of miR-1, miR-9, miR-18b, miR-20b, miR-92b, miR-146b, miR-181d, miR-193b, miR-194, miR-206, miR-291a-3p, miR-291b-3p, miR-301b, miR-329, miR-346, miR-351, miR-362, miR-362-3p, miR-369-5p, miR-384, miR-409-3p, miR-409-5p, miR-425-5p, miR-449b, miR-455, miR-483, miR-484, miR-485-3p, miR-485-5p, miR-486, miR-487b, miR-488, miR-489, miR-490, miR-491, miR-493-3p, miR-494, miR-495, miR-497, miR-499, miR-500, miR-501, miR-503, miR-505, miR-519a, miR-519b, miR-519c, miR-519d, miR-520a, miR-520b, miR-520d, miR-520e, miR-520f, miR-532, miR-539, miR-542-3p, miR-542-5p, miR-615, miR-652, miR-668, miR-671, miR-675-5p, miR-699, miR-721 and miR-758. In one embodiment, at least one of the universal forward and reverse PCR primers includes at least one nucleic acid molecule comprising a 2'-O,4'-C-methylene-β-D-ribofuranosyl moiety.

In another aspect, the present invention provides a method for discriminating between two or more mammalian target microRNA that have a similar sequence in a sample from a living organism. The method comprises the steps of: (a) producing a first DNA molecule that is complementary to the first microRNA molecule using a first extension primer specific to the first microRNA molecule; (b) amplifying the first DNA molecule to produce a first population of amplified DNA molecules using a universal forward primer and a first reverse primer; (c) producing a second DNA molecule that is complementary to the second microRNA molecule using a second extension primer specific to the second microRNA molecule; (d) amplifying the second DNA molecule to produce a second population of amplified DNA molecules using a universal forward primer and a second reverse primer; and (e) measuring the amount of the first and second population of amplified DNA molecules, wherein the first and second extension primers or the first and second reverse primers differ by one or more nucleotides in the portion that is complementary to the target microRNA.

In another aspect, the present invention provides methods for amplifying a microRNA molecule to produce cDNA molecules. The methods include the steps of: (a) producing a first DNA molecule that is complementary to a target microRNA molecule using primer extension; and (b) amplifying the first DNA molecule to produce amplified DNA molecules using a universal forward primer and a reverse primer. In some embodiments of the method, at least one of the forward primer and the reverse primer comprise at least one locked nucleic acid molecule. It will be understood that, in the practice of the present invention, typically numerous (e.g., millions) of individual microRNA molecules are amplified in a sample (e.g., a solution of RNA molecules isolated from living cells).

In another aspect, the present invention provides methods for measuring the amount of a target microRNA in a sample from a living organism. The methods of this aspect of the invention include the step of measuring the amount of a target microRNA molecule in a multiplicity of different cell types within a living organism, wherein the amount of the target microRNA molecule is measured by a method including the steps of: (1) producing a first DNA molecule complementary to the target microRNA molecule in the sample using primer extension; (2) amplifying the first DNA molecule to produce amplified DNA molecules using a universal forward primer and a reverse primer; and (3) measuring the amount of the amplified DNA molecules. In some embodiments of the method, at least one of the forward primer and the reverse primer comprise at least one locked nucleic acid molecule.

In another aspect, the invention provides nucleic acid primer molecules consisting of sequence SEQ ID NO:1 to SEQ ID NO: 499, as shown in TABLE 1, TABLE 2, TABLE 6, and TABLE 7. The primer molecules of the invention can be used as primers for detecting mammalian microRNA target molecules, using the methods of the invention described herein.

In another aspect, the present invention provides kits for detecting at least one mammalian target microRNA, the kits comprising one or more primer sets specific for the detection of a target microRNA, each primer set comprising (1) an extension primer for producing a cDNA molecule complementary to a target microRNA, (2) a universal forward PCR primer for amplifying the cDNA molecule and (3) a reverse PCR primer for amplifying the cDNA molecule. The extension primer comprises a first portion that hybridizes to the target microRNA molecule and a second portion that includes a hybridization sequence for a universal forward PCR primer. The reverse PCR primer comprises a sequence selected to hybridize to a portion of the cDNA molecule. In some embodiments of the kit, at least one of the universal forward and reverse primers include at least one locked nucleic acid molecule. The kits of the invention may be used to practice various embodiments of the methods of the invention.

The methods, compositions and kits of the present invention are useful, for example, for quantitating specific microRNA molecules within different types of cells in a living organism, or, for example, for measuring changes in the amount of specific microRNAs in living cells in response to a stimulus (e-g., in response to administration of a drug).

### BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
FIGURE 1 shows a flow chart of a representative method of the present invention;
FIGURE 2 graphically illustrates the standard curves for assays specific for the detection of microRNA targets miR-95 and miR-424 as described in EXAMPLE 3;
FIGURE 3A is a histogram plot showing the expression profile of miR-1 across a panel of total RNA isolated from twelve tissues as described in EXAMPLE 5;
FIGURE 3B is a histogram plot showing the expression profile of miR-124 across a panel of total RNA isolated from twelve tissues as described in EXAMPLE 5; and
FIGURE 3C is a histogram plot showing the expression profile of miR-150 across a panel of total RNA isolated from twelve tissues as described in EXAMPLE 5.

### DETAILED DESCRIPTION OF THE PREFERRED EMBODIMENT

In accordance with the foregoing, in one aspect, the present invention provides methods for amplifying a microRNA molecule to produce cDNA molecules. The methods include the steps of: (a) using primer extension to make a DNA molecule that is complementary to a target microRNA molecule; and (b) using a universal forward primer and a reverse primer to amplify the DNA molecule to produce amplified DNA molecules. In some embodiments of the method, at least one of the universal forward primer and the reverse primer comprises at least one locked nucleic acid molecule.

As used herein, the term "locked nucleic acid molecule" (abbreviated as LNA molecule) refers to a nucleic acid molecule that includes a 2'-O,4'-C-methylene-β-D-ribofuranosyl moiety. Exemplary 2'-0,4'-C-methylene-β-D-ribofuranosyl moieties, and exemplary LNAs including such moieties, are described, for example, in Petersen, M., and Wengel, J., Trends in Biotechnology 21(2):74-81 (2003) which publication is incorporated herein by reference in its entirety.

As used herein, the term "microRNA" refers to an RNA molecule that has a length in the range of from 21 nucleotides to 25 nucleotides. Some microRNA molecules (e.g., siRNA molecules) function in living cells to regulate gene expression.

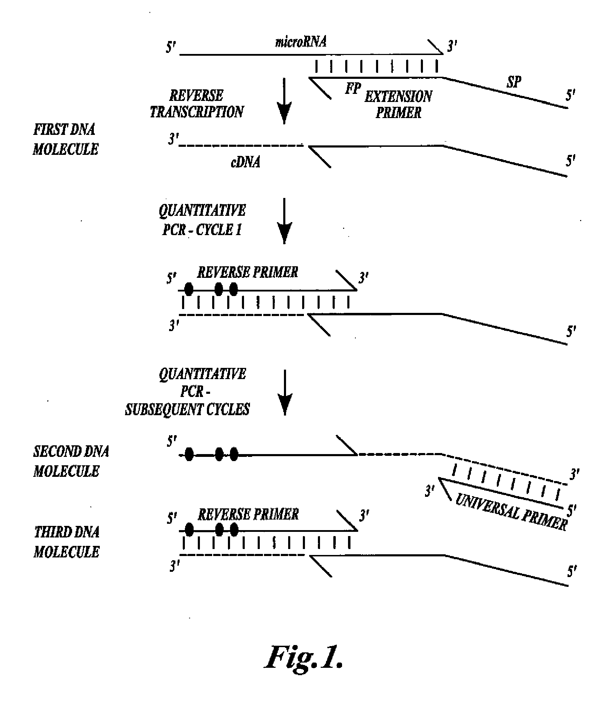

Representative Method of the Invention. FIGURE 1 shows a flowchart of a representative method of the present invention. In the method represented in FIGURE 1, a microRNA is the template for synthesis of a complementary first DNA molecule. The synthesis of the first DNA molecule is primed by an extension primer, and so the first DNA molecule includes the extension primer and newly synthesized DNA (represented by a dotted line in FIGURE 1). The synthesis of DNA is catalyzed by reverse transcriptase.

The extension primer includes a first portion (abbreviated as FP in FIGURE 1) and a second portion (abbreviated as SP in FIGURE 1). The first portion hybridizes to the microRNA target template, and the second portion includes a nucleic acid sequence that hybridizes with a universal forward primer, as described *infra.*

A quantitative polymerase chain reaction is used to make a second DNA molecule that is complementary to the first DNA molecule. The synthesis of the second DNA molecule is primed by the reverse primer that has a sequence that is selected to specifically hybridize to a portion of the target first DNA molecule. Thus, the reverse primer does not hybridize to nucleic acid molecules other than the first DNA molecule, The reverse primer may optionally include at least one LNA molecule located within the portion of the reverse primer that does not overlap with the extension primer. In FIGURE 1, the LNA molecules are represented by shaded ovals.

A universal forward primer hybridizes to the 3' end of the second DNA molecule and primes synthesis of a third DNA molecule. It will be understood that, although a single microRNA molecule, single first DNA molecule, single second DNA molecule, single third DNA molecule and single extension, forward and reverse primers are shown in FIGURE 1, typically the practice of the present invention uses reaction mixtures that include numerous copies (e.g., millions of copies) of each of the foregoing nucleic acid molecules.

The steps of the methods of the present invention are now considered in more detail.

Reparation of microRNA Molecules Useful as Templates. microRNA molecules useful as templates in the methods of the invention can be isolated from any organism (e.g., eukaryote, such as a mammal) or part thereof, including organs, tissues, and/or individual cells (including cultured cells). Any suitable RNA preparation that includes microRNAs can be used, such as total cellular RNA.

RNA may be isolated from cells by procedures that involve lysis of the cells and denaturation of the proteins contained therein. Cells of interest include wild-type cells, drug-exposed wild-type cells, modified cells, and drug-exposed modified cells,

Additional steps may be employed to remove some or all of the DNA. Cell lysis may be accomplished with a nonionic detergent, followed by microcentrifugation to remove the nuclei and hence the bulk of the cellular DNA. In one embodiment, RNA is extracted from cells of the various types of interest using guanidinium thiocyanate lysis followed by CsCl centrifugation to separate the RNA from DNA (see, Chirgwin et al., 1979, Biochemistry 18:5294-5299). Separation of RNA from DNA can also be accomplished by organic extraction, for example, with hot phenol or phenol/chloroform/isoamyl alcohol.

If desired, RNase inhibitors may be added to the lysis buffer. Likewise, for certain cell types, it may be desirable to add a protein denaturation/digestion step to the protocol.

The sample of RNA can comprise a multiplicity of different microRNA molecules, each different microRNA molecule having a different nucleotide sequence. In a specific embodiment, the microRNA molecules in the RNA sample comprise at least 100 different nucleotide sequences. In other embodiments, the microRNA molecules of the RNA sample comprise at least 500, 1,000, 5,000, 10,000, 20,000, 30,000, 40,000, 50,000, 60,000, 70,000, 80,000 90,000, or 100,000 different nucleotide sequences.

The methods of the invention may be used to detect the presence of any microRNA. For example, the methods of the invention can be used to detect one or more of the microRNA targets described in a database such as "the miRBase sequence database" as described in Griffith-Jones et al. (2004), Nucleic Acids Research. 32:D109-D111, and Griffith-Jones et al. (2006), Nucleic Acids Research 34:D140-D144, which is publically accessible on the World Wide Web at the Wellcome Trust Sanger Institute website at http://microrna.sanger.ac.uk/sequences/. A list of exemplary microRNA targets is also described in the following references: Lagos-Quintana et al., Curr. Biol. 12(9):735-9 (2002).

Synthesis of DNA Molecules Using microRNA Molecules As Templates. In the practice of the methods of the invention, first DNA molecules are synthesized that are complementary to the microRNA target molecules, and that are composed of an extension primer and newly synthesized DNA (wherein the extension primer primes the synthesis of the newly synthesized DNA). Individual first DNA molecules can be complementary to a whole microRNA target molecule, or to a portion thereof; although typically an individual first DNA molecule is complementary to a whole microRNA target molecule. Thus, in the practice of the methods of the invention, a population of first DNA molecules is synthesized that includes individual DNA molecules that are each complementary to all, or to a portion, of a target microRNA molecule.

The synthesis of the first DNA molecules is catalyzed by reverse transcriptase. Any reverse transcriptase molecule can be used to synthesize the first DNA molecules, such as those derived from Moloney murine leukemia virus (MMLV-RT), avian myeloblastosis virus (AMV-RT), bovine leukemia virus (BLV-RT), Rous sarcoma virus (RSV) and human immunodeficiency virus (HIV-RT). A reverse transcriptase lacking RNaseH activity (e.g., SUPERSCRIPT III™ sold by Invitrogen, 1600 Faraday Avenue, P.O. Box 6482, Carlsbad, California 92008) is preferred in order to minimize the amount of double-stranded cDNA synthesized at this stage. The reverse transcriptase molecule should also preferably be thermostable so that the DNA synthesis reaction can be conducted at as high a temperature as possible, while still permitting hybridization of primer to the microRNA target molecules.

Priming the Synthesis of the First DNA Molecules. The synthesis of the first DNA molecules is primed using an extension primer. Typically, the length of the extension primer is in the range of from 10 nucleotides to 100 nucleotides, such as 20 to 35 nucleotides. The nucleic acid sequence of the extension primer is incorporated into the sequence of each, synthesized, DNA molecule. The extension primer includes a first portion that hybridizes to a portion of the microRNA molecule. Typically the first portion of the extension primer includes the 3'-end of the extension primer. The first portion of the extension primer typically has a length in the range of from 6 nucleotides to 20 nucleotides, such as from 10 nucleotides to 12 nucleotides. In some embodiments, the first portion of the extension primer has a length in the range of from 3 nucleotides to 25 nucleotides.

The extension primer also includes a second portion that typically has a length of from 18 to 25 nucleotides. For example, the second portion of the extension primer can be 20 nucleotides long. The second portion of the extension primer is located 5' to the first portion of the extension primer. The second portion of the extension primer includes at least a portion of the hybridization site for the universal forward primer. For example, the second portion of the extension primer can include all of the hybridization site for the universal forward primer, or, for example, can include as little as a single nucleotide of the hybridization site for the universal forward primer (the remaining portion of the hybridization site for the forward primer can, for example, be located in the first portion of the extension primer). An exemplary nucleic acid sequence of a second portion of an extension primer is 5' CATGATCAGCTGGGCCAAGA 3' (SEQ ID NO:1).

Amplification of the DNA Molecules. In the practice of the methods of the invention, the fist DNA molecules are enzymatically amplified using the polymerase chain reaction. A universal forward primer and a reverse primer are used to prime the polymerase chain reaction. The reverse primer includes a nucleic acid sequence that is selected to specifically hybridize to a portion of a first DNA molecule.

The reverse primer typically has a length in the range of from 10 nucleotides to 100 nucleotides. In some embodiments, the reverse primer has a length in the range of from 12 nucleotides to 20 nucleotides. The nucleotide sequence of the reverse primer is selected to hybridize to a specific target nucleotide sequence under defined hybridization conditions. The reverse primer and extension primer are both present in the PCR reaction mixture, and so the reverse primer should be sufficiently long so that the melting temperature (Tm) is at least 50°C, but should not be so long that there is extensive overlap with the extension primer which may cause the formation of "primer dimers." "Primer dimers" are formed when the reverse primer hybridizes to the extension primer, and uses the extension primer as a substrate for DNA synthesis, and the extension primer hybridizes to the reverse primer, and uses the reverse primer as a substrate for DNA synthesis. To avoid the formation of "primer dimers," typically the reverse primer and the extension primer are designed so that they do not overlap with each other by more than 6 nucleotides. If it is not possible to make a reverse primer having a Tm of at least 50°C, and wherein the reverse primer and the extension primer do not overlap by more than 6 nucleotides, then it is preferable to lengthen the reverse primer (since Tm usually increases with increasing oligonucleotide length) and decrease the length of the extension primer.

The reverse primer primes the synthesis of a second DNA molecule that is complementary to the first DNA molecule. The universal forward primer hybridizes to the portion of the second DNA molecule that is complementary to the second portion of the extension primer which is incorporated into all of the first DNA molecules. The universal forward primer primes the synthesis of third DNA molecules. The universal forward primer typically has a length in the range of from 16 nucleotides to 100 nucleotides. In some embodiments, the universal forward primer has a length in the range of from 16 nucleotides to 30 nucleotides. The universal forward primer may include at least one locked nucleic acid molecule. In some embodiments, the universal forward primer includes from 1 to 25 locked nucleic acid molecules. The nucleic acid sequence of an exemplary universal forward primer is set forth in SEQ ID NO:13.

In general, the greater the number of amplification cycles during the polymerase chain reaction, the greater the amount of amplified DNA that is obtained. On the other hand, too many amplification cycles (e.g., more than 35 amplification cycles) may result in spurious and unintended amplification of non-target double-stranded DNA. Thus, in some embodiments, a desirable number of amplification cycles is between one and 45 amplification cycles, such as from one to 25 amplification cycles, or such as from five to 15 amplification cycles, or such as ten amplification cycles.

Use of LNA Molecules and Selection of Primer Hybridization Conditions. Hybridization conditions are selected that promote the specific hybridization of a primer molecule to the complementary sequence on a substrate molecule. With respect to the hybridization of a 12 nucleotide first portion of an extension primer to a microRNA, it has been found that specific hybridization occurs at a temperature of 50°C. Similarly, it has been found that hybridization of a 20 nucleotide universal forward primer to a complementary DNA molecule, and hybridization of a reverse primer (having a length in the range of from 12-20 nucleotides, such as from 14-16 nucleotides) to a complementary DNA molecule occurs at a temperature of 50°C. By way of example, it is often desirable to design extension, reverse and universal forward primers that each have a hybridization temperature in the range of from 50°C to 60°C.

In some embodiments, LNA molecules can be incorporated into at least one of the extension primer, reverse primer, and universal forward primer to raise the Tm of one, or more, of the foregoing primers to at least 50°C. Incorporation of an LNA molecule into the portion of the reverse primer that hybridizes to the target first DNA molecule, but not to the extension primer, may be useful because this portion of the reverse primer is typically no more than 10 nucleotides in length. For example, the portion of the reverse primer that hybridizes to the target first DNA molecule, but not to the extension primer, may include at least one locked nucleic acid molecule (e.g., from 1 to 25 locked nucleic acid molecules). In some embodiments, two or three locked nucleic acid molecules are included within the first 8 nucleotides from the 5' end of the reverse primer.

The number of LNA residues that must be incorporated into a specific primer to raise the Tm to a desired temperature mainly depends on the length of the primer and the nucleotide composition of the primer. A tool for determining the effect on Tm of one or more LNAs in a primer is available on the Internet Web site of Exiqon, Bygstubben 9, DK-2950 Vedbaek, Denmark.

Although one or more LNAs can be included in any of the primers used in the practice of the present invention, it has been found that the efficiency of synthesis of cDNA is low if an LNA is incorporated into the extension primer. While not wishing to be bound by theory, LNAs may inhibit the activity of reverse transcriptase.

Detecting and Measuring the Amount of the Amplified DNA Molecules. The amplified DNA molecules can be detected and quantitated by the presence of detectable marker molecules, such as fluorescent molecules. For example, the amplified DNA molecules can be detected and quantitated by the presence of a dye (e.g., SYBR green) that preferentially or exclusively binds to double stranded DNA during the PCR amplification step of the methods of the present invention. For example, Molecular Probes, Inc. (29851 Willow Creek Road, Eugene, OR 97402) sells quantitative PCR reaction mixtures that include SYBR green dye. By way of further example, another dye (referred to as "BEBO") that can be used to label double stranded DNA produced during real-time PCR is described by Bengtsson, M., et al., Nucleic Acids Research 31(8):e45 (April 15, 2003), which publication is incorporated herein by reference. Again by way of example, a forward and/or reverse primer that includes a fluorophore and quencher can be used to prime the PCR amplification step of the methods of the present invention. The physical separation of the fluorophore and quencher that occurs after extension of the labeled primer during PCR permits the fluorophore to fluoresce, and the fluorescence can be used to measure the amount of the PCR amplification products. Examples of commercially available primers that include a fluorophore and quencher include Scorpion primers and Uniprimers, which are both sold by Molecular Probes, Inc.

Representative Uses of the Present Invention. The present invention is useful for producing cDNA molecules from microRNA target molecules. The amount of the DNA molecules can be measured which provides a measurement of the amount of target microRNA molecules in the starting material. For example, the methods of the present invention can be used to measure the amount of specific microRNA molecules (e.g., specific siRNA molecules) in living cells. Again by way of example, the present invention can be used to measure the amount of specific microRNA molecules (e.g., specific siRNA molecules) in different cell types in a living body, thereby producing an "atlas" of the distribution of specific microRNA molecules within the body. Again by way of example, the present invention can be used to measure changes in the amount of specific microRNA molecules (e.g., specific siRNA molecules) in response to a stimulus, such as in response to treatment of a population of living cells with a drug.

Thus, in another aspect, the present invention provides methods for measuring the amount of a target microRNA in a multiplicity of different cell types within a living organism (e.g., to make a microRNA "atlas" of the organism). The methods of this aspect of the invention each include the step of measuring the amount of a target microRNA molecule in a multiplicity of different cell types within a living organism, wherein the amount of the target microRNA molecule is measured by a method comprising the steps of: (1) using primer extension to make a DNA molecule complementary to the target microRNA molecule isolated from a cell type of a living organism; (2) using a universal forward primer and a reverse primer to amplify the DNA molecule to produce amplified DNA molecules, and (3) measuring the amount of the amplified DNA molecules. In some embodiments of the methods, at least one of the forward primer and the reverse primer comprises at least one locked nucleic acid molecule. The measured amounts of amplified DNA molecules can, for example, be stored in an interrogatable database in electronic form, such as on a computer-readable medium(e.g., a floppy disc).

In some embodiments, the methods may be used to discriminate between two or more mammalian target microRNA that have a similar sequence in a sample from a living g organism, the method comprising the steps of: (a) producing a first DNA molecule that is complementary to the first microRNA molecule using a first extension primer specific to the first microRNA molecule; (b) amplifying the first DNA molecule to produce a first population of amplified DNA molecules using a universal forward primer and a first reverse primer; (c) producing a second DNA molecule that is complementary to the second microRNA molecule using a second extension primer specific to the second microRNA molecule; (d) amplifying the second DNA molecule to produce a second population of amplified DNA molecules using a universal forward primer and a second reverse primer; (e) measuring the amount of the first and second population of amplified DNA molecules, wherein the first and second extension primers or the first and second reverse primers differ by one or more nucleotides in the portion that is complementary to the target microRNA. This method may be used to discriminate between microRNA targets that differ by one, two, three or more nucleotides, by designing the gene-specific region of the first and second extension primers to hybridize to the region of the microRNA targets that are not identical.

In another aspect, the invention provides nucleic acid primer molecules consisting of sequence SEQ ID NO:1 to SEQ ID NO: 499, as shown in TABLE 1, TABLE 2, TABLE 6, and TABLE 7. The primer molecules of the invention can be used as primers for detecting mammalian microRNA target molecules, using the methods of the invention described herein.

In another aspect, the invention provides sets of nucleic acid primers consisting of SEQ ID NO:500 to SEQ ID NO: 965, as shown in TABLE 8. The sets of primer molecules of the invention can be used for the detection of microRNA target molecules from human, mouse, and rat, using the methods of the invention described herein.

In another aspect, the present invention provides kits for detecting at least one mammalian target microRNA, the kits comprising one or more primer sets specific for the detection of a target microRNA, each primer set comprising (1) an extension primer for producing a cDNA molecule complementary to a target microRNA, (2) a universal forward PCR primer, and (3) a reverse PCR primer for amplifying the cDNA molecule. The extension primer comprises a first portion that hybridizes to the target microRNA molecule and a second portion that includes a hybridization sequence for a universal forward PCR primer. The reverse PCR primer comprises a sequence selected to hybridize to a portion of the cDNA molecule. In some embodiments of the kits, at least one of the universal forward and reverse primers includes at least one locked nucleic acid molecule.

The extension primer, universal forward and reverse primers for inclusion in the kit may be designed to detect any mammalian target microRNA in accordance with the methods described herein. Nonlimiting examples of human target microRNA target molecules and exemplary target-specific extension primers and reverse primers are listed below in TABLE 1, TABLE 2, and TABLE 6. Nonlimiting examples of murine target microRNA target molecules and exemplary target-specific extension primers and reverse primers are listed below in TABLE 7. A nonlimiting example of a universal forward primer is set forth as S EQ ID NO: 13.

In certain embodiments, the kit includes a set of primers comprising an extension primer, reverse and universal forward primers for a selected target micraRNA molecule that each have a hybridization temperature in the range of from 50°C to 60°C.

In certain embodiments, the kit includes a plurality of primer sets that may be used to detect a plurality of mammalian microRNA targets, such as two microRNA targets up to several hundred microRNA targets.

In certain embodiments, the kit comprises one or more primer sets capable of detecting at least one or more of the following human microRNA target templates: of miR-1, miR-7, miR-9*, miR-10a, miR-10b, miR-15a, miR-15b, miR-16, miR-17-3p, miR-17-5p. miR-18, miR-19a, miR-19b, miR-20, miR-21, miR-22, miR-23a, miR-23b, miR-24, miR-25, miR-26a, miR-26b, miR-27a, miR-28, miR-29a, miR-29b, miR-29c, miR-30a-5p, miR-30b, miR-30c, miR-30d, miR-30e-5p, miR-30e-3p, miR-31, miR-32, miR-33, miR-34a, miR-34b, miR-34c, miR-92, miR-93, miR-95, miR-96, miR-98, miR-99a, miR-99b, miR-100, miR-101, miR-103, miR-105, miR-106a, miR-107, miR-122, miR-122a, miR-124, miR-124, miR-124a, miR-125a, miR-125b, miR-126, miR-1.26*, miR-127, miR-128a, miR-128b, miR-129, miR-130a, miR-130b, miR-132, miR-133a, miR-133b, miR-134, miR-135a, miR-135b, miR-136, miR-137, miR-138, miR-139, miR-140, miR-141, miR-142-3p, miR-143, miR-144, miR-145, miR-146, miR-147, miR-148a, miR-148b, miR-149, miR-150, miR-151, miR-152, miR-153, miR-154*, miR-154, miR-155, miR-181a, miR-181b, miR-181c, miR-182*, miR-182, miR-183, miR-184, miR-185, miR-186, miR-187, miR-188, miR-189, miR-190, miR-191, miR-192, miR-193, miR-194, miR-195, miR-196a, miR-196b, miR-197, miR-198, miR-199a*, miR-199a, miR-199b, miR-200a, miR-200b, miR-200c, miR-202, miR-203, miR-204, miR-205, miR-206, miR-208, miR-210, miR-211, miR-212, miR-213, miR-213, miR-214, miR-215, miR-216, miR-217, miR-218, miR-220, miR-221, miR-222, miR-223, miR-224, miR-296, miR-299, miR-301, miR-302a*, miR-302a, miR-302b*, miR-302b, miR-302d, miR-302c*, miR-302c, miR-320, miR-323, miR-324-3p, miR-324-5p, miR-325, miR-326, miR-328, miR-330, miR-331, miR-337, miR-338, miR-339, miR-340, miR-342, miR-345, miR-346, miR-363, miR-367, miR-368, miR-370, miR-371, miR-372, miR-373*, miR-373, miR-374, miR-375, miR-376b, miR-378, miR-379, miR-380-5p, miR-380-3p, miR-381, miR-382, miR-383, miR-410, miR-412, miR-422a, miR-422b, miR-423, miR-424, miR-425, miR-429, miR-431, miR-448, miR-449, miR-450, miR-451, let7a, let7b, let7c, let7d, let7e, let7f, let7g, let7i, miR-376a, and miR-377. The sequences of the above-mentioned microRNA targets are provided in "the miRBase sequence database" as described in Griffith-Jones et al. (2004), Nucleic Acids Research 32:D109-D111, and Griffith-Jones et al. (2006), Nucleic Acids Research 34:D140-D144, which is publically accessible on the World Wide Web at the Wellcome Trust Sanger Institute website at http://microrna.sanger.ac.uk/sequences/.

Exemplary primers for use in accordance with this embodiment of the kit are provided in TABLE 1, TABLE 2, and TABLE 6 below.

In another embodiment, the kit comprises one or more primer sets capable of detecting at least one or more of the following human microRNA target templates: miR-1, miR-7, miR-10b, miR-26a, miR-26b, miR-29a, miR-30e-3p, miR-95, miR-107, miR-141, miR-143, miR-154*, miR-154, miR-155, miR-181a, miR-181b, miR-181c, miR-190, miR-193, miR-194, miR-195, miR-202, miR-206, miR-208, miR-212, miR-221, miR-222, miR-224, miR-296, miR-299, miR-302c*, miR-302c, miR-320, miR-339, miR363, miR-376b, miR379, miR410, miR412, miR424, miR429, miR431, miR449, miR451, let7a, let7b, let7c, let7d, let7e, let7f, let7g, and let7i. Exemplary primers for use in accordance with this embodiment of the kit are provided in TABLE 1, TABLE 2, and TABLE 6 below.

In another embodiment, the kit comprises one or more primer sets capable of detecting at least one or more of the following human, mouse or rat microRNA target templates: miR-1, miR-9, miR-18b, miR-20b, miR-92b, miR-146b, miR-181d, miR-193b, miR-194, miR-206, miR-291a-3p, miR-291b-3p, miR-301b, miR-329, miR-346, miR-351, miR-362, miR-362-3p, miR-369-5p, miR-384, miR-409-3p, miR-409-5p, miR-425-5p, miR-449b, miR-455, miR-483, miR-484, miR-485-3p, miR-485-5p, miR-486, miR-487b, miR-488, miR-489, miR-490, miR-491, miR-493-3p, miR-494, miR-495, miR-497, miR-499, miR-500, miR-501, miR-503, miR-.505, miR-519a, miR-519b, miR-519c, miR-519d, miR-520a, miR-520b, miR-520d, miR-520e, miR-520f, miR-532, miR-539, miR-542-3p, miR-542-5p, miR-615, miR-652, miR-668, miR-671, miR-675-5p, miR-699, miR-721, and miR-758.

Exemplary primers for use in accordance with this embodiment of the kit are provided in TABLE 8.

In another embodiment, the kit comprises at least one oligonucleotide primer selected from the group consisting of SEQ ID NO: 2 to SEQ ID NO: 493, as shown in TABLE 1, TABLE 2, TABLE 6, and TABLE 7.

In another embodiment, the kit comprises at least one oligonucleotide primer selected from the group consisting of SEQ ID NO: 47, 48, 49, 50, 55, 56, 81, 82, 83, 84, 91, 92, 103, 104, 123, 124, 145, 146, 193, 194, 197, 198, 221, 222, 223, 224, 225, 226, 227, 228, 229, 230, 239, 240, 247, 248, 253, 254, 255, 256, 257, 258, 277, 278, 285, 286, 287, 288, 293, 294, 301, 302, 309, 310, 311, 312, 315, 316, 317, 318, 319, 320, 333, 334, 335, 336, 337, 338, 359, 360, 369, 370, 389, 390, 393, 394, 405, 406, 407, 408, 415, 416, 419, 420, 421, 422, 425, 426, 429, 430, 431, 432, 433, 434, 435, 436, 437, 438, 439, 440, 441, 442, 443, 444, 461 and 462, as shown in TABLE 6.

In another embodiment, the kit comprises at least one oligonucleotide primer selected from the group consisting of SEQ ID NO: 500 to SEQ ID NO: 965, as shown in TABLE 8.

A kit of the invention can also provide reagents for primer extension and amplification reactions. For example, in some embodiments, the kit may further include one or more of the following components: a reverse transcriptase enzyme, a DNA polymerase enzyme, a Tris buffer, a potassium salt (e.g., potassium chloride), a magnesium salt (e.g., magnesium chloride), a reducing agent (e.g., dithiothreitol), and deoxynucleoside triphosphates (dNTPs).

In various embodiments, the kit may include a detection reagent such as SYBR green dye or BEBO dye that preferentially or exclusively binds to double stranded DNA during a PCR amplification step. In other embodiments, the kit may include a forward and/or reverse primer that includes a fluorophore and quencher to measure the amount of the PCR amplification products.

The kit optionally includes instructions for using the kit in the detection and quantitation of one or more mammalian microRNA targets. The kit can also be optionally provided in a suitable housing that is preferably useful for robotic handling in a high throughput manner.

The following examples merely illustrate the best mode now contemplated for practicing the invention, but should not be construed to limit the invention.

### Example 1

This Example describes a representative method of the invention for producing DNA molecules from microRNA target molecules.

Primer extension was conducted as follows (using InVitrogen Superscript III^{®} reverse transcriptase and following the guidelines that were provided with the enzyme). The following reaction mixture was prepared on ice:
1 µl of 10 mM dNTPs
1 µl of 2 µM extension primer
1- 5 µl of target template
4 µl of "5X cDNA buffer"
1 µl of 0.1 M DTT
1 µl of RNAse OUT
1 µl of SuperScript III^{®} enzyme
water to 20 µl

The mixture was incubated at 50°C for 30 minutes, then 85°C for 5 minutes, then cooled to room temperature and diluted 10-fold with TE (10 mM Tris, pH 7.6, 0.1mM EDTA).

Real-time PCR was conducted using an ABI 7900 HTS detection system (Applied Biosystems, Foster City, California, U.S.A.) by monitoring SYBR^{®} green fluorescence of double-stranded PCR amplicons as a function of PCR cycle number. A typical 10 µl PCR reaction mixture contained:
5 µl of 2X SYBR^{®} green master mix (ABI)
0.8 µl of 10 µM universal forward primer
0.8 µl of 10 µM reverse primer
1.4 µl of water
2.0 µl of target template (10-fold diluted RT reaction)

The reaction was monitored through 40 cycles of standard "two cycle" PCR (95°C ― 15 sec, 60°C ― 60 sec) and the fluorescence of the PCR products was measured.

The foregoing method was successfully used in eleven primer extension PCR assays for quantitation of endogenous microRNAs present in a sample of total RNA. The DNA sequences of the extension primers, the universal forward primer sequence, and the LNA substituted reverse primers, used in these 11 assays are shown in TABLE 1.

**TABLE 1**

| Target microRNA | Primer number | Primer Name | DNA sequence (5' to 3') | SEQ ID NO |
|---|---|---|---|---|
| *gene-specific* extension primers¹ | | | | |
| humanb let7a | 357 | let7aP4 | *CATGATCAGCTGGGCCAAGA*AACTATACAACCT | 2 |
| human miR-1 | 337 | miR 1P5 | *CATGATCAGCTGGGCCAAGA*TACATACTTCT | 3 |
| human miR-15a | 344 | miR15aP3 | *CATGATCAGCTGGGCCAAGA*CACAAACCATTATG | 4 |
| human miR-16 | 351 | miR16P2 | *CATGATCAGCTGGGCCAAGA*CGCCAATATTTACGT | 5 |
| human miR-21 | 342 | miR21P6 | *CATGATCAGCTGGGCCAAGA*TCAACATCAGT | 6 |
| human miR-24 | 350 | miR24P5 | *CATGATCAGCTGGGCCAAGA*CTGTCCTGCTG | 7 |
| human miR-122 | 222 | 122-E5F | *CATGATCAGCTGGGCCAAGA*ACAAACACCATTGTC A | 8 |
| human miR-124 | 226 | 124-E5F | *CATGATCAGCTGGGCCAAGA*TGGCATTCACCGCGTG | 9 |
| human miR-143 | 362 | miR143P5 | *CATGATCAGCTGGGCCAAGA*TGAGCTACAGTG | 10 |
| human miR-145 | 305 | miR145P2 | *CATGATCAGCTGGGCCAAGA*AAGGGATTCCTGGGAA | 11 |
| human miR-155 | 367 | miR155P3 | C*ATGATCAGCTGGGCCAAGA*CCCCTATCACGAT | 12 |
| ¹ - Universal forward primer binding sites are shown in italics. The overlap with the RNA-specific reverse primers are underlined. | | | | |
| universal forward primer | | | | |
| | 230 | E5F | CATGATCAGCTGGGCCAAGA | 13 |
| RNA species-specific reverse primers² | | | | |
| human let7a | 290 | miRlet7a-1,2,3R | TG+AGGT+AGTAGGTTG | 14 |
| human miR-1 | 285 | miR1-1,2R | TG+GAA+TG+TAA AGAAGTA | 15 |
| human miR-15a | 287 | miR15aR | TAG+CAG+CACATAATG | 16 |
| human miR-16 | 289 | miR16-1,2R | T+AGC+AGCACGTAAA | 17 |
| human miR-21 | 286 | miR21R | T+AG+CT+TATCAGACTGAT | 18 |
| human miR-24 | 288 | miR24-1,2R | TGG+CTCAGTTCAGC | 19 |
| human miR-122 | 234 | 122LNAR | T+G+GAG+TGTGACAA | 20 |
| human miR-124 | 235 | 124LNAR | T+TAA+GGCACGCG | 21 |
| human miR-143 | 291 | miR143R | TG+AGA+TGAAGCACTG | 22 |
| human miR-145 | 314 | miR145R2 | GT+CCAGTTTTCCCA | 23 |
| human miR-155 | 293 | miR155R | T+TAA+TG+CTAATCGTGA | 24 |
| ² - LNA molecules are preceded by a "+". Region of overlap of the reverse primers with the corresponding extension primers are underlined. | | | | |

The assay was capable of detecting microRNA in a concentration range of from 2 nM to 20 fM. The assays were linear at least up to a concentration of 2 nM of synthetic microRNA (>1,000,000 copies/cell).

### Example 2

This Example describes the evaluation of the minimum sequence requirements for efficient primer-extension mediated cDNA synthesis using a series of extension primers for microRNA assays having gene specific regions that range in length from 12 to 3 base pairs.

Primer Extension Reactions. Primer extension was conducted using the target molecules miR-195 and miR-215 as follows. The target templates miR-195 and miR-215 were diluted to InM RNA (100,000 copies/cell) in TE zero plus 100ng/µl total yeast RNA. A no template control (NTC) was prepared with TE zero plus 100ng/µl total yeast RNA.

The reverse transcriptase reactions were carried out as follows (using InVitrogen SuperScript III^{®} reverse transcriptase and following the guidelines that were provided with the enzyme) using a series of extension primers for miR-195 (SEQ ID NO: 25-34) and a series of extension primers for miR-215 (SEQ ID NO: 35-44) the sequences of which are shown below in TABLE 2.

The following reaction mixtures were prepared on ice:
Set 1: No Template Control
   37.5 µl water
   12.5 µl of 10mM dNTPs
   12.5 µl 0.1 mM DTT
   50 µl of "5X cDNA buffer"
   12.5 µl RNAse OUT
   12.5 µl Superscript III^{®} reverse transcriptase enzyme
   12.5 µl 1µg/µl Hela cell total RNA (Ambion)
   plus 50 µl of 2 µM extension primer
   plus 50 µl TEzero + yeast RNA
Set 2: Spike-in Template
   37.5 µl water
   12.5 µl of 10mM dNTPs
   12.5 µl 0.1 mM DTT
   50 µl of "5X cDNA buffer"
   12.5 µl RNAse OUT
   12.5 µl Superscript III^{®} reverse transcriptase enzyme (InVitrogen)
   12.5 µl 1µg/µl Hela cell total RNA (Ambion)
   plus 50 µl of 2 µM extension primer
   plus 50 µl 1 nM RNA target template (miR-195 or miR-215) serially diluted in 10-fold increments

The reactions were incubated at 50°C for 30 minutes, then 85°C for 5 minutes, and cooled to 4°C and diluted 10-fold with TE (10mM Tris, pH 7.6, 0.1 mM EDTA).

Quantitative Real-Time PCR Reactions. Following reverse transcription, quadruplicate measurements of cDNA were made by quantitative real-time (qPCR) using an ABI 7900 HTS detection system (Applied Biosystems, Foster City, California, U.S.A.) by monitoring SYBR^{®} green fluorescence of double-stranded PCR amplicons as a function of PCR cycle number. The following reaction mixture was prepared:
5 µl of 2X SYBR green master mix (ABI)
0.8 µl of 10 µM universal forward primer (SEQ ID NO: 13)
0.8 µl of 10 µM reverse primer (miR-195RP:SEQ ID NO: 45 or miR215RP: SEQ ID NO: 46)
1.4 µl of water
2.0µl of target template (10-fold diluted miR-195 or miR-215
RT reaction)

Quantitative real-time PCR was performed for each sample in quadruplicate, using the manufacturer's recommended conditions. The reactions were monitored through 40 cycles of standard "two cycle" PCR (95°C- 15 sec, 60°C- 60 sec) and the fluorescence of the PCR products were measured and disassociation curves were generated. The DNA sequences of the extension primers, the universal forward primer sequence, and the LNA substituted reverse primers, used in the miR-195 and miR-215 assays are shown below in TABLE 2. The assay results for miR-195 are shown below in TABLE 3 and the assay results for miR-215 are shown below in TABLE 4.

**TABLE 2**

| **Target microRNA** | **Primer number** | **Primer Name** | **DNA sequence (5' to 3')** | **SEQ ID NO:** |
|---|---|---|---|---|
| gene-specific extension primers¹ | | | | |
| miR-195 | 646 | mir195-GS1 | *CATGATCAGCTGGGCCAAGA*GCCAATATTTCT | 25 |
| miR-195 | 647 | mir195-GS2 | *CATGATCAGCTGGGCCAAGA*GCCAATATTTC | 26 |
| miR-195 | 648 | mir195-GS3 | *CATGATCAGCTGGGCCAAGA*GCCAATATTT | 27 |
| miR-195 | 649 | mir195-GS4 | *CATGATCAGCTGGGCCAA*GAGCCAATATT | 28 |
| núR-195 | 650 | mir195-GS5 | *CATGATCAGCTGGGCCAA*GAGCCAATAT | 29 |
| miR-195 | 651 | mir195-GS6 | *CATGATCAGCTGGGCCAA*GAGCCAATA | 30 |
| miR-195 | 652 | mir195-GS7 | *CATGATCAGCTGGGCCAA*GAGCCAAT | 31 |
| miR-195 | 653 | l1Ùf195-GS8 | *CATGATCAGGI'GGGCCAAGA*GCCAA | 32 |
| nuR-195 | 654 | mirx95-GS9 | *CATGATCAGCTGGGCCAAGA*GCCA | 33 |
| miR-195 | 655 | mir195-GS10 | *CATGATCAGCTGGGCCAAGA*GCC | 34 |
| miR-215 | 656 | mir215-GS1 | *CATGATCAGCTGGGCC.4AGA*GTCTGTCAATTC | 35 |
| miR-215 | 657 | mir215-GS2 | *CATGATCAGCTGGGCCAAGA*GTCTGTCAATT | 36 |
| miR-215 | 658 | mir215-GS3 | *CATGATCAGCTGGGCCAAGA*GTCTGTCAAT | 37 |
| miR-215 | 659 | mir215-GS4 | *CATGATCAGCTGGGCCAAGAGTCTGTCAA* | 38 |
| miR-215 | 660 | mir215-GSS | *CATGATCAGCTGGGCCAAGAGTCTGTCA* | 39 |
| miR-215 | 661 | mir215-GS6 | *CATGATCAGCTGGGCCAAGAGTCTGTC* | 40 |
| miR-215 | 662 | mir215-GS7 | *CATGATCAGCTGGGCCAAGAGTCTGT* | 41 |
| miR-215 | 663 | mir215-GS8 | *CATGATCAGCTGGGCCAAGA*GTCTG | 42 |
| miR-215 | 664 | mir215-GS9 | *CATGATCAGCTGGGCCAAGAGTCT* | 43 |
| miR-215 | 665 | mir215-GS10 | *CATGATCAGCTGGGCCAAGA*GTC | 44 |
| ¹ - Universal forward primer binding sites are shown in italics. | | | | |
| RNA species-specific reverse primers² | | | | |
| miR-195 | 442 | mir195RP | T+AGC+AGCACAGAAAT | 45 |
| miR-215 | 446 | mir215RP | A+T+GA+CCTATGAATTG | 46 |
| ² - The "+" symbol precedes the LNA molecules. | | | | |

### Results:

The sensitivity of each assay was measured by the cycle threshold (Ct) value which is defined as the cycle count at which fluorescence was detected in an assay containing microRNA target template. The lower this Ct value (e.g. the fewer number of cycles), the more sensitive was the assay. For microRNA samples, it was generally observed that while samples that contain template and no template controls both eventually cross the detection threshold, the samples with template do so at a much lower cycle number. The ACt value is the difference between the number of cycles (Ct) between template containing samples and no template controls, and serves as a measure of the dynamic range of the assay. Assays with a high dynamic range allow measurements of very low microRNA copy numbers. Accordingly, desirable characteristics of a microRNA detection assay include high sensitivity (low Ct value) and broad dynamic range (ΔCt ≥ 12) between the signal of a sample containing target template and a no template background control sample.

The results of the miR195 and miR215 assays using extension primers having a gene specific portion ranging in size from 12 nucleotides to 3 nucleotides are shown below in TABLE 3 and TABLE 4, respectively. The results of these experiments unexpectedly demonstrate that gene-specific priming sequences as short as 3 nucleotides exhibit template specific priming. For both the miR-195 assay sets (shown in TABLE 3) and the miR-215 assay sets (shown in TABLE 4), the results demonstrate that the dynamic range (ΔCt) for both sets of assays are fairly consistent for extension primers having gene specific regions that are greater or equal to 8 nucleotides in length. The dynamic range of the assay (ΔCt) begins to decrease for extension primers having gene specific regions below 8 nucleotides, with a reduction in assay specificity below 7 nucleotides in the miR-195 assays, and below 6 nucleotides in the miR-215 assays. A melting point analysis of the miR-215 samples demonstrated that even at 3 nucleotides, there is specific PCR product present in the plus template samples (data not shown). Taken together, these data demonstrate that the gene specific region of extension primers is ideally ≥ 8 nucleotides, but can be as short as 3 nucleotides in length.

**TABLE 3: MIR195 ASSAY RESULTS**

| GS Primer Length | Ct: No Template Control | Ct: Plus Template | Δ Ct |
|---|---|---|---|
| 12 | 34.83 | 20.00 | 14.82 |
| 12 | 34.19 | 19.9 | 14.3 |
| 11 | 40.0 | 19.8 | 20.2 |
| 10 | 36.45 | 21.2 | 15.2 |
| 9 | 36.40 | 22.2 | 14.2 |
| 8 | 40.0 | 23.73 | 16.27 |
| 7 | 36.70 | 25.96 | 10.73 |
| 6 | 30.95 | 26.58 | 4.37 |
| 5 | 30.98 | 31.71 | -0.732 |
| 4 | 32.92 | 33.28 | -0.364 |
| 3 | 35.98 | 35.38 | -0.605 |
| Ct=the cycle count where the fluorescence exceeds the threshold of detection. ΔCt = the difference between the Ct value with template and no template. | | | |

**TABLE 4: MIR215 ASSAY RESULTS**

| GS Primer Length | Ct: No Template Control | Ct: Plus Template | Δ Ct |
|---|---|---|---|
| 12 | 33.4 | 13.57 | 19.83 |
| 12 | 33.93 | 14.15 | 19.77 |
| 11 | 35.51 | 15.76 | 19.75 |
| 10 | 35.33 | 15.49 | 19.84 |
| 9 | 36.02 | 16.84 | 19.18 |
| 8 | 35.79 | 17.07 | 18.72 |
| 7 | 32.29 | 17.58 | 14.71 |
| 6 | 34.38 | 20.62 | 13.75 |
| 5 | 34.41 | 28.65 | 5.75 |
| 4 | 36.36 | 33.92 | 2.44 |
| 3 | 35.09 | 33.38 | 1.70 |
| Ct=the cycle count where the fluorescence exceeds the threshold of detection. ΔCt = the difference between the Ct value with template and no template. | | | |

### Example 3

This Example describes assays and primer sets designed for quantitative analysis of human microRNA expression patterns.

### Primer Design:

*microRNA target templates:* the sequence of the target templates as described herein are publically available accessible on the World Wide Web at the Wellcome Trust Sanger Institute Web site in the "miRBase sequence database" as described in Griffith-Jones et al. (2004), Nucleic Acids Research 32:D109-D111, and Griffith-Jones et al. (2006), Nucleic Acids Research 34:D 140-D144.

*Extension primers:* gene specific primers for primer extension of a microRNA to form a cDNA followed by quantitative PCR (qPCR) amplification were designed to (1) convert the RNA template into cDNA; (2) to introduce a "universal" PCR binding site (SEQ ID NO:1) to one end of the cDNA molecule; and (3) to extend the length of the cDNA to facilitate subsequent monitoring by qPCR.

*Reverse primers:* unmodified reverse primers and locked nucleic acid (LNA) containing reverse primers (RP) were designed to quantify the primer-extended, full length cDNA in combination with a generic universal forward primer (SEQ ID NO:13). For the locked nucleic acid containing reverse primers, two or three LNA modified bases were substituted within the first 8 nucleotides from the 5' end of the reverse primer oligonucleotide, as shown below in the exemplary reverse primer sequences provided in TABLE 6. The LNA base substitutions were selected to raise the predicted Tm of the primer by the highest amount, and the final predicted Tm of the selected primers were specified to be preferably less than or equal to 55°C.

An example describing an assay utilizing an exemplary set of primers the detection of miR-95 and miR-424 is described below.

Primer Extension Reactions: primer extension was conducted using DNA templates corresponding to miR-95 and miR-424 as follows. The DNA templates were diluted to 0 nM, 1 nM, 100 pM, 10 pM, and 1 pM dilutions in TE zero (10 mM Tris pH 7.6, 0.1 mM EDTA) plus 100ng/µl yeast total RNA (Ambion, Austin, TX).

The reverse transcriptase reactions were carried out using the following primers:
Extension primers: (diluted to 500 nM)
   miR-95GSP CATGATCAGCTGGGCCAAGATGCTCAATAA (SEQ ID NO:123)
   miR-424GSP CATGATCAGCTGGGCCAAGATTCAAAACAT (SEQ ID NO:415)
Reverse primers: (diluted to 10 mM)
   miR-95_RP4 TT+CAAC+GGGTATTTATTGA (SEQ ID NO:124)
   miR-424RP2 C+AG+CAGCAATTCATGTTTT (SEQ ID NO:416)
Reverse Transcription (per reaction):
   2 µl water
   2 µl of "5X cDNA buffer" (InVitrogen, Carlsbad, CA)
   0.5 µl of 0.1 mM DTT (InVitrogen, Carlsbad, CA)
   0.5 µl of 10 mM dNTPs (InVitrogen, Carlsbad, CA)
   0.5 µl RNAse OUT (InVitrogen, Carlsbad, CA)
   0.5 µl Superscript III^{®} reverse transcriptase enzyme (InVitrogen, Carlsbad, CA)
   2 µl of extension primer plus 2 µl of template dilution
   The reactions were mixed and incubated at 50°C for 30 minutes, then 85°C for 5 minutes, and cooled to 4°C and diluted 10-fold with TE zero.
Quantitative Real-Time PCR Reactions (per reaction):
   5 µl 2X SYBR mix (Applied Biosystems, Foster City, CA)
   1.4 µl water
   0.8 µl universal primer (CATGATCAGCTGGGCCAAGA (SEQ
   ID NO: 13))
   2.0 µl of diluted reverse transcription (RT) product from above.

Quantitative real-time PCR was performed for each sample in quadruplicate, using the manufacturer's recommended conditions. The reactions were monitored through 40 cycles of standard "two cycle" PCR (95°C - 15 sec, 60°C - 60 sec) and the fluorescence of the PCR products were measured and disassociation curves were generated. The DNA sequences of the extension primers, the universal forward primer sequence, and the LNA substituted reverse primers, used in the representative miR-95 and miR-424 assays as well as primer sets for 212 different human microRNA templates are shown below in TABLE 6, Primer sets for assays requiring extensive testing and design modification to achieve a sensitive assay with a high dynamic range are indicated in TABLE 6 with the symbol # following the primer name.

### Results:

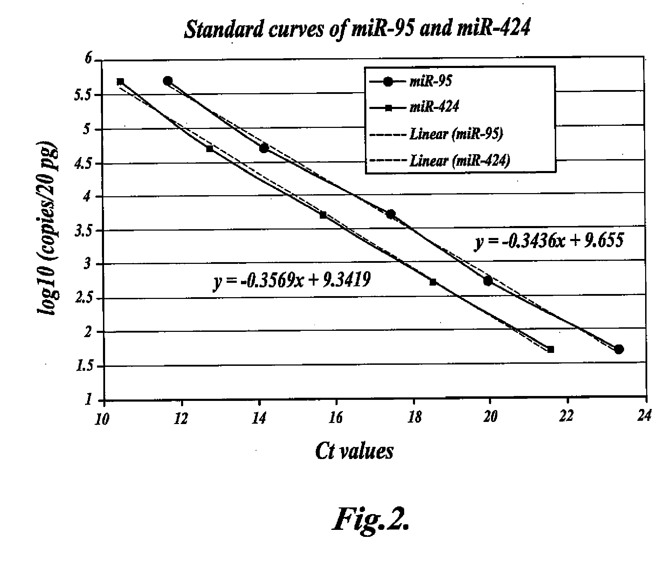

TABLE 5 shows the Ct values (averaged from four samples) from the miR-95 and miR-424 assays, which are plotted in the graph shown in FIGURE 2. The results of these assays are provided as representative examples in order to explain the significance of the assay parameters shown in TABLE 6 designated as slope (column 6), intercept (column 7) and background (column 8).

As shown in TABLE 5, the Ct value for each template at various concentrations is provided. The Ct values (x-axis) are plotted as a function of template concentration (y-axis) to generate a standard curve for each assay, as shown in FIGURE 2. The slope and intercept define the assay measurement characteristics that permit an estimation of number of copies/cell for each microRNA. For example, when the Ct values for 50 µg total RNA input for the miR-95 assay are plotted, a standard curve is generated with a slope and intercept of -.03569 and 9.655, respectively. When these standard curve parameters are applied to the Ct of an unknown sample (x), they yield log 10 (copies/20pg total RNA) (y). Because the average cell yields 20 pg of total RNA, these measurements equate to copies of microRNA/cell The background provides an estimate of the minimum copy number that can be measured in a sample and is computed by inserting the no template control (NTC) value into this equation. In this example, as shown in TABLE 6, miR-95 yields a background of 1.68 copies/20 pg at 50 µg of RNA input.

As further shown in TABLE 6, reverse primers that do not contain LNA may also be used in accordance with the methods of the invention. See, e.g., SEQ ID NO:494-499. The sensitivity and dynamic range of the assays using non-LNA containing reverse primers SEQ ID NO:494-499, yielded similar results to the corresponding assays using LNA-containing reverse primers.

**TABLE 5**

| Ct Values (averaged from four samples) | | | | | | |
|---|---|---|---|---|---|---|
| Template concentration | 10 nM | 1 nM | 0.1 nM | 0.01 nM | 0.001 nM | NTC |
| copies/20 pg RNA (50 µg input) | 500,000 | 50,000 | 5000 | 500 | 50 | |
| copies/20 pg RNA (5 µg input) | 5,000,000 | 500,000 | 50,000 | 5000 | 500 | |
| miR-95 | 11.71572163 | 14.17978 | 17.46353 | 19.97259 | 23.33171 | 27.44383 |
| miR-424 | 10.47708975 | 12.76806 | 15.69251 | 18.53729 | 21.56897 | 23.2813 |
| log10 (copies for 50 µg input) | 5.698970004 | 4.69897 | 3.69897 | 2.69897 | 1.69897 | |

**TABLE 6: PRIMERS TO DETECT HUMAN MICRORNA TARGET TEMPLATES**

| **Human Target micro RNA** | **Extension Primer Name** | **Extension Primer Sequence** | **Reverse Primer Name** | **Reverse Primer Sequence** | **Slope** | **lntercept** | **Background RNA input** | |
|---|---|---|---|---|---|---|---|---|
| | | | | | | | **50ug** | **5ug** |
| # denotes primers for assays that required extensive testing and primer design modification to achieve optimal assay results including high sensitivity and high dynamic range. | | | | | | | | |
| miR-1 | miR1GSP10^{#} | CATGATCAGCTGGGCCAAGATACATACTTC | miR-1RP^{#} | T+G+GAA+TG+TAAAGAAGT | -0.2758 | 8,3225 | 2.44 | 24.36 |
| | | SEQ ID NO:47 | | SEQ ID NO:48 | | | | |
| miR-7 | miR-7GSP10^{#} | CATGATCAGCTGGGCCAAGACAACAAAATC | miR-7_RP6^{#} | T+GGAA+GACTAGTGATTTT | -0.2982 | 10.435 | 11.70 | 116.99 |
| | | SEQ ID NO:49 | | SEQ ID NO·50 | | | | |
| miR-9* | miR-9*GSP | CATGATCAGCTGGGCCAAGAACTTTCGGTT | miR-9*RP | TAAA+GCT+AGATAACCG | -0,2405 | 8.9145 | 3.71 | 37.15 |
| | | SEQ ID NO:51 | | SEQ ID NO.52 | | | | |
| miR-10a | miR-10aGSP | CATGATCAGCTGGGCCAAGACACAAATTCG | miR-10aRP | T+AC+CCTGTAGATCCG | -0,2755 | 8.6976 | 0.09 | 0.94 |
| | | SEQ ID NO:53 | | SEQ ID NO:54 | | | | |
| miR-10b | miR-10b_GSP11^{#} | CATGATCAGCTGGGCCAAGAACAAATTCGGT | miR-10b_RP2^{#} | TA+CCC+TGT+AGAACCGA | -0,3505 | 8.7109 | 0.55 | 5.52 |
| | | SEQ ID NO:55 | | SEQ ID NO:56 | | | | |
| miR-15a | miR-15aGSP | CATGATCAGCTGGGCCAAGACACAAACCAT | miR-15aRP | T+AG+CAGCACAT AATG | -0.2831 | 8.4519 | 4.40 | 44.01 |
| | | SEQ ID NO:57 | | SEQ ID NO:58 | | | | |
| miR-15b | miR-15bGSP2 | CATGATCAGCTGGGCCAAGATGTAAACCA | miR-15bRP | T+AG+CAGCACATCAT | -0.2903 | 8.4206 | 0.18 | 1.84 |
| | | SEQ ID NO:59 | | SEQ lD NO:60 | | | | |
| miR-16 | miR-16GSP2 | CATGATCAGCTGGGCCAAGACGCCAATAT | miR-16RP | T+AG+CAGCACGTAAA | -0.2542 | 9.3689 | 1.64 | 16.42 |
| | | SEQ ID NO.61 | | SEQ ID NO 62 | | | | |
| miR-17-3p | miR-17-3pGSP | CATGATCAGCTGGGCCGAACAACAAGTGCCT | miR-17-3pRP | A+CT+GCAGTGAAGGC | -0.2972 | 8.2625 | 1.08 | 10.78 |
| | | SEQ ID NO:63 | | SEQ ID NO:64 | | | | |
| miR-17-5p | miR-17-5pGSP2 | CATGATCAGCTGGGCCAAGAACTACCTGC | miR-17-5pRP | C+AA+AGTGCTTACAGTG | -0.2956 | 7.9101 | 0.13 | 1.32 |
| | | SEQ ID NO:65 | | SEQ ID NO:66 | | | | |
| miR-19a | miR-19aGSP2 | CATGATCAGCTGGGCCAAGATCAGTTTTG | miR-19aRP | TG+TG+CAAATCTATGC | -0.2984 | 9.461 | 0,02 | 0.23 |
| | | SEQ ID NO:67 | | SEQ ID NO:68 | | | | |
| miR-19b | miR-19bGSP | CATGATCAGCTGGGCCAAGATCAGTTTTGC | miR-19bRP | TG+TG+CAAATCCATG | -0.294 | 8.1434 | 2.26 | 22.55 |
| | | SEQ ID NO:69 | | SEQ ID NO:70 | | | | |
| miR-20 | miR-20GSP3 | CATGATCAGCTGGGCCAAGACTACCTGC | miR-20RP | T+AA+AGTGCTTATAGTGCA | 0.2979 | 7.9929 | 0.16 | 1.60 |
| | | SEQ ID NO:71 | | SEQ ID NO:72 | | | | |
| miR-21 | miR-21GSP2 | CATGATCAGCTGGGCCAAGATCAACATCA | miR-21 RP | T+AG+CTTATCAGACTGATG | -0.2849 | 8.1624 | 1.80 | 17.99 |
| | | SEQ ID NO:73 | | SEQ ID NO:74 | | | | |
| miR-23a | miR-23aGSP | CATGATCAGCTGGGCCAAGAGGAAATCCCT | miR-23aRP | A+TC+ACATTGCCAGG | -0.3172 | 9.4253 | 2.41 | 2408 |
| | | SEQ ID NO:75 | | SEQ ID NO:76 | | | | |
| miR-23b | miR-23bGSP | CATGATCAGCTGGGCCAAGAGGTAATCCCT | miR-23bRP | A+TC+ACATTGCCAGG | -0.2944 | 9.0985 | 5.39 | 53.85 |
| | | SEQ ID NO:77 | | SEQ ID NO:78 | | | | |
| miR-25 | miR-25GSP | CATGATCAGCTGGGCCAAGATCAGACCGAG | miR-25RP | C+AT+TGCACTTGTCTC | -03009 | 9.2482 | 1.52 | 15.19 |
| | | SEQ ID NO:79 | | SEQ ID NO:80 | | | | |
| miR-26a | miR-26aGSP9^{#} | CATGATCAGCTGGGCCAAGAGCCTATCCT | miR-26aRP2^{#} | TT+CA+AGTAATCCAGGAT | -0.2807 | 8.558 | 0.26 | 2.56 |
| | | SEQ ID NO:81 | | SEQ ID NO:82 | | | | |
| miR-26b | miR-26bGSP9^{#} | CATGATCAGCTGGGCCAAGAAACCTATCC | miR-26bRP2^{#} | TT+CA+AGT+AATTCAGGAT | -0.2831 | 8.7885 | 0.37 | 3.67 |
| | | SEQ ID NO:83 | | SEQ ID NO:84 | | | | |
| miR 27a | miR-27aGSP | CATGATCAGCTGGGCCAAGAGCGGAACTTA | miR-27aRP | TT+CA+CAGTGGCTAA | -0.2765 | 9.5239 | 5.15 | 51.51 |
| | | SEQ ID NO:85 | | SEQ ID NO:86 | | | | |
| miR-27b | miR-27bGSP | CATGATCAGCTGGGCCAAGAGCAGAACTTA | miR-27bRP | TT+CA+CAGTGGCTAA | -0.28 | 9.5483 | 5.97 | 59.71 |
| | | SEQ ID NO:87 | | SEQ ID NO:88 | | | | |
| miR-28 | miR-28GSP | CATGATCAGCTGGGCCAAGACTCAATAGAC | miR-28RP | A+AG+GAGCTCACAGT | -0.3226 | 10.071 | 7.19 | 71.87 |
| | | SEQ ID NO:89 | | SEQ ID NO:90 | | | | |
| miR-29a | miR-29aGSP8^{#} | CATGATCAGCTGGGCCAAGAAACCGATT | miR- 29aRP2^{#} | T+AG+CACCATCTGAAAT | -0.29 | 8.8731 | 0.04 | 0.38 |
| | | SEQ ID NO:91 | | SEQ m NO:92 | | | | |
| miR-29b | miR-29bGSP2 | CATGATCAGCTGGGCCAAGAAACACTGAT | miR-29bRP2 | T+AG+CACCATTTGAAATCAG | -0.3162 | 9.6276 | 3.56 | 3557 |
| | | SEQ ID NO:93 | | SEQ ID NO.94 | | | | |
| miR-30a- 5p | miR-30a- 5pGSP | CATGATCAGCTGGGCCAAGACTTCCAGTCG | miR-30a- 5pRP | T+GT+AAACATCCTCGAC | -0.2772 | 9.0694 | 1.92 | 19.16 |
| | | SEQ ID NO:95 | | SEQ ID NO:96 | | | | |
| miR-30b | miR-30bGSP | CATGATCAGCTGGGCCAAGAAGCTGAGTGT | miR-30bRP | TGT+AAA+CATCCTACACT | -0.2621 | 8.5974 | 0.11 | 1.13 |
| | | SEQ ID NO:97 | | SEQ ID NO:98 | | | | |
| nuR-30c | miR-30cGSP | CATGATCAGCTGGGCCAAGAGCTGAGAGTG | miR-30cRP | TGT+AAA+CATCCTACACT | -0.2703 | 8.699 | 0.15 | 1.48 |
| | | SEQ ID NO:99 | | SEQ ID NO·100 | | | | |
| miR-30d | miR-30dGSP | CATGATCAGCTGGGCCAAGACTTCCAGTCG | miR-30dRP | T+GTAAA+CATCCCCG | -0.2506 | 9.3875 | 0.23 | 2.31 |
| | | SEQ ID NO:101 | | SEQ ID NO:102 | | | | |
| miR-30e- | miR-30e- 3pGSP9^{#} | CATGATCAGCTGGGCCAAGAGCTGTAAAC | miR-30e- 3pRP5^{#} | CTTT+CAGT+CGGATGTTT | -0.325 | 11.144 | 6.37 | 63.70 |
| 3p | | SEQ ID NO:103 | | SEQ ID NO:104 | | | | |
| miR-30e- 5p | miR-30e- 5pGSP | CATGATCAGCTGGGCCAAGATCCAGTCAAG | miR-30e- 5pRP | TG+TAAA+CATCCTTGAC | -0.2732 | 8.1604 | 8.50 | 85.03 |
| | | SEQ ID NO:105 | | SEQ ID NO·106 | | | | |
| miR-31 | miR-31GSP | CATGATCAGCTGGGCCAAGACAGCTATGCC | miR-31RP | G+GC+AAGATGCTGGC | -0.3068 | 8.2605 | 3.74 | 37.43 |
| | | SEQ ID NO:107 | | SEQ ID NO:108 | | | | |
| miR-32 | miR 32GSP | CATGATCAGCTGGGCCAAGAGCAACTTAGT | miR-32RP | TATTG+CA+CATTACTAAG | -0.2785 | 8.9581 | 0.39 | 3.93 |
| | | SEQ ID NO:109 | | SEQ ID NO: 110 | | | | |
| miR-33 | miR-33GSP2 | CATGATCAGCTGGGCCAAGACAATGCAAC | miR-33RP | G+TG+CATTGTAGTTGC | -0.3031 | 8.42 | 2.81 | 28.14 |
| | | SEQ ID NO:111 | | SEQ ID NO:112 | | | | |
| miR-34a | miR-34aGSP | CATGATCAGCTGGGCCAAGAAACAACCAGC | miR-34aRP | T+GG+CAGTGTCTTAG | -0.3062 | 9.1522 | 2.40 | 23.99 |
| | | SEQ ID NO:113 | | SEQ ID NO:114 | | | | |
| miR-34b | miR-34bGSP | CATGATCAGCTGGGCCAAGACAATCAGCTA | miR-34bRP | TA+GG+CAGTGTCATT | -0.3208 | 9.054 | 0.04 | 0.37 |
| | | SEQ ID NO:115 | | SEQ ID NO:116 | | | | |
| miR-34c | miR-34cGSP | CATGATCAGCTGGGCCAAGAGCAATCAGCT | miR-34cRP | A+GG+CAGTGTAGTTA | -0.2995 | 1014 | 1.08 | 10 83 |
| | | SEQ ID NO:117 | | SEQ ID NO:118 | | | | |
| miR-92 | miR-92GSP | CATGATCAGCTGGGCCAAGACAGGCCGGGA | miR-92RP | T+AT+TGCACTTGTCCC | -0.3012 | 8.6908 | 8.92 | 89.17 |
| | | SEQ ID NO:119 | | SEQ ID NO:120 | | | | |
| miR-93 | miR-93GSP | CATGATCAGCTGGGCCAAGACTACCTGCAC | miR-93RP | AA+AG+TGCTGTTCGT | -0.3025 | 7.9933 | 4.63 | 46.30 |
| | | SEQ ID NO:121 | | SEQ ID NO:122 | | | | |
| miR-95 | miR-95GSP^{#} | CATGATCAGCTGGGCCAAGATGCTCAATAA | miR-95_RP4^{#} | TT+CAAC+GGGTATTTATTGA | -0.3436 | 9.655 | 1.68 | 16.80 |
| | | SEQ ID NO:123 | | SEQ ID NO:124 | | | | |
| miR-96 | miR-95GSP | CATGATCAGCTGGGCCAAGAGCAAAAATGT | miR-96RP | T+TT+GGCACTAGCAC | -0.2968 | 9.2611. | 0.00 | 0.05 |
| | | SEQ ID NO:125 | | SEQ ID NO:126 | | | | |
| miR-98 | miR-98GSP | CATGATCAGCTGGGCCAAGAAACAATACAA | miR-98RP | TGA+GGT+AGTAAGTTG | -0.2797 | 9.5654 | 1.05 | 10.48 |
| | | SEQ ID NO:127 | | SEQ ID NO:128 | | | | |
| miR-99a | miR-99aGSP | CATGATCAGCTGGGCCAAGACACAAGATCG | miR-99aRP | A+AC+CCGTAGATCCG | -0.2768 | 8.781 | 0.21 | 2.08 |
| | | SEQ ID NO:129 | | SEQ ID NO:130 | | | | |
| miR-99b | miR-99bGSP | CATGATCAGCTGGGCCAAGACGCAAGGTCG | miR-99bRP | C+AC+CCGT AGAACCG | -0.2747 | 7 9855 | 0.25 | 2.53 |
| | | SEQ ID NO:131 | | SEQ ID NO:132 | | | | |
| miR-100 | miR 100GSP | CATGATCAGCTGGGCCAAGACACAAGTTCG | miR 100RP | A+AC+CCGTAGATCCG | -0.2902 | 8.669 | 0.04 | 0.35 |
| | | SEQ ID NO:133 | | SEQ ID NO:134 | | | | |
| miR-101 | miR-101GSP | CATGATCAGCTGGGCCAAGACTTCAGTTAT | miR-101RP | TA+CAG+TACTGTGATAACT | -0.3023 | 8.2976 | 0.46 | 4.63 |
| | | SEQ ID NO:135 | | SEQ ID NO:136 | | | | |
| miR-103 | miR-103GSP | CATGATCAGCTGGGCCAAGATCATAGCCCT | miR-103RP | A+GC+AGCATTGTACA | -0.3107 | 8.5776 | 0.02 | 0.21 |
| | | SEQ ID NO:137 | | SEQ ID NO:138 | | | | |
| miR-105 | miR-105GSP | CATGATCAGCTGGGCCAAGAACAGGAGTCT | miR-105RP | T+CAAA+TGCTCAGACT | -0.2667 | 8.9832 | 0.93 | 9.28 |
| | | SEQ ID NO:139 | | SEQ ID NO:140 | | | | |
| miR-106a | miR-106aGSP | CATGATCAGCTGGGCCAAGAGCTACCTGCA | miR-106aRP | AAA+AG+TGCTTACAGTG | -0.3107 | 8.358 | 0.03 | 0.31 |
| | | SEQ ID NO:141 | | SEQ ID NO:142 | | | | |
| miR-106b | miR-106bGSP | CATGATCAGCTGGGCCAAGAATCTGCACTG | miR-106bRP | T+AAAG+TGCTGACAGT | -0.2978 | 8.7838 | 0.10 | 1.04 |
| | | SEQ ID NO:143 | | SEQ ID NO:144 | | | | |
| miR-107 | miR-107GSP8^{#} | CATGATCAGCTGGGCCAAGATGATAGCC | miR-107RP2^{#} | A+GC+AGCATTGTACAG | -0.304 | 9.1666 | 0.34 | 3.41 |
| | | SEQ ID NO:145 | | SEQ ID NO:146 | | | | |
| miR-122a | miR-122aGSP | CATGATCAGCTGGGCCAAGAACAAACACCA | miR-122aRP | T+GG+AGTGTGACAAT | -0.3016 | 8.1479 | 0.06 | 0.58 |
| | | SEQ ID NO:147 | | SEQ ID NO:148 | | | | |
| miR-124a | miR-124aGSP | CATGATCAGCTGGGCCAAGATGGCATTCAC | miR-124aRP | T+TA+AGGCACGCGGT | -0.3013 | 8.6906 | 0.56 | 5.63 |
| | | SEQ ID NO:149 | | SEQ ID NO:150 | | | | |
| miR-125a | miR-125aGSP | CATGATCAGCTGGGCCAAGACACAGGTTAA | miR-125aRP | T+CC+CTGAGACCCTT | -0.2938 | 8.6754 | 0.09 | 0.91 |
| | | SEQ ID NO:151 | | SEQ ID NO.152 | | | | |
| miR-125b | miR-125bGSP | CATGATCAGCTGGGCCAAGATCACAAGTTA | miR-125bRP | T+CC+CTGAGACCCTA | -0.283 | 8.1251 | 0.20 | 1.99 |
| | | SEQ ID NO:153 | | SEQ ID NO 154 | | | | |
| miR-126 | miR-126GSP | CATGATCAGCTGGGCCAAGAGCATTATTAC | miR-126RP | T+CG+TACCGTGAGTA | -0.26 | 8.937 | 0.18 | 1.80 |
| | | SEQ ID NO:155 | | SEQ ID NO:156 | | | | |
| miR-126* | miR-126*GSP3 | CATGATCAGCTGGGCCAAGACGCGTACC | miR-126*RP | C+ATT+ATTA+CTTTTGGTACG | -0.2969 | 8.184 | 3.58 | 35.78 |
| | | SEQ ID NO:157 | | SEQ ID NO:158 | | | | |
| miR-127 | miR-127GSP | CATGATCAGCTGGGCCAAGAAGCCAAGCTC | miR-127RP | T+CG+GATCCGTCTGA | -0.2432 | 9.1013 | 1.11 | 11.13 |
| | | SEQ ID NO:159 | | SEQ ID NO:160 | | | | |
| miR-128a | miR-128aGSP | CATGATCAGCTGGGCCAAGAAAAAGAGACC | miR-128aRP | T+CA+CAGTGAACCGG | -0.2866 | 8.0867 | 0.16 | 1.60 |
| | | SEQ ID NO:161 | | SEQ ID NO:162 | | | | |
| miR-128b | miR-128bGSP | CATGATCAGCTGGGCCAAGAGAAAGAGACC | miR-128bRP | T+CA+CAGTGAACCGG | -0.2923 | 8.0608 | 0.07 | 0.74 |
| | | SEQ ID NO:163 | | SEQ ID NO:164 | | | | |
| miR-129 | miR-129GSP | CATGATCAGCTGGGCCAAGAGCAAGCCCAG | miR-129RP | CTTTT+TG+CGGTCTG | -0.2942 | 9.7 731 | 0.88 | 8.85 |
| | | SEQ ID NO:165 | | SEQ ID NO:166 | | | | |
| miR-130u | miR-130aGSP | CATGATCAGCTGGGCCAAGAATGCCCTTTT | miR-130aRP | C+AG+TGCAATGTTAAAAG | -0.2943 | 8.7465 | 1.28 | 12.78 |
| | | SEQ ID NO:167 | | SEQ ID NO.168 | | | | |
| miR-130b | miR-130bGSP | CATGATCAGCTGGGCCAAGAATGCCCTTTC | miR-130bRP | C+AG+TGCAATGATGA | -0.2377 | 9.1403 | 3.14 | 31.44 |
| | | SEQ ID NO:169 | | SEQ ID NO:170 | | | | |
| miR-132 | miR-132GSP | CATGATCAGCTGGGCCAAGACGACCATGGC | miR-132RP | T+AA+CAGTCTACAGCC | -0.2948 | 8.1167 | 0.11 | 1.13 |
| | | SEQ ID NO:171 | | SEQ ID NO:172 | | | | |
| miR-133a | miR-133aGSP | CATGATCAGCTGGGCCAAGAACAGCTGGTT | miR-133aRP | T+ TG+GTCCCCTTCAA | -0.295 | 9.3679 | 0.10 | 1.04 |
| | | SEQ ID NO:173 | | SEQ ID NO:174 | | | | |
| miR-133b | miR-133bGSP | CATGATCAGCTGGGCCAAGATAGCTGGTTG | miR-133bRP | T+TG+GTCCCCTTCAA | -0.3062 | 8.3649 | 0 02 | 0.18 |
| | | SEQ ID NO:175 | | SEQ ID NO:176 | | | | |
| miR-134 | miR-134GSP | CATGATCAGCTGGGCCAAGACCCTCTGGTC | miR-134RP | T+GT+GACTGGTTGAC | -0.2965 | 9.0483 | 0.14 | 1.39 |
| | | SEQ ID NO:177 | | SEQ ID NO·178 | | | | |
| miR-135a | miR-135aGSP | CATGATCAGCTGGGCCAAGATCACATAGGA | miR-135aRP | T+AT+GGCTTTTTATTCCT | -0.2914 | 8.092 | 1.75 | 17.50 |
| | | SEQ ID NO:179 | | SEQ ID NO:18D | | | | |
| miR-135b | miR-135bGSP | CATGATCAGCTGGGCCAAGACACATAGGAA | miR-135bRP | T+AT+GGCTTTTCATTCC | -0.2962 | 7.8986 | 0.05 | 0.49 |
| | | SEQ ID NO:181 | | SEQ ID NO:182 | | | | |
| miR-136 | miR-136GSP | CATGATCAGCTGGGCCAAGATCCATCATCA | miR-136RP | A+CT+CCATTTGTTTTGATG | -0.3616 | 10.229 | 0.68 | 6.77 |
| | | SEQ ID NO:183 | | SEQ ID NO:184 | | | | |
| miR-137 | miR-137GSP | CATGATCAGCTGGGCCAAGACTACGCGTAT | miR-137RP | T+AT+TGCTTAAGAATACGC | -0.2876 | 8.234 | 8.57 | 85.71 |
| | | SEQ ID NO:185 | | SEQ ID NO:186 | | | | |
| miR-138 | miR-138GSP2 | CATGATCAGCTGGGCCAAGACGGCCTGAT | miR-138RP | A+GC+TGGTGTTGTGA | -0.3023 | 9.0814 | 0.22 | 2.19 |
| | | SEQ ID NO:187 | | SEQ ID NO:188 | | | | |
| miR-139 | miR-139GSP | CATGATCAGCTGGGCCAAGAAGACACGTGC | miR-139RP | T+CT+ACAGTGCACGT | -0.2983 | 8.1141 | 6.92 | 69.21 |
| | | SEQ ID NO:189 | | SEQ ID NO:190 | | | | |
| miR-140 | miR-140GSP | CATGATCAGCTGGGCCAAGACTACCATAGG | miR 140RP | A+GT+GGTTTTACCCT | -0.2312 | 8.3231 | 0.13 | 1.34 |
| | | SEQ ID NO:191 | | SEQ ID NO:192 | | | | |
| miR-141 | miR 141GSP9^{#} | CATGATCAGCTGGGCCAAGACCATCTTTA | miR- | TAA+CAC+TGTCTGGTAA | -0.2805 | 9.6671 | 0.13 | 1.26 |
| | | SEQ ID NO:193 | 141RP2^{#} | SEQ ID NO:194 | | | | |
| miR-142-3p | miR-142- 3pGSP3 | CATGATCAGCTGGGCCAAGATCCATAAA | miR-142- 3pRP | TGT+AG+TGTTTCCTACT | -0.2976 | 8.4046 | 0.03 | 0.27 |
| | | SEQ ID NO:195 | | SEQ ID NO:196 | | | | |
| miR-143 | miR-143GSP8^{#} | CATGATCAGCTGGGCCAAGATGAGCTAC | miR- 143RP2^{#} | T+GA+GATGAAGCACTG | -0.3008 | 9.2675 | 0.37 | 3.71 |
| | | SEQ ID NO:197 | | SEQ ID NO:198 | | | | |
| miR-144 | miR.144GSP2 | CATGATCAGCTGGGCCAAGACTAGTACAT | miR-144RP | TA+CA+GTAT+AGATGATG | -0.2407 | 9.4441 | 0.95 | 9.52 |
| | | SEQ ID NO:199 | | SEQ ID NO:200 | | | | |
| miR-145 | miR-145GSP2 | CATGATCAGCTGGGCCAAGAAAGGGATTC | miR-145RP | G+TC+CAGTTTCCCA | -0.2937 | 8.0791 | 0.39 | 3.86 |
| | | SEQ ID NO:201 | | SEQ ID NO:202 | | | | |
| miR-146 | miR-146GSP3 | CATGATCAGCTGGGCCAAGAAACCCATG | miR-146RP | T+GA+GAACTGAATTCCA | -0.2861 | 8.8246 | 0.08 | 0.75 |
| | | SEQ ID NO:203 | | SEQ ID NO:204 | | | | |
| miR-147 | miR-147GSP | CATGATCAGCTGGGCCAAGAGCAGAAGCAT | miR-147RP | C+TG+TGTGGAAATGC | -0.2989 | 8.8866 | 1.65 | 16.47 |
| | | SEQ ID NO:205 | | SEQ ID NO:206 | | | | |
| miR-148a | miR-148aGSP2 | CATGATCAGCTGGGCCAAGAACAAAGTTC | miR-148aRP2 | T+CA+GTGCACT ACAGAACT | -0.2928 | 9.4654 | 1.27 | 12.65 |
| | | SEQ ID NO:207 | | SEQ ID NO:208 | | | | |
| miR-148b | miR-148bGSP2 | CATGATCAGCTGGGCCAAGAACAAAGTTC | miR-148bRP | T+CA+GTGCATCACAG | -0.2982 | 10.417 | 0.24 | 2.44 |
| | | SEQ ID NO:209 | | SEQ ID NO:210 | | | | |
| miR-149 | miR-149GSP2 | CATGATCAGCTGGGCCAAGAGGAGTGAAG | miR-149RP | T+CT+GGCTCCGTGTC | -0.2996 | 8.3392 | 2.15 | 21.50 |
| | | SEQ ID NO:211 | | SEQ ID NO:212 | | | | |
| miR-150 | miR-150GSP3 | CATGATCAGCTGGGCCAAGACACTGGTA | miR-150RP | T+CT+CCCAACCCTTG | -0.2943 | 8.3945 | 0.06 | 0.56 |
| | | SEQ ID NO:213 | | SEQ ID NO:214 | | | | |
| miR-151 | miR-151GSP2 | CATGATCAGCTGGGCCAAGACCTCAAGGA | miR 151RP | A+CT+AGACTGAAGCTC | -0.2975 | 8.651 | 0.16 | 1.60 |
| | | SEQ ID NO:215 | | SEQ ID NO:216 | | | | |
| miR-152 | miR-152GSP2 | CATGATCAGCTGGGCCAAGACCCAAGTTC | miR-152RP | T+CA+GTGCATGACAG | -0.2741 | 8.7404 | 0.33 | 3.25 |
| | | SEQ ID NO:217 | | SEQ ID NO:218 | | | | |
| miR-153 | miR-153GSP2 | CATGATCAGCTGGGCCAAGATCACTTTTG | miR-153RP | TTG+CAT+AGTCACAAAA | -0.2723 | 9.5732 | 3.32 | 33.19 |
| | | SEQ ID NO:219 | | SEQ ID NO:220 | | | | |
| miR-154^{*} | miR-154*GSP9^{#} | CATGATCAGCTGGGCCAAGAAATAGGTCA | miR-154*RP2^{#} | AATCA+TA+CACGGTTGAC | -0.3056 | 8.8502 | 0.07 | 0.74 |
| | | SEQ ID NO:221 | | SEQ ID NO:222 | | | | |
| miR-154 | miR-154GSP9^{#} | CATGATCAGCTGGGCCAAGACGAAGGCAA | miR-154RP3^{#} | TA+GGTTA+TCCGTGTT | -0.3062 | 9.3947 | 0.10 | 0.96 |
| | | SEQ ID NO:223 | | SEQ ID NO:224 | | | | |
| miR-155 | miR-155GSP8^{#} | CATGATCAGCTGGGCCAAGACCCCTATC | miR-155RP2^{#} | TT+AA+TGCTAATCGTGATAGG | -0.3201 | 8.474 | 5.49 | 54.91 |
| | | SEQ ID NO:225 | | SEQ ID NO:226 | | | | |
| miR-181a | miR-181aGSP9^{#} | CATGATCAGCTGGGCCAAGAACTCACCGA | miR-181aRP2^{#} | AA+CATT+CAACGCTGTC | -0.2919 | 7.968 | 1.70 | 17.05 |
| | | SEQ ID NO:227 | | SEQ ID NO:228 | | | | |
| miR-181c | miR 181cGSP9^{#} | CATGATCAGCTGGGCCAAGAACTCACCGA | miR-181cRP2^{#} | AA+CATT+CAACCTGTCG | -0.3102 | 7.9029 | 1.08 | 10.78 |
| | | SEQ ID NO:229 | | SEQ ID NO:230 | | | | |
| miR-182^{*} | miR-182^{*}GSP | CATGATCAGCTGGGCCAAGATAGTTGGCAA | miR-182^{*}RP | T+GG+TTCTAGACTTGC | -0.2978 | 8.5876 | 4.25 | 42.47 |
| | | SEQ ID NO:231 | | SEQ ID NO:232 | | | | |
| miR-182 | miR-182GSP2 | CATGATCAGCTGGGCCAAGATGTGAGTTC | miR-182RP | TTT+GG+CAATGGTAG | -0.2863 | 9.0854 | 1.52 | 15.20 |
| | | SEQ ID NO:233 | | SEQ ID NO:234 | | | | |
| miR-183 | miR-183GSP2 | CATGATCAGCTGGGCCAAGACACTGAATT | miR-183RP | T+AT+GGCACTGGTAG | -0.2774 | 9.9254 | 1.95 | 19.51 |
| | | SEQ ID NO:235 | | SEQ ID NO:236 | | | | |
| miR-184 | miR-184GSP2 | CATGATCAGCTGGGCCAAGAACCCTTATC | miR-184RP | T+GG+ACGGAGAACTG | -0.2906 | 7.9585 | 0.05 | 0.49 |
| | | SEQ ID NO:237 | | SEQ ID NO:238 | | | | |
| miR-186 | miR-186GSP9^{#} | CATGATCAGCTGGGCCAAGAAAGCCCAAA | miR-186RP3^{#} | CA+AA+GAATT+CTCCTTTGG | -0.2861 | 8.6152 | 0.32 | 3.18 |
| | | SEQ ID NO:239 | | SEQ ID NO:240 | | | | |
| miR-187 | miR-187GSP | CATGATCAGCTGGGCCAAGACGGCTGCAAC | miR-187RP | T+CG+TGTCTTGTGTT | -0.2953 | 7.9329 | 1.23 | 12.31 |
| | | SEQ ID NO:241 | | SEQ ID NO:242 | | | | |
| miR-188 | miR-188GSP | CATGATCAGCTGGGCCAAGAACCCTCCACC | miR-188RP | C+AT+CCCTTGCATGG | -0.2925 | 8.0782 | 8.49 | 84.92 |
| | | SEQ ID NO:243 | | SEQ ID NO:244 | | | | |
| miR-189 | miR-189GSP2 | CATGATCAGCTGGGCCAAGAACTGATATC | miR-189RP | G+TG+CCTACTGAGCT | -0.2981 | 8.8964 | 0.21 | 2.08 |
| | | SEQ ID NO:245 | | SEQ ID NO:246 | | | | |
| miR-190 | miR-190GSP9^{#} | CATGATCAGCTGGGCCAAGAACCTAATAT | miR-190RP4^{#} | T+GA+TA+TGTTTGATATATT AG | -0.3317 | 9.8766 | 0.43 | 4.34 |
| | | SEQ ID NO:247 | | | | | | |
| | | | | SEQ ID NO:248 | | | | |
| miR-191 | miR-191GSP2 | CATGATCAGCTGGGCCAAGAAGCTGCTTT | miR-191RP2 | C+AA+CGGAATCCCAAAAG | -0.299 | 9.0317 | 0.41 | 4.07 |
| | | SEQ ID NO:249 | | SEQ ID NO:250 | | | | |
| miR-192 | miR-192GSP2 | CATGATCAGCTGGGCCAAGAGGCTGTCAA | miR-192RP | C+TGA+CCTATGAATTGAC | -0.2924 | 9.5012 | 1.10 | 10.98 |
| | | SEQ ID NO:251 | | SEQ ID NO:252 | | | | |
| miR-193 | miR-193GSP9^{#} | CATGATCGACTGGGCCAAGACTGGGACTT | miR-193RP2^{#} | AA+CT+GGCCTACAAAG | -0.3183 | 8.9942 | 0.17 | 1.72 |
| | | SEQ ID NO:253 | | SEQ ID NO:254 | | | | |
| miR-194 | mir194GSP8^{#} | CATGATCAGCTGGGCCAAGATCCACATG | mir194RP^{#} | TG+TAA+CAGCAACTCCA | -0.3078 | 9.8045 | 0.37 | 3.69 |
| | | SEQ ID NO:255 | | SEQ ID NO:256 | | | | |
| miR-195 | miR-195GSP9^{#} | CATGATCAGCTGGGCCAAGAGCCAATATT | miR-195RP3^{#} | T+AG+CAG+CACAGAAATA | -0.2955 | 10.213 | 0.76 | 7.58 |
| | | SEQ ID NO:257 | | SEQ ID NO:258 | | | | |
| miR-196b | miR-196bGSP | CATGATCAGCTGGGCCAAGACCAACAACAC | miR-196bRP | TA+GGT+AGTTTCCTGT | -0.301 | 8.1641 | 1.47 | 14.66 |
| | | SEQ ID NO:259 | | SEQ ID NO:260 | | | | |
| miR-196a | miR-196aGSP | CATGATCAGCTGGGCCAAGACCAACAACAT | miR-196aRP | TA+GG+TAGTTTCATGTTG | -0 2932 | 8.0448 | 8.04 | 80.37 |
| | | SEQ ID NO:261 | | SEQ ID NO:262 | | | | |
| miR-197 | miR-197GSP2 | CATGATCAGCTGGGCCAAGAGCTGGGTGG | miR-197RP | TT+CA+CCACCTTCTC | -0.289 | 8.2822 | 0.71 | 7.10 |
| | | SEQ ID NO:263 | | SEQ ID NO:264 | | | | |
| miR-198 | miR-198GSP3 | CATGATCAGCTGGGCCAAGACCTACTC | miR-198RP | G+GT+CCAGAGGGGAG | -0.2986 | 8.1359 | 0.31 | 3.15 |
| | | SEQ ID NO:265 | | SEQ ID NO 266 | | | | |
| miR- | miR-199a^{*}GSP2 | CATGATCAGCTGGGCCAAGAAACCAATGT | miR-199a^{*}RP | T+AC+AGTAGTCTGCAC | -0.3029 | 9.0509 | 0.25 | 2.52 |
| 199a^{*} | | SEQ ID NO:267 | | SEQ ID NO:268 | | | | |
| miR-199a | miR-199aGSP2 | CATGATCAGTGGGCCAAGAGAACAGGTA | miR-199aRP | C+CC+AGTGTTCAGAC | -0.3187 | 9.2268 | 0.12 | 1.16 |
| | | SEQ ID NO:269 | | SEQ ID NO:270 | | | | |
| miR-199b | miR-199bGSP | CATGATCAGCTGGGCCAAGAGAACAGATAG | miR-199bRP | C+CC+AGTGTTTAGAC | -0,3165 | 9.3935 | 2.00 | 20.04 |
| | | SEQ ID NO:271 | | SEQ ID NO:272 | | | | |
| miR-200a | miR.200aGSP2 | CATGATCAGCTGGGCCAAGAACATCGTTA | miR-200aRP | TAA+CAC+TGTCTGGT | -0.2754 | 9.1227 | 0.08 | 0.78 |
| | | SEQ ID NO:273 | | SEQ ID NO:274 | | | | |
| miR-200b | miR-200bGSP2 | CATGATCAGCTGGGCCAAGAGTCATCATT | miR-200bRP | TAATA+CTG+CCTGGTAAT | -0.2935 | 8.5461 | 0.08 | 0.85 |
| | | SEQ ID NO:275 | | SEQ ID NO:276 | | | | |
| miR-202 | miR-202 GSP10^{*} | CATGATCAGCTGGGCCAAGATTTTCCCATG | miR-202RP^{#} | A+GA+GGTATA+GGGCAT | -0.2684 | 9.056 | 0.25 | 2.48 |
| | | SEQ ID NO:277 | | SEQ ID NO:278 | | | | |
| miR-203 | miR-203GSP2 | CATGATCAGCTGGGCCAAGACTAGTGGTC | miR-203RP | G+TG+AAATGTTTAGGACC | -0.2852 | 8.1279 | 1.60 | 16.03 |
| | | SEQ ID NO:279 | | SEQ ID NO:280 | | | | |
| miR-204 | miR-204GSP2 | CATGATCAGCTGGGCCAAGAAGGCATAGG | miR-204RP | T+TC+CCTTTGTCATCC | -0.2925 | 8.7648 | 0.16 | 1.59 |
| | | SEQ ID NO:281 | | SEQ ID NO:282 | | | | |
| miR-205 | miR-205GSP | CATGATCAGCTGGGCCAAGACAGACTCCGG | miR-205RP | T+CCTT+CATTCCACC | -0.304 | 8.2407 | 9.21 | 92.15 |
| | | SEQ ID NO:283 | | SEQ ID NO:284 | | | | |
| miR-206 | mir206GSP7^{#} | CATGATCAGCTGGGCCAAGACCACACA | miR-206RP^{#} | T+G+GAA+TGTAAGGAAGTGT | -0.2815 | 8.2206 | 0.29 | 2.86 |
| | | SEQ ID NO:285 | | SEQ ID NO:286 | | | | |
| miR-208 | miR-208_GSP13^{#} | CATGATCAGCTGGGCCAAGAACAAGCTTTTTGC | miR 208_RP4^{#} | ATAA+GA+CG+AGCAAAAAG | -0.2072 | 7.9097 | 57.75 | 577.52 |
| | | SEQ ID NO:287 | | SEQ ID NO:288 | | | | |
| miR-210 | miR-210GSP | CATGATCAGCTGGGCCAAGATCAGCCGCTG | miR-2 10RP | C+TG+TGCGTGTGACA | -0.2717 | 8.249 | 0.18 | 1.77 |
| | | SEQ ID NO:289 | | SEQ ID NO:290 | | | | |
| miR-211 | miR-211GSP2 | CATGATCAGCTGGGCCAAGAAGGCGAAGG | miR-211RP | T+TC+CCTTTGTCATCC | -0.2926 | 8.3106 | 0.10 | 1.00 |
| | | SEQ ID NO:291 | | SEQ ID NO:292 | | | | |
| miR-212 | miR-212GSP9^{#} | CATGATCAGCTGGGCCAAGAGCCGTGAC | miR-212RP2^{#} | T+AA+CAGTCTCCAGTCA | -0.2916 | 8.0745 | 0.59 | 5.86 |
| | | SEQ ID NO:293 | | SEQ ID NO:294 | | | | |
| miR-213 | miR-213GSP | CATGATCAGCTGGGCCAAGAGGTACAATCA | miR-213RP | A+CC+ATCGACCGTTG | -0.2934 | 8.1848 | 2.96 | 29.59 |
| | | SEQ ID NO:295 | | SEQ ID NO:296 | | | | |
| miR-214 | miR-214GSP | CATGATCAGCTGGGCCAAGACTGCCTCT | miR-214RP | A+CA+GCAGGCACAGA | -0.2947 | 7.82 | 0.84 | 8.44 |
| | | SEQ ID NO:297 | | SEQ ID NO:298 | | | | |
| miR-215 | miR-215GSP2 | CATGATCAGCTGGGCCAAGAGTCTGTCAA | miR-215RP | A+TGA+CCTATGAATTGAC | -0.2932 | 8.9273 | 1.51 | 15.05 |
| | | SEQ ID NO:299 | | SEQ ID NO:300 | | | | |
| miR-216 | miR-216GSP^{#} | CATGATCAGCTGGGCCAAGACACAGTTGC | mir216RP^{#} | TAA+TCT+CAGCTGGCA | -0.273 | 8.5829 | 0.95 | 9.50 |
| | | SEQ ID NO:301 | | SEQ ID NO:302 | | | | |
| miR-217 | miR-217GSP2 | CATGATCAGCTGGGCCAAGAATCCAATCA | miR-2 17RP2 | T+AC+TGCATCAGGAACTGA | -0.3089 | 9.6502 | 0.07 | 0.71 |
| | | SEQ ID NO:303 | | SEQ ID NO:304 | | | | |
| miR-218 | miR-218GSP2 | CATGATCAGCTGGGCCAAGAACATGGTTA | miR-218RP | TTG+TGCTT+GATCTAAC | -0.2778 | 8.4363 | 1.00 | 10.05 |
| | | SEQ ID NO:305 | | SEQ ID NO:306 | | | | |
| miR-220 | miR-220GSP | CATGATCAGCTCGGGCCAAGAAAAGTGTCAG | miR-220RP | C+CA+CACCGTATCTG | -0.2755 | 9.0728 | 8.88 | 88.75 |
| | | SEQ ID NO:307 | | SEQ ID NO:308 | | | | |
| miR-221 | miR-221GSP9^{#} | CATGATCAGCTGGGCCAAGAGAAACCCAG | miR-221RP^{#} | A+CC+TACATTGTCTGC | -0,2886 | 8.5743 | 0.12 | 1.17 |
| | | SEQ ID NO:309 | | SEQ ID NO:310 | | | | |
| miR-222 | miR-222GSP8^{#} | CATGATCAGCTGGGCCAAGAGAGACCCA | miR-222RP^{#} | A+GC+TACATCTGGCT | -0.283 | 8.91 | 1.64 | 16.41 |
| | | SEQ ID NO:311 | | SEQ ID NO:312 | | | | |
| miR-223 | miR-223GSP | CATGATCAGCTGGGCCAAGAGGGGTATTTG | miR-223RP | TG+TC+AGTTTGTCAAA | -0.2998 | 8.6669 | 0.94 | 9.44 |
| | | SEQ ID NO:313 | | SEQ ID NO:314 | | | | |
| miR-224 | miR-224GSP8^{#} | CATGATCAGCTGGGCCAAGATAAACGGA | miR-224RP2^{#} | C+AAG+TCACTAGTGGTT | -0.2802 | 7.5575 | 0.56 | 5.63 |
| | | SEQ ID NO:315 | | (SEQ ID NO:316 | | | | |
| miR-296 | miR-296GSP9^{#} | CATGATCAGCTGGGCCAAGAACAGGATTG | miR-296RP2^{#} | A+GG+GCCCCCCCTCAA | -0.3178 | 8.3856 | 0.10 | 0.96 |
| | | SEQ ID NO:317 | | SEQ ID NO:318 | | | | |
| miR-299 | miR-299GSP9^{#} | CATGATCAGCTGGGCCAAGAATGTATGTG | miR-299RP^{#} | T+GG+TTTACCGTCCC | -0.3155 | 7.9383 | 1.30 | 12.96 |
| | | SEQ ID NO:319 | | SEQ ID NO:320 | | | | |
| miR-301 | miR-301GSP | CATGATCAGCTGGGCCAAGAGCTTTGACAA | miR-301RP | C+AG+TGCAATAGTATTGT | -0.2839 | 8.314 | 2.55 | 25.52 |
| | | SEQ ID NO:321 | | SEQ ID NO:322 | | | | |
| miR-302a^{*} | miR-302a^{*}GSP | CATGATCAGCTGGGCCAAGAAAAGCAAGTA | miR-302a^{*}RP | TAAA+CG+TGGATGTAC | -0 2608 | 8.3921 | 0.04 | 0.41 |
| | | SEQ ID NO:323 | | SEQ ID NO:324 | | | | |
| miR-302a | miR 302aGSP | CATGATCAGCTGGGCCAAGATCACCAAAAC | miR 302aRP | T+AAG+TGCTTCCATGT | -0.2577 | 9.6657 | 2.17 | 21.67 |
| | | SEQ ID NO:325 | | SEQ ID NO:326 | | | | |
| miR-302b* | miR-302b^{*}GSP | CATGATCAGCTGGGCCAAGAAGAAAGCACT | miR-302b^{*}RP | A+CTTTAA+CATGGAAGTG | -0.2702 | 8.5153 | 0.02 | 0.24 |
| | | SEQ ID NO:327 | | SEQ ID NO:328 | | | | |
| miR-302b | miR-302bGSP | CATGATCAGCTGGGCCAAGACTACTAAAAC | miR-302bRP | T+AAG+TGCTTCCATGT | -0.2398 | 9.1459 | 5.11 | 51.11 |
| | | SEQ ID NO:329 | | SEQ ID NO:330 | | | | |
| miR-302d | miR-302dGSP | CATGATCAGCTGGGCCAAGAACACTCAAAC | miR-302dRP | T+AAG+TGCTTCCATGT | -0.2368 | 8.5602 | 5.98 | 59.78 |
| | | SEQ ID NO:331 | | SEQ ID NO:332 | | | | |
| miR- | miR-302c*_GSP9^{#} | CATGATCAGCTGGGCCAAGACAGCAGGTA | miR-302c*_RP2^{#} | TT+TAA+CAT+GGGGGTACC | -0.312 | 8.2904 | 0.33 | 3.28 |
| 302c* | | SEQ ID NO:333 | | SEQ ID NO:334 | | | | |
| miR-302c | miR 302cGSP9^{#} | CATGATCAGCTGGGCCAAGACCACTGAAA | miR-302cRP5^{#} | T+AAG+TGCTTCCATGTTTCA | -0.2945 | 8.381 | 14.28 | 142.76 |
| | | SEQ ID NO:335 | | SEQ ID NO:336 | | | | |
| miR-320 | miR-320_GsP8^{#} | CATGATCAGCTGGGCCAAGATTCGCCCT | miR-320-RP3^{#} | AAAA+GCT+GGGTTGAGAGG | -0.2677 | 7.8956 | 6.73 | 67.29 |
| | | SEQ ID NO:337 | | SEQ ID NO:338 | | | | |
| miR-323 | miR-323GSP | CATGATCAGCTGGGCCAAGAAGAGGTCGAC | miR-323RP | G+CA+CATTACACGGT | -0.2878 | 8.2546 | 0.19 | 1.92 |
| | | SEQ ID NO:339 | | SEQ ID NO:340 | | | | |
| miR-324-3p | miR-324-3pGSP | CATGATCAGCTGGGCCAAGACCAGCAGCAC | miR-324-3pRP | C+CA+CTGCCCCAGGT | -0.2698 | 8.5223 | 2.54 | 25.41 |
| | | SEQ ID NO:341 | | SEQ ID NO:342 | | | | |
| miR-324-5p | miR-324-5pGSP | CATGATCAGCTGGGCCAAGAACACCAATGC | miR-324-5pRP | C+GC+ATCCCCTAGGG | -0.2861 | 7.6865 | 0.06 | 0.62 |
| | | SEQ ID NO:343 | | SEQ ID NO:344 | | | | |
| miR-325 | miR-325GSP | CATGATCAGCTGGGCCAAGAACACTTACTG | miR-325RP | C+CT+AGTAGGTGTCC | -0.2976 | 8.1925 | 0.01 | 0.14 |
| | | SEQ ID NO:345 | | SEQ ID NO:346 | | | | |
| miR-326 | miR-326GSP | CATGATCAGCTGGGCCAAGATGGAGGAAG | miR-326RP | C+CT+CTGGGCCCTTC | -0.2806 | 7.897 | 0.59 | 5.87 |
| | | SEQ ID NO:347 | | SEQ ID NO:348 | | | | |
| miR 328 | miR-328GSP | CATGATCAGCTGGGCCAAGAACGGAAGGGC | miR-328RP | C+TG+GCCCTCTCTGC | -0.293 | 7.929 | 3.17 | 31.69 |
| | | SEQ ID NO:349 | | SEQ ID NO:350 | | | | |
| miR-330 | miR-330GSP | CATGATCAGCTGGGCCAAGATCTCTGCAGG | miR-330RP | G+CA+AAGCACACGGC | -0.3009 | 7.7999 | 0.13 | 1.30 |
| | | SEQ ID NO:351 | | SEQ ID NO:352 | | | | |
| miR-331 | miR-331GSP | CATGATCAGCTGGGCCAAGATTCTAGGATA | miR-331RP | G+CC+CCTGGGCCTAT | -0.2816 | 8.1643 | 0.45 | 4.54 |
| | | SEQ ID NO:353 | | SEQ ID NO:354 | | | | |
| miR-337 | miR-337GSP | CATGATCAGCTGGGCCAAGAAAAGGCCATCA | miR-337RP | T+CC+AGCTCCTATATG | -0.2968 | 8.7313 | 0.10 | 1.02 |
| | | SEQ ID NO:355 | | SEQ ID NO:356 | | | | |
| miR-338 | miR-338GSP | CATGATCAGCTGGGCCAAGATCAACAAAAT | miR-338RP2 | T+CC+AGCATCAGTGATTT | -0.2768 | 8.5618 | 0.52 | 5.17 |
| | | SEQ ID NO:357 | | SEQ ID NO:358 | | | | |
| miR-339 | miR-339GSP9^{#} | CATGATCAGCTGGGCCCAAGATGAGCTCCT | miR-339RP2^{#} | T+CC+CTGTCCTCCAGG | -0.303 | 8.4873 | 0.27 | 2.72 |
| | | SEQ ID NO:359 | | SEQ ID NO:360 | | | | |
| miR-340 | miR-340GSP | CATGATCAGCTGGGCCAAGAGGCTATAAAG | miR-340RP | TC+CG+TCTCAGTTAC | -0.2846 | 9.6673 | 0.15 | 1.45 |
| | | SEQ ID NO:361 | | SEQ ID NO:362 | | | | |
| miR-342 | miR-342GSP3 | CATGATCAGCTGGGCCAAGAGACGGGTG | miR-342RP | T+CT+CACACAGAAATCG | -0.293 | 8.1553 | 4.69 | 46.85 |
| | | SEQ ID NO.363 | | SEQ ID NO:364 | | | | |
| miR-345 | miR-345GSP | CATGATCAGCTGGGCCAAGAGCCCTGGACT | miR-345RP | T+GC+TGACTCCTAGT | -0.2909 | 8.468 | 0.04 | 0.40 |
| | | SEQ ID NO:365 | | SEQ ID NO:366 | | | | |
| miR-346 | miR-346GSP | CATGATCAGCTGGGCCAAGAAGAGGCAGGC | miR-346RP | T+GT+CTGCCCGCATG | -0.2959 | 8.1958 | 0.25 | 2.54 |
| | | SEQ ID NO:367 | | SEQ ID NO.368 | | | | |
| miR-363 | miR-363 GSP10^{#} | CATGATCAGCTGGGCCAAGATACAGATGGA | miR-363RP^{#} | AAT+TG+CAC+GGTATCC | -0.2362 | 8.9762 | 0.44 | 4.36 |
| | | SEQ ID NO:369 | | SEQ ID NO:370 | | | | |
| miR-367 | miR-367GSP | CATGATCAGCTGGGCCAAGATCACCATTGC | miR-367RP | AAT+TG+CACTTTAGCAAT | -0.2819 | 8.6711 | 0.00 | 0.03 |
| | | SEQ ID NO:371 | | SEQ ID NO:372 | | | | |
| miR-368 | miR 368GSP | CATGATCAGCTGGGCCAAGAAAACGTGGAA | miR-368RP2 | A+CATAGA+GGAAATTCCAC | -0.2953 | 8.0067 | 6.01 | 60.11 |
| | | SEQ ID NO:373 | | SEQ ID NO:374 | | | | |
| miR-370 | miR-370GSP | CATGATCAGCTGGGCCAAGACCAGGTTCCA | miR-370RP | G+CC+TGCTGGGGTGG | -0.2825 | 8.3162 | 1.45 | 14.55 |
| | | SEQ ID NO:375 | | SEQ ID NO:376 | | | | |
| miR-371 | miR-371GSP | CATGATCAGCTGGGCCAAGAACACTCAAAA | miR-371RP | G+TG+CCGCCATCTTT | -0.295 | 7.8812 | 2.51 | 25.12 |
| | | SEQ ID NO:377 | | SEQ ID NO:378 | | | | |
| miR-372 | miR-372GSP | CATGATCAGCTGGGCCAAGAACGCTCAAAT | miR-372RP | A+AA+GTGCTGCGACA | -0.2984 | 8.9183 | 0.05 | 0.53 |
| | | SEQ ID NO:379 | | SEQ ID NO:380 | | | | |
| miR-373* | miR-373*GSP | CATGATCAGCTGGGGCCAAGAGGAAAGCGCC | miR-373*RP | A+CT+CAAAATGGGGG | -0.2705 | 8.4513 | 0.20 | 1.99 |
| | | SEQ ID NO:381 | | SEQ ID NO:382 | | | | |
| miR-373 | miR-373GSP | CATGATCAGCTGGGCCAAGAACACCCCAAA | miR-373RP2 | GA+AG+TGCTTCGATTTGG | -0.307 | 7.9056 | 9.13 | 91.32 |
| | | SEQ ID NO:383 | | SEQ ID NO:384 | | | | |
| miR-374 | miR-374GSP2 | CATGATCAGCTGGGCCAAGACACTTATCA | miR-374RP | TT+AT+AATA+CAACCTGATA | -0.2655 | 9.3795 | 9.16 | 91.60 |
| | | SEQ ID NO:385 | | AG | | | | |
| | | | | SEQ ID NO:386 | | | | |
| miR-375 | miR-375GSP | CATGATCAGCTGGGCCAAGATCACGCGAGC | miR-375RP | TT+TG+TTCGTTCGGC | -0.3041 | 8.1181 | 0.09 | 0.90 |
| | | SEQ ID NO:387 | | SEQ ID NO:388 | | | | |
| miR-376b | miR-376b GSP8^{#} | CATGATCAGCTGGGCCAAGAAACATGGA | miR-376bRP^{#} | AT+CAT+AGA+GGAAAATCCA | -0.2934 | 9.0188 | 1.07 | 10.74 |
| | | SEQ ID NO:389 | | SEQ ID NO:390 | | | | |
| miR-378 | miR-378GSP | CATGATCAGCTGGGCCAAGAACACAGGACC | miR-378RP | C+TC+CTGACTCCAGG | -0.2899 | 8.1467 | 0.07 | 0.73 |
| | | SEQ ID NO:391 | | SEQ ID NO:392 | | | | |
| miR-379 | miR-. 379_GSP7^{#} | CATGATCAGCTGGGCCAAGATACGTTC | miR-379RP2# | T+GGT+AGACTATGGAACG | -0.2902 | 8.2149 | 10.89 | 108.86 |
| | | SEQ ID NO:393 | | SEQ ID NO:394 | | | | |
| miR-380-5p | miR-380-5pGSP | CATGATCAGCTGGGCCAAGAGCGCATGTTC | miR-380-5pRP | T+GGT+TGACCATAGA | -0.2462 | 9.4324 | 1.30 | 13.04 |
| | | SEQ ID NO:395 | | SEQ ID NO:396 | | | | |
| miR-380-3p | miR-380-3pGSP | CATGATCAGCTGGGCCAAGAAAGATGTGGA | miR-380-3pRP | TA+TG+TAATATGGTCCACA | -0.3037 | 8.0356 | 3.69 | 36.89 |
| | | SEQ ID NO:397 | | SEQ ID NO:398 | | | | |
| miR-381 | miR-381GSP2 | CATGATCAGCTGGGCCAAGAACAGAGAGC | miR-381RP2 | TATA+CAA+GGGCAAGCT | -0.3064 | 8.8704 | 1.72 | 17.16 |
| | | SEQ ID NO:399 | | SEQ ID NO:400 | | | | |
| miR-382 | miR-382GSP | CATGATCAGCTGGGCCAAGACGAATCCACC | miR-382RP | G+AA+GTTGTTCGTGGT | -0.2803 | 7.6738 | 0.66 | 6.57 |
| | | SEQ ID NO:401 | | SEQ ID NO:402 | | | | |
| miR-383 | miR-383GSP | CATGATCAGCTGGGCCAAGAAGCCACAATC | miR-383RP2 | A+GATC+AGAAGGTGATTGT | -0.2866 | 8.1463 | 0.54 | 5.45 |
| | | SEQ ID NO:403 | | SEQ ID NO:404 | | | | |
| miR-410 | miR-410 GSP9^{#} | CATGATCAGCTGGGCCAAGAACAGGCCAT | miR-410RP^{#} | AA+TA+TAA+CA+CAGATGGC | -0.2297 | 8.5166 | 4.27 | 42.71 |
| | | SEQ ID NO:405 | | SEQ ID NO:406 | | | | |
| miR-412 | miR-412 GSP10^{#} | CATGATCAGCTGGGCCAAGAACGGCTAGTG | miR-412RP^{#} | A+CTT+CACCTGGTCCACTA | -0.3001 | 7.9099 | 4.24 | 42.37 |
| | | SEQ ID NO:407 | | SEQ ID NO:408 | | | | |
| miR-422a | miR-422aGSP | CATGATCAGCTGGGCCAAGAGGCCTTCTGA | miR-422aRP | C+TG+GACTTAGGGTC | -0.3079 | 9.3108 | 5.95 | 59.54 |
| | | SEQ ID NO:409 | | SEQ ID NO:410 | | | | |
| miR-422b | miR-422bGSP | CATGATCAGCTGGGCCAAGAGGCCTTCTGA | miR-422bRP | C+TG+GACTTGGAGTC | -0.2993 | 8.9437 | 4.86 | 48 56 |
| | | SEQ ID NO:411 | | SEQ ID NO:412 | | | | |
| miR-423 | miR-423GSP | CATGATCAGCTGGGCCAAGACTGAGGGGCC | miR-423RP | A+GC+TCGGTCTGAGG | -0.3408 | 9.2274 | 6.06 | 60.62 |
| | | SEQ ID NO:413 | | SEQ ID NO:414 | | | | |
| miR-424 | miR-424GSP^{#} | CATGATCAGCTGGGCCAAGATTCAAAACAT | miR-424RP2^{#} | C+AG+CAGCAATTCATGTTTT | -0.3569 | 9.3419 | 10.78 | 107 85 |
| | | SEQ ID NO:415 | | SEQ ID NO:416 | | | | |
| miR-425 | miR-425GSP | CATGATCAGCTGGGCCAAGAGGCGGACACG | miR-425RP | A+TC+GGGAATGTCGT | -0.2932 | 7.9786 | 0.39 | 3.93 |
| | | SEQ ID NO:417 | | SEQ ID NO.418 | | | | |
| miR-429 | miR-429_GSP11^{#} | CATGATCAGCTGGGCCAAGAACGGTTTTACC | miR-429RP5^{#} | T+AATAC+TG+TCTGGTAAAA | -0.2458 | 8.2805 | 16.21 | 162 12 |
| | | SEQ ID NO:419 | | SEQ ID NO:420 | | | | |
| miR-431 | miR-431 GSP10^{#} | CATGATCAGCTGGGCCAAGATGCATGACGG | miR-431RP^{#} | T+GT+CTTGCAGGCCG | -0.3107 | 7.7127 | 7.00 | 70.05 |
| | | SEQ ID NO:421 | | SEQ ID NO:422 | | | | |
| miR-448 | miR-448GSP | CATGATCAGCTGGGCCAAGAATGGGACATC | miR-448RP | TTG+CATA+TGTAGGATG | -0.3001 | 8.4969 | 0.12 | 1.16 |
| | | SEQ ID NO:423 | | SEQ ID NO:424 | | | | |
| miR-449 | miR-449GSP10^{#} | CATGATCAGCTGGGCCAAGAACCAGCTAAC | miR-449RP2# | T+GG+CAGTGTATTGTTAGC | -0.3225 | 8.4953 | 2.57 | 25.70 |
| | | SEQ ID NO:425 | | SEQ ID NO:426 | | | | |
| miR-450 | miR-450GSP | CATGATCAGCTGGGCCAAGATATTAGGAAC | miR-450RP | TTTT+TG+CGATGTGTT | -0.2906 | 8.1404 | 0.48 | 4.82 |
| | | SEQ ID NO:427 | | SEQ ID NO:428 | | | | |
| miR-451 | miR-451 GSP10^{#} | CATGATCAGCTGGGCCAAGAAAACTCAGTA | miR-451R^{#} | AAA+CCG+TTA+CCATTACTGA | -0.2544 | 8.0291 | 1.73 | 17 35 |
| | | SEQ ID NO:429 | | SEQ ID NO:430 | | | | |
| let7a | let7a-GSP2^{#} | CATGATCAGCTGGGCCAAGAAACTATAC | let7a-RP^{#} | T+GA+CGTAGTAGGTTG | -0.3089 | 9.458 | 0.04 | 0.38 |
| | | SEQ ID NO:431 | | SEQ ID NO:432 | | | | |
| let7b | let7b-GSP2^{#} | CATGATCAGCTGGGCCAAGAAACCACAC | let7b-RP^{#} | T+GA+GGTAGTAGGTTG | -0.2978 | 7.9144 | 0.05 | 0.54 |
| | | SEQ ID NO:433 | | SEQ ID NO.432 | | | | |
| let7c | let7c-GSP2^{#} | CATGATCAGCTGGGCCAAGAAACCATAC | let7c-RP^{#} | T+GA+GGTAGTAGGTTG | -0.308 | 7.9854 | 0.01 | 0.14 |
| | | SEQ ID NO:434 | | SEQ ID NO:432 | | | | |
| let7d | let7d-GSP2^{#} | CATGATCAGCTGGGCCAAGAACTATGCA | let7d-RP^{#} | A+GA+GGTAGTAGGTTG | -0.3238 | 8.3359 | 0.06 | 0.57 |
| | | SEQ ID NO:435 | | SEQ ID NO:436 | | | | |
| let7e | let7e-GSP2^{#} | CATGATCAGCTGGGCCAAGAACTATACA | let7e-RP^{#} | T+GA+GGTAGGAGGTTG | -0.3284 | 9.7594 | 0.22 | 2.20 |
| | | SEQ ID NO:437 | | SEQ ID NO:438 | | | | |
| let7f | let7f-GSP2^{#} | CATGATCAGCTGGGCCAAGAAACTATAC | let7f-RP^{#} | T+GA+GGTAGTAGATTG | -0.2901 | 11.107 | 0.32 | 3.18 |
| | | SEQ ID NO:439 | | SEQ ID NO.440 | | | | |
| let7g | let7g-GSP2^{#} | CATGATCAGCTGGGCCAAGAACTGTACA | let7g-RP^{#} | T+GA+GGTAGTAGTTTG | -0.3469 | 9.8235 | 0.16 | 1.64 |
| | | SEQ ID NO:441 | | SEQ ID NO:442 | | | | |
| let7i | let7i-GSP2^{#} | CATGATCAGCTGGGCCAAGAACAGCACA | let7i-RP^{#} | T+GA+GGTAGTAGTTTG | -0.321 | 10.82 | 0.20 | 1.99 |
| | | SEQ ID NO:443 | | SEQ ID NO:444 | | | | |
| miR-377 | miR-377GSP | CATGATCAGCTGGGCCAAGAACAAAAGTTG | miR-377RP2 | AT+CA+CACAAAGGCAAC | -0.2979 | 10.612 | 13.45 | 134.48 |
| | | SEQ ID NO:445 | | SEQ ID NO:446 | | | | |
| miR-376a | miR-376a_GSP7 | CATGATCAGCTGGGCCAAGAACGTGGA | miR-376a_RP5 | AT+CAT+AGA+GGAAAATCC | -0.2938 | 10.045 | 63.00 | 630.00 |
| | | SEQ ID NO:447 | | SEQ ID NO:448 | | | | |
| miR-22 | miR 22GSP | CATGATCAGCTGGGCCAAGAACAGTTCTTC | miR 22RP | A+AG+CTGCCAGTTGA | -0.2862 | 8.883 | 20.46 | 204.58 |
| | | SEQ ID NO:449 | | SEQ ID NO:450 | | | | |
| miR-200c | miR-200cGSP2 | CATGATCAGCTGGGCCAAGACCATCATTA | miR-200cRP | T+AA+TACTGCCGGGT | -0.3094 | 11.5 | 15.99 | 159.91 |
| | | SEQ ID NO:451 | | SEQ ID NO:452 | | | | |
| mix-24 | miR-24GSP | CATGATCAGCTGGGCCAAGACTGTTCCTGC | miR-24RP | T+GG+CTCAGTTCAGC | -0.3123 | 8.6824 | 24.34 | 243.38 |
| | | SEQ ID NO:453 | | SEQ ID NO.454 | | | | |
| miR- | miR-29cGSP10 | CATGATCAGCTGGGCCAAGAACCGATTTCA | miR-29cRP | T+AG+CACCATTTGAAAT | -0.2975 | 8.8441 | 23.22 | 232.17 |
| 29cDNA | | SEQ ID NO:455 | | SEQ ID NO:456 | | | | |
| miR-18 | miR-18GSP | CATGATCAGCTGGGCCAAGATATCTGCACT | miR-18RP | T+AA+GGTGCATCTAGT | -0.3209 | 9.0999 | 14.90 | 149.01 |
| | | SEQ ID NO:457 | | SEQ ID NO.458 | | | | |
| miR-185 | miR-185GSP | CATGATCAGCTGGGCCAAGAGAACTGCCTT | miR-185RP | T+GG+AGAGAAAGGCA | -0.3081 | 8.9289 | 15.73 | 157.32 |
| | | SEQ ID NO:459 | | SEQ ID NO:460 | | | | |
| miR-181b | miR-181bGSP8^{#} | CATGATCAGCTGGGCCAAGACCCACCGA | miR-181bRP2^{#} | AA+CATT+CATTGCTGTC | -0.3115 | 10.846 | 15.87 | 159.67 |
| | | SEQ ID NO:461 | | SEQ ID NO:462 | | | | |
| miR-128a | miR-128aGSP | CATGATCAGCTGGGCCAAGAAAAAGAGACC | miR-128anLRP | TCACAGTGAACCGGT | approx. | approx. | approx. | approx. |
| | | SEQ ID NO:161 | | SEQ ID NO:494 | -0.2866 | 8.0867 | 0.16 | 1.60 |
| miR-138 | miR-138GSP2 | CATGATCAGCTGGGCCAAGACGGCCTGAT | miR- 138nLRP | AGCTGGTGTTGTGAA | approx. | approx. | approx. | approx. |
| | | SEQ ID NO:187 | | SEQ ID NO:495 | -0.3023 | 9.0814 | 0.22 | 2.19 |
| miR-143 | miR-143GSP8^{#} | CATGATCAGCTGGGCCAAGATGAGCTAC | miR-143nLRP | TGAGATGAAGCACTGT | approx. | approx. | approx. | approx. |
| | | SEQ ID NO:197 | | SEQ ID NO:496 | -0.3008 | 9.2675 | 0.37 | 3.71 |
| miR-150 | miR-150GSP3 | CATGATCAGCTGGGCCAAGACACTGGTA | miR-150nLRP | TCTCCCAACCCTTGTA | approx. | approx. | approx. | approx. |
| | | SEQ ID NO:213 | | SEQ ID NO:497 | -0.2943 | 8.3945 | 0.06 | 0.56 |
| miR-181a | miR-181aGSP9^{#} | CATGATCAGCTGGGCCAAGAACTCACCGA | miR-181anLRP | AACATTCAACGCTGT | approx. | approx. | approx. | approx. |
| | | SEQ ID NO:227 | | SEQ ID NO:498 | -0.2919 | 7.968 | 1.70 | 17.05 |
| miR-194 | mir194GSP8^{#} | CATGATCAGCTGGGCCAAGATCCACATG | miR-194nLRP | TGTAACAGCAACTCCA | approx. | approx. | approx. | approx. |
| | | SEQ ID NO:255 | | SEQ ID NO:499 | -0.3078 | 8.8045 | 0.37 | 3.69 |

### Example 4

This Example describes assays and primers designed for quantitative analysis of murine miRNA expression patterns.

Methods: The representative murine microRNA target templates described in TABLE 7 are publically available accessible on the World Wide Web at the Wellcome Trust Sanger Institute website in the "miRBase sequence database" as described in Griffith-Jones et al. (2004), Nucleic Acids Research 32:D109-D111 and Griffith-Jones et al. (2006), Nucleic Acids Research 34: D140-D144. As indicated below in TABLE 7, the murine microRNA templates are either totally identical to the corresponding human microRNA templates, identical in the overlapping sequence with differing ends, or contain one or more base pair changes as compared to the human microRNA sequence. The murine microRNA templates that are identical or that have identical overlapping sequence to the corresponding human templates can be assayed using the same primer sets designed for the human microRNA templates, as indicated in TABLE 7. For the murine microRNA templates with one or more base pair changes in comparison to the corresponding human templates, primer sets have been designed specifically for detection of the murine microRNA, and these primers are provided in TABLE 7. The extension primer reaction and quantitative PCR reactions for detection of the murine microRNA templates may be carried out as described in EXAMPLE 3.

**TABLE 7: PRIMERS TO DETECT MURINE MICRORNA TARGET TEMPLATES**

| **Mouse Target microRNA:** | **Extension Primer Name** | **Extension Primer Sequence** | **Reverse Primer Name** | **Reverse Primer Sequence** | **Mouse microRNA as compared to Human microRNA** |
|---|---|---|---|---|---|
| **miR-1** | miR1GSP10 | CATGATCAGCTGGGCCAAGATACATACTTC | miR-1RP | T+G+GAA+TG+TAAAGAAGT | Identical |
| | | SEQ ID NO:47 | | SEQ ID NO:48 | |
| **miR-7** | miR-7GSP10 | CATGATCAGCTGGGCCAAGAAACAAAATC | miR-7_RP6 | T+GGAA+GACTTGTGATTTT | one or more base pairs differ |
| | | SEQ ID NO: 486 | | SEQ ID NO: 487 | |
| **miR-9*** | miR-9*GSP | CATGATCAGCTGGGCCAAGAACTTTCGGTT | miR-9*RP | TAAA+GCT+AGATAACCG | Identical overlapping sequence, |
| | | SEQ ID NO:51 | | SEQ ID NO:52 | ends differ |
| **miR-10a** | miR-10aGSP | CATGATCAGCTGGGCCAAGACACAAATTCG | miR-10aRP | T+AC+CCTGTAGATCCG | Identical |
| | | SEQ ID NO:53 | | SEQ ID NO:54 | |
| **miR-10b** | miR-10b_GSP11 | CATGATCAGCTGGGCCAAGAACACAAATTCG | miR-10b_RP2 | C+CC+TGT+AGAACCGAAT | one or more base pairs differ |
| | | SEQ ID NO: 492 | | SEQ ID NO: 493 | |
| **miR-15a** | miR-15aGSP | CATGATCAGCTGGGCCAAGACACAAACCAT | miR-15aRP | T+AG+CAGCACATAATG | Identical |
| | | SEQ ID NO:57 | | SEQ ID NO:58 | |
| **miR-15b** | miR-15bGSP2 | CATGATCAGCTGGGCCAAGATGTAAACCA | miR-15bRP | T+AG+CAGCACATCAT | Identical |
| | | SEQ ID NO:59 | | SEQ ID NO:60 | |
| **miR-16** | miR-16GSP2 | CATGATCAGCTGGGCCAAGACGCCAATAT | miR-16RP | T+AG+CAGCACGTAAA | Identical |
| | | SEQ ID NO:61 | | SEQ ID NO:62 | |
| **miR-17-3p** | miR-17-3pGSP | CATGATCAGCTGGGCCAAGAACAAGTGCCC | miR-17-3pRP | A+CT+GCAGTGAGGGC | one or more base pairs differ |
| | | SEQ ID NO: 463 | | SEQ ID NO: 464 | |
| **miR-17-5p** | miR-17-5pGSP2 | CATGATCAGCTGGGCCAAGAACTACCTGC | miR-17-5pRP | C+AA+AGTGCTTACAGTG | Identical |
| | | SEQ ID NO:65 | | SEQ ID NO:66 | |
| **miR-19a** | miR-19aGSP2 | CATGATCAGCTGGGCCAAGATCAGTTTTG | miR-19aRP | TG+TG+CAAATCTATGC | Identical |
| | | SEQ ID NO:67 | | SEQ ID NO:68 | |
| **miR-19b** | miR-19bGSP | CATGATCAGCTGGGCCAAGATCAGTTTTGC | miR-19bRP | TG+TG+CAAATCCATG | Identical |
| | | SEQ ID NO:69 | | SEQ ID NO:70 | |
| **miR-20** | miR-20GSP3 | CATGATCAGCTGGGCCAAGACTACCTGC | miR-20RP | T+AA+AGTGCTTATAGTGCA | Identical |
| | | SEQ ID NO:71 | | SEQ ID NO:72 | |
| **miR-21** | miR-21GSP2 | CATGATCAGCTGGGCCAAGATCAACATCA | miR-21RP | T+AG+CTTATCAGACTGATG | Identical |
| | | SEQ ID NO: 73 | | SEQ ID NO:74 | |
| **miR-23a** | miR-23aGSP | CATGATCAGCTGGGCCAAGAGGAAATCCCT | miR-23aRP | A+TC+ACATTGCCAGG | Identical |
| | | SEQ ID NO:75 | | SEQ ID NO:76 | |
| **miR-23b** | miR-23bGSP | CATGATCAGCTGGGCCAAGAGGTAATCCCT | miR-23bRP | A+TC+ACATTGCCAGG | Identical |
| | | SEQ ID NO:77 | | SEQ ID NO:78 | |
| **miR-24** | miR-24P5 | CATGATCAGCTGGGCCAAGACTGTTCCTGC TG | miR24-1,2R | TGG+CTCAGTTCAGC | Identical |
| | | SEQ ID NO: 7 | | SEQ ID NO: 19 | |
| **miR-25** | miR-25GSP | CATGATCAGCTGGGCCAAGATCAGACCGAG | miR-25RP | C+AT+TGCACTTGTCTC | Identical |
| | | SEQ ID NO:79 | | SEQ ID NO:80 | |
| **miR-26a** | miR-26aGSP9 | CATGATCAGCTGGGCCAAGAGCCTATCCT | miR-26aRP2 | TT+CA+AGTAATCCAGGAT | Identical |
| | | SEQ ID NO:81 | | SEQ ID NO:82 | |
| **miR-26b** | miR-26bGSP9 | CATGATCAGCTGGGCCAAGAAACCTATCC | miR-26bRP2 | TT+CA+AGT+AATTCAGGAT | Identical |
| | | SEQ ID NO:83 | | SEQ ID NO:84 | |
| **miR-27a** | miR-27aGSP | CATGATCAGCTGGGCCAAGAGCGGAACTTA | miR-27aRP | TT+CA+CAGTGGCTAA | Identical |
| | | SEQ ID NO:85 | | SEQ ID NO:86 | |
| **miR-27b** | miR-27bGSP | CATGATCAGCTGGGCCAAGAGCAGAACTTA | miR-27bRP | TI+CA+CAGTGGCTAA | Identical |
| | | SEQ ID NO:87 | | SEQ ID NO:88 | |
| **miR-28** | miR-28GSP | CATGATCAGCTGGGCCAAGACTCAATAGAC | miR-28RP | A+AG+GAGCTCACAGT | Identical |
| | | SEQ ID NO:89 | | SEQ ID NO:90 | |
| **miR-29a** | miR-29aGSP8 | CATGATCAGCTGGGCCAAGAAACCGATT | miR-29aRP2 | T+AG+CACCATCTGAAAT | Identical |
| | | SEQ ID NO:91 | | SEQ ID NO:92 | |
| **miR-29b** | miR-29bGSP2 | CATGATCAGCTGGGCCAAGAAACACTGAT | miR-29bRP2 | T+AG+CACCATTTGAAATCAG | Identical |
| | | SEQ ID NO:93 | | SEQ ID NO:94 | |
| **miR-30a-5p** | miR-30a-5pGSP | CATGATCAGCTGGGCCAAGACTTCCAGTCG | miR-30a-bpRP | T+GT+AAACATCCTCGAC | Identical |
| | | SEQ ID NO:95 | | SEQ ID NO:96 | |
| **miR-30b** | miR-30bGSP | CATGATCAGCTGGGCCAAGAAGCTGAGTGT | miR-30bRP | TGT+AAA+CATCCTACACT | Identical |
| | | SEQ ID NO:97 | | SEQ ID NO:98 | |
| miR-30c | miR-30cGSP | CATGATCAGCTGGGCCAAGAGCTGAGAGTG | miR-30cRP | TGT+AAA+CATCCTACACT | Identical |
| | | SEQ ID NO:99 | | SEQ ID NO:100 | |
| miR-30d | miR-30dGSP | CATGATCAGCTGGGCCAAGACTTCCAGTCG | miR-30dRP | T+GTAAA+CATCCCCG | Identical |
| | | SEQ ID NO:101 | | SEQ ID NO:102 | |
| miR-30e-3p | miR-30e-3pGSP9 | CATGATCAGCTGGGCCAAGAGCTGSAAAC | miR-30e-3pRP5 | CTTT+CAGT+CGGATGTTT | Identical |
| | | SEQ ID NO:103 | | SEQ ID NO:104 | |
| miR-31 | miR-31GSP | CATGATCAGCTGGGCCAAGACAGCTATGCC | miR-31RP | G+GC+AAGATGCTGGC | Identical overlapping sequence, ends differ |
| | | SEQ ID NO:107 | | SEQ ID NO:108 | |
| miR-32 | miR-32GSP | CATGATCAGCTGGGCCAAGAGCAACTTAGT | miR-32RP | TATTG+CA+CATTACTAAG | Identical |
| | | SEQ ID NO:109 | | SEQ ID NO:110 | |
| miR-33 | miR-33GSP2 | CATGATCAGCTGGGCCAAGACAATGCAAC | miR 33RP | G+TG+CATTGTAGTTGC | Identical |
| | | SEQ ID NO:111 | | SEQ ID NO:112 | |
| miR-34a | miR-34aGSP | CATGATCAGCTGGGCCAAGAAACAACCAGC | miR-34aRP | T+GG+CAGTGTCRTAG | Identical |
| | | SEQ ID NO:113 | | SEQ ID NO:114 | |
| miR-34b | miR-34bGSP | CATGATCAGCTGGGCCAAGACAATCAGCTA | miR-34bRP | TA+GG+CAGTGTAATT | one or more base pairs differ |
| | | SEQ ID NO: 115 | | SEQ ID No: 482 | |
| miR-34c | miR-34cGSP | CATGATCAGCTGGGCCAAGAGCAATCAGCT | miR-34cRP | A+GG+CAGTGTAGTTA | Identical |
| | | SEQ ID NO:117 | | SEQ ID NO:118 | |
| miR-92 | miR-92GSP | CATGATCAGCTGGGCCAAGACAGGCCGGGA | miR-92RP | T+AT+TGCACTTGTCCC | Identical |
| | | SEQ ID NO:119 | | SEQ ID NO:120 | |
| miR-93 | miR-93GSP | CATGATCAGCTGGGCCAAGACTACCTGCAC | miR-93RP | AA+AG+TGCTGTTCGT | Identical overlapping sequence, ends differ |
| | | SEQ ID NO:121 | | SEQ ID NO:122 | |
| miR-96 | miR-96GSP | CATGATCAGCTGGGCCAAGAGCAAAAATGT | miR-96RP | T+TT+GGCACTAGCAC | ldentical overlapping sequence, ends differ |
| | | SEQ ID NO:125 | | SEQ ID NO:126 | |
| miR-98 | miR-98GSP | CATGATCAGCTGGGCCAAGAAACAATACAA | miR-98RP | TGA+GGT+AGTAAGTTG | Identical |
| | | SEQ ID NO:127 | | SEQ ID NO:128 | |
| miR-99a | miR-99aGSP | CATGATCAGCTGGGCCAAGACACAAGATCG | miR-99aRP | A+AC+CCGTAGATCCG | Identical overlapping sequence, ends differ |
| | | SEQ ID NO:129 | | SEQ ID NO:130 | |
| miR-99b | miR-99bGSP | CATGATCFGCTGGGCCAAGACGCAAGGTCG | miR-99bRP | C+AC+CCGTAGAACCG | Identical |
| | | SEQ ID NO:131 | | SEQ ID NO:132 | |
| miR-100 | miR-100GSP | CATGATCAGCTGGGCCAAGACACAAGTTCG | miR-100RP | A+AC+CCGTACATCCG | Identical |
| | | SEQ ID NO:133 | | SEQ ID NO:134 | |
| miR-101 | miR-101GSP | CATGATCAGCTGGGCCAAGACTTCACTTAT | miR-101RP | TA+CAG+TACTGTGATAACT | Identical |
| | | SEQ ID NO:135 | | SEQ ID NO:136 | |
| miR-103 | miR-103GSP | CATGATCAGCTGGGCCAAGATCATAGCCCT | miR-103RP | A+GC+AGCATTGTACA | Identical |
| | | SEQ ID NO:137 | | SEQ ID NO:138 | |
| miR-106a | miR-106aGSP | CATGATCRGCTGGGCCAAGATACCTGCAC | miR-106aRP | CAATAGTTGCTAACAGTG | one or more base pairs differ |
| | | SEQ ID NO: 472 | | SEQ ID NO: 473 | |
| miR-106b | miR-106bGSP | CATGATCAGCTGGGCCAAGAATCTGCACTG | miR-106bRP | T+AAAG+TGCTGACAGT | Identical |
| | | SEQ ID NO:143 | | SEQ ID NO:144 | |
| miR-107 | miR-107GSP8 | CATGATCAGCTGGGCCAAGATGATAGCC | miR-107RP2 | A+GC+AGCATTGTACAG | Identical |
| | | SEQ ID NO:145 | | SEQ ID NO:146 | |
| miR-122a | miR-122aGSP | CATGATCAGCTGGGCCAAGAACAAACACCA | miR-122aRP | T+GG+AGTGTGACAAT | Identical |
| | | SEQ ID NO:147 | | SEQ ID NO:148 | |
| miR-124a | miR-124aGSP | CATGATCAGCTGGGCCAAGATGGCATTCAC | miR-124aRP | T+TA+AGGCACGCGGT | Identical overlapping sequence, ends differ |
| | | SEQ ID NO:149 | | SEQ ID NO:150 | |
| miR-125a | miR-125aGSP | CATGATCAGCTGGGCCAAGACACAGGTTAA | miR-125aRP | T+CC+CTGAGACCCTT | Identical |
| | | SEQ ID NO:151 | | SEQ ID NO:152 | |
| miR-125b | miR-125bGSP | CATGATCAGCTGGGCCAAGATCACAAGTTA | miR-125bRP | T+CC+CTGAGACCTA | Identical |
| | | SEQ ID NO:153 | | SEQ ID NO:154 | |
| miR-126 | miR-126GSP | CATGATCAGCTGGGCCAAGAGCATTATTAC | miR-126RP | T+CG+TACCGTGAGTA | Identical |
| | | SEQ ID NO: 155 | | SEQ ID NO:156 | |
| miR-126* | miR-126*GSP3 | CATGATCAGCTGGGCCAAGACGCGTACC | miR-126*RP | C+ATT+ATTA+CTTTTGGTACG | Identical |
| | | SEQ ID NO:157 | | SEQ ID NO:158 | |
| miR-127 | miR-127GSP | CATGATCAGCTGGGCCAAGAAGCCAAGCTC | miR-127RP | T+CG+GATCCGTCTGA | Identical overlapping sequence, ends differ |
| | | SEQ ID NO:159 | | SEQ ID NO:160 | |
| miR-128a | miR-128aGSP | CATGATCAGCTGGGCCAAGAAAAAGAGACC | miR-128aRP | T+CA+CAGTGAACCGG | Identical |
| | | SEQ ID NO:161 | | SEQ ID NO:162 | |
| miR-128b | miR-128bGSP | CATGATCAGCTGGGCCAAGAGAAAGAGACC | miR-128bRP | T+CA+CAGTGAACCGG | Identical |
| | | SEQ ID NO:163 | | SEQ ID NO:164 | |
| miR-130a | miR-130aGSP | CATGATCAGCTGGGCCAAGAATGCCCTTTT | miR-130aRP | C+AG+TGCAATGTTAAAAG | identical |
| | | SEQ ID NO:167 | | SEQ ID NO:168 | |
| miR-130b | miR-130bGSP | CATGATCAGCTGGGCCAAGAATGCCCTTTC | miR-130bRP | C+AG+TGCAATGATGA | Identical |
| | | SEQ ID NO:169 | | SEQ ID NO:170 | |
| miR-132 | miR-132GSP | CATGATCAGCTGGGCCAAGACGACCATGGC | miR-132RP | T+AA+CAGTCTACAGCC | Identical |
| | | SEQ ID NO:171 | | SEQ ID NO:172 | |
| miR-133a | miR-133aGSP | CATGATCAGCTGGGCCAAGAACAGCTGGTT | miR-133aRP | T+TG+TGTCCCCTTCAA | Identical |
| | | SEQ ID NO:173 | | SEQ ID NO:174 | |
| miR-133b | miR-133bGSP | CATGATCAGCTGGGCCAAGATAGCTGGTTG | miR-133bRP | T+TG+GTCCCCTTCAA | Identical |
| | | SEQ ID NO:175 | | SEQ ID NO:176 | |
| miR-134 | miR-134GSP | CATGATCAGCTGGGCCAAGACCCTCTGGTC | miR-134RP | T+GT+GACTGGTTGAC | Identical overlapping sequence, ends differ |
| | | SEQ ID NO:177 | | SEQ ID NO:178 | |
| miR-135a | miR-135aGSP | CATGATCAGCTGGGCCAAGATCACATAGGA | miR-135aRP | T+AT+GGCTTTTTATTCCT | Identical |
| | | SEQ ID NO:179 | | SEQ ID NO:180 | |
| miR-135b | miR-135bGSP | CATGATCAGCTGGGCCAAGACACATAGGAA | miR-135bRP | T+AT+GGCTTTTCATTCC | Identical |
| | | SEQ ID NO:181 | | SEQ ID NO:162 | |
| miR-136 | miR-136GSP | CATGATCAGCTGGGCCAAGATCCATCATCA | miR-136RP | A+CT+CCATTTGTTTTGATG | Identical |
| | | SEQ ID NO:183 | | SEQ ID NO:184 | |
| miR-137 | miR-137GSP | CATGATCAGCTGGGCCAAGACTACGCGTAT | miR-137RP | T+AT+TGCTTAAGAATACGC | Identical overlapping sequence, ends differ |
| | | SEQ ID NO:185 | | SEQ ID NO:186 | |
| miR-138 | miR-138GSP2 | CATGATCAGCTGGGCCAAGACGGCCTGAT | miR-138RP | A+GC+TGGTGTTGTGA | Identical |
| | | SEQ ID NO:187 | | SEQ ID NO:188 | |
| miR-139 | miR-139GSP | CATGATCAGCTGGGCCAAGAAGACACGTGC | miR-139RP | T+CT+ACAGTGCACGT | Identical |
| | | SEQ ID NO:189 | | SEQ ID NO:190 | |
| miR-140 | miR-140GSP | CATGATCAGCTGGCCCAAGACTACCATAGG | miR-140RP | A+GT+GGTTTTACCCT | Identical overlapping sequence. ends differ |
| | | SEQ ID NO:191 | | SEQ ID NO:192 | |
| miR-141 | miR-141GSP9 | CATGATCAGCTGGGCCAAGACCATCTTTA | miR-141RP2 | TAA+CAC+TGTCTGGTAA | Identical |
| | | SEQ ID NO:193 | | SEQ ID NO:l94 | |
| miR-142-3p | miR-142-3pGSP3 | CATGATCAGCTGGGCCAAGATCCATAAA | miR-142-3pRP | TGT+AG+TGTTTCCTACT | Identical overlapping sequence, ends differ |
| | | SEQ ID NO:195 | | SEQ ID NO:196 | |
| miR-143 | miR-143GSP8 | CATGATCAGCTGGGCCAAGATGAGCTAC | miR-143RP2 | T+GA+GATGAAGCACTG | Identical |
| | | SEQ ID NO:197 | | SEQ ID NO:198 | |
| miR-144 | miR-144GSP2 | CATGATCAGCTGGGCCAAGACTAGTACAT | miR-144RP | TA+CA+GTAT+AGATGATG | Identical |
| | | SEQ ID NO:199 | | SEQ ID NO:200 | |
| miR-145 | miR-145GSP2 | CATGATCACCTGGGCCAAGAAAGGGATTC | miR-145RP | G+TC+CAGTTTTCCCA | Identical |
| | | SEQ ID NO:201 | | SEQ ID NO:202 | |
| miR-146 | miR-146GSP3 | CATGATCAGCTGGGCCAAGAAACCCATG | miR-146RP | T+GA+GAACTGAATTCCA | Identical |
| | | SEQ ID NO:203 | | SEQ ID NO:204 | |
| miR-148a | miR-148aGSP2 | CATGATCAGCTGGGCCAAGAACAAAGTTC | miR-148aRP2 | T+CA+GTGCACTACAGAACT | Identical |
| | | SEQ ID NO:207 | | SEQ ID NO:208 | |
| miR-148b | miR-198bGSP2 | CATGATCAGCTGGGCCAAGAACAAAGTTC | miR-148bRP | T+CA+GTGCATCACAG | Identical |
| | | SEQ ID NO:209 | | SEQ ID NO:210 | |
| miR-149 | miR-149GSP2 | CATGATCAGCTGGGCCAAGAGGAGTGAAG | miR-149RP | T+CT+GGCTCCGTGTC | Identical |
| | | SEQ ID NO:211 | | SEQ ID NO:212 | |
| miR-150 | miR-150GSP3 | CATGATCAGCTGGGCCAAGACACTGGTA | miR-150RP | T+CT+CCCAACCCTTG | Identical |
| | | SEQ ID NO:213 | | SEQ ID NO:214 | |
| miR-151 | miR-151GSP2 | CATGATCAGCTGGGCCAAGACCTCAAGGA | miR-151RP | A+CT+AGACTGAGGCTC | one or more base pairs differ |
| | | SEQ ID NO: 215 | | SEQ ID No: 477 | |
| miR-152 | miR-152GSP2 | CATGATCAGCTGGGCCAGACCCAAGTTC | miR-152RP | T+CA+GTGCATGACAG | Identical |
| | | SEQ ID NO:217 | | SEQ ID NO:218 | |
| miR-153 | miR-153GSP2 | CATGATCAGCTGGGCCAAGATCACTTTTG | miR-153RP | TTG+CAT+AGTCACAAAA | Identical overlapping sequence, ends differ |
| | | SEQ ID NO:219 | | SEQ ID NO:220 | |
| miR-154 | miR-154GSP9 | CATGATCAGCTGGGCCAAGACGAAGGCAA | miR-154RP3 | TA+GGTTA+TCCGTGTT | Identical |
| | | SEQ ID NO:223 | | SEQ ID NO:224 | |
| miR-155 | miR-155GSP8 | CATGATCAGCTGGGCCAAGACCCCTAFC | miR-155RP2 | TT+AA+TGCTAATTGTGATAGG | one or more base pairs differ |
| | - | SEQ ID NO: 225 | | SEQ ID NO: 489 | |
| miR-181a | miR-181aGSP9 | CATGATCAGCTGGGCCAAGAACTCACCGA | miR-181aRP2 | AA+CATT+CFACGCTGTC | Identical |
| | | SEQ ID NO:227 | | SEQ ID NO:228 | |
| miR-181c | miR-181cGSP9 | CATGATCAGCTGGGCCAAGAACTCACCGA | miR-181cRP2 | AA+CATT+CAACCTGTCG | Identical |
| | | SEQ ID NO:229 | | SEQ ID NO:230 | |
| miR-182 | miR-182*GSP | CATGATCAGTGGGCCAAGATAGTTGGCAA | miR-182*RP | T+GG+TTCTAGACTTGC | Identical |
| | | SEQ ID NO:231 | | SEQ ID NO:232 | |
| miR-183 | miR-183GSP2 | CATGATCAGCTGGGCCAAGACAGTGAATT | miR-183RP | T+AT+GGCACtGGTAG | Identical |
| | | SEQ ID NO:235 | | SEQ ID NO:236 | |
| miR-184 | miR-184GSP2 | CATGATCAGCTGGGCCAAGAACCCTTATC | miR-189RP | T+GG+ACGGAGAACTG | Identical |
| | | SEQ ID NO:237 | | SEQ ID NO:238 | |
| miR-186 | miR-186GSP9 | CATGATCAGCTGGGCCAAGAAAGCCCAAA | miR-186RP3 | CA+AA+GAATT+CTCCTTTTGG | Identical |
| | | SEQ ID NO:239 | | SEQ ID NO:240 | |
| miR-187 | miR-187GSP | CATGATCAGCTGGGCCAAGACGGCTGCAAC | miR-187RP | T+CG+TGTCTTGTGTT | Identical overlapping sequence, ends differ |
| | | SEQ ID NO:243 | | SEQ ID NO:242 | |
| miR-188 | miR-108GSP | CATGATCAGCTGGGCCAAGAACCCTCCACC | miR-188RP | C+AT+CCCTTGCATGG | Identical |
| | | SEQ ID NO:243 | | SEQ ID NO:244 | |
| miR-189 | miR-189GSP2 | CATGATCAGCTGGGCCAAGAACTGATATC | miR-189RP | G+TG+CCTACTGAGCT | Identical |
| | | SEQ ID NO:245 | | SEQ ID NO:246 | |
| miR-190 | miR-290GSP9 | CATGATCAGCTGGGCCAAGAACCTAATAT | miR-190RP4 | T+GA+TA+TGTTTGATATATTAG | Identical |
| | | SEQ ID NQ:247 | | SEQ ID NO:248 | |
| miR-191 | miR-191GSP2 | CATGATCAGCTGGGCCAAGAAGCTGCTT | miR-191RP2 | C+AA+CGGAATCCCAAAAG | Identical |
| | | SEQ ID NO:249 | | SEQ ID NO:250 | |
| miR-192 | miR-192GSP2 | CATGATCAGCTGGGCCAAGAGGCTGTCAA | miR-192RP | C+TGA+CCTATGAATTGAC | Identical overlapping sequence, ends differ |
| | | SEQ ID NO:251 | | SEQ ID NO:252 | |
| miR-193 | miR-193GSP9 | CATGATCAGCTGGGCCAAGACTGGGACTT | miR-193RP2 | AA+CT+GGCCTACAAAG | Identical |
| | | SEQ ID NO:253 | | SEQ ID NO:254 | |
| miR-194 | mir194GSP8 | CATGATCAGCTGGGCCAAGATCCACATG | mir194RP | TG+TAA+CAGCAACTCCA | Identical |
| | | SEQ ID NO:255 | | SEQ ID NO:256 | |
| miR-195 | miR-195GSP9 | CATGATCAGCTGGGCCAAGAGCCAATATT | miR-195RP3 | T+AG+CAG+CACAGAAATA | Identical |
| | | SEQ ID NO:257 | | SEQ ID NO:258 | |
| miR-196a | miR-196aGSP | CATGATCAGCTGGGCCAAGACCAACAACAT | miR-196aRP | TA+GG+TAGTTTCAFGTTG | Identical |
| | | SEQ ID NO:261 | | SEQ ID NO:262 | |
| miR-196b | miR-196bGSP | CATGATCAGCTGGGCCAAGACCAACAACAG | miR-196bRP | TA+GGT+AGTTTCCTGT | Identical |
| | | SEQ ID NO:259 | | SEQ ID NO:260 | |
| miR-199a* | miR-199a*GSP2 | CATGATCAGCTGGGCCAAGAAACCAATGT | miR-199a*RP | T+AC+AGTAGTCTGCAC | Identical |
| | | SEQ ID NO:267 | | SEQ ID NO:268 | |
| miR-199a | miR-199aGSP2 | CATGATCAGCTGGGCCAAGAGAACAGGTA | miR-199aRP | C+CC+AGTGTTCAGAC | Identical |
| | | SEQ ID NO:269 | | SEQ ID NO:270 | |
| miR-199b | miR-299bGSP | CATGATCAGCTGGGCCAAGAGAACAGGTAG | miR-199bRP | C+CC+AGTGTTTAGAC | one or more base pairs differ |
| | | SEQ ID NO: 475 | | SEQ ID NO: 272 | |
| miR-200a | miR-200aGSP2 | CATGATCAGCTGGGCCAAGAACATCGTTA | miR-200aRP | TAA+CAC+TGTCTGGT | Identical |
| | | SEQ ID NO:273 | | SEQ ID NO:274 | |
| miR-200b | miR-200bGSP2 | CATGATCAGCTGGGCCAAGAGTCATCATT | miR-200bRP | TAATA+CTG+CCTGGTAAT | Identical |
| | | SEQ ID NO:275 | | SEQ ID NO:276 | |
| miR-203 | miR-203GSP2 | CATGATCAGCTGGGCCAAGACTAGTGGTC | miR-203RP | G+TG+AAATGTTTAGGACC | Identical overlapping sequence, ends differ |
| | | SEQ ID NO:279 | | SEQ ID NO:280 | |
| miR-204 | miR-204GSP2 | CATGATCAGCTGGGCCAAGAAGGCATAGG | miR-204RP | T+TC+CCTTTGTCATCC | Identical overlapping sequence, ends differ |
| | | SEQ ID NO:281 | | SEQ ID NO:282 | |
| miR-205 | miR-205GSP | CATCGATCAGCTGGGCAA GACAGACTCCGG | miR-205RP | T+CCTT+CATTCCACC | Identical |
| | | SEQ ID NO:283 | | SEQ ID NO:284 | |
| miR-206 | miR-206GSP7 | CATGATCAGCTGGGCCAAGACCACACA | miR-206RP | T+G+GAA+TGTAAGGAAGIGI | Identical |
| | | SEQ ID NO:285 | | SEQ ID NO:286 | |
| **miR-208** | miR-208_GSP13 | CATGATCAGCTGGGCCAAGAACAAGCTTTTTGC | miR-208_RP4 | ATAA+GA+CG+AGCAAAAAG | Identical |
| | | SEQ ID No:287 | | SEQ ID NO:288 | |
| **miR-210** | miR-210GSP | CATGATCAGCTGGGCCAAGATCAGCCGCTG | miR-210RP | C+TG+TGCGTGTGACA | Identical |
| | | SEQ ID NO:289 | | SEQ ID NO:290 | |
| **MiR-211** | miR-211GSP2 | CATGATCAGCTGGGCCAAGAAGGCAAAGG | miR-211RP | T+TC+CCTTTGTCATCC | one or more base pairs differ |
| | | SEQ ID NO: 491 | | SEQ ID NO: 292 | |
| **miR-212** | miR-212GSP9 | CATGATCAGCTGGGCCAAGAGGCCGTGAC | miR-212RP2 | T+AA+CAGTCTCCAGTCA | Identical |
| | | SEQ ID NO:293 | | SEQ ID NO:294 | |
| **miR-213** | miR-213GSP | CATGATCAGCTGGGCCAAGPGGTACTATCA | miR-213RP | A+CC+ATCGACCGTTG | Identical |
| | | SEQ ID NO:295 | | SEQ ID NO:296 | |
| **miR-214** | miR-214GSP | CATGATCAGCTCTGGCCAAGACTGCCTGTCT | miR-214RP | A+CA+GCAGGCACAGA | Identical |
| | | SEQ ID NO:297 | | SEQ ID NO:298 | |
| **miR-215** | miR-215GSP2 | CATGATCAGCTGGGCCAAGAGTCTGTCAA | miR-215RP | A+TGA+CCTATGATTTGAC | one or more base pairs differ |
| | | SEQ ID NO: 299 | | SEQ ID NO: 469 | |
| **miR-216** | miR-216GSP9 | CATGATCAGCTGGGCCAAGACACAGTTGC | mir216RP | TAA+TCT+CAGCTGGCA | Identical |
| | | SEQ ID NO:301 | | SEQ ID NO:302 | |
| **miR-217** | miR-217GSP2 | CATGATCAGCTGGGCCAAGAATCCAGTCA | miR-217RP2 | T+AC+TGCATCAGGAACTGA | one or more base pairs differ |
| | | SEQ ID NO:481 | | SEQ ID NO: 304 | |
| **miR-218** | miR-218GSP2 | CATGATCAGCTGGGCCAAGAACATGGTTA | miR-218RP | TTG+TGCTT+GATCTAAC | Identical |
| | | SEQ ID NO:305 | | SEQ ID NO:306 | |
| **miR-221** | miR-221GSP9 | CATGATCAGCTGGGCCAAGAGAAACCCAG | miR-221RP | A+GC+TACATTGTCTGC | Identical overlapping sequence, ends differ |
| | | SEQ ID NO:309 | | SEQ ID NO:310 | |
| **miR-222** | miR-222GSP8 | CATGATCAGCTGGGCCAAGAGAGACCCA | miR-222RP | A+GC+TACATCTGGCT | Identical |
| | | SEQ ID NO:311 | | SEQ ID NO:312 | |
| **miR-223** | miR-223GSP | CATGATCAGCTGGGCCAAGAGGGGTATTTG | miR-223RP | TG+TC+AGTTTGTCAAA | Identical |
| | | SEQ ID NO:313 | | SEQ ID NO:314 | |
| **miR-224** | miR-224GSP8 | CATGATCAGCTGGGCCAAGATAAACGGA | miR-224RP2 | C+AAG+TCACTAGTGGTT | Identical overlapping sequence, ends differ |
| | | SEQ ID NO:315 | | SEQ ID NO:316 | |
| **miR-296** | miR-296GSP9 | CATGATCAGCTGGGCCAAGAACAGGATTG | miR-296RP2 | A+GG+GCCCCCCCTCAA | Identical |
| | | SEQ ID NO:317 | | SEQ ID NO:318 | |
| **miR-299** | miR-299GSP9 | CATGATCAGCTGGGCCAAGAATGTATGTG | miR-299RP | T+GG+TTTACCGTCCC | Identical |
| | | SEQ ID NO:319 | | SEQ ID NO:320 | |
| **miR-301** | miR-301GSP | CATGATCAGCTGGGCCAAGAGCTTTGACAA | miR-301RP | C+AG+TGCAATAGTATTGT | Identical |
| | | SEQ TO NO:321 | | SEQ ID NO:322 | |
| **miR-302a** | miR-302aGSP | CATGATCAGCTGGGCCAAGATCACCAAAAC | miR-302aRP | T+AAG+TGCTTCCATGT | Identical |
| | | SEQ ID NO:325 | | SEQ ID NO:326 | |
| **miR-320** | miR-320_GSP8 | CATGATCAGCTGGGCCAAGATTCGCCCT | miR-320_RP3 | AAAA+GCT+GGGTTGAGAGG | Identical |
| | | SEQ ID NO:337 | | SEQ ID NO:338 | |
| **miR-323** | miR-323GSP | CATGATCAGCTGGGCCAAGAAGAGGTCGAC | miR-323RP | G+CA+CATTACACGGT | Identical |
| | | SEQ ID NO:339 | | SEQ ID NO:340 | |
| **miR-324-3p** | miR-324-3pGSP | CATGATCAGCTGGGCCAAGACCAGCAGCAC | miR-324-3pRP | C+CA+CTGCCCCAGGT | Identical |
| | | SEQ ID NO:341 | | SEQ ID NO:342 | |
| **miR-324-5p** | miR-3Z4-5pGSP | CATGATCAGCTGGGCCAAGAACACCAATGC | miR-329-5pRP | C+GC+ATCCCCTAGGG | Identical overlapping sequence, ends differ |
| | | SEQ ID NO:343 | | SEQ ID NO:344 | |
| **miR-325** | miR-325GSP | CATGATCAGCTGGGCCAAGAACACTTACTG | miR-325RP | C+CT+AGTAGGTGCTC | one or more base pairs differ |
| | | SEQ ID NO: 345 | | SEQ ID NO: 476 | |
| **miR-326** | miR-326GSP | CATGATCAGCTGGGCCAAGACTGGAGGAAG | miR-326RP | C+CT+CTGGGCCCTTC | Identical overlapping sequence, ends differ |
| | | SEQ ID NO:347 | | SEQ ID NO:348 | |
| **miR-328** | miR-328GSP | CATGATCAGCTGGGCCAAGAACGGAAGGGC | miR-328RP | C+TG+GCCCTCTCTGC | Identical |
| | | SEQ ID NO:349 | | SEQ ID NO:350 | |
| **miR-330** | miR-330GSP | CATGATCAGCTGGGCCAAGATCTCTGCAGG | miR-330RP | G+CA+AAGCACAGGGC | one or more base pairs differ |
| | | SEQ ID NO: 351 | | SEQ ID NO: 478 | |
| **miR-331** | miR-331GSP | CATGATCAGCTGGGCCAAGATTCTAGGATA | miR-331RP | G+CC+CCTGGGCCTAT | Identical |
| | | SEQ ID NO:353 | | SEQ ID NO:354 | |
| **miR-337** | miR-337GSP | CATGATCAGCTGGGCCAAGAAAAGGCATCA | miR-337RP | T+TC+AGCTCCTATATG | one or more base pairs differ |
| | | SEQ ID NO:355 | | SEQ ID NO: 490 | |
| **miR-338** | miR-338GSP | CATGATCAGCTGGGCCAAGATCAACAAAAT | miR-338RP2 | T+CC+AGCATCAGTGATTT | Identical |
| | | SEQ ID NO:357 | | SEQ ID NO:358 | |
| **miR-339** | miR-339GSP9 | CATGATCAGCTGGGCCAAGATGAGCTCCT | miR-339RP2 | T+CC+CTGTCCTCCAGG | Identical |
| | | SEQ ID NO:359 | | SEQ ID NO:360 | |
| **miR-340** | miR-340GSP | CATGATCAGCTGGGCCAAGAGGCTATAAAG | miR-340RP | TC+CG+TCTCAGTTAC | Identical |
| | | SEQ ID NO:361 | | SEQ ID NO:362 | |
| **miR-342** | miR-342GSP3 | CATGATCAGCTGGGCCAAGAGACGGGTG | miR-342RP | T+CT+CACACAGAAATCG | Identical |
| | | SEQ ID NO:363 | | SEQ ID NO:364 | |
| **miR-345** | miR-345GSP | CATGATCAGCTGGGCCAAGAGCACTGGACT | miR-345RP | T+GC+TGACCCCTAGT | one or more base pairs differ |
| | | SEQ ID NO: 484 | | SEQ ID NO: 485 | |
| **miR-346** | miR-346GSP | CATGATCAGCTCGGGCCAAGAAGAGGCAGGC | miR-346RP | T+GT+CTGCCCGAGTG | one or more base pairs differ |
| | | SEQ ID NO: 367 | | SEQ ID NO: 488 | |
| **miR-363** | miR-363 GSP10 | CATGATCAGCTGGGCCAAGATACAGATGGA | miR-363RP | AAT+TG+CAC+GGTATCC | Identical |
| | | SEQ ID NO:369 | | SEQ ID NO:370 | |
| **miR-370** | miR-370GSP | CATGATCAGCTGGGCCAAGACCAGGTTCCA | miR-370RP | G+CC+TGCTGGGGTGG | Identical overlapping sequence, ends differ |
| | | SEQ ID NO:375 | | SEQ ID NO:376 | |
| **miR-375** | miR-375GSP | CATGATCAGCTGGGCCAAGATCACGCGAGC | miR-375RP | TT+TG+TTCGTTCGGC | Identical |
| | | SEQ ID NO:387 | | SEQ ID NO:388 | |
| **miR-376a** | miR-376aGSP3 | CATGATCAGCTGGGCCAAGAACGTGGAT | miR-376aRP2 | A+TCGTAGA+GGAAAATCCAC | one or more base pairs differ |
| | | SEQ ID NO: 467 | | SEQ ID NO:468 | |
| **miR-378** | miR-378GSP | CATGATCAGCTGGGCCAAGAACACAGGACC | miR-378RP | C+TC+CTGACTCCAGG | Identical |
| | | SEQ ID NO:391 | | SEQ ID NO:392 | |
| **miR-379** | miR-379_GSP7 | CATGATCAGCTGGGCCAAGATACGCGTTC | miR-379RP2 | T+GGT+AGACTATGGAACG | Identical overlapping sequence, ends differ |
| | | SEQ ID NO:393 | | SEQ ID NO:394 | |
| **miR-380-5p** | miR-380-5pGSP | CATGATCAGCTGGGCCAAGAGCGCATGTTC | miR-380-5pRP | T+GGT+TGACCATAGA | Identical |
| | | SEQ ID NO:395 | | SEQ ID NO:396 | |
| miR-380-3p | miR-380-3pGSP | CATGATCAGCTGGGCCAAGAAAGATGTGGA | miR-380-3pRP | TA+TG+TAGTATGGTCCACA | one or more base pairs differ |
| | | SEQ ID NO: 395 | | SEQ ID NO: 483 | |
| **miR-381** | miR-381GSP2 | CATGATCAGCTGGGCCAAGAACAGAGAGC | miR-381RP2 | TATA+CAA+GGGCAAGCT | Identical |
| | | SEQ ID NO:399 | | SEQ ID NO:400 | |
| **miR-382** | miR-382GSP | CATGATCAGCTGGGCCAAGACGAATCCACC | miR-382RP | G+AA+GTTGTTCGTGGT | Identical |
| | | SEQ ID NO:401 | | SEQ ID NO:402 | |
| **miR-383** | miR-383GSP | CATGATCAGCTGGGCCAAGAAGCCACAGTC | miR-383RP2 | A+GATC+AGAAGGTGACTGT | one or more base pairs differ |
| | | SEQ ID NO:465 | | SEQ ID NO: 466 | |
| **miR-384** | miR-384_GSP9 | CATGATCAGCTGGGCCAAGATGTGAACAA | miR-384_RP5 | ATT+CCT+AG+AAATTGTTC | one or more base pairs differ |
| | | SEQ ID NO:470 | | SEQ ID NO: 471 | |
| **miR-410** | miR-410 GSP9 | CATGATCAGCTGGGCCAAGAACAGGCCAT | miR-410RP | AA+TA+TAA+CA+CAGATGGC | Identical |
| | | SEQ ID NO:405 | | SEQ ID NO:406 | |
| **miR-412** | miR-412 GSP10 | CATGATCAGCTGGGCCAAGAACGGCTAGTG | miR-412RP | A+CTT+CACCTGGTCCACTA | Identical |
| | | SEQ ID NO:407 | | SEQ ID NO:408 | |
| **miR-424** | miR-424GSP | CATGATCAGCTGGGCCAAGATCCAAAACAT | miR-424RP2 | C+AG+CAGCAATTCATGTTTT | one or more base pairs differ |
| | | SEQ ID NO:474 | | SEQ ID NO: 414 | |
| **miR-425** | miR-425GSP | CATGATCAGCTGGGCCAAGAGGCGGACACG | miR-425RP | A+TC+GGGAATGTCGT | Identical |
| | | SEQ ID NO:417 | | SEQ ID NO:418 | |
| **miR-429** | miR-429_GSP11 | CATGATCAGCTGGGCCAAGAACGGCATTACC | miR-429RP5 | T+AATAC+TG+TCTGGTAATG | one or more base pairs differ |
| | | SEQ ID NO:479 | | SEQ ID NO: 480 | |
| **miR-431** | miR-431 GSP10 | CATGATCAGCTGGGCCAAGATGCATGACGG | miR-431RP | T+GT+CTTGCAGGCCG | Identical overlapping sequence, ends differ |
| | | SEQ ID NO:421 | | SEQ ID NO:422 | |
| **miR-448** | miR-448GSP | CATGATCAGCTGGGCCAAGAATGGGACATC | miR-448RP | TTG+CATA+TGTAGGATG | Identical |
| | | SEQ ID NO:423 | | SEQ ID NO:424 | |
| **miR-449** | miR-449GSP10 | CATGATCAGCTGGGCCAAGAACCAGCTAAC | miR-449RP2 | T+GG+CAGTGTATTGTTAGC | Identical |
| | | SEQ ID NO:425 | | SEQ ID NO:426 | |
| **miR-450** | miR-450GSP | CATGATCAGCTGGGCCAAGATATTAGGAAC | miR-450RP | TTTT+TG+CGATGTGTT | Identical |
| | | SEQ ID NO:427 | | SEQ ID NO:428 | |
| **miR-451** | miR-451 GSP10 | CATGATCAGCTGGGCCAAGAAAACTCAGTA | miR-451RP | AAA+CCG+TTA+CCATTACTGA | Identical overlapping sequence, ends differ |
| | | SEQ ID NO:429 | | SEQ ID NO:430 | |
| **let 7a** | let 7a-GSP2 | CATGATCAGCTGGGCCAAGAAACTATAC | let 7a-RP | T+GA+GGTAGTAGGTTG | Identical overlapping sequence, ends differ |
| | | SEQ ID NO:431 | | SEQ ID NO:432 | |
| **let 7b** | let 7b-GSP2 | CATGATCAGCTGGGCCAAGAAACCACAC | let 7b-RP | T+GA+GGTAGTAGGTTG | Identical |
| | | SEQ ID NO:433 | | SEQ ID NO:432 | |
| **let 7c** | Let 7c-GSP2 | CATGATCAGCTGGGCCAAGAAACCATAC | let 7c-RP | T+GA+GGTAGTAGGTTG | Identical |
| | | SEQ ID NO:434 | | SEQ ID NO:432 | |
| **let 7d** | let 7d-GSP2 | CATGATCAGCTGGGCCAAGAACTATGCA | let 7d-RP | A+GA+GGTAGTAGGTTG | Identical |
| | | SEQ ID NO:435 | | SEQ ID NO:436 | |
| **let 7e** | let 7e-GSP2 | CATGATCAGCTGGGCCAAGAACTATACA | let 7e-RP | T+GA+GGTAGGAGGTTG | Identical |
| | | SEQ ID NO:437 | | SEQ ID NO:438 | |
| **let 7f** | let 7f-GSP2 | CATGATCAGCTGGGCCAAGAAACTATAC | let 7f-RP | T+GA+GGTAGTAGATTG | Identical overlapping sequence, ends differ |
| | | SEQ ID NO:439 | | SEQ ID NO:440 | |
| **let 7g** | let 7g-GSP2 | CATGATCAGCTGGGCCAAGAACTGTACA | let 7g-RP | T+GA+GGTAGTAGTTTG | Identical |
| | | SEQ ID NO:441 | | SEQ ID NO:442 | |
| **let 7i** | let 7i-GSP2 | CATGATCAGCTGGGCCAAGAACAGCACA | let 7i-RP | T+GA+GGTAGTAGTTTG | Identical |
| | | SEQ ID NO:443 | | SEQ ID NO:444 | |

### Example 5

This Example describes the detection and analysis of expression profiles for three microRNAs in total RNA isolated from twelve different tissues using methods in accordance with an embodiment of the present invention.

Methods: Quantitative analysis of miR-1, miR-124 and miR-150 microRNA templates was determined using 0.5 µg of First Choice total RNA (Ambion, Inc.) per 10 µl primer extension reaction isolated from the following tissues: brain, heart, intestine, kidney, liver, lung, lymph, ovary, skeletal muscle, spleen, thymus and uterus. The primer extension enzyme and quantitative PCR reactions were carried out as described above in EXAMPLE 3, using the following PCR primers:
miR-1 Template:
   extension primer: CATGATCAGCTGGGCCAAGATACATACTTC (SEQ ID NO:47)
   reverse primer: T+G+GAA+TG+TAAAGAAGT (SEQ ID NO:48)
   forward primer: CATGATCAGCTGGGCCAAGA (SEQ ID NO:13)
miR-124 Template:
   extension primer: CATGATCAGCTGGGCCAAGATGGCATTCAC (SEQ ID NO:149)
   reverse primer: T+TA+AGGCACGCGGT (SEQ ID NO:150)
   forward primer: CATGATCAGCTGGGCCAAGA (SEQ ID NO:13)
miR-150 template:
   extension primer: CATGATCAGCTGGGCCAAGACACTGGTA (SEQ ID NO:213)
   reverse primer: T+CT+CCCAACCCTTG (SEQ ID NO:214)
   forward primer: CATGATCAGCTGGGCCAAGA (SEQ ID NO:13)

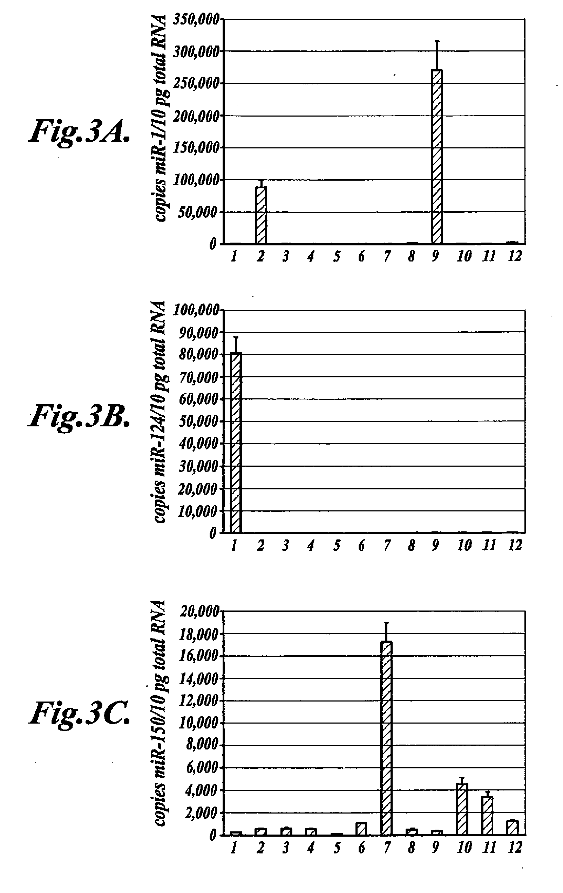

Results. The expression profiles for miR-1, miR-124 and miR-150 are shown in FIGURE 3A, 3B, and 3C, respectively. The data in FIGURES 3A-3C are presented in units of microRNA copies per 10 pg of total RNA (y-axis). These units were chosen since human cell lines typically yield ≤ 10 pg of total RNA per cell. Hence the data shown are estimates of microRNA copies per cell. The numbers on the x-axis correspond to the following tissues: (1) brain, (2) heart, (3) intestine, (4) kidney, (5) liver, (6) lung, (7) lymph, (8) ovary, (9) skeletal muscle, (10) spleen, (11) thymus and (12) uterus.

Consistent with previous reports, very high levels of striated muscle-specific expression were found for miR-1 (as shown in FIGURE 3A), and high levels of brain expression were found for miR-124 (as shown in FIGURE 3B) (see Lagos-Quintana et al., RNA 9:175-179, 2003). Quantitative analysis reveals that these microRNAs are present at tens to hundreds of thousands of copies per cell. These data are in agreement with quantitative Northern blot estimates of miR-1 and miR-124 levels (see Lim et al., Nature 433:769-773, 2005). As shown in FIGURE 3C, miR-150 was found to be highly expressed in the immune-related lymph node, thymus and spleen samples which is also consistent with previous findings (see Baskerville et al., RNA 11:241-247, 2005).

### Example 6

This Example describes the selection and validation of primers for detecting mammalian microRNAs of interest.

Rationale: In order to perform multiple assays to detect a plurality of microRNA targets in a single sample (i.e., multiplex PCR), it is important that the assays work under uniform reverse transcriptase and PCR cycling conditions in a common buffer system with a single universal primer. The following primer design principles and high throughput assays were utilized to identify useful primer sets for desired microRNA targets that work well under the designated reaction conditions.

### Primer Design:

As described in Example 2, the sensitivity of an assay to detect mammalian microRNA targets using the methods of the invention may be measured by the cycle threshold (Ct) value. The lower the Ct value (e.g., the fewer number of cycles), the more sensitive is the assay. The Δ Ct value is the difference between the number of cycles (Ct) between template containing samples and no template controls, and serves as a measure of the dynamic range of the assay. Assays with a high dynamic range allow measurements of very low microRNA copy numbers. Accordingly, desirable characteristics of a microRNA detection assay include high sensitivity (low Ct value) (preferably in the range of from about 5 to about 25, such as from about 10 to about 20), and broad dynamic range (preferably in the range of from about 10 and 35, such as Δ Ct ≥ 12) between the signal of a sample containing target template and a no template background control sample.

*microRNA Target Templates:* Representative mammalian microRNA target templates (h= human, r= rat, m= mouse) are provided in Table 9 (SEQ ID NO:966 to SEQ ID NO:1043) which are publically available and accessible on the World Wide Web at the Wellcome Trust Sanger Institute website in the "miRBase sequence database" as described in Griffith-Jones et al. (2004), Nucleic Acids Research 32:D109-D111 and Griffith-Jones et al. (2006), Nucleic Acids Research 34:D140-D144.

### Extension Primers:

Empirical data generated as described in Examples 1-5 suggests that gene specific (GS) extension primers are primarily responsible for the dynamic range of the assays for detecting mammalian microRNA targets using the methods described herein. As described in Example 2, it was determined that the dynamic range (Δ Ct) and specificity of the assays tested decreased for extension primers having gene specific regions below 6 to 7 nucleotides. Therefore, in order to optimize microRNA detection assays, extension primers were designed that have 7 to 10 nucleotide overlap with the microRNA target of interest. Exemplary extension primers for the microRNA targets listed in TABLE 9 are provided in TABLE 8 (SEQ ID NO:500 to SEQ ID NO:965). These exemplary extension primers have a gene specific (GS) region from 7 to 10 nucleotide overlap with the microRNA target of interest.

### Reverse Primers:

Unmodified and locked nucleic acid (LNA)-containing reverse primers were designed to quantify the primer-extended, full length cDNA in combination with a generic universal forward primer (SEQ ID NO:13). Based on the data generated as described in Examples 1-5, it was determined that the design of the reverse primers contributes to the efficiency of the PCR reactions, with the observation that the longer the reverse primer, the better the PCR performance. However, it was also observed that the longer the overlap with the extension primer, the higher the background. Therefore, the reverse primers were designed to be as long as possible while minimizing the overlap with the gene specific portion of the extension primer, in order to reduce the non-specific background signal.

In addition, as described in Example 3, LNA base substitutions may be selected to raise the predicted Tm of the primer, with two or three LNA base substitutions typically substituted within the first 8 nucleotides from the 5' end of the reverse primer oligonucleotide. Exemplary reverse primers for the microRNA targets listed in TABLE 9 are provided in TABLE 8. While these exemplary reverse primers contain LNA base substitutions (the "+" symbol preceding a nucleotide designates an LNA substitution), this feature is optional and not required.

### Selection and validation of primers for a desired target:

### Assay oligonucleotide selection is made in two steps as follows:

1) Primer designs were determined using the principles described above. Typically, 4 extension primer candidates and 2 reverse primer candidates were designed for each microRNA target of interest. The extension primers in each set overlap the gene specific region by 7, 8, 9 and 10 nucleotides, respectively, at the 3' end. Exemplary primers designed according to these design principles are provided in TABLE 8 for the microRNA targets listed in TABLE 9.
*Assay design to validate the candidate primer sets (Assay #1)*
*microRNA Target:*
Exemplary target microRNA miR-495 has an RNA target sequence (SEQ ID NO:966) that is conserved across human (h), mouse (m) and rat (r), as indicated by the designation "hmr"-miR-495 in TABLE 9. Therefore, the primer designed for this target sequence would be expected to be useful to detect miR-495 in samples obtained from human, mouse, and rat.
microRNA miR-495 target RNA sequence: 5'
AAACAAACAUGGUGCACUUCUU 3' (SEQ ID NO:966)
*Extension Primers (4 candidates)*
hmr-miR-495GS10: 5' CATGATCAGCTGGGCCAAGAAAGAAGTGCA 3' (SEQ ID NO:500)
hmr.miR-495GS9: 5' CATGATCAGCTGGGCCAAGAAAGAAGTGC 3' (SEQ ID NO:501)
hmr-miR-495GS8: 5' CATGATCAGCTGGGCCAAGAAAGAAGTG 3' (SEQ ID NO:502)
hmr-miR-495GS7: 5' CATGATCAGCTGGGCCAAGAAAGAAGT (SEQ ID NO:503)
*Reverse Primers (2 candidates)*
hmr-miR-495RP1: 5'AAA+CAAA+CA+TGGTGCAC3' (SEQ ID NO:504)
hmr-miR-495RP2: 5' AAA+C+AAA+CATGGTGC 3' (SEQ ID NO:505)
2) The primers designed as described above were tested to find pairs that showed both high sensitivity and high dynamic range in quantitative PCR assays, using the assay methods described in Example 2. The optimal combination of extension primer and reverse primer was determined for the target microRNA by testing all combinations of primers in the presence or absence of DNA template. It is preferable to use DNA rather than RNA template to test the oligo pairs because it is less likely to degrade than RNA. Degraded templates result in misleading assay data. Therefore, HPLC purified DNA template molecules are preferred.
TABLE 8 shows exemplary primer sets for use in detection assays for 78 microRNA targets (shown in TABLE 9). The candidate primers for use in these assays were designed to specifically detect human (h), mouse (m) and rat (r) microRNAs, or microRNAs from one or more species. For example, assays with the "hmr" prefix are designed to detect a perfectly conserved microRNA in all three species, whereas a "mr" prefix means the assay is designed to detect a microRNA conserved between mouse and rat, but not human. Nucleotides preceded by a plus (+) sign may be optionally locked (LNA). TABLE 9 shows the microRNA target sequence for each assay.

**TABLE 8: EXEMPLARY PRIMER SETS FOR DETECTING MAMMALIAN MICRORNA TARGETS**

| **Assay Number** | **Target microRNA** | **Extension Primer Name** | **Extension Primer Sequence** | **Reverse Primer Name** | **Reverse Primer Sequence** | **Comments** |
|---|---|---|---|---|---|---|
| 1 | hmr-miR-495 | Hmr-miR- 495GS10 | CATGATCAGCTGGGCCAAGAAAGAAGTGCA | Hmr-miR- 495RP1 | AAA+CAAA+CA+TGGTGCAC | Conserved across all three species |
| | | | SEQ ID NO: 500 | | SEQ ID NO: 504 | |
| | | Hmr-miR- 495GS9 | CATGATCAGCTGGGCCAAGAAGAAGTGC | Hmr-miR- 495RP2 | AAA+C+AAA+CATGGTGC | |
| | | | SEQ ID NO:501 | | SEQ ID NO:505 | |
| | | Hmr-miR- 495GS8 | CATGATCAGCTGGGCCAAGAAAGAAGTG | | | |
| | | | SEQ ID NO: 502 | | | |
| | | Hmr-miR- 495GS7 | CATGATCAGCTGGGCCAAGAAAGAAGT | | | |
| | | | SEQ ID NO: 503 | | | |
| 2 | mr-miR-291a-3p | mr-mIR- 291a- 3pGS10 | CATGATCAGCTGGGCCAAGAGGCACACAAA | mr-mIR-291a- 3pRF1 | AA+AG+TGCTTCCACTTTGT | Mouse/rat specific; seed region ortholog to human miR-371/2 |
| | | | SEQ ID NO: 506 | | SEQ ID NO:510 | |
| | | mr-mIR- 291a-3pGS9 | CATGATCAGCTGGGCCAAGAGGCACACAA | mr-mIR-291a- 3pRP2 | AA+AG+TG+CTTCCACTTT | |
| | | | SEQ ID NO:507 | | SEQ ID NO:511 | |
| | | mr-mIR- 291a-3pGS8 | CATGATCAGCTGGGCCAAGAGGCACACA | | | |
| | | | SEQ ID NO:508 | | | |
| | | mr-mIR- 291a-3pGS7 | CATGATCAGCTGGGCCAAGAGGCACAC | | | |
| | | | SEQ ID NO: 509 | | | |
| 3 | m-miR-291b- 3p | m-mIR- 291b- 3pGS10 | CATGATCAGCTGGGCCAAGAGACAAACAAA | m mIR-291b- 3pRF1 | AA+AG+TG+CAT+CCATTTTGT | Mouse specific; seed region ortholog to human miR-371/2 |
| | | | SEC ID NO:512 | | SEQ ID NO:516 | |
| | | m-mIR- 291b-3pGS9 | CATGATCAGCTGGGCCAAGAGACAAACAA | m-mIR-291b- 3pRP2 | AA+AG+TG+CATCCATTTT | |
| | | | SEQ ID NO:513 | | SEQ ID NO:517 | |
| | | m-mIR- 291b-3pGS8 | CATGATCAGCTGGGCCAAGAGACAAACA | | | |
| | | | SEQ ID NO:514 | | | |
| | | m-mIR-291b-3pGS7 | CATGATCAGCTGGGCCAAGAGACAAAC | | | |
| | | | SEQ ID NO:515 | | | |
| 4 | h-miR-519a | h-miR-519aGS10 | CATGATCAGCTGGGCCAAGAGTAACACTCT | h-miR-519aRP1 | AA+AG+TG+CATCCTTTTAGAGT | Human specific; implicated in oncogenesis |
| | | | SEQ ID NO:518 | | SEQ ID NO:522 | |
| | | h-miR-519aGS9 | CATGATCAGCTGGGCCAAGAGTAACACTC | h-miR-519aRP2 | AA+AG+TG+CATCCTTTTAGA | |
| | | | SEQ ID NO:519 | | SEQ ID NO:523 | |
| | | h-miR-519aGS8 | CATGATCAGCTGGGCCAAGAGTAACACT | | | |
| | | | SEQ ID NO:520 | | | |
| | | h-miR-519aGS7 | CATGATCAGCTGGGCCAAGAGTAACAC | | | |
| | | | SEQ ID NO:521 | | | |
| 5 | h-miR-519b | h-miR-519bGS 10 | CATGATCAGCTGGGCCAAGAAAACCTCTAA | h-miR-519bRP1 | AA+AG+TG+CATCCTTTTAG | Human specific; implicated in oncogenesis |
| | | | SEQ ID NO:524 | | SEQ ID NO:528 | |
| | | h-miR-519bGS9 | CATGATCAGCTGGGCCAAGAAAACCTCTA | h-miR-519bRP2 | AA+AG+TG+CATCCTTTT | |
| | | | SEQ ID NO:525 | | SEQ ID NO:529 | |
| | | h-miR-519hGS8 | CATGATCAGCTGGGCCAAGAAAACCTCT | | | |
| | | | SEQ ID NO:526 | | | |
| | | h-miR-519bGS7 | CATGATCAGCTGGGCCAAGAAAACCTC | | | |
| | | | SEQ ID NO: 527 | | | |
| 6 | h-miR-519e | h-miR-519cGS10 | CATGATCAGCTGGGCCAAGAATCCTCTAAA | h miR-514eRP1 | AA+AG+TG+CATCTTTTAGA | Human specific; implicated in oncogenesis |
| | | | SEQ ID NO: 530 | | SEQ ID NO:534 | |
| | | h-miR-519cGS9 | CATGATCAGCTGGGCCAAGAATCCTCTAA | h-miR-519cRP2 | AA+AG+TG+CATCTTTTTA | |
| | | | SEQ ID NO: 531 | | SEQ ID NO:535 | |
| | | h-miR-519cGS8 | CATGATCAGCTGGGCCAAGAATCCTCTA | | | |
| | | | SEQ ID NO:532 | | | |
| | | h-mir-519cGS7 | CATGATCAGCTGGGCCAAGAATCCTCT | | | |
| | | | SEQ ID NO:533 | | | |
| 7 | h-miR-519d | h-miR-519dGS 10 | CATGATCAGCTGGGCCAAGAACACTCTAAA | h-miR-519dRP1 | C+AAAG+TGCCTTTAG | Human specific; implicated in oncogenesis |
| | | | SEQ ID NO:536 | | SEQ ID NO:540 | |
| | | h-miR-519dGS9 | CATGATCAGCTGGGCCAAGAACACTCTAA | h-miR-519dRP2 | C+AA+AG+TGCCTCCCTTT | |
| | | | SEQ ID NO:537 | | SEQ ID NO:541 | |
| | | h-miR-519dGS8 | CATGATCAGCTGGGCCAAGAACACTCTA | | | |
| | | | SEQ ID NO:538 | | | |
| | | h-miR-519dGS7 | CATGATCAGCTGGGCCAAGAACACTCT | | | |
| | | | SEQ ID NO:539 | | | |
| 8 | h-miR-520a | h-miR-520aGS 10 | CATGATCAGCTGGGCCAAGAACAGTCCAAA | h-miR-520aRP1 | AA+AG+TGCTTCCCTTTGG | Human specific; implicated in oncogenesis |
| | | | SEQ ID NO:542 | | SEQ ID NO:546 | |
| | | h-miR-520aGS9 | CATGATCAGCTGGGCCAAGAACAGTCCAA | h-miR-520aRP2 | AA+AG+T+GCTTCCCTTT | |
| | | | SEQ ID NO:543 | | SEQ ID NO:547 | |
| | | h-miR-520aGS8 | CATGATCAGCTGGGCCAAGAACAGTCCA | | | |
| | | | SEQ ID NO:544 | | | |
| | | h-miR-520aGS7 | CATGATCAGCTGGGCCAAGAACAGTCC | | | |
| | | | SEQ ID NO:545 | | | |
| 9 | h-miR-520b | h-miR-520bGS10 | CATGATCAGCTGGGCCAAGACCCTAAAA | h-miR-520bRP1 | AA+AG+T+GCTTCCTTTTAG | Human specific; implicated in oncogenesis |
| | | | SEQ ID NO: 548 | | SEQ ID NO:552 | |
| | | h-miR-520bGS9 | CATGATCAGCTGGGCCAAGACCCTCTAAA | h-miR-520bRP2 | AA+AG+TG+CTTCCTTTTA | |
| | | | SEQ ID NO:549 | | SEQ ID NO:553 | |
| | | h-miR-520bGS8 | CATGATCAGCTGGGCCAAGACCCTCTAA | | | |
| | | | SEQ ID NO:550 | | | |
| | | h-miR-520bGS7 | CATGATCAGCTGGGCCAAGACCCTCTA | | | |
| | | | SEQ ID NO:551 | | | |
| 10 | h-miR-520d | h-miR-520dGS10 | CATGATCAGCTGGGCCAAGAAACCCACCAA | h-miR-520dRP1 | AA+AG+TGCTTCTTTGGT | Human specific; implicated in oncogenesis |
| | | | SEQ ID NO:554 | | SEQ ID NO:558 | |
| | | h-miR-520dGS9 | CATGATCAGCTGGGCCAAGAAACCCACCA | h-miR-520dRP2 | AA+AG+TG+CTTCTTTG | |
| | | | SEQ ID NO:555 | | SEQ ID NO:559 | |
| | | h-miR-520dGS8 | CATGATCAGCTGGGCCAAGAAACCCACC | | | |
| | | | SEQ ID NO:556 | | | |
| | | h-miR 520dGS7 | CATGATCAGCTGGGCCAAGAAACCCAC | | | |
| | | | SEQ ID NO:557 | | | |
| 11 | h-miR-520e | h-miR-520eGS10 | CATGATCAGCTGGGCCAAGACCCTCAAAAA | h-miR-520eRP1 | AA+AG+TGCTTCCTTTTTG | Human specific; implicated in oncogenesis |
| | | | SEQ ID NO:560 | | SEQ ID NO:564 | |
| | | h-miR-520eGS9 | CATGATCAGCTGGGCCAAGACCCTCAAAA | h-miR-520eRP2 | AA+AG+T+GCTTCCTTTTT | |
| | | | SEQ ID NO:561 | | SEQ ID NO:565 | |
| | | h-miR-520eGS8 | CATOATCAGTOOOCCAAGACCCTCAAA | | | |
| | | | SEQ ID NO:562 | | | |
| | | h-miR-520eGS7 | CATGATCAGCTGGGCCAAGACCCTCAA | | | |
| | | | SEQ ID NO:563 | | | |
| 12 | h-miR-520f | h-miR-520fGS10 | CATGATCAGCTGGGCCAAGAAACCCTCTAA | h-miR-520fRP1 | A+AG+TGCTTCCTTTTAGA | Human specific; implicated in oncogenesis |
| | | | SEQ ID NO:566 | | SEQ ID NO:570 | |
| | | h-miR-520fGS9 | CATGATCAGCTGGGCCAAGAAACCCTCTA | h-miR-520fRP2 | A+AG+T+GCTTCCTTTTA | |
| | | | SEQ ID NO:567 | | SEQ ID NO:571 | |
| | | h-miR-520fGS8 | CATGATCAGCTGGGCCAAGAAACCCTCT | | | |
| | | | SEQ ID NO:568 | | | |
| | | h-miR-520fGS7 | CATGATCAGCTGGGCCAAGAAACCCTC | | | |
| | | | SEQ ID NO:569 | | | |
| 13 | mr-miR-329 | mr-miR-329GS10 | CATGATCAGCTGGGCCAAGAAAAAAGGTTA | mr-miR-329RP1 | AA+CA+CACCCAGCTAACC | Specific for mouse/rat ortholog |
| | | | SEQ ID NO:572 | | SEQ ID NO:576 | |
| | | mr-miR-329GS9 | CATGATCAGCTGGGCCAAGAAAAAAGGTT | mr-miR-329RP2 | AA+CA+CACCCAGCTAA | |
| | | | SEQ ID NO:573 | | SEQ ID NO:577 | |
| | | mr-miR-329GS8 | CATGATCAGCTGGGCCAAGAAAAAAGGT | | | |
| | | | SEQ ID NO:574 | | | |
| | | mr-miR-329GS7 | CATGATCAGCTGGGCCAAGAAAAAAGG | | | |
| | | | SEQ ID NO:575 | | | |
| 14 | hmr-miR-181d | hmr-miR -181dGS10 | CATGATCAGCTGGGCCAAGAAACCCACCGA | hmr-miR-181dRP1 | AA+CATT+CATTGTTGTCGGT | Conserved across all three species |
| | | | SEQ ID NO:578 | | SEQ ID NO:582 | |
| | | hmr miR 181dGS9 | CATGATCAGCTGGGCCAAGAAACCCACCG | hmr miR 181dRP2 | AA+CA+TT+CATTGTTGTCG | |
| | | | SEQ ID NO:579 | | SEQ ID NO:583 | |
| | | hmr-miR-181dGS8 | CATGATCAGCTGGGCCAAGAAACCCACC | | | |
| | | | SEQ ID NO:580 | | | |
| | | hmr-miR- | CATGATCAGCTGGGCCAAGAAACCCAC | | | |
| | | 181dGS7 | SEQ ID NO:581 | | | |
| 15 | has-miR-193b | hmr-miR-193bGS10 | CATGATCAGCTGGGCCAAGAAAAGCGGGAC | hmr-miR-193bRP1 | AA+CT+GGCCCTCAAAGTCCC | Conserved across all three species |
| | | | SEQ ID NO:584 | | SEQ ID NO:588 | |
| | | hmr-miR-193bGS9 | CATGATCAGCTGGGCCAAGAAAAGCGGGA | hmr-miR-193bRP2 | AA+CT+GGCCCTCAAAGTC | |
| | | | SEQ ID NO:585 | | SEQ ID NO:589 | |
| | | hmr-miR-193bGS8 | CATGATCAGCTGGGCCAAGAAAAGCGGG | | | |
| | | | SEQ ID NO:586 | | | |
| | | hmr-miR-193bGS7 | CATGATCAGCTGGGCCAAGAAAAGCGG | | | |
| | | | SEQ ID NO:587 | | | |
| 16 | h-miR-362 | h-miR-362GS 10 | CATGATCAGCTGGGCCAAGAACTCACACCT | h-miR-362RP1 | AAT+CCTT+GGAACCTAGGTG | Assay specific for human ortholog |
| | | | SEQ ID NO:590 | | SEQ ID NO:594 | |
| | | h-miR-362GS9 | CATGATCAGCTGGGCCAAGAACTCACACC | h-miR-362RP2 | AA+TC+CTT+GGAACCTAGG | |
| | | | SEQ ID NO:591 | | SEQ ID NO:595 | |
| | | h-miR-362GS8 | CATGATCAGCTGGGCCAAGAACTCACAC | | | |
| | | | SEQ ID NO:592 | | | |
| | | h-miR-362GS7 | CATGATCAGCTGGGCCAAGAACTCACA | | | |
| | | | SEQ ID NO:593 | | | |
| 17 | mr-miR-362 | mr-mIR-362-3pGS10 | CATGATCAGCTGGGCCAAGATTCACACCTA | mr-mIR-362-3pRP1 | AA+TCCTT+GGAACCTAGGT | Assay specific for rodent ortholog |
| | | | SEQ ID NO:596 | | SEQ ID NO:600 | |
| | | mr-mIR-362-3pGS9 | CATGATCAGCTGGGCCAAGATTCACACCT | mr-mIR-362-3pRP2 | AA+TC+CTT+GGAACCTAG | |
| | | | SEQ ID NO:597 | | SEQ ID NO:601 | |
| | | mr-mIR-362-3pGS8 | CATGATCAGCTGGGCCAAGATTCACACC | | | |
| | | | SEQ ID NO:598 | | | |
| | | mr mIR-362-3pGS7 | CATGATCAGCTGGGCCAAGATTCACAC | | | |
| | | | SEQ ID NO:599 | | | |
| 18 | h-miR-500 | h-miR-500GS10 | CATGATCAGCTGGGCCAAGACAGAATCCTT | h-miR-500RP1 | A+TG+CACCTGGGCAAGGA | Assay specific for human ortholog |
| | | | SEQ ID NO:602 | | SEQ ID NO: 606 | |
| | | h-miR-500GS9 | CATGATCAGCTGGGCCAAGACAGAATCCT | h-miR-500RP2 | A+TG+CACCT GGGCAAG | |
| | | | SEQ ID NO:603 | | SEQ ID NO:607 | |
| | | h-miR-500GS8 | CATGATCAGCTGGGCCAAGACAGAATCC | | | |
| | | | SEQ ID NO: 604 | | | |
| | | h-miR-500GS7 | CATGATCAGCTGGGCCAAGACAGAATC | | | |
| | 500GS7 | | SEQ ID NO:605 | | | |
| 19 | mmu-miR-500 | mr-miR-5000GS10 | CATGATCAGCTGGGCCAAGACTGAACCCTT | mr-miR-500RP1 | A+TGCA+CCTGGGCAAGGG | Assay specific for rodent ortholog |
| | | | SEQ ID NO:608 | | SEQ ID NO:612 | |
| | | mr-miR-500GS9 | CATGATCAGCTGGGCCAAGACTGAACCCT | mr-miR-500RP2 | A+TGCA+CCTGGGCCAAG | |
| | | | SEQ ID NO:609 | | SEQ ID NO:613 | |
| | | mr-miR-50OGS8 | CATGATCAGCTGGGCCAAGACTGAACCC | | | |
| | | | SEQ ID NO:610 | | | |
| | | mr-miR-500GS7 | CATGATCAGCTGGGCCAAGACTGAACC | | | |
| | | | SEQ ID NO:611 | | | |
| 20 | h-miR 501 | h-miR-501GS10 | CATGATCAGCTGGGCCAAGATCTCACCCAG | h-miR-501RP1 | AA+T+CCTT+TGTCCCTGGG | Assay specific for human ortholog |
| | | | SEQ ID NO:614 | | SEQ ID NO:618 | |
| | | h-miR-501GS9 | CATGATCAGCTGGGCCAAGATCTCACCCA | h-miR-501RP2 | AAT+CCTT+TGTCCCTGG | |
| | | | SEQ ID NO: 615 | | SEQ ID NO:619 | |
| | | h-miR-501GS8 | CATGATCAGCTGGGCCAAGATCTCACCC | | | |
| | | | SEQ ID NO:616 | | | |
| | | h-miR-501GS7 | CATGATCAGCTGGGCCAAGATCTCACC | | | |
| | | | SEQ ID NO:617 | | | |
| 21 | mr-miR-501 | mr-miR-501GS10 | CATGATCAGCTGGGCCAAGATTTCACCCAG | mr-miR-501RP1 | AA+T+CC+TTTGTCCCTGGG | Assay specific for rodent ortholog |
| | | | SEQ ID NO:620 | | SEQ ID NO:624 | |
| | | mr-miR 501GS9 | CATGATCAGCTGGGCCAAGATTTCACCCA | mr-miR 501RP2 | AA+T+CC+TTTGTCCCTG | |
| | | | SEQ ID NO:621 | | SEQ ID NO:625 | |
| | | mr-miR-501GS8 | CATGATCAGCTGGGCCAAGATTTCACCC | | | |
| | | | SEQ ID NO:622 | | | |
| | | mr-miR-501GS7 | CATGATCAGCTGGGCCAAGATTTCACC | | | |
| | | | SEQ ID NO: 623 | | | |
| | hmr-miR-487b | hmr-miR-487bGS10 | CATGATCAGCTGGGCCAAGAAGTGGATGAC | hmr-miR-487bRP1 | AAT+CG+TACAGGGTCAT | Conserved across all three species |
| 22 | | | SEQ ID NO:626 | | SEQ ID NO:630 | |
| | | hmr-miR-487bGS9 | CATGATCAGCTGGGCCAAGAAGTGGATGA | hmr-miR-487bRP2 | A+AT+CG+TACAGGGTC | |
| | | | SEQ ID NO:627 | | SEQ ID NO:631 | |
| | | hmr-miR-487bGS8 | CATGATCAGCTGGGCCAAGAAGTGGATG | | | |
| | | | SEQ ID NO:628 | | | |
| | | hmr-miR-487bGS7 | CATGATCAGCTGGGCCAAGAAGTGGAT | | | |
| | | | SEQ ID NO:629 | | | |
| 23 | h-miR-489 | h-miR-489GS10 | CATGATCAGCTGGGCCAAGAGCTGTCCGTAT | h-miR-489RP1 | AG+TGA+CATCACATATACG | Assay specific for human ortholog |
| | | | SEQ ID NO:632 | | SEQ ID NO:636 | |
| | | h-miR-489GS9 | CATGATCAGCTGGGCCAAGAGCTGCCGTA | h-miR-489RP2 | A+G+TGA+CATCACATATAC | |
| | | | SEQ ID NO: 633 | | SEQ ID NO:637 | |
| | | h-miR-489GS8 | CATGATCAGCTGGGCCAAGCTGCCGT | | | |
| | | | SEQ ID NO:634 | | | |
| | | h-miR-489GS7 | CATGATCAGCTGGGCCAAGACTGCCG | | | |
| | | | SEQ ID NO:635 | | | |
| 24 | m-miR-489 | m-miR-489GS10 | CATGATCAGCTGGGCCAAGAGCTGCCATAT | m-miR-489RP1 | AATGA+CA+CCACATATATG | Assay specific for mouse ortholog |
| | | | SEQ ID NO:638 | | SEQ ID NO:642 | |
| | | m-miR-489GS9 | CATGATCAGCTGGGCCAAGAGCTGCCATA | m-miR-489RP2 | AA+TGA+CA+CCACATAT | |
| | | | SEQ ID NO: 639 | | SEQ ID NO: 643 | |
| | | 489GS8 | CATGATCAGCTGGGCCAAGAGCTGCCAT | | | |
| | | | SEQ ID NO:640 | | | |
| | | m miR-489GS7 | CATGATCAGCTGGGCCAAGAGCTGCCA | | | |
| | | | SEQ ID NO:641 | | | |
| 25 | r-miR-489 | r-miR-489GS10 | CATGATCAGCTGGGCCAAGAGCTGCCATAT | r-miR-489RP1 | AA+TGA+CA+TCACATATATG | Assay specific for rat ortholog |
| | | | SEQ ID NO:644 | | SEQ ID NO: 648 | |
| | | r-miR-489GS9 | CATGATCAGCTGGGCCCAAGAGCTGCCATA | r-miR-489RP2 | AAT+GA+CA+TCACATATAT | |
| | | | SEQ ID NO:645 | | SEQ ID NO:649 | |
| | | r-miR-489GS8 | CATGATCAGCTGGGCCAAGAGCTGCCAT | | | |
| | | | SEQ ID NO:646 | | | |
| | | r-miR-489GS7 | CATGATCAGCTGGGCCAAGAGCTGCCA | | | |
| | | | SEQ ID NO:647 | | | |
| 26 | hmr-miR-425-5p | hmr-miR-425-5pGS10 | CATGATCAGCTGGGCCAAGATCAACGGGAG | hmr-miR-425-5pRP1 | AA+TGA+CACGATCACTCCC | Conserved across all three species |
| | | | SEQ ID NO:650 | | SEQ ID NO:654 | |
| | | hmr-miR-425-5pGS9 | CATGATCAGCTGGGCCAAGATCAACGGGA | hmr-miR-425-5pRP2 | AA+T+GA+CACGATCACTC | |
| | | | SEQ ID NO:651 | | SEQ ID NO: 655 | |
| | | hmr-miR-425-5pGS8 | CATGATCAGCTGGGCCAAGATCAACGGG | | | |
| | | | SEQ ID NO:652 | | | |
| | | hnu-mir-425-5pGS7 | CATGATCAGCTGGGCCAAGATCAACGG | | | |
| | | | SEQ ID NO:653 | | | |
| 27 | hmr-miR-652 | hmr miR-652GS10 | CATGATCAGCTGGGCCAAGATGCACAACCC | hmr-miR-652RP1 | AAT+GGCGCCACT AGGGTT | Conserved across all three species |
| | | | SEQ ID NO:656 | | SEQ ID NO:660 | |
| | | hmr-miR-652GS9 | CATGATCAGCTGGGCCAAGATGCACAACC | hmr- miR-652RP2 | AAT+GG+CGCCACT AGGG | |
| | | | SEQ ID NO:657 | | SEQ ID NO:661 | |
| | | hmr-miR-652GS8 | CATGATCAGCTGGGCCAAGATGCACAAC | | | |
| | | | SEQ ID NO: 658 | | | |
| | | hmr-miR-652GS7 | CATGATCAGCTGGGCCAAGATGCACAA | | | |
| | | | SEQ ID NO: 659 | | | |
| 28 | hmr-miR-485-5p | hmr-miR-485-5pGS10 | CATGATCAGCTGGGCCAAGAGAATTCATCA | hmr-miR-485-5pRP1 | AGA+GGCTGGCCGTGATG | Conserved across all three species |
| | | | SEQ ID NO:662 | | SEQ ID NO: 666 | |
| | | hmr-miR-485-5pGS9 | CATGATCAGCTGGGCCAAGAGAATTCATC | hmr-miR-485-5pRP2 | AGA+GGCTGGCCGTGA | |
| | | | SEQ ID NO: 663 | | SEQ ID NO: 667 | |
| | | hmr-miR-485-5pGS8 | CATGATCAGCTGGGCCAAGAGAATTCAT | | | |
| | | | SEQ ID NO: 664 | | | |
| | | hmr-miR-485-5pGS7 | CATGATCAGCTGGGCCAAGAGAATTCA | | | |
| | | | SEQ ID NO: 665 | | | |
| 29 | has-miR-485-3p | hmr-miR-485-3pGS10 | CATGATCAGCTGGGCCAAGAAGAGAGGAGA | hmr-miR-485-3pRP1 | AG+TCATA+CACGGCTCTCC | Conserved across all three species |
| | | | SEQ ID NO: 668 | | SEQ ID NO: 672 | |
| | | hmr-miR-485-3pGS9 | CATGATCAGCTGGGCCAAGAAGAGAGGAG | hmr-miR-485-3pRP2 | AG+TC+ATACACGGCTCT | |
| | | | SEQ ID NO: 669 | | SEQ ID NO: 673 | |
| | | hmr-miR-485-3pGS8 | CATGATCAGCTGGGCCAAGAAGAGAGGA | | | |
| | | | SEQ ID NO: 670 | | | |
| | | hmr-miR-485-3pGS7 | CATGATCAGCTGGGCCAAGAAGAGAGG | | | |
| | | | SEQ ID NO: 671 | | | |
| 30 | hmr-miR-369-5p | hmr-miR-369-5pGS10 | CATGATCAGCTGGGCCAAGACGAATATAAC | hmr-miR-369-5pRP1 | A+GA+TC+GACCGTGTTAT | Conserved across all three species |
| | | | SEQ ID NO: 674 | | SEQ ID NO: 678 | |
| | | hmr-miR-369-5pGS9 | CATGATCAGCTGGGCCAAGACGAATATAA | hmr-miR-369-5pRP2 | A+GA+TCGACCGTGTT | |
| | | | SEQ ID NO: 675 | | SEQ ID NO: 679 | |
| | | hmr-miR 369-5pGS8 | CATGATCAGCTGGGCCAAGACGAATATA | | | |
| | | | SEQ ID NO: 676 | | | |
| | | hmr-miR-369-5pGS7 | CATGATCAGCTGGGCCAAGACGAATAT | | | |
| | | | SEQ ID NO: 677 | | | |
| 31 | hmr-miR-671 | hmr-miR-671GS10 | CATGATCAGCTGGGCCAAGACCTCCAGCCC | hmr-miR-671RP1 | A+GGAAGCCCTGGAGGGGCT | Conserved across all three species |
| | | | SEQ ID NO: 680 | | SEQ ID NO: 684 | |
| | | hmr-miR-671GS9 | CATGATCAGCTGGGCCAAGACCTCCAGCC | hmr-miR-671RP2 | A+GGAAGCCCTGGAGGGG | |
| | | | SEQ ID NO: 681 | | SEQ ID NO: 685 | |
| | | hmr-miR-671GS8 | CATGATCAGCTGGGCCAAGACCTCCAGC | | | |
| | | | SEQ ID NO: 682 | | | |
| | | hm-miR 671GS7 | CATGATCAGCTGGGCCAAGACCTCCAG | | | |
| | | | SEQ ID NO: 683 | | | |
| 32 | h-miR-449b | h-miR-449bGS10 | CATGATCAGCTGGGCCAAGAGCCAGCTAAC | h-miR-449bRP1 | A+GGC+AGTGTATTGTTAG | Assay specific for human ortholog |
| | | | SEQ ID NO: 686 | | SEQ ID NO: 690 | |
| | | h-miR-449bGS9 | CATGATCAGCTGGGCCAAGAGCCAGCTAA | h-miR-449bRP2 | AG+GC+AG+TGTATTGTT | |
| | | | SEQ ID NO: 687 | | SEQ ID NO: 691 | |
| | | h-miR-449bGS8 | CATGATCAGCTGGGCCAAGAGCCAGCTA | | | |
| | | | SEQ ID NO: 688 | | | |
| | | h-miR-449bGS7 | CATGATCAGCTGGGCCAAGAGCCAGCT | | | |
| | | | SEQ ID NO: 689 | | | |
| 33 | mr-miR-449b | mr-miR-449bGS10 | CATGATCAGCTGGGCCAAGACCAGCTAGCA | mr-miR-449bRP1 | A+GGC+AGTGCATTGCTA | Assay specific for rodent ortholog |
| | | | SEQ ID NO: 692 | | SEQ ID NO: 696 | |
| | | mr-miR-449bGS9 | CATGATCAGCTGGGCCAAGACCAGCTAGC | mr-miR-449bRP2 | A+GG+CAGTGCATTGC | |
| | | | SEQ ID NO: 693 | | SEQ ID NO: 697 | |
| | | mr-miR-449bGS8 | CATGATCAGCTGGGCCAAGACCAGCTAG | | | |
| | | | SEQ ID NO: 694 | | | |
| | | mr-miR-449bGS7 | CATGATCAGCTGGGCCAAGACCAGCTA | | | |
| | | | SEQ ID NO: 695 | | | |
| 34 | m-miR-699 | m-miR-699GS10 | CATGATCAGCTGGGCCAAGACGAGCCAGGT | m-miR-699RP1 | A+GGCAGTGCGACCTG | Mouse specific; ortholog to miR-34c |
| | | | SEQ ID NO: 698 | | SEQ ID NO: 702 | |
| | | m-miR-699GS9 | CATGATCAGCTGGGCCAAGACGAGCCAGG | m-miR-699RP2 | A+GG+CAGTGCGACC | |
| | | | SEQ ID NO: 699 | | SEQ ID NO: 703 | |
| | | m-miR-699GS8 | CATGATCAGCTGGGCCAAGACGAGCCAG | | | |
| | | | SEQ ID NO: 700 | | | |
| | | m-miR-699GS7 | CATGATCAGCTGGGCCAAGACGAGCCA | | | |
| | | | SEQ ID NO: 701 | | | |
| 35 | hmr-miR-409-5p | hmr-miR-409-5pGS10 | CATGATCAGCTGGGCCAAGACAAAGTTGCT | hmr-miR-409-5pRP1 | A+GGT+ TACCCGAGCAACT | Conserved across all three species |
| | | | SEQ ID NO: 704 | | SEQ ID NO: 708 | |
| | | hmr-miR-409-5pGS9 | CATGATCAGCTGGGCCAAGACAAAGTTGC | hmr miR-409-5pRP2 | A+GG+TTACCCGAGCAA | |
| | | | SEQ ID NO: 705 | | SEQ ID NO: 709 | |
| | | hmr-miR-409-5pGS8 | CATGATCAGCTGGGCCAAGACAAAGTTG | | | |
| | | | SEQ 10 NO: 706 | | | |
| | | hmr-miR-409-5pGS7 | CATGATCAGCTGGGCCAAGACAAAGTT | | | |
| | | | SEQ ID NO: 707 | | | |
| 36 | hmr-miR-409-3p | hmr-miR-409-3pGS10 | CATGATCAGCTGGGCCAAGAAAGGGGTTCA | hmr-miR-409-3pRP1 | G+AA+TGTTGCTCGGTGAAC | Conserved across all three species |
| | | | SEQ ID NO: 710 | | SEQ ID NO: 714 | |
| | | hmr-miR-409-3pGS9 | CATGATCAGCTGGGCCAAGAAAGGGGTTC | hmr-miR-409-3pRP2 | G+AA+TGTTGCTCGGTGA | |
| | | | SEQ ID NO: 711 | | SEQ ID NO: 715 | |
| | | hmr-miR-409-3pGS8 | CATGATCAGCTGGGCCAAGAAAGGGGTT | | | |
| | | | SEQ ID NO: 712 | | | |
| | | hmr-miR-409-3pGS7 | CATGATCAGCTGGGCCAAGAAAGGGGT | | | |
| | | | SEQ ID NO: 713 | | | |
| 37 | hmr-miR-491 | hmr-miR-491GS10 | CATGATCAGCTGGGCCAAGACCTCATGGAA | hmr-miR-491RP1 | AG+TGG+GGAACCCTTCCA | Conserved across all three species |
| | | | SEQ ID NO: 716 | | SEQ ID NO: 720 | |
| | | hmr-miR-491GS9 | CATGATCAGCTGGGCCAAGACCTCATGGA | hmr-miR-491RP2 | AG+TG+GGGAACCCTTC | |
| | | | SEQ ID NO: 717 | | SEQ ID NO : 721 | |
| | | hmr-miR-491GS8 | CATGATCAGCTGGGCCAAGACCTCATGG | | | |
| | | | SEQ ID NO: 718 | | | |
| | | hmr-miR-491GS7 | CATGATCAGCTGGGCCAAGACCTCATG | | | |
| | | | SEQ ID NO: 719 | | | |
| 38 | h-miR-384 | h-miR-384GS10 | CATGATCAGCTGGGCCAAGATATGAACAAT | h-miR-384RP1 | A+TT+CCT+AGAAATTGTTC | Assay specific for human ortholog |
| | | | SEQ ID NO: 722 | | SEQ ID NO: 726 | |
| | | h-miR-384GS9 | CATGATCAGCTGGGCCAAGATATGAACAA | h-miR-384RP2 | A+TT+CCT+AG+AAATTGT | |
| | | | SEQ ID NO: 723 | | SEQ ID NO: 727 | |
| | | h-miR-384GS8 | CATGATCAGCTGGGCCAAGAT ATGAACA | | | |
| | | | SEQ ID NO: 724 | | | |
| | | h-miR- | CATGATCAGCTGGGCCAAGATATGAAC | | | |
| | | 384GS7 | SEQ ID NO: 725 | | | |
| 39 | mr-miR-384 | mr-miR-384GS10 | CATGATCAGCTGGGCCAAGATGTGAACAAT | mr-miR-384RP1 | A+TT+CCT+AGAAATTGTT | Assay specific for rodent ortholog |
| | | | SEQ ID NO: 728 | | SEQ ID NO: 732 | |
| | | mr-miR-384GS9 | CATGATCAGCTGGGCCAAGATGTGAACAA | mr-miR-384RP2 | A+TT+CCT+AG+AAATTGTT | |
| | | | SEQ ID NO: 729 | | SEQ ID NO: 733 | |
| | | mr-miR-384GS8 | CATGATCAGCTGGGCCAAGATGTGAACA | | | |
| | | | SEQ ID NO: 730 | | | |
| | | mr-miR-384GS7 | CATGATCAGCTGGGCCAAGATGTGAAC | | | |
| | | | SEQ ID NO: 731 | | | |
| 40 | hmr-miR-20b | hmr-miR-20bGS10 | CATGATCAGCTGGGCCAACAACCTGCACTA | hmr-miR-20bRP1 | C+AA+AG+TGCTCATAGTGCA | Conserved across all three species |
| | | | SEQ ID NO: 734 | | SEQ ID NO: 738 | |
| | | hmr-miR-20bGS9 | CATGATCAGCTGGGCCAAGAACCTGACT | hmr-miR-20bRP2 | CAA+AG+TG+CTCATAGTG | |
| | | | SEQ ID NO: 735 | | SEQ ID NO: 739 | |
| | | hmr-miR-20bGS8 | CATGATCAGCTGGGCCAAGAACCTGCAC | | | |
| | | | SEQ ID NO: 736 | | | |
| | | hmr-miR-20bGS7 | CATGATCAGCTGGGCCAAGAACCTGCA | | | |
| | | | SEQ ID NO: 737 | | | |
| 41 | hmr miR-490 | hmr-miR-490GS10 | CATGATCAGCTGGGCCAAGACAGCATGGAG | hmr miR-490RP1 | C+AA+CCTGGAGGACTCCA | Conserved across all three species |
| | | | SEQ ID NO: 740 | | SEQ ID NO: 744 | |
| | | hmr-miR-490GS9 | CATGATCAGCTGGGCCAAGACAGCATGGA | hmr-miR-490RP2 | CAA+CCT+GGAGGACCTC | |
| | | | SEQ ID NO: 741 | | SEQ ID NO: 745 | |
| | | hmr-miR-490GS8 | CATGATCAGCTGGGCCAAGACAGCATGG | | | |
| | | | SEQ ID NO: 742 | | | |
| | | hmr-miR-490GS7 | CATGATCAGCTGGGCCAAGACAGCATG | | | |
| | | | SEQ ID NO: 743 | | | |
| 42 | hmr-miR-497 | hmr-miR-497GS 10 | CATGATCAGCTGGGCCAAGAACAAACCACA | hmr-miR-497RP1 | C+AG+CAGCACACTGTGG | Conserved across all three species |
| | | | SEQ ID NO: 746 | | SEQ ID NO: 750 | |
| | | hmr miR 497GS9 | CATGATCAGCTGGGCCAAGAACAAACCAC | hmr-miR-497RP2 | C+AG+CAGCACACTGTG | |
| | | | SEQ ID NO: 747 | | SEQ ID NO: 751 | |
| | | hmr-miR-497GS8 | CATGATCAGCTGGGCCAAGAACAAACCA | | | |
| | | | SEQ ID NO: 748 | | | |
| | | hmr-miR-497GS7 | CATGATCAGCTGGGCCAAGAACAAACC | | | |
| | | | SEQ ID NO: 749 | | | |
| 43 | h-miR-301b | h-miR-301bGS10 | CATGATCAGCTGGGCCAAGATGCTTTGACA | h-miR-301bRP1 | C+AG+TG+CAATGATATTGTCA | Assay specific for human ortholog |
| | | | SEQ ID NO: 752 | | SEQ ID NO: 756 | |
| | | h-miR-301bGS9 | CATGATCAGCTGGGCCAAGATGCTTTGAC | h-mir-301bRP2 | C+AG+TG+CAATGATATTGT | |
| | | | SEQ ID NO: 753 | | SEQ ID NO: 757 | |
| | | h-miR-301bGS8 | CATGATCAGCTGGGCCAAGATGCTTTGA | | | |
| | | | SEQ ID NO: 754 | | | |
| | | h-miR-301bGS7 | CATGATCAGCTGGGCCAAGATGCTTTG | | | |
| | | | SEQ ID NO: 755 | | | |
| 44 | mr-miR-301b | mr-miR-301bGS10 | CATGATCAGCTGGGCCAAGATGCTTTGACA | mr-miR-301bRP1 | C+AG+TG+CAATGGTATTGTCA | Assay specific for rodent ortholog |
| | | | SEQ ID NO: 758 | | SEQ ID NO: 762 | |
| | | 301bGS9 | CATGATCAGCTGGGCCAAGATGCTTTGAC | mr-miR-301bRP2 | C+AG+TG+CAATGGTATTGT | |
| | | | SEQ ID NO: 759 | | SEQ ID NO: 763 | |
| | | mr miR-301bGS8 | CATGATCAGCTGGGCCAAGATGCTTTGA | | | |
| | | | SEQ ID NO: 760 | | | |
| | | mr-miR-301bGS7 | CATGATCACTGGGCCAAGATGCTTTG | | | |
| | | | SEQ ID NO: 761 | | | |
| 45 | hmr-miR-721 | hmr-miR-721GS10 | CATGATCAGCTGGGCCAAGATTCCCCCTTT | hmr-miR-721RP1 | C+AG+TG+CAATTAAAAGGG | Conserved across all three species |
| | | | SEQ ID NO: 764 | | SEQ ID NO: 768 | |
| | | hmr-miR-721GS9 | CATGATCAGCTGGGCCAAGATTCCCCCTT | hmr-miR-721RP2 | C+AG+TG+CAATTAAAAG | |
| | | | SEQ ID NO: 765 | | SEQ ID NO: 769 | |
| | | hmr-miR-721GS8 | CATGATCAGCTGGGCCAAGATTCCCCCT | | | |
| | | | SEQ ID NO: 766 | | | |
| | | hmr miR-721GS7 | CATGATCAGCTGGGCCAAGATTCCCCC | | | |
| | | | SEQ ID NO:767 | | | |
| 46 | hmr-miR-532 | hmr-miR-532GS10 | CATGATCAGCTGGGCCAAGAACGGTCCTAC | hmr-miR-532RP1 | CA+TG+CCTTGAGTGTAGG | Conserved across all three species |
| | | | SEQ ID NO: 770 | | SEQ ID NO: 774 | |
| | | hmr-miR-532GS9 | CATGATCAGCTGGGCCAAGAACGGTCCTA | hmr-miR-532RP2 | CA+TG+CCTTGAGTGTA | |
| | | | SEQ ID NO: 771 | | SEQ ID NO: 775 | |
| | | hmr-miR-532GS8 | CATGATCAGCTGGGCCAAGAACGGTCCT | | | |
| | | | SEQ ID NO: 772 | | | |
| | | hmr-miR-532GS7 | CATGATCAGCTGGGCCAAGAACGGTCC | | | |
| | | | SEQ ID NO: 773 | | | |
| 47 | h-miR-488 | h-miR-488GS10 | CATGATCAGCTGGGCCAAGATTGAGAGTGC | h-miR-488RP1 | C+CCA+GATAATGGCACT | Assay specific for human ortholog |
| | | | SEQ ID NO: 776 | | SEQ ID NO: 780 | |
| | | h-miR-488GS9 | CATGATCAGCTGGGCCAAGATTGAGAGTG | h-miR-488RP2 | G+CC+A+GATAATGGCA | |
| | | | SEQ ID NO: 777 | | SEQ ID NO: 781 | |
| | | h-miR-488GS8 | CATGATCAGCTGGGCCAAGATTGAGAGT | | | |
| | | | SEQ ID NO: 778 | | | |
| | | h-miR- | CATGATCAGCTGGGCCAAGATTGAGAG | | | |
| | | 488GS7 | SEQ ID NO: 779 | | | |
| 48 | mr-miR-488 | mr-miR-488GS10 | CATGATCAGCTGGCCAAGATTGAGAGTGC | mr-miR-488RP1 | C+CCA+GATAATAGCACT | Assay specific for :rodent ortholog |
| | | | SEQ ID NO: 782 | | SEQ ID NO: 785 | |
| | | mr-miR-488GS9 | CATGATCAGCTGGGCCAAGATTGAGAGTG | mr-miR-488RP2 | C+CC+A+GATAATAGCA | |
| | | | SEQ ID NO: 783 | | SEQ ID NO: 787 | |
| | | mr-miR-488GS8 | CATGATCAGCTGGGCCAAGATTGAGAGT | | | |
| | | | SEQ ID NO: 784 | | | |
| | | mr-miR-488GS7 | CATGATCAGCTGGGCCAAGATTGAGAG | | | |
| | | | SEQ ID NO: 785 | | | |
| 49 | hmr-miR-539 | hmr-miR-539GS10 | CATGATCAGCTGGGCCAAGAACACACCAAG | hmr-miR-539RP1 | GG+AG+AAATTATCCTTGGT | Conserved across all three species |
| | | | SEQ ID NO: 788 | | SEQ ID NO: 792 | |
| | | hmr-miR-539GS9 | CATGATCAGCTGGGCCAAGAACACACCAA | hmr-miR-539RP2 | G+GA+G+AAATT ATCCTTGG | |
| | | | SEQ ID NO: 789 | | SEQ ID NO. 793 | |
| | | hmr-miR-539GS8 | CATGATCAGCTGGGCCAAGAACACACCA | | | |
| | | | SEQ ID NO: 790 | | | |
| | | hmr-miR-539GS7 | CATGATCAGCTGGGCCAAGAACACACC | | | |
| | | | SEQ ID NO: 791 | | | |
| 50 | h-miR-505 | h-miR-505GS10 | CATGATCAGCTGGGCCAAGAGAGGAAACCA | h-miR-505RP1 | GT+CAA+CACTTGCTGGTT | Assay specific for human ortholog |
| | | | SEQ ID NO: 794 | | SEQ ID NO: 798 | |
| | | h-miR-505GS9 | CATGATCAGCTGGGCCAAGAGAGGAAACC | h-miR-505RP2 | G+T+CAA+CACTTGCTGG | |
| | | | SEQ ID NO: 795 | | SEQ ID NO: 799 | |
| | | h-miR-505GS8 | CATGATCAGCTGGGGCAAGAGAGGAAAC | | | |
| | | | SEQ ID NO: 796 | | | |
| | | h-miR-505GS7 | CATGATCAGCTGGGCCAAGAGAGGAAA | | | |
| | | | SEQ ID NO: 797 | | | |
| 51 | mr-miR-505 | mr-miR-505GS10 | CATGATCAGCTGGGCCAAGAGGAAACCAGC | mr-miR-505RP1 | CG+T+CAA+CA+CTTGCTGGT | Assay speafic for rodent ortholog |
| | | | SEQ ID NO: 800 | | SEQ ID NO: 804 | |
| | | mr-miR-505GS9 | CATGATCAGCTGGGCCAAGAGGAAACCAG | mr-miR-505RP2 | CG+T+CAA+CA+CTTGCTG | |
| | | | SEQ ID NO: 801 | | SEQ ID NO: 805 | |
| | | mr-miR-505GS 8 | CATGATCAGCTGGGCCAAGAGGAAACCA | | | |
| | | | SEQ ID NO: 802 | | | |
| | | mr-miR-505GS7 | CATGATCAGCTGGGCCAAGAGGAAACC | | | |
| | | | SEQ ID NO: 803 | | | |
| 52 | h-miR-18b | h-miR-18bGS10 | CATGATCAGCTGGGCCAAGATAACTGCACT | h-miR-18bRP1 | TAA+GG+TGCATCTAGTGC | Assay specific for human ortholog |
| | | | SEQ ID NO: 806 | | SEQ ID NO: 810 | |
| | | h-miR-18bGS9 | CATGATCAGCTGGGCCAAGATAACTGCAC | h-miR-18bRP2 | T+AA+GG+TGCATCTAGT | |
| | | | SEQ ID NO: 807 | | SEQ ID NO: 811 | |
| | | h-miR-18bGS8 | CATGATCAGCTGGGCCAAGATAACTGCA | | | |
| | | | SEQ ID NO: 808 | | | |
| | | h-miR-18bGS7 | CATGATCAGCTGGGCCAAGATAACTGC | | | |
| | | | SEQ ID NO: 809 | | | |
| 53 | mr-miR-18b | mr-miR-l8bGS10 | CATGATCAGCTGGGCCAAGATAACAGCACT | mr-miR-18bRP1 | T+AA+GG+TGCATCTAGTGC | Assay specific for rodent ortholog |
| | | | SEQ ID NO: 812 | | SEQ ID NO: 816 | |
| | | mr-miR-18bGS9 | CATGATCAGCTGGGCCAAGATAACAGCAC | mr-miR-18bRP2 | TAA+GG+TG+CATCTAGT | |
| | | | SEQ ID NO: 813 | | SEQ ID NO: 817 | |
| | | mr-miR-18bGS8 | CATGATCAGCTGGGCCAAGATAACAGCA | | | |
| | | | SEQ ID NO: 814 | | | |
| | | mr-miR-18bCS7 | CATGATCAGCTGGGCCAAGATAACAGC | | | |
| | | | SEQ ID NO: 815 | | | |
| 54 | hmr-miR-503 | hmr-miR-503GS10 | CATCATCAGCTGGGCCAAGACAGTACTGTT | hmr-miR-503RP1 | T+AGC+AGCGGGAACAGT | Conserved across all three species |
| | | | SEQ ID NO: 818 | | SEQ ID NO: 822 | |
| | | hmr-miR-503GS9 | CATGATCAGCTGGGCCAAGACAGTACTGT | hmr-miR-503RP2 | T+AGC+AGCGGGAACA | |
| | | | SEQ ID NO: 819 | | SEQ ID NO: 823 | |
| | | hmr-miR-503GS8 | CATGATCAGCTGGGCCAAGACAGTACTG | | | |
| | | | SEQ ID NO: 820 | | | |
| | | hmr-miR-503GS7 | CATGATCAGCTGGGCCAAGACAGTACT | | | |
| | | | SEQ ID NO: 821 | | | |
| 55 | hmr-miR-455 | hmr-miR 455GS10 | CATGATCAGCTGGGCCAAGACGATGTAGTC | hmr-miR-455RP1 | TA+TG+ TGCCTTTGGACTA | Conserved across all three species |
| | | | SEQ ID NO: 824 | | SEQ ID NO: 828 | |
| | | hmr-miR-455GS9 | CATGATCAGCTGGGCCAAGACGATGTAGT | hmr-miR-455RP2 | TA+TG+TGCCTTTGGAC | |
| | | | SEQ ID NO: 825 | | SEQ ID NO: 829 | |
| | | hmr-miR-455GS8 | CATGATCAGCTGGGCCAAGACGATGTAG | | | |
| | | | SEQ ID NO: 826 | | | |
| | | hmr-miR-455GS7 | CATGATCAGCTGGGCCAAGACGATGTA | | | |
| | | | SEQ ID NO: 827 | | | |
| 56 | hmr-miR 92b | hmr-miR-92bGS 10 | CATGATCAGCTGGGCCAAGAGAGGCCGGGA | hmr-miR-92bRP1 | TAT+TG+CACTCGTCCCG | Conserved across all three species |
| | | | SEQ ID NO: 830 | | SEQ ID NO: 834 | |
| | | hmr-miR-92bGS9 | CATGATCAGCTGGGCCAAGAGAGGCCGGG | hmr-miR-92bRP2 | TAT+TG+CACTCGTCCC | |
| | | | SEQ ID NO: 831 | | SEQ ID NO: 835 | |
| | | hmr-miR-92bGS 8 | CATGATCAGCTGGGCCAAGAGAGGCCGG | | | |
| | | | SEQ ID NO: 832 | | | |
| | | hmr-miR-92bGS7 | CATGATCAGCTGGGCCAAGAGAGGCCG | | | |
| | | | SEQ ID NO: 833 | | | |
| 57 | h-miR-483 | h-miR-483GS10 | CATGATCAGCTGGGCCAAGAAGAAGACGGG | h-miR-483RP1 | T+CAC+TCCTCTCCTCCCGT | Assay specific for human ortholog |
| | | | SEQ ID NO: 836 | | SEQ ID NO: 840 | |
| | | h-miR-493GS9 | CATGATCAGCTGGGCCAAGAAGAAGACGG | h-miR-483RP2 | T+CAC+TCCTCTCCTCCC | |
| | | | SEQ ID NO: 837 | | SEQ ID NO: 841 | |
| | | h-miR-483GS8 | CATGATCAGCTGGGCCAAGAAGAAGACG | | | |
| | | | SEQ ID NO: 838 | | | |
| | | h-miR-483GS7 | CATGATCAGCTGGGCCAAGAAGAAGAC | | | |
| | | | SEQ ID NO: 839 | | | |
| 58 | mr-miR-483 | mr-miR-483GS10 | CATGATCAGCTGGGCCAAGAACAAGACGGG | mr-miR-483RP1 | TC+ACTCCTCCCCTCCCGT | Assay specific for rodent ortholog |
| | | | SEQ ID NO: 842 | | SEQ ID NO: 846 | |
| | | mr-miR-483GS9 | CATGATCAGCTGGGCCAAGAACAAGACGG | mr-miR-483RP2 | TC+ACTCCTCCCCTCCC | |
| | | | SEQ ID NO: 843 | | SEQ ID NO: 847 | |
| | | mr-miR-483GS8 | CATGATCAGCTGGGCCAAGAACAAGACG | | | |
| | | | SEQ ID NO: 844 | | | |
| | | mr-miR-483GS7 | CATGATCAGCTGGGCCAAGAACAAGAC | | | |
| | | | SEQ ID NO: 845 | | | |
| 59 | hmr-miR-484 | hmr-miR-484GS10 | CATGATCAGCTGGGCCAAGAATCGGGAGGG | hmr-miR-484RP1 | TCA+GGCTCAGTCCCCTC | Conserved across all three species |
| | | | SEQ ID NO: 848 | | SEQ ID NO: 852 | |
| | | hmr-miR-484GS9 | CATGATCAGCTGGGCCAAGAATCGGGAGG | hmr-miR-484RP2 | TC+AGGCTCAGTCCCC | |
| | | | SEQ ID NO: 849 | | SEQ ID NO: 853 | |
| | | hmr-miR-484GS8 | CATGATCAGCTGGGCCAAGAATCGGGAG | | | |
| | | | SEQ ID NO: 850 | | | |
| | | hmr-miR-484GS7 | CATGATCAGCTGGGCCAAGAATCGGGA | | | |
| | | | SEQ ID NO: 851 | | | |
| 60 | mmu-miR-351 | hmr-miR-351GS10 | CATGATCAGCTGGGCCAAGACAGGCTCAAA | hmr-miR-351RP1 | TC+CCTGAGGAGCCCTTTGA | Rodent specific; ortholog to human miR-125 |
| | | | SEQ ID NO: 854 | | SEQ ID NO: 858 | |
| | | hmr-miR-351GS9 | CATGATCAGCTGGGCCAAGACAGGCTCAA | hmr-miR-351RP2 | TC+CCTGAGGAGCCCTTT | |
| | | | SEQ ID NO: 855 | | SEQ ID NO: 859 | |
| | | hmr-miR- | CATGATCAGCTGGGCCAAGACAGGCTCA | | | |
| | | 351GS8 | SEQ ID NO: 856 | | | |
| | | hmr-miR-351GS7 | CATGATCAGCTGGGCCAAGACAGGCTC | | | |
| | | | SEQ ID NO: 857 | | | |
| 61 | hmr-miR-615 | hmr-miR-615GS10 | CATGATCAGCTGCTGGGCCAAGAAGAAGAGGGAGAC | hmr-miR-615RP1 | TC+CGAGCCTGGGTCTC | Conserved across all three species |
| | | | SEQ ID NO: 860 | | SEQ ID NO: 864 | |
| | | hmr-miR-615GS9 | CATGATCAGCTGGGCCAAGAAGAGGGAGA | hmr-miR-615RP2 | TC+CGAGCCTGGGTC | |
| | | | SEQ ID NO: 861 | | SEQ ID NO: 865 | |
| | | hmr-miR-615GS8 | CATGATCAGCTGGGCCAAGAAGAGGGAG | | | |
| | | | SEQ ID NO: 862 | | | |
| | | hmr-miR-615GS7 | CATGATCAGCTGGGCCAAGAAGAGGGA | | | |
| | | | SEQ ID NO: 863 | | | |
| 62 | hmr-miR-486 | hmr-miR-486GS10 | CATGATCAGCTGGGCCAAGACTCGGGGCAG | hmr-miR-486RP1 | T+CC+TGTACTGAGCTGCC | Conserved across all three species |
| | | | SEQ ID NO: 866 | | SEQ ID NO: 870 | |
| | | hmr-miR-486GS9 | CATGATCAGCTGGGCCAAGACTCGGGGCA | hmr-miR-486RP2 | T+CC+TGTACTGAGCTG | |
| | | | SEQ ID NO: 867 | | SEQ ID NO: 871 | |
| | | hmr-miR-486GS8 | CATGATCAGCTGGGCCAAGACTCGGGGC | | | |
| | | | SEQ ID NO: 868 | | | |
| | | hmr-miR -486GS7 | CATGATCAGCTGGGCCAAGACTCGGGG | | | |
| | | | SEQ ID NO: 869 | | | |
| 63 | hmr-miR-494 | hmr-miR-494GS10 | CATGATCAGCTGGGCCAAGAAGGTTTCCCG | hmr-miR-494RP1 | T+GA+AA+CATACACGGGA | Conserved across all three species |
| | | | SEQ ID NO: 872 | | SEQ ID NO: 876 | |
| | | hmr-miR-494GS9 | CATGATCAGCTGGGCCAAGAAGGTTTCCC | hmr miR 494RP2 | T+GA+AA+CATACACGG | |
| | | | SEQ ID NO: 873 | | SEQ ID NO: 877 | |
| | | hmr-miR-494GS8 | CATGATCAGCTGGGCCAAGAATTTCC | | | |
| | | | SEQ ID NO: 874 | | | |
| | | hmr-miR-494GS7 | CATGATCAGCTGGGCCAAGAAGGTTTC | | | |
| | | | SEQ ID NO: 875 | | | |
| 64 | hmr-miR-493-3p | hmr-miR-493-3pGS10 | CATGATCAGCTGGGCCAAGACTGGCACACA | hmr-miR-493-3pRP1 | T+GAA+GGTCTACTGTG | Conserved across all three species |
| | | | SEQ ID NO: 878 | | SEQ ID NO: 882 | |
| | | hmr-miR -493-3pGS9 | CATGATCAGCTGGGCCAAGACTGGCACAC | hmr-miR-493-3pRP2 | T+GAA+GGTCTACTGT | |
| | | | SEQ ID NO: 879 | | SEQ ID NO: 883 | |
| | | hmr-miR -493-3pGS8 | CATGATCAGCTGGGCCAACTGGCACA | | | |
| | | | SEQ ID NO: 880 | | | |
| | | hmr-miR -493-3pGS7 | CATGATCAGCTGGGCCAAGACTGGCAC | | | |
| | | | SEQ ID NO: 881 | | | |
| 65 | hmr-miR-146b | hmr-miR-146bGS10 | CATGATCAGCTGGGCCAAGAAGCCTATGGA | hmr-miR-146bRP1 | T+GA+GAAC+TGAATTCCATA | Conserved across all three species |
| | | | SEQ ID NO: 884 | | SEQ ID NO: 888 | |
| | | hmr-miR-146bGS9 | CATGATCAGCTGGGCCAAGAAGCCTATGG | hmr-miR-146bRP2 | T+GA+GAAC+TGAATTCCA | |
| | | | SEQ ID NO: 885 | | SEQ ID NO: 889 | |
| | | hmr-miR-146bGS8 | CATGATCAGCTGGGCCAAGAAGCCTATG | | | |
| | | | SEQ ID NO: 886 | | | |
| | | hmr-miR-146bGS7 | CATGATCAGCTGGGCCAAGAAGCCTAT | | | |
| | | | SEQ ID NO: 887 | | | |
| 66 | r-miR-1 | r-miR-1GS10 | CATGATCAGCTGGGCCAAGATACACACTTC | r-miR-1RP1 | T+G+GAA+TGTAAAGAAGTG | Assay specific for rat |
| | | | SEQ ID NO: 890 | | SEQ ID NO: 894 | ortholog |
| | | r-miR-1 GS9 | CATGATCAGCTGGGCCAAGATACACACTT | r-miR-1RP2 | T+G+GAA+TGT AAAGAAG | |
| | | | SEQ ID NO: 891 | | SEQ ID NO: 895 | |
| | | r-miR-1GS8 | CATGATCAGCTGGGCCAAGATACACACT | | | |
| | | | SEQ ID NO: 892 | | | |
| | | r miR 1GS7 | CATGATCAGCTGGGCCAAGATACACAC | | | |
| | | | SEQ ID NO: 893 | | | |
| 67 | h-miR-675-5p | h-miR-675- 5pGS10 | CATGATCAGCTGGGCCAAGACACTGTGGGC | h-miR-675- 5pRP1 | T+GGTGCGGAGAGGGCCCA | Assay specific for human ortholog |
| | | | SEQ ID NO: 896 | | SEQ ID NO: 900 | |
| | | h-miR-675- 5pGS9 | CATGATCAGCTGGGCCAAGACACTGTGGG | h-miR-675- 5pRP2 | T+GGTGCGGAGAGGGC | |
| | | | SEQ ID NO: 897 | | SEQ ID NO: 901 | |
| | | h-miR-675- 5pGS8 | CATGATCAGCTGGGCCAAGACACTGTGG | | | |
| | | | SEQ ID NO: 898 | | | |
| | | h-miR-675- 5pGS7 | CATGATCAGCTGGGCCAAGACACTGTG | | | |
| | | | SEQ ID NO: 899 | | | |
| 68 | mr-miR-675- 5p | mr-miR- 675-5pGS10 | CATGATCAGCTGGGCCAAGAACTGTGGGCC | mr-miR-675- 5pRP1 | T+GGTGCGGAAAGGGCC | Assay specific for rodent ortholog |
| | | | SEQ ID NO: 902 | | SEQ ID NO: 906 | |
| | | mr-miR- 675-5pGS9 | CATGATCAGCTGGGCCAAGAACTGTGGGC | mr-miR-675- 5pRP2 | T+GGTGCGGAAAGGG | |
| | | | SEQ ID NO: 903 | | SEQ ID NO: 907 | |
| | | mr-miR- 675-5pGS8 | CATGATCAGCTGGGCCAAGAACTGTGGG | | | |
| | | | SEQ ID NO: 904 | | | |
| | | mr-miR- 675-5pGS7 | CATGATCAGCTGGGCCAAGAACTGTGG | | | |
| | | | SEQ ID NO: 905 | | | |
| 69 | hmr-miR-668 | hmr-miR 668GS10 | CATGATCAGCTGGGCCAAGAGTAGTGGGCC | hmr miR 668RP1 | TG+TCACTCGGCTCGGCC | Conserved across all three species |
| | | | SEQ ID NO: 908 | | SEQ ID NO: 912 | |
| | | hmr-miR- 668GS9 | CATGATCAGCTGGGCCAAGAGTAGTGGGC | hmr-miR- 668RP2 | TG+TCACTCGGCTCGG | |
| | | | SEQ ID NO: 909 | | SEQ ID NO: 913 | |
| | | hmr-miR- 668GS8 | CATGATCAGCTGGGCCAAGAGTAGTGGG | | | |
| | | | SEQ ID NO: 910 | | | |
| | | hmr-miR- 669GS7 | CATGATCAGCTGGGCCAAGAGTAGTGG | | | |
| | | | SEQ ID NO: 911 | | | |
| 70 | r-miR-346 | r-miR- 346GS10 | CATGATCAGCTGGGCCAAGAAGAGGCAGGC | r-miR-346RP1 | TGTC+TGCCTGAGTGCCTG | Assay specific for rat ortholog |
| | | | SEQ ID NO: 914 | | SEQ ID NO: 918 | |
| | | r-miR 346GS9 | CATGATCAGCTGGGCCAAGAAGAGGCAGG | r-miR-346RP2 | TGTC+TGCCTGAGTGCC | |
| | | | SEQ ID NO: 915 | | SEQ ID NO: 919 | |
| | | r-miR-346GS8 | CATGATCAGCTGGGCCAAGAAGAGGCAG | | | |
| | | | SEQ ID NO; 916 | | | |
| | | r-miR-346GS7 | CATGATCAGCTGGGCCAAGAAGAGGCA | | | |
| | | | SEQ ID NO: 917 | | | |
| 71 | hmr-miR-542-3p | hmr-miR-542-3pGS10 | CATGATCAGCTGGGCCAAGATTCAGTTATC | hmr-miR-542-3pRP1 | TG+TGA+CAGATTGATAACT | Conserved across all three species |
| | | | SEQ ID NO: 920 | | SEQ ID NO: 924 | |
| | | hmr-miR-542-3pGS9 | CATGATCAGCTGGGCCAAGATTCAGTTAT | hmr-miR-542-3pRP2 | TG+T+GA+CAGATTGATAA | |
| | | | SEQ ID NO: 921 | | SEQ ID NO: 925 | |
| | | hmr-miR-542-3pGS8 | CATGATCAGCTGGGCCAAGATTCAGTTA | | | |
| | | | SEQ ID NO: 922 | | | |
| | | hmr-miR-542-3pGS7 | CATGATCAGCTGGGCCAAGATTCAGTT | | | |
| | | | SEQ ID NO: 923 | | | |
| 72 | hmr-miR-542-5p | hmr-miR-542-5pGS10 | CATGATCAGCTGGGCCAAGACGTGACATGATG | hmr-miR-542-5pRP1 | CTC+GG+GGATCATCATG | Conserved across all three species |
| | | | SEQ ID NO: 926 | | SEQ ID NO: 930 | |
| | | hmr-miR-542-5pGS9 | CATGATCAGCTGGGCCAAGACGTGACATG | hmr-miR-542-5pRP2 | C+TC+GGGGATCATCAT | |
| | | | SEQ ID NO: 927 | | SEQ ID NO: 931 | |
| | | hmr-miR-542-5pGS8 | CATGATCAGCTGGGCCAAGACGTGACAT | | | |
| | | | SEQ ID NȮ: 928 | | | |
| | | hmr-miR-542-5pGS7 | CATGATCAGCTGGGCCAAGACGTGACA | | | |
| | | | SEQ ID NO: 929 | | | |
| 73 | hmr-miR-499 | hmr-miR-499GS10 | CATGATCAGCTGGGCCAAGAAAACATCACT | hmr-miR-499RP1 | T+TAA+GA+CTTGCAGTGAT | Conserved across all three species |
| | | | SEQ ID NO: 932 | | SEQ ID NO: 936 | |
| | | hmr-miR-499GS9 | CATGATCAGCTGGGCCAAGAAAACATCAC | hmr-miR-499RP2 | T+TAA+GA+CTTGCAGTG | |
| | | | SEQ ID NO: 933 | | SEQ ID NO: 937 | |
| | | hmr-miR-499GS8 | CATGATCAGCTGGGCCAAGAAAACATCA | | | |
| | | | SEQ ID NO: 934 | | | |

| **Assay Number** | **Target microRNA** | **Extesion Primer Name** | **Extension Primer Sequence** | **Reverse Primer Name** | **Reverse Primer Sequence** | **Comments** |
|---|---|---|---|---|---|---|
| | | hmr-miR-499GS7 | CATGATCAGCTGGGCCAAGAAAACATC | | | |
| | | | SEQ ID NO: 935 | | | |
| 74 | hmr-miR-758 | hmr-mir-758GS10 | CATGATCAGCTGGGCCAAGAGTTAGTGGAC | hmr-miR-758RP1 | TT+TG+TGACCTGGTCCAC | Conserved across all three species |
| | | | SEQ ID NO: 938 | | SEQ ID NO: 942 | |
| | | hmr-miR-758GS9 | CATGATCAGCTGGGCCAAGAGTTAGTGGA | hmr-miR-758RP2 | TT+TG+T+GACCTGGTCC | |
| | | | SEQ ID NO: 939 | | SEQ ID NO: 943 | |
| | | hmr-miR-758GS8 | CATGATCAGCTGGGCCAAGAGTTAGTGG | | | |
| | | | SEQ ID NO: 940 | | | |
| | | hmr-miR-758GS7 | CATGATCAGCTGGGCCAAGAGTTAGTG | | | |
| | | | SEQ ID NO: 941 | | | |
| 75 | hmr-miR-194 | miR-194GSP10 | CATGATCAGCTGGGCCAAGATCCACATGGA | miR-194RP1 | TG+TAA+CAGCAACTCCA | Conserved across all three species |
| | | | SEQ ID NO: 944 | | SEQ ID NO: 948 | |
| | | miR-194GSP9 | CATGATCAGCTGGGCCAAGATCCACATGG | miR-RP2 | TG+TAA+CA+GCAACTCCAT | |
| | | | SEQ ID NO: 945 | | SEQ ID NO: 949 | |
| | | miR-194GSP8 | CATGATCAGCTGGGCCAAGATCCACATG | | | |
| | | | SEQ ID NO: 946 | | | |
| | | miR-194GSP7 | CATGATCAGCTGGGCCAAGATCCACAT | | | |
| | | | SEQ ID NO: 947 | | | |
| 76 | hmr-miR-206 | mir-206GSP10 | CATGATCAGCTGGGCCAAGACCACACACTT | mir-206RP1 | T+GGAA+TGTAAGGAAGT | Conserved across all three species |
| | | | SEQ ID NO: 950 | | SEQ ID NO: 954 | |
| | | mir-206GSP9 | CATGATCAGCTGGGCCAAGACCACACACT | miR-206RP2 | T+G+GAA+TGTAAGGAAGTGT | |
| | | | SEQ ID NO: 951 | | SEQ ID NO: 955 | |
| | | mir-206GSP8 | CATGATCAGCTGGGCCAAGACCACACAC | | | |
| | | | SEQ ID NO: 952 | | | |
| | | mir-206GSP7 | CATGATCAGCTGGGCCAAGACCACACA | | | |
| | | | SEQ ID NO: 953 | | | |
| 77 | hmr-miR-1 | miR-1GS10 | CATGATCAGCTGGGCCAAGATACATACTTC | miR-1RP1 | TG+GAA+TG+TAAAGAAGTA | Conserved across all three species |
| | | | (SEQ ID NO: 47) | | (SEQ ID NO:959) | |
| | | miR-1GS9 | CATGATCAGCTGGGCCAAGATACATACTT (SEQ ID NO:956) | miR-1RP2 | T+G+GAA+TG+TAAAGAAGT (SEQ ID NO:48) | |
| | | miR-1GS8 | CATGATCAGCTGGGCCAAGATACATACT (SEQ ID NO:957) | | | |
| | | miR-1GS7 | CATGATCAGCTGGGCCAAGATACATAC (SEQ ID NO:958) | | | |
| 78 | hmr-miR-9 | miR-9GS10 | CATGATCAGCTGGGCCAAGATCATACAGCT (SEQ ID NO:960) | miR-9RP1 | T+CTTT+GGTTATCTAGCT (SEQ ID NO:964) | Conserved across all three species |
| | | miR-9GS9 | CATGATCAGCTGGGCCAAGATCATACAGC (SEQ ID NO:961) | miR-9RP2 | TC+TTT+GGTT+ATCTAGCTGTA (SEQ ID NO:965) | |
| | | miR-9GS8 | CATGATCAGCTGGGCCAAGATCATACAG (SEQ ID NO:962) | | | |
| | | miR-9GS7 | CATGATCAGCTGGGCCAAGATCATACA (SEQ ID NO:963) | | | |

**TABLE 9**

| **Assay Number** | **Target MicroRNA Name** | **RNA target sequence** | **SEQ ID NO:** |
|---|---|---|---|
| 1. | hmr-miR-495 | AAACAAACAUGGUGCACUUCUU | 966 |
| 2. | mr-miR-291a-3p | AAAGUGCUUCCACUUUGUGUGCC | 967 |
| 3. | m-mIR-291b-3p | AAAGUGCAUCCAUUUUGUUUGUC | 968 |
| 4. | h-miR-519a | AAAGUGCAUCCUUUUAGAGUGUUAC | 969 |
| 5. | h-miR-519b | AAAGUGCAUCCUUUUAGAGGUUU | 970 |
| 6. | h-miR-519c | AAAGUGCAUCUUUUUAGAGGAU | 971 |
| 7. | h-miR-519d | CAAAGUGCCUGCCUUUAGAGUGU | 972 |
| 8. | h-miR-520a | AAAGUGCUUCCCUUUGGACUGU | 973 |
| 9. | h-miR-520b | AAAGUGCUUCCUUUUAGAGGG | 974 |
| 10. | h-miR-520d | AAAGUGCUUCUCUUUGGUGGGUU | 975 |
| 11. | h-miR-520e | AAAGUGCUUCCUUUUUGAGGG | 976 |
| 12. | h-miR-520f | AAGUGCUUCCUUUUAGAGGGUU | 977 |
| 13. | mr-miR-329 | AACACACCCAGCUAACCUUUUU | 978 |
| 14. | hmr-miR-181d | AACAUUCAUUGUUGUCGGUGGGUU | 979 |
| 15. | hmr-miR-193b | AACUGGCCCUCAAAGUCCCGCUUU | 980 |
| 16. | h-miR-362 | AAUCCUUGGAACCUAGGUGUGAGU | 981 |
| 17. | mr-mIR-362-3p | AAUCCUUGGAACCUAGGUGUGAA | 982 |
| 18. | h-miR-500 | AUGCACCUGGGCAAGGAUUCUG | 983 |
| 19. | mr-miR-500 | AUGCACCUGGGCAAGGGUUCAG | 984 |
| 20. | h-miR-501 | AAUCCUUUGUCCCUGGGUGAGA | 985 |
| 21. | mr-miR-501 | AAUCCUUUGUCCCUGGGUGAAA | 986 |
| 22. | hmr-miR-487b | AAUCGUACAGGGUCAUCCACU | 987 |
| 23. | h-miR-489 | AGUGACAUCACAUAUACGGCAGC | 988 |
| 24. | m-miR-489 | AAUGACACCACAUAUAUGGCAGC | 989 |
| 25. | r-miR-489 | AAUGACAUCACAUAUAUGGCAGC | 990 |
| 26. | hmr-miR-425-5p | AAUGACACGAUCACUCCCGUUGA | 991 |
| 27. | hmr-miR-652 | AAUGGCGCCACUAGGGUUGUGCA | 992 |
| 28. | hmr-miR-485-5p | AGAGGCUGGCCGUGAUGAAUUC | 993 |
| 29. | hmr-miR-485-3p | AGUCAUACACGGCUCUCCUCUCU | 994 |
| 30. | hmr-miR-369-5p | AGAUCGACCGUGUUAUAUUCG | 995 |
| 31. | hmr-miR-671 | AGGAAGCCCUGGAGGGGCUGGAGG | 996 |
| 32. | h-miR-449b | AGGCAGUGUAUUGUUAGCUGGC | 997 |
| 33. | mr-miR-449b | AGGCAGUGCAUUGCUAGCUGG | 998 |
| 34. | m-miR-699 | AGGCAGUGCGACCUGGCUCG | 999 |
| 35. | hmr-miR-409-5p | AGGUUACCCGAGCAACUUUGCA | 1000 |
| 36. | hmr-miR-409-3p | GAAUGUUGCUCGGUGAACCCCUU | 1001 |
| 37. | hmr-miR-491 | AGUGGGGAACCCUUCCAUGAGG | 1002 |
| 38. | h-miR-384 | AUUCCUAGAAAUUGUUCAUA | 1003 |
| 39. | mr-miR-384 | AUUCCUAGAAAUUGUUCACA | 1004 |
| 40. | hmr-miR-20b | CAAAGUGCUCAUAGUGCAGGUAG | 1005 |
| 41. | hmr-miR-490 | CAACCUGGAGGACUCCAUGCUG | 1006 |
| 42. | hmr-miR-497 | CAGCAGCACACUGUGGUUUGU | 1007 |
| 43. | h-miR-301b | CAGUGCAAUGAUAUUGUCAAAGCA | 1008 |
| 44. | mr-miR-301b | CAGUGCAAUGGUAUUGUCAAAGCA | 1009 |
| 45. | hmr-miR-721 | CAGUGCAAUUAAAAGGGGGAA | 1010 |
| 46. | hmr-miR-532 | CAUGCCUUGAGUGUAGGACCGU | 1011 |
| 47. | h-miR-488 | CCCAGAUAAUGGCACUCUCAA | 1012 |
| 48. | mr-miR-488 | CCCAGAUAAUAGCACUCUCAA | 1013 |
| 49. | hmr-miR-539 | GGAGAAAUUAUCCUUGGUGUGU | 1014 |
| 50. | h-miR-505 | GUCAACACUUGCUGGUUUCCUC | 1015 |
| 51. | mr-miR-505 | CGUCAACACUUGCUGGUUUUCU | 1016 |
| 52. | h-miR-18b | UAAGGUGCAUCUAGUGCAGUUA | 1017 |
| 53. | mr-miR-18b | UAAGGUGCAUCUAGUGCUGUUA | 1018 |
| 54. | hmr-miR-503 | UAGCAGCGGGAACAGUACUGC | 1019 |
| 55. | hmr-miR-455 | UAUGUGCCUUUGGACUACAUCG | 1020 |
| 56. | hmr-miR-92b | UAUUGCACUCGUCCCGGCCUC | 1021 |
| 57. | h-miR-483 | UCACUCCUCUCCUCCCGUCUUCU | 1022 |
| 58. | mr-miR-483 | UCACUCCUCCCCUCCCGUCUUGU | 1023 |
| 59. | hmr-miR-484 | UCAGGCUCAGUCCCCUCCCGAU | 1024 |
| 60. | hmr-miR-351 | UCCCUGAGGAGCCCUUUGAGCCUG | 1025 |
| 61. | hmr-miR-615 | UCCGAGCCUGGGUCUCCCUCU | 1026 |
| 62. | hmr-miR-486 | UCCUGUACUGAGCUGCCCCGAG | 1027 |
| 63. | hmr-miR-494 | UGAAACAUACACGGGAAACCU | 1028 |
| 64. | hmr-miR-493-3p | UGAAGGUCUACUGUGUGCCAG | 1029 |
| 65. | hmr-miR-146b | UGAGAACUGAAUUCCAUAGGCU | 1030 |
| 66. | r-miR-1 | UGGAAUGUAAAGAAGUGUGUA | 1031 |
| 67. | h-miR-675-5p | UGGUGCGGAGAGGGCCCACAGUG | 1032 |
| 68. | mr-miR-675-5p | UGGUGCGGAAAGGGCCCACAGU | 1033 |
| 69. | hmr-miR-668 | UGUCACUCGGCUCGGCCCACUAC | 1034 |
| 70. | r-miR-346 | UGUCUGCCUGAGUGCCUGCCUCU | 1035 |
| 71. | hmr-miR-542-3p | UGUGACAGAUUGAUAACUGAAA | 1036 |
| 72. | hmr-miR-542-5p | CUCGGGGAUCAUCAUGUCACG | 1037 |
| 73. | hmr-miR-499 | UUAAGACUUGCAGUGAUGUUU | 1038 |
| 74. | hmr-miR-758 | UUUGUGACCUGGUCCACUAACC | 1039 |
| 75. | hmiR-194 | UGUAACAGCAACUCCAUGUGGA | 1040 |
| 76. | hmiR-206 | UGGAAUGUAAGGAAGUGUGUGG | 1041 |
| 77. | hmiR-1 | UGGAAUGUAAAGAAGUAUGUA | 1042 |
| 78. | hmiR-9 | UCUUUGGUUAUCUAGCUGUAUGA | 1043 |

### Assay Format:

Several candidate primer sets shown above in TABLE 8 were tested in a high-throughput assay testing format as follows:

Each test assay (e.g., assay #75, #76, #77 and #78 listed in TABLE 8) was run in 4x4 wells of a 96 well plate, with 6 assays per 96 well plate, thereby allowing for rapid determination of the optimal primer pair for each target.

For each assay, each of the 4 candidate extension (GS) primers were tested in a separate row of the 96 well plate. Each of the 2 reverse primers were tested plus (1nM DNA) or minus template (10mM Tris pH 7.6, 0.1 mM EDTA, 100ng/ul yeast total RNA). Following reverse transcription, one set of duplicate non-template control and template samples was tested against reverse primer 1 (RP1) and the other against reverse primer 2 (RP2).

### Reverse Transcriptase Assay Conditions:

6µl of RT master mix was added to all 96 wells
2µl of 0.5 µM GS primers was added to four successive wells
yeast RNA in TE (10mM Tris pH 7.6, 0.1 mM EDTA) was added to all odd-numbered wells and pre-diluted DNA templates was added to even-numbered wells

Samples were mixed well and the reverse transcriptase step was carried out, followed by dilution with 80 µl TE (10mM Tris pH 7.6, 0.1 mM EDTA).

2µl of the reverse transcription mixture was transferred into quadruplicate wells of a 384 well PCR plate preloaded with 8µl PCR mix per well containing universal primer plus the appropriate reverse primers.

The quantitative PCR reaction results were evaluated on a real-time PCR instrument compatible with 384 well plates.

Ct values for the PCR reactions were determined based on a baseline threshold of 0.01. The sensitivity (Ct value of 1 nM template) and dynamic range (Ct of no-template control minus the Ct of the 1 nM template) were determined for each primer pair in each assay. The results of exemplary assays #75, #76, #77 and #78, listed in TABLE 8, are shown in TABLE 10 below.

**TABLE 10: ASSAY RESULTS USING CANDIDATE PRIMER SETS FOR DETECTING MIR-1. MIR-9; MIR-194 AND MIR-206**

| **microRNA target** | **Extension primer** | **Reverse primer** | **Sensitivity** | **Dynamic Range** | **Selected for use in profiling** |
|---|---|---|---|---|---|
| miR-9 (SEQ ID NO: 1043) | miR-9GS10 (SEQ ID NO:960) | miR_{'}9RPl(SEQID NO:964) | 13 | 9 | - |
| | miR-9GS9 (SEQ ID NO: 961) | miR-9 RP1 (SEQ ID NO:964) | 13 | 4 | - |
| | miR-9GS8 (SEQ ID NO:962) | miR-9 RP1 (SEQ ID N0:964) | 10 | 0 | - |
| | miR-9GS7 (SEQ ID NO:963) | miR-9 RP1 (SEQ ID NO:964) | 16 | 8 | - |
| | miR-9GS10 (SEQ ID NO:960) | miR-9 RP2 (SEQ ID NO: 965) | 13 | 5 | - |
| | miR-9GS9 (SEQ ID NO: 961) | miR-9 RP2 (SEQ ID NO: 965) | 14 | 4 | - |
| | miR-9GS8 (SEQ ID N0:962) | miR-9 RP2 (SEQ ID NO: 965) | 10 | 0 | - |
| | miR-9GS7 (SEQ ID NO:963) | miR-9 RP2 (SEQ ID NO: 965) | 17 | 8 | - |
| miR-194 (SEQ ID NO: 1040) | miR-194GS10 (SEQ ID NO: 944) | miR-194RP1 (SEQ ID NO:948) | 9 | 6 | - |
| | miR-194GS9 (SEQ TD NO:945) | miR-194RP1 (SEQ ID NO:948) | 11 | 5 | - |
| | miR-194GS8 (SEQ ID NO:946) | miR-194RP1 (SEQ ID NO:948) | 13 | 17 | + |
| | miR-194GS7 (SEQ ID NO:947) | miR-194RP1 (SEQ ID NO:948) | 15 | 17 | - |
| | miR-194GS10 (SEQ ID NO: 944) | miR-194RP2 (SEQ ID NO:949) | 10 | 6 | - |
| | miR-194GS9 (SEQ ID NO:945) | miR-194RP2 (SEQ ID NO:949) | 11 | 6 | - |
| | miR-194GS8 (SEQ ID NO:946) | miR-194RP2 (SEQ ID NO:949) | 13 | 16 | - |
| | miR-194GS7 (SEQ ID NO:947) | miR-194RP2 (SEQ ID NO:949) | 17 | 16 | - |
| miR-1 (SEQ ID NO:1042) | miR-1 GS10 (SEQ ID NO:47) | miR-1 RP1 (SEQ ID NO:959) | 15 | 15 | - |
| | miR-1 GS9 (SEQ ID NO:956) | miR-1 RP1 (SEQ ID NO:959) | 17 | 8 | - |
| | miR-1 GS8 (SEQ ID NO:957) | miR-1 RP1 (SEQ ID NO:959) | 19 | 11 | - |
| | miR-1 GS7 (SEQ ID NO:958) | miR-1 RP1 (SEQ ID NO:959) | 22 | 11 | - |
| | miR-1 GS10 (SEQ ID NO:47) | miR-1 RP2 (SEQ ID NO: 48) | 13 | 15 | + |
| | miR-1 GS9 (SEQ ID NO:956) | miR-1 RP2 (SEQ ID *NO:* 48) | 15 | 8 | - |
| | miR-1 GS8 (SEQ ID NO:957) | miR-1 RP2 (SEQ ID NO: 48) | 17 | 1 1 | - |
| | miR-1 GS7 (SEQ ID NO: 958) | miR-1 RP2 (SEQ ID NO: 48) | 19 | 10 | - |
| miR-206 (SEQ ID NO: 1041) | miR-206 GS10 (SEQ ID NO:950) | miR-206RP1 (SEQ ID NO: 954) | 15 | 10 | - |
| | miR-206 GS9 (SEQ ID NO:951) | miR-206RP1 (SEQ ID NO: 954) | 16 | 10 | - |
| | miR-206 GS8 (SEQ ID NO: 952) | miR-206RP1 (SEQ ID NO: 954) | 17 | 14 | - |
| | miR-206 GS7 (SEQ ID NO:953) | miR-206RP1 (SEQ ID NO: 954) | 20 | 20 | - |
| | miR-206 GS10 (SEQ ID NO:950) | miR-206RP2 (SEQ ID NO:955) | 10 | 8 | - |
| | miR-206 GS9 (SEQ ID NO:951) | miR-206RP2 (SEQ ID NO:955) | 11 | 9 | - |
| | miR-206 GS8 (SEQ ID NO: 952) | miR-206RP2 (SEQ ID NO:955) | 11 | 11 | - |
| | miR-206 GS7 (SEQ ID NO:953) | miR-206RP2 (SEQ ID NO:955) | 13 | 20 | + |

Optimal primer pairs were identified based on superior sensitivity (e.g., a preferred range between 5 and 25) and dynamic range (e.g., a preferred range between 10 and 35) characteristics. As shown above in TABLE 10, an optimal primer pair was identified for miR-194: GS8 (SEQ ID NO:946) and RP1 (SEQ ID NO:948) with a sensitivity of 13 and a dynamic range of 17. An optimal primer pair was identified for miR-1: GS10 (SEQ ID NO:47) and RP2 (SEQ ID NO:48) with a sensitivity of 13 and a dynamic range of 15. An optimal primer pair was identified for miR-206: GS7 (SEQ ID NO:953) and RP2 (SEQ ID NO:955) with a sensitivity of 13 and a dynamic range of 20. As also shown in TABLE 10, the GS primers control specificity, as shown by the significant increase in dynamic range (driven by a decrease in background) in going from GS9 to GS8 (see, e.g., miR-194).

Candidate primers designed based on the principles described above, such as the additional exemplary primers listed in TABLE 8, or other candidate primers designed using the design principles described herein, may be tested using the screening methods described above. The assays may be further optimized by using HPLC purified templates to avoid problems associated with degraded templates.

It has also been determined that microRNAs that differ from each other in sequence by only 1, 2 or 3 nucleotide changes can be readily distinguished from one another through the use of the primers designed according to the design principles and methods described herein.

While the preferred embodiment of the invention has been illustrated and described, it will be appreciated that various changes can be made therein without departing from the spirit and scope of the invention.

## Claims

1. A kit for detecting at least one mammalian microRNA comprising at least one oligonucleotide primer selected from the group consisting of SEQ ID NO:506 to SEQ ID NO:511.

2. The kit of Claim 1, the kit being for detecting at least two mammalian microRNA targets, the kit comprising at least two oligonucleotide primer sets, wherein at least one set comprises a primer selected from the group consisting of SEQ ID NO:506 to SEQ ID NO:511, and a second primer set comprises a primer selected from the group consisting of SEQ ID NO: 500 to SEQ ID NO: 505 and SEQ ID NO:512 to SEQ ID NO:965, wherein the primers are capable of use in an assay with a common buffer system with a single universal primer to detect at least two mammalian microRNA targets.

3. The kit of Claim 2, wherein at least one of the at least two oligonucleotide primers comprises a 2'-O,4'-C-methylene-β-D-ribofuranosyl moiety.

4. An oligonucleotide primer for detecting a mammalian microRNA selected from the group consisting of SEQ ID NO:506 to SEQ ID NO:511.

5. At least two oligonucleotide primers for detecting a mammalian miR-291a-3p microRNA and at least one additional microRNA, wherein at least one primer is selected from the group consisting of SEQ ID NO:506 to SEQ ID NO:511 as claimed in Claim 4, and a second primer is selected from the group consisting of SEQ ID NO: 500 to SEQ ID NO: 505 and SEQ ID NO:512 to SEQ ID NO:965, wherein the at least two primers are capable of use in an assay with a common buffer system with a single universal primer to detect at least two mammalian microRNA targets.

6. The kit according to claim 2, wherein the at least one primer set is specific for the detection of a mammalian miR-291a-3p microRNA, and the second primer set is specific for the detection of at least one additional microRNA, each primer set comprising:
(1) an extension primer for producing a cDNA molecule complementary to a target microRNA, the extension primer comprising a first portion that hybridizes to a target microRNA and a second portion having a hybridization sequence for a universal forward PCR primer;
(2) a universal forward PCR primer for amplifying the cDNA molecule, comprising a sequence selected to hybridize to the hybridization sequence on the extension primer; and
(3) a reverse PCR primer for amplifying the cDNA molecule, comprising a sequence selected to hybridize to a portion of the cDNA molecule,
wherein the primers in the kit are capable of use in an assay with a common buffer system with a single universal primer to detect the at least two mammalian microRNA targets, and
wherein the at least one additional target microRNA is selected from the group consisting of miR-1, miR-9, miR-18b, miR-20b, miR-92b, miR-146b, miR-181d, miR-193b, miR-194, miR-206, miR-291b-3p, miR-301b, miR-329, miR-346, miR-351, miR-362, miR-362-3p, miR-369-5p, miR-384, miR-409-3p, miR-409-5p, miR-425-5p, miR-449b, miR-455, miR-483, miR-484, miR-485-3p, miR-485-5p, miR-486, miR-487b, miR-488, miR-489, miR-490, miR-491, miR-493-3p, miR-494, miR-495, miR-497, miR-499, miR-500, miR-501, miR-503, miR-505, miR-519a, miR-519b, miR-519c, miR-519d, miR-520a, miR-520b, miR-520d, miR-520e, miR-520f, miR-532, miR-539, miR-542-3p, miR-542-5p, miR-615, miR-652, miR-668, miR-671, miR-675-5p, miR-699, miR-721 and miR-758.

7. The kit according to Claim 6, wherein at least one of the universal forward and reverse PCR primers includes at least one nucleic acid molecule comprising a 2'-O,4'-C-methylene-β-D-ribofuranosyl moiety.

8. The kit according to Claim 6, wherein the extension primers have a length in the range of from 10 to 100 nucleotides.

9. The kit according to Claim 6, wherein the first portion of the extension primers have a length in the range of from 7 to 10 nucleotides, or wherein the second portion of the extension primers have a length in the range of from 18 to 25 nucleotides.

10. The kit according to Claim 6, wherein the second portion of the extension primers have a nucleic acid sequence comprising the nucleic acid sequence of SEQ ID NO:1.

11. The kit according to Claim 6, wherein the universal forward PCR primer has a length in the range of from 16 to 100 nucleotides, or wherein the reverse PCR primers have a length in the range of from 10 to 100 nucleotides.

12. The kit according to Claim 6, wherein the universal forward primer consists of the nucleic acid sequence set forth in SEQ ID NO:13.

13. The kit according to Claim 6, wherein at least one of the reverse PCR primers comprises from 1 to 25 nucleic acid molecules comprising a 2'-O,4'-C-methylene-β-D-ribofuranosyl moiety.

14. The kit according to Claim 6, wherein the at least two mammalian target microRNAs are human microRNAs, or wherein the at least two target microRNAs are murine microRNAs.

15. The kit according to Claim 6, comprising at least one or more oligonucleotide primers selected from the group consisting of SEQ ID NO: 500 to SEQ ID NO: 505 and SEQ ID NO:512 to SEQ ID NO:965.
